# EUROPEAN PATENT SPECIFICATION

(11) **EP 3 400 220 B1**
(45) Date of publication and mention of the grant of the patent: **24.03.2021**
(21) Application number: 16882855.6
(22) Date of filing: 05.01.2016
(51) Int. Cl.: C07D 401/06, C07D 205/04, C07D 207/10, A61K 31/397, A61P 25/00, A61P 29/00

(54) **PYRAZOLE DERIVATIVES**
PYRAZOLDERIVATE
DÉRIVÉS DE PYRAZOLE

(43) Date of publication of application: 14.11.2018
(73) Proprietor: Hua Medicine (Shanghai) Ltd., Pudong, Shanghai 201203 (CN)
(72) Inventor: CHEN, Li, Shanghai 201203 (CN); JIN, Xiaowei, Shanghai 201203 (CN)
(74) Representative: Gill, Siân Victoria
(86) International application number: PCT/CN2016/070115
(87) International publication number: WO 2017/117708

(56) References cited:
- WO-A1-02/46166
- WO-A1-2004/080998
- CN-A- 105 121 424
- US-A1- 2006 030 559
- EGLE ARBACIAUSKIENE ET AL.: 'Pd-catalyzed cross-coupling reactions of halogenated 1- phenylpyrazol-3-ols and related triflates' TETRAHEDRON vol. 65, no. 37, 09 July 2009, pages 7817 - 7824, XP055288662

## Description

### Field of the Invention

This invention relates generally to compounds of formula Ia: 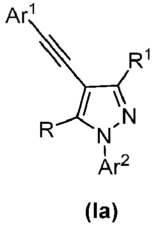 or a pharmaceutically acceptable salt thereof, and to pharmaceutical compositions comprising said compounds or a pharmaceutically acceptable salt thereof, wherein the definitions of Ar¹, Ar², R¹, A and B are as defined below. The compounds and compositions disclosed herein are mGlu5 receptor antagonists useful for the treatment or prevention of mGluR5 mediated disorders, such as acute and/or chronic neurological disorders, cognitive disorders and memory deficits, as well as acute and chronic pain.

### Background of the Invention

Glutamate is the most prominent neurotransmitter in the body, being present in over 50% of nervous tissue. Glutamate mediates its effects through two major groups of receptors: ionotropic and metabotropic. Ionotropic glutamate receptors are ion channel receptors which are often responsible for fast excitatory transmission. They are generally divided into N-methyl-D-aspartate (NMDA), α-amino-3-hydroxy-5-methyl-4-isoxazole propionic acid (AMPA) and kainite receptors. By contrast, metabotropic glutamate receptors (mGluRs) belong to the class C G-protein-coupled receptor (GPCR) protein family and are mainly involved in the modulation of fast excitatory transmission. As such, they are attractive therapeutic targets for treatment of disorders involving malfunction of glutamate signaling. The mGluRs are further divided into three groups (Group I, II and III) based on amino acid sequence homology, signal transduction mechanism and pharmacological properties. Group I receptors include mGluR1 and mGluR5, Group II includes mGluR2 and mGluR3 and Group III includes mGluR4, mGluR6, mGluR7 and mGluR8. The Group I mGluR1 and mGluR5 receptors couple to G-proteins of the Gq family, Gq and G11, and their activation leads to activation of phospholipase C, resulting in the hydrolysis of membrane phosphatidylinositol (4, 5)-bisphosphate to diacylglycerol, which subsequently activates protein kinase C, and inositol trisphosphate, which in turn activates the inositol trisphosphate receptor to promote the release of intracellular calcium.

Anatomical studies demonstrate a broad and selective distribution of mGluRs in the mammalian nervous system. For example, mGlu5 receptors are abundantly expressed in the striatum, cortex, hippocampus, caudate-putamen and nucleus accumbens; see for example: Shigemoto, R., Nomura, S., Hidemitsu, S., et al. Neuroscience Lett 1993, 163, 53-57. As these brain areas have been shown to be involved in emotion, motivational processes, learning and memory, as well as motor control, mGluR5 modulators have long been regarded as possessing therapeutic potential for a wide range of indications.

mGlu5 receptor antagonists can be used for modulating the activity of the mGlu5 receptor and for use in the treatment or prevention of mGluR5 mediated disorders, such as acute and/or chronic neurological disorders, cognitive disorders and memory deficits, acute and chronic pain, protection against drug or disease induced liver damage or failure, urinary inconsistence. Other diseases contemplated include cerebral ischemia, chronic neurodegeneration including Huntington's chorea, amyotrophic lateral sclerosis, Alzheimer's disease, Parkinson's disease, levodopa-induced dyskinesia in Parkinson's disease (PD-LID), psychiatric disorders, schizophrenia, mood disorders, emotion disorders, disorders of extrapyramidal motor function, obesity, disorders of pulmonary system and respiration, motor control and function, attention deficit disorders, concentration disorders, mental retardation (including mental retardation related to Fragile X syndrome), autism spectrum disorders (ASDs), pain disorders, neurodegenerative disorders, epilepsy, convulsive disorders, migraine, dyskinesia, eating disorders, vomiting, muscle spasms, urinary inconsistence, sleep disorders, sexual disorders, circadian disorders, drug withdrawal, drug addiction, compulsive disorders, anxiety, panic disorders, depression disorders, skin disorders, retinal ischemia, retinal degeneration, glaucoma, disorders associated with organ transplantation, asthma, ischemia and astrocytomas, diseases of the cardiovascular system, diseases of the gastrointestinal system such as gastroesophageal reflux disease (GERD) and irritable bowel syndrome, diseases of the endocrine system, diseases of the exocrine system, diseases of the skin, cancer and diseases of the ophthalmic system. The development and use of mGLuR5 antagonists has been summarized in numerous review articles for example: Gasparini, F., Bilbe, G., Gomez-Mancilla, G., and Spooren, W., Current Opinion in Drug Discovery & Development. 11(5), 655-665, 2008; Rocher, J.-P., Bonnet, B., Boléa, C., et al., Current Topics in Medicinal Chemistry. 11, 680-695, 2011; Dekundy, A., Gravius, A., Hechenberger, M, et al., J. Neural Transm. 118, 1703-1716, 2011; Niswender, C. M. and Conn, P. J., Annu Rev Pharmacol Toxicol. 50, 295-322, 2010; Emmitte KA. mGluR5 negative allosteric modulators, a patent review (2010-2012). Expert Opin Ther Pat. 2013 Apr; 23(4):393-408 and Guiying Li, Morten Jørgensen and Brian M Campbell. Metabotropic glutamate receptor 5-negative allosteric modulators for the treatment of psychiatric and neurological disorders (2009-July 2013), Pharmaceutical Patent Analyst. 2(6), 767-802.

### Summary of the Invention

The present invention is directed to compounds of the formula Ia, or a pharmaceutically acceptable salt thereof, pharmaceutical compositions containing them and to methods of treating diseases and disorders. The compounds and compositions disclosed herein are mGlu5 receptor antagonists useful for the treatment of mGluR5 mediated disorders, including acute and/or chronic neurological disorders, cognitive disorders and memory deficits, as well as acute and chronic pain.

### Detailed Description of the Invention

In an embodiment, the present invention provides a compound according to formula Ia: 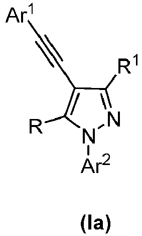 or a pharmaceutically acceptable salt thereof, wherein:
R is -H, -halogen, -alkyl, -cycloalkyl, -haloalkyl, -OR², or -N(CH₃)₂, wherein R² is a branched or straight-chain alkyl radical of one to nine carbon atoms which may be optionally substituted with 1-3 substituents selected from the group consisting of -halogen, -OH, -alkoxy, and -N(CH3)₂;
R¹ is -H, -halogen, -alkyl, -cycloalkyl, -haloalkyl, -OR², or -N(CH₃)₂, wherein R^{2'} is a branched or straight-chain alkyl radical of one to nine carbon atoms which may be optionally substituted with 1-3substituents selected from the group consisting of -halogen, -OH, -alkoxy, and -N(CH3)2;
Ar¹ is a 5- to 10-membered mono- or bicyclic heteroaryl ring that contains 1-3 N heteroatoms, wherein the 5- to 10-membered mono- or bicyclic heteroaryl ring is optionally substituted with 1-3 substituents independently selected from the group consisting of -alkyl, -halogen, -OH, -CN, nitro, -CF3, -O-CF₃, -O-alkyl, - O-aryl, -S-alkyl, -S-aryl, -S(O)-alkyl, -S(O)-aryl, -S(O₂)-alkyl, -S(O₂)-aryl, - CH₂-aryl, aryl, heteroaryl, -O-CH₂-aryl, -N(CH₃)₂, cycloalkyl, heterocycloalkyl, -C(O)-alkyl, -C(O)-cycloalkyl, -C(O)-heterocycloalkyl, -C(O)-aryl, -C(O)-heteroaryl, -C(O)O-alkyl, -C(O)O-cycloalkyl, -C(O)O-heterocycloalkyl, - C(O)O-aryl, -C(O)O-heteroaryl, -C(O)NH₂, -C(O)NH-alkyl, -C(O)N(alkyl)₂, - C(O)NH-cycloalkyl, -C(O)N(cycloalkyl)₂, -C(O)NH-heterocycloalkyl, - C(O)N(heterocycloalkyl)₂, -C(O)NH-aryl, -C(O)N(aryl)₂, -C(O)NH-heteroaryl, -C(O)N(heteroaryl)₂, and substituted lower alkyl, wherein the substituents may combine to form an optionally substituted 5-7 membered fused carbacyclic or heterocyclic ring, or
   a 5- to 10-membered mono- or bicyclic aryl ring, wherein the 5- to 10-membered mono- or bicyclic aryl ring is optionally substituted with 1-3 substituents independently selected from the group consisting of alkyl, -halogen, -OH, -CN, nitro, -CF₃, -O-CF₃, -O-alkyl, -O-aryl, -S-alkyl, -S-aryl, -S(O)-alkyl, -S(O)-aryl, -S(O₂)-alkyl, -S(O₂)-aryl, -CH₂-aryl, aryl, heteroaryl, -O-CH₂-aryl, -N(CH₃)₂, cycloalkyl, heterocycloalkyl, -C(O)-alkyl, -C(O)-cycloalkyl, -C(O)-heterocycloalkyl, -C(O)-aryl, -C(O)-heteroaryl, -C(O)O-alkyl, -C(O)O-cycloalkyl, -C(O)O-heterocycloalkyl, -C(O)O-aryl, -C(O)O-heteroaryl, - C(O)NH₂, -C(O)NH-alkyl, -C(O)N(alkyl)₂, -C(O)NH-cycloalkyl, - C(O)N(cycloalkyl)₂, -C(O)NH-heterocycloalkyl, -C(O)N(heterocycloalkyl)₂, - C(O)NH-aryl, -C(O)N(aryl)₂, -C(O)NH-heteroaryl, -C(O)N(heteroaryl)₂, and substituted lower alkyl, wherein the substituents may combine to form a 5-7 membered fused and optionally substituted carbacyclic or heterocyclic ring;
Ar² is a 5- to 10-membered mono- or bicyclic heteroaryl ring that contains 1-3 N heteroatoms, wherein the 5- to 10-membered mono- or bicyclic heteroaryl ring is optionally substituted with 1-3 substituents independently selected from the group consisting of -alkyl, -halogen, -OH, -CN, nitro, -CF₃, -O-CF₃, -O-alkyl, - O-aryl, -S-alkyl, -S-aryl, -S(O)-alkyl, -S(O)-aryl, -S(O₂)-alkyl, -S(O₂)-aryl, -O-alkyl-OH, -O-alkyl-O-alkyl, -CH₂-aryl, aryl, heteroaryl, -O-CH₂-aryl, -N(CH₃)₂, cycloalkyl, heterocycloalkyl, -C(O)-alkyl, -C(O)-cycloalkyl, -C(O)-heterocycloalkyl, -C(O)-aryl, -C(O)-heteroaryl, -C(O)O-alkyl, -C(O)O-cycloalkyl, -C(O)O-heterocycloalkyl, -C(O)O-aryl, -C(O)O-heteroaryl, - C(O)NH₂, -C(O)NH-alkyl, -C(O)N(alkyl)₂, -C(O)NH-cycloalkyl, - C(O)N(cycloalkyl)₂, -C(O)NH-heterocycloalkyl, -C(O)N(heterocycloalkyl)₂, - C(O)NH-aryl, -C(O)N(aryl)₂, -C(O)NH-heteroaryl, -C(O)N(heteroaryl)₂, and substituted lower alkyl wherein the substituents may combine to form an optionally substituted 5-7 membered fused carbacyclic or heterocyclic ring, or a 5- to 10-membered mono- or bicyclic aryl ring, wherein the 5- to 10-membered mono- or bicyclic aryl ring is optionally substituted with 1-3 substituents independently selected from the group consisting of -alkyl, -halogen, -OH, -CN, nitro, -CF₃, -O-CF₃, -O-alkyl, -O-aryl, -S-alkyl, -S-aryl, -S(O)-alkyl, -S(O)-aryl, -S(O₂)-alkyl, -S(O₂)-aryl, -O-alkyl-OH, -O-alkyl-O-alkyl, -CH₂-aryl, aryl, heteroaryl, -O-CH₂-aryl, -N(CH₃)₂, cycloalkyl, heterocycloalkyl, -C(O)-alkyl, - C(O)-cycloalkyl, -C(O)-heterocycloalkyl, -C(O)-aryl, -C(O)-heteroaryl,-C(O)O-alkyl, -C(O)O-cycloalkyl, -C(O)O-heterocycloalkyl, -C(O)O-aryl,-C(O)O-heteroaryl, -C(O)NH₂, -C(O)NH-alkyl, -C(O)N(alkyl)₂, -C(O)NH-cycloalkyl, -C(O)N(cycloalkyl)₂, -C(O)NH-heterocycloalkyl,-C(O)N(heterocycloalkyl)₂, -C(O)NH-aryl, -C(O)N(aryl)₂, -C(O)NH-heteroaryl, -C(O)N(heteroaryl)₂, and substituted lower alkyl wherein the substituents may combine to form an optionally substituted 5-7 membered fused carbacyclic or heterocyclic ring.

In a further embodiment, provided is a compound according to formula Ia, or a pharmaceutically acceptable salt thereof, wherein:
R is -H, -halogen, -alkyl, -cycloalkyl, -haloalkyl, -OR², or -N(lower alkyl)₂, wherein R² is a branched or straight-chain alkyl radical of one to nine carbon atoms which may be optionally substituted with 1-2 substituents selected from the group consisting of -OH and -alkoxy;
R¹ is -H, -halogen, -alkyl, -cycloalkyl, -haloalkyl, -OR^{2'}, or -N(lower alkyl)₂, wherein R^{2'} is a branched or straight-chain alkyl radical of one to nine carbon atoms which may be optionally substituted with 1-3substituents selected from the group consisting of -halogen, -OH, -alkoxy, and -N(CH3)2;
Ar¹ is a 5- or 6-membered mono-heteroaryl ring that contains 1-3 N heteroatoms, wherein the 5- or 6-membered mono-heteroaryl ring is optionally substituted with 1-3 substituents independently selected from the group consisting of -alkyl, -halogen, -OH, -CN, nitro, -CF₃, -O-CF₃, and -O-alkyl, or
   a 6-membered aryl ring, wherein the 6-membered aryl is optionally substituted with 1-3 substituents independently selected from the group consisting of -alkyl, -halogen, -OH, -CN, nitro, -CF₃, -O-CF₃, and -O-alkyl;
Ar² is a 5- or 6-membered mono-heteroaryl ring that contains 1-3 N, wherein the 5- or 6-membered mono-heteroaryl ring is optionally substituted with 1-3 substituents independently selected from the group consisting of -alkyl, -halogen, -OH, -CN, nitro, -CF₃, -O-CF₃, -O-alkyl, -S-alkyl, -S(O)-alkyl, -S(O₂)-alkyl, -O-alkyl-O-alkyl, aryl, -C(O)O-alkyl, -C(O)NH₂, -C(O)NH-alkyl, and -C(O)N(alkyl)₂, or a 6-membered aryl ring, wherein the 6-membered aryl is optionally substituted with 1-3 substituents independently selected from the group consisting of -alkyl, -halogen, -OH, -CN, nitro, -CF₃, -O-CF₃, -O-alkyl, -S-alkyl, -S(O)-alkyl, -S(O₂)-alkyl, -O-alkyl-O-alkyl, aryl, -C(O)O-alkyl, -C(O)NH₂, -C(O)NH-alkyl, and - C(O)N(alkyl)₂;

In a further embodiment, provided is a compound according to formula Ia, or a pharmaceutically acceptable salt thereof, wherein:
R is -H, -halogen, -alkyl, -cycloalkyl, -haloalkyl, -OR², or -N(lower alkyl)₂, wherein R² is lower alkyl which may be optionally substituted with 1-2 substituents selected from the group consisting of -OH and -alkoxy;
R¹ is -H, -halogen, -alkyl, -cycloalkyl, -haloalkyl, -OR² , or -N(lower alkyl)₂, wherein R^{2'} is lower alkyl which may be optionally substituted with 1-3 substituents selected from the group consisting of -halogen, -OH, -alkoxy, and -N(CH₃)₂;
Ar¹ is a 6-membered aryl ring or a 5- or 6-membered mono-heteroaryl ring that contains 1, 2, or 3 N heteroatoms, wherein the 6-membered aryl or the 5- or 6-membered mono-heteroaryl ring is optionally substituted with 1, 2 or 3 substituents independently selected from the grouppp consisting of lower alkyl, and halogen;
Ar² is a 6-membered aryl ring or a 5- or 6-membered mono-heteroaryl ring that contains 1, 2, or 3 N heteroatoms, wherein the 6-membered aryl ring or the 5- or 6-membered mono-heteroaryl ring is optionally substituted with 1, 2 or 3 substituents independently selected from the group consisting of -C₁-C₄alkyl, -F, -Cl, -Br, -OH, -CN, nitro, -CF₃, -OCF₃, -O-C₁-C₄alkyl, -SCH₃, -S(O)-CH₃, - S(O₂)-CH₃, -O-C₁-C₄alkyl-O-C₁-C₄alkyl, -C(O)OCH₃, -C(O)NH₂, - C(O)NH(CH₃), -C(O)N(CH₃)₂, phenyl.

In a further embodiment, provided is a compound according to formula Ia, or a pharmaceutically acceptable salt thereof, wherein:
R is -H, -halogen, -C₁-C₄alkyl, -CF₃, -O-C₁-C₄alkyl, or -O-C₁-C₄alkyl-O-C₁-C₄alkyl;
R¹ is -H, -halogen, -C₁-C₄alkyl, -C₃-C₆cycloalkyl, -halo-C₁-C₄alkyl, -OR^{2'}, or -N(C₁-C4alkyl)2, wherein R^{2'} is C₁-C₄alkyl which may be optionally substituted with 1-2 substituents selected from the group consisting of -halogen, -OH, -C₁-C₄alkoxy, and -N(CH₃)₂;
Ar¹ is optionally substituted pyridinyl, pyrimidinyl, pyrazinyl, or phenyl, wherein the pyridinyl, pyrimidinyl, pyrazinyl, or phenyl is optionally substituted with a substituent selected from the group consisting of -C₁-C₄alkyl, and halogen, wherein the halogen is preferably chloro;
Ar² is optionally substituted pyridinyl, or phenyl, wherein the pyridinyl, or phenyl is optionally substituted with 1-2 substituents independently selected from the group consisting of -C₁-C₄alkyl, -halogen, -CN, -CF₃, -O-CF₃, -S(O₂)-C₁-C₄alkyl, and -O-C₁-C₄alkyl-O-C₁-C₄alkyl.

In a still further embodiment of the present invention, provided is a pharmaceutical composition, comprising a therapeutically effective amount of a compound according to formula Ia or a pharmaceutically acceptable salt thereof and a pharmaceutically acceptable carrier.

It is to be understood that the terminology employed herein is for the purpose of describing particular embodiments, and is not intended to be limiting. Further, although any methods, devices and materials similar or equivalent to those described herein can be used in the practice or testing of the invention, the preferred methods, devices and materials are now described.

As used herein, for simplicity, the term "Cₓ-C_{y}" denotes the number of atoms, including carbon atoms and heteroatoms where present, where x and y are integers. So, for example, C₁-C₄alkyl refers to an alkyl group having 1 to 4 carbon atoms, C₅-C₁₂heterocyclyl refers to a 5 to 12 membered ring system having at least one heteroatom but not a ring system containing 5 to 12 annular carbon atoms.

As used herein, the term "alkyl", alone or in combination with other groups, refers to a branched or straight-chain monovalent saturated aliphatic hydrocarbon radical of one to twenty carbon atoms, preferably one to sixteen carbon atoms, more preferably one to ten, one to eight, one to six, one to five or one to four carbon atoms.

As used herein, the term "alkenyl", alone or in combination with other groups, refers to a straight-chain or branched hydrocarbon residue having an olefinic bond of two to twenty carbon atoms, preferably two to sixteen carbon atoms, more preferably two to ten, two to eight, two to six, two to five or two to four carbon atoms.

The term "cycloalkyl" refers to a mono- or polycarbocyclic saturated aliphatic hydrocarbonyl radical of three to ten, preferably three to six carbon atoms. This term is further exemplified by radicals such as cyclopropyl, cyclobutyl, cyclopentyl, cyclohexyl, cycloheptyl, norbornyl, adamantyl, indanyl and the like. In a preferred embodiment, the "cycloalkyl" moieties can optionally be substituted with one, two, three or four substituents, with the understanding that said substituents are not, in turn, substituted further unless indicated otherwise in the Examples or claims below. Each substituent can independently be, alkyl, alkoxy, halogen, amino, hydroxyl or oxygen (O=) unless otherwise specifically indicated. Examples of cycloalkyl moieties include, but are not limited to, optionally substituted cyclopropyl, optionally substituted cyclobutyl, optionally substituted cyclopentyl, optionally substituted cyclohexyl, optionally substituted cycloheptyl, and the like or those which are specifically exemplified herein.

The term "heterocycloalkyl" denotes a mono- or polycyclic alkyl ring of three to ten, preferably three to six ring atoms, wherein one, two or three of the ring atoms is a heteroatom such as N, O or S. Examples of heterocycloalkyl groups include, but are not limited to, morpholinyl, thiomorpholinyl, piperazinyl, piperidinyl, pyrrolidinyl, tetrahydropyranyl, tetrahydrofuranyl, 1,3-dioxanyl and the like. The heterocycloalkyl groups may be unsubstituted or substituted and attachment may be through their carbon frame or through their heteroatom(s) where appropriate, with the understanding that said substituents are not, in turn, substituted further unless indicated otherwise in the Examples or claims below.

The term "lower alkyl", alone or in combination with other groups, refers to a branched or straight-chain alkyl radical of one to nine carbon atoms, preferably one to six carbon atoms, more preferably one to five carbon atoms or one to four carbon atoms. This term is further exemplified by radicals such as methyl, ethyl, *n*-propyl, iso-propyl, *n*-butyl, *s*-butyl, *iso*-butyl, *t-*butyl, *n*-pentyl, 3-methylbutyl, *n*-hexyl, 2-ethylbutyl and the like.

The term "aryl" refers to an aromatic mono- or polycarbocyclic radical of 6 to 12 carbon atoms having at least one aromatic ring. Examples of such groups include, but are not limited to, phenyl, naphthyl, 1,2,3,4-tetrahydronaphthalene, 1,2-dihydronaphthalene, indanyl, 1H-indenyl and the like.

The alkyl, lower alkyl and aryl groups may be substituted or unsubstituted. When substituted, there will generally be, for example, 1 to 4 substituents present, with the understanding that said substituents are not, in turn, substituted further unless indicated otherwise in the Examples or claims below. These substituents may optionally form a ring with the alkyl, lower alkyl or aryl group with which they are connected. Substituents may include, for example: carbon-containing groups such as alkyl, aryl, arylalkyl (e.g. substituted and unsubstituted phenyl, substituted and unsubstituted benzyl); halogen atoms and halogen-containing groups such as haloalkyl (e.g. trifluoromethyl); oxygen-containing groups such as alcohols (e.g. hydroxyl, hydroxyalkyl, aryl(hydroxyl)alkyl), ethers (e.g. alkoxy, aryloxy, alkoxyalkyl, aryloxyalkyl, more preferably, for example, methoxy and ethoxy), aldehydes (e.g. carboxaldehyde), ketones (e.g. alkylcarbonyl, alkylcarbonylalkyl, arylcarbonyl, arylalkylcarbonyl, arycarbonylalkyl), acids (e.g. carboxy, carboxyalkyl), acid derivatives such as esters(e.g. alkoxycarbonyl, alkoxycarbonylalkyl, alkylcarbonyloxy, alkylcarbonyloxyalkyl), amides (e.g. aminocarbonyl, mono- or di-alkylaminocarbonyl, aminocarbonylalkyl, mono-or di-alkylaminocarbonylalkyl, arylaminocarbonyl), carbamates (e.g. alkoxycarbonylamino, aryloxycarbonylamino, aminocarbonyloxy, mono-or di-alkylaminocarbonyloxy, arylaminocarbonloxy) and ureas (e.g. mono- or di- alkylaminutesocarbonylamino or arylaminutesocarbonylamino); nitrogen-containing groups such as amines (e.g. amino, mono- or di-alkylamino, aminoalkyl, mono- or di-alkylaminoalkyl), azides, nitriles (e.g. cyano, cyanoalkyl), nitro; sulfur-containing groups such as thiols, thioethers, sulfoxides and sulfones (e.g. alkylthio, alkylsulfinyl, alkylsulfonyl, alkylthioalkyl, alkylsulfillylalkyl, alkylsulfonylalkyl, arylthio, arysulfinyl, arysulfonyl, arythioalkyl, arylsulfinylalkyl, arylsulfonylalkyl); and heterocyclic groups containing one or more heteroatoms, (e.g. thienyl, furanyl, pyrrolyl, imidazolyl, pyrazolyl, thiazolyl, isothiazolyl, oxazolyl, oxadiazolyl, thiadiazolyl, aziridinyl, azetidinyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, piperidyl, hexahydroazepinyl, piperazinyl, morpholinyl, thianaphthyl, benzofuranyl, isobenzofuranyl, indolyl, oxyindolyl, isoindolyl, indazolyl, indolinyl, 7-azaindolyl, benzopyranyl, coumarinyl, isocoumarinyl, quinolinyl, isoquinolinyl, naphthridinyl, cinnolinyl, quinazolinyl, pyridopyridyl, benzoxazinyl, quinoxalinyl, chromenyl, chromanyl, isochromanyl, phthalazinyl and carbolinyl).

The term "heteroaryl", refers to an aromatic mono- or polycyclic radical of 5 to 12 atoms having at least one aromatic ring containing one, two, or three ring heteroatoms selected from N, O, and S, with the remaining ring atoms being C, preferably an aromatic monocycylic radical of 5 to 6 atoms, and preferably an aromatic bicyclic radical of 11 to 12 atoms. One or two ring carbon atoms of the heteroaryl group may be replaced with a carbonyl group. Examples of such groups include, but are not limited to, pyrimidinyl, pyridyl, indoyl, quinolinyl, pyridon-2-yl, isoquinolinyl, 5,6,7,8-tetrahydroquinolinyl, thienyl, furanyl, imidazolyl, pyrazolyl, thiazolyl, isothiazolyl, oxazolyl, oxadiazolyl, thiadiazolyl, pyrazolidinyl, pyrazinyl, pyridazinyl, thianaphthyl, benzofuranyl, isobenzofuranyl, oxyindolyl, isoindolyl, indazolyl, indolinyl, 7-azaindolyl, benzopyranyl, coumarinyl, isocoumarinyl, isoquinolinyl, naphthridinyl, cinnolinyl, quinazolinyl, pyridopyridyl, benzoxazinyl, quinoxalinyl, chromenyl, chromanyl, isochromanyl, phthalazinyl and the like.

The heteroaryl group described above may be substituted independently with one, two, or three substituents, with the understanding that said substituents are not, in turn, substituted further unless indicated otherwise in the Examples or claims below. These substituents may optionally form a ring with the heteroaryl group to which they are connected. Substituents may include, for example: carbon-containing groups such as alkyl, aryl, arylalkyl (e.g. substituted and unsubstituted phenyl, substituted and unsubstituted benzyl); halogen atoms and halogen-containing groups such as haloalkyl (e.g. trifluoromethyl); oxygen-containing groups such as alcohols (e.g. hydroxyl, hydroxyalkyl, aryl(hydroxyl)alkyl), ethers (e.g. alkoxy, aryloxy, alkoxyalkyl, aryloxyalkyl), aldehydes (e.g. carboxaldehyde), ketones (e.g. alkylcarbonyl, alkylcarbonylalkyl, arylcarbonyl, arylalkylcarbonyl, arylcarbonylalkyl), acids (e.g. carboxy, carboxyalkyl), acid derivatives such as esters (e.g. alkoxycarbonyl, alkoxycarbonylalkyl, alkylcarbonyloxy, alkylcarbonyloxyalkyl), amides (e.g. aminocarbonyl, mono- or di-alkylaminocarbonyl, aminocarbonylalkyl, mono-or di-alkylaminocarbonylalkyl, arylaminocarbonyl), carbamates (e.g. alkoxycarbonylamino, aryloxycarbonylamino, aminocarbonyloxy, mono-or di-alkylaminocarbonyloxy, arylminocarbonloxy) and ureas (e.g. mono- or di- alkylaminocarbonylamino or arylaminocarbonylamino); nitrogen-containing groups such as amines (e.g. amino, mono- or di-alkylamino, aminoalkyl, mono- or di-alkylaminoalkyl), azides, nitriles (e.g. cyano, cyanoalkyl), nitro; sulfur-containing groups such as thiols, thioethers, sulfoxides and sulfones (e.g. alkylthio, alkylsulfinyl, alkylsulfonyl, alkylthioalkyl, alkylsulfinylalkyl, alkylsulfonylalkyl, arylthio, arysulfinyl, arysulfonyl, arythioalkyl, arylsulfinylalkyl, arylsulfonylalkyl); and heterocyclic groups containing one or more heteroatoms, (e.g. thienyl, furanyl, pyrrolyl, imidazolyl, pyrazolyl, thiazolyl, isothiazolyl, oxazolyl, oxadiazolyl, thiadiazolyl, aziridinyl, azetidinyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, piperidyl, hexahydroazepinyl, piperazinyl, morpholinyl, thianaphthyl, benzofuranyl, isobenzofuranyl, indolyl, oxyindolyl, isoindolyl, indazolyl, indolinyl, 7-azaindolyl, benzopyranyl, coumarinyl, isocoumarinyl, quinolinyl, isoquinolinyl, naphthridinyl, cinnolinyl, quinazolinyl, pyridopyridyl, benzoxazinyl, quinoxalinyl, chromenyl, chromanyl, isochromanyl, phthalazinyl, benzothiazoyl and carbolinyl).

As used herein, the term "alkoxy" means -O-alkyl; and "alkanoyl" means -CO-alkyl, wherein the alkyl moiety contains 1 to 8 carbon atoms, preferably 1 to 6 carbon atoms, more preferably 1 to 5 carbon atoms, or 1 to 4 carbon atoms. Alkoxy substituent groups or alkoxy-containing substituent groups may be substituted by, for example, one or more alkyl groups, with the understanding that said substituents are not, in turn, substituted further unless indicated otherwise in the Examples or claims below.

As used herein, the term "halogen" or "halo" means a fluorine, chlorine, bromine or iodine radical, preferably a fluorine, chlorine or bromine radical, and more preferably a fluorine or chlorine radical.

As used herein, the term "pharmaceutically acceptable salt" means any pharmaceutically acceptable salt of the compound of formula Ia and Ib. Salts may be prepared from pharmaceutically acceptable non-toxic acids and bases including inorganic and organic acids and bases. Such acids include, for example, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfuric, tartaric, oxalic, *p*-toluenesulfonic and the like. Particularly preferred are fumaric, hydrochloric, hydrobromic, phosphoric, succinic, sulfuric and methanesulfonic acids. Acceptable base salts include alkali metal (e.g. sodium, potassium), alkaline earth metal (e.g. calcium, magnesium) and aluminum salts.

In the practice of the method of the present invention, an effective amount of any one of the compounds of this invention or a combination of any of the compounds of this invention or a pharmaceutically acceptable salt thereof, is administered via any of the usual and acceptable methods known in the art, either singly or in combination. The compounds or compositions can thus be administered orally (e.g., buccal cavity), sublingually, parenterally (e.g., intramuscularly, intravenously, or subcutaneously), rectally (e.g., by suppositories or washings), transdermally (e.g., skin electroporation) or by inhalation (e.g., by aerosol), and in the form of solid, liquid or gaseous dosages, including tablets and suspensions. The administration can be conducted in a single unit dosage form with continuous therapy or in a single dose therapy ad libitum. The therapeutic composition can also be in the form of an oil emulsion or dispersion in conjunction with a lipophilic salt such as pamoic acid, or in the form of a biodegradable sustained-release composition for subcutaneous or intramuscular administration.

Useful pharmaceutical carriers for the preparation of the compositions hereof, can be solids, liquids or gases. Thus, the compositions can take the form of tablets, pills, capsules, suppositories, powders, enterically coated or other protected formulations (e.g. binding on ionexchange resins or packaging in lipid-protein vesicles), sustained release formulations, solutions, suspensions, elixirs, aerosols, and the like. The carrier can be selected from the various oils including those of petroleum, animal, vegetable or synthetic origin, e.g., peanut oil, soybean oil, mineral oil, sesame oil, and the like. Water, saline, aqueous dextrose, and glycols are preferred liquid carriers, particularly (when isotonic with the blood) for injectable solutions. For example, formulations for intravenous administration comprise sterile aqueous solutions or of the active ingredient(s) which are prepared by dissolving solid active ingredient(s) in water to produce an aqueous solution, and rendering the solution sterile. Suitable pharmaceutical excipients include starch, cellulose, talc, glucose, lactose, talc, gelatin, malt, rice, flour, chalk, silica, magnesium stearate, sodium stearate, glycerol monostearate, sodium chloride, dried skim milk, glycerol, propylene glycol, water, ethanol, and the like. The compositions may be subjected to conventional pharmaceutical additives such as preservatives, stabilizing agents, wetting or emulsifying agents, salts for adjusting osmotic pressure, buffers and the like. Suitable pharmaceutical carriers and their formulation are described in **Reminutesgton's Pharmaceutical Sciences** by E. W. Martin. Such compositions will, in any event, contain an effective amount of the active compound together with a suitable carrier so as to prepare the proper dosage form for proper administration to the recipient.

The dose of a compound of the present invention depends on a number of factors, such as, for example, the manner of administration, the age and the body weight of the subject, and the condition of the subject to be treated, and ultimately will be decided by the attending physician or veterinarian. Such an amount of the active compound as determined by the attending physician or veterinarian is referred to herein, and in the claims, as a "therapeutically effective amount". For example, the dose of a compound of the present invention is typically in the range of about 1 to about 1000 mg per day. Preferably, the therapeutically effective amount is in an amount of from about 1 mg to about 500 mg per day.

It will be appreciated, that the compounds of general formula I in this invention may be derivatized at functional groups to provide derivatives which are capable of conversion back to the parent compound *in vivo.* Physiologically acceptable and metabolically labile derivatives, which are capable of producing the parent compounds of general formula I *in vivo* are also within the scope of this invention.

Compounds of the present invention can be prepared beginning with commercially available starting materials and utilizing general synthetic techniques and procedures known to those skilled in the art. Chemicals may be purchased from companies such as for example Aldrich, Argonaut Technologies, VWR and Lancaster.

The present compounds of formula I can be prepared by the methods described below, by the methods given in the schemes or in the examples. Appropriate reaction conditions for the individual reaction steps are known to a person skilled in the art. The reaction sequence is not limited to the one displayed in the schemes, however, depending on the starting materials and their respective reactivity, the sequence of reaction steps can be freely altered. Starting materials are either commercially available or can be prepared by methods analogous to the methods given below, by methods described in the examples, or by methods known in the art.

The present compounds of formula I can be prepared by the following schemes described below.

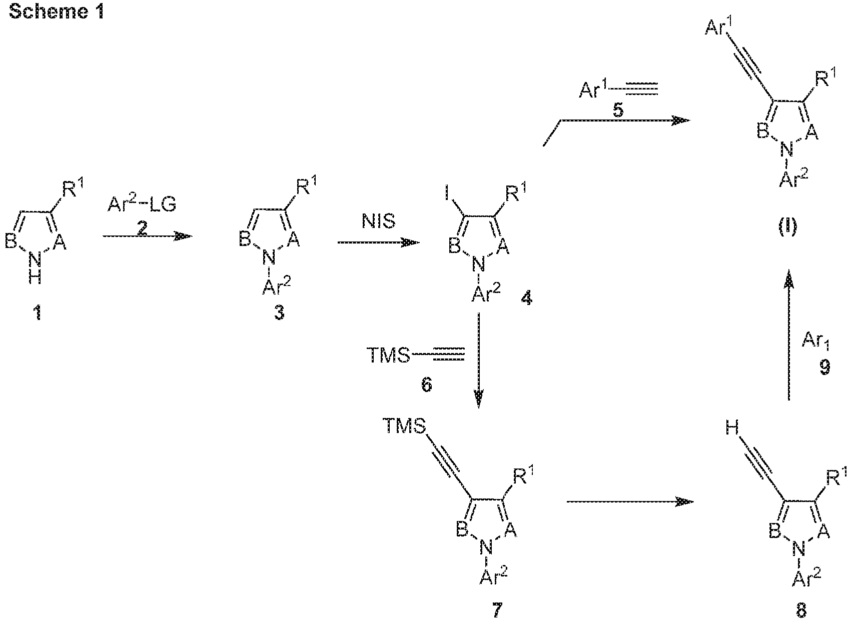

In scheme 1 compounds of formula 1 are known, and in many cases, commercially available compounds or can be prepared using well established methodology. For example, B= C-CH₃ or C-CF₃, A is N and R¹ is CH₃ are commercial available from several suppliers including Sigma-Aldrich. In case compound 1 are not commercially available, it can be synthesized through Knorr pyrazole synthesis. The Knorr pyrazole synthesis is an organic reaction used to convert a 1,3-dicarbonyl compound and a hydrazine or its derivatives to a pyrazole. Knorr, L. **(1884),** *Berichte der deutschen chemischen Gesellschaft* 17: 1635.

In scheme 1 compounds 5, for example, Ar¹= 2-pyridinyl or 3-pyridinyl are available from several suppliers including Sigma-Aldrich. In the case that commercial supplies are not readily available, aryl- and heteroaryl alkynes can be prepared from the corresponding aryl or heteroaryl carboxaldehydes using the Corey-Fuchs procedure (Corey, E. J. and Fuchs, P. L., Tetrahedron Lett. 1972, 3769; reviewed in: Han, Xiaojun. Editor(s): Li, Jie Jack. Name Reactions for Homologations (2009), (Pt. 1), 393-403. Publisher: John Wiley & Sons, Inc.). Alternatively, aryl and heteroaryl carboxaldehydes may be converted to aryl or heteroaryl alkynes by treatment with C-silylated -diazophosphines under neutral conditions (Ona, I., Xavier, B., Cazoria, A. M., et al., Journal of Organic Chemistry, 2006, 71, 5320).

In cases where the appropriate carboxaldehydes are not readily available, aryl or heteroaryl aryl alkynes may also be prepared from aryl or heteroaryl compounds functionalized with groups capable of undergoing transition metal catalyzed cross-coupling reactions with alkynes. Those skilled in the art will appreciate how to select the appropriate reaction partners. For example 3-alkynyl pyridine can be synthesized from either 3-bromopyridine or 3-trifluoromethanesulfonyl pyridine through reactions with suitably functionalized alkynes catalyzed by transition metals, followed by deprotection of terminal alkynes bearing a protecting group at the terminal position. In the event the protecting group is trimethylsilyl (TMS), the compound may be treated with an aqueous base, for example potassium hydroxide or tetrabutylammonium fluoride (TBAF) in methanol to effect its removal. In the case where the alkyne is formed through a transition metal catalyzed reaction between an aryl or heteraryl ring bearing a suitable functionality for cross coupling reactions, for example a bromide, and 2-methyl-3-butyne-2-ol, deprotection to give a terminal alkyne can be achieved through heating in a suitable solvent, for example toluene, in the presence of catalytic amounts of a base, for example sodium hydride. The following references are among the many examples of such transformations in the published literature: Uttam Dutta, Soham Maity, Rajesh Kancherla, and Debabrata Maiti, Organic Letters, 2014, 16(24), 6302-6305; Holmes, B. T., Pennington, W. T., Hanks, T. W., Synthetic Communications, 2003, 33, 2447-2461; Negishi, E.-i., Xu, C., Tan, Z., Kotora, M., Heterocycles, 1997, 46, 209-214. One common variant is known as the Sonogashira coupling reaction, reviewed in Chinchilla, R., Nájera, C., Recent Advances in Sonogashira reactions, Chemical Society Reviews, 2011, 40, 5084-5121.

In scheme 1 compound of structure **2** in which LG is a leaving group, which can be B(OH)₂ or halogen like F, Cl, Br and I. Reaction condition for compounds **1** and **2** to form compound **3** depending on the LG types, 1) for example, when LG is B(OH)₂, compound **1** and **2** go through Chan-Lam Coupling reaction in an suitable inert solvent, such as DCM in the presence of catalyst copper (II) like Cu(OAC)₂ at a suitable temperature, for example room temperature in air, after reaction is complete the newly formed compound **3** can be isolated using conventional technics, for example by filtering and concentrating under vacuo, the reaction residue was purified through chromatography over silica gel (P. Y. S. Lam, C. G. Clark, S. Saubern, J. Adams, M. P. Winters, D. M. T. Chan, A. Combs, Tetrahedron Lett., 1998, 39, 2941 - 2944.); 2) for example, when LG is halogen like F, Cl, Br and I, compound **1** and **2** go through SNAr reaction in an suitable inert solvent, such as DMF with a base like Cs₂CO₃ or NaH at a suitable temperature, for example 90°C, after reaction is complete and the newly formed compound 3 can be isolated using conventional technics, for example by quenching the reaction with an aqueous solution followed by extraction of the products into an organic solvent, washing with brine, drying and chromatography over silica gel, if necessary.

Conversion of compounds of structure **3** to the iodides **4** can be achieved by nucleophilic substitution reaction, like combination of a solution of **3** to a iodination reagent, for example N-iodosuccinimide (NIS) in a suitable inert solvent, for example, chloroform at a medium temperature, for example 60°C. Such reactions may be worked up using common procedures, for example, the reaction mixture was concentrated under vacuo and the residue was purified by silica gel chromatography, if necessary to obtain the nuclear substituted products **4** (Tetrahedron Letters, 42 (15), 863-865, 2001; Tetrahedron Letters, 68 (15), 3165-3171, 2012).

Compound **4** could go through two pathways to get the final product of formula **I.**
1) Reaction of compounds **4** and aryl alkyne **5** to form final product of formula I can be achieved by Sonogashira coupling of the alkyne **5** and halohydrocarbon **4** in a suitable inert solvent, for example THF, by adding Pd(PPH₃)₂Cl₂, Et₃N and CuI, then the reaction mixture microwaved at a medium temperature, for example 90°C, after reaction is complete and the newly formed compound **I** can be isolated using conventional technics, for example The reaction mixture was concentrated to dryness and the residue was purified by pre-HPLC to afford the final product **I** (Sonogashira, K. (2002), "Development of Pd-Cu catalyzed cross-coupling of terminal acetylenes with sp2-carbon halides", J. Organomet. Chem. 653: 46-49; King, A. O.; Yasuda, N. (2004), "Palladium-Catalyzed Cross-Coupling Reactions in the Synthesis of Pharmaceuticals Organometallics in Process Chemistry", Top. Organomet. Chem. 6: 205-245).
2)Reaction of compounds **4** and ethynyltrimethylsilane **6** to form compound **7** can be achieved by Sonogashira coupling of the alkyne **6** and halohydrocarbon **4** in a suitable inert solvent, for example THF, by adding Pd(PPH₃)₂Cl₂, Et₃N and CuI, then the reaction mixture was stirred at a medium temperature, for example 90°C, after reaction is complete and the newly formed compound **7** can be isolated using conventional technics, for example reaction mixture was concentrated under vacuo and the residue was added organic solvent like EtOAc, followed by washing with brine, drying and chromatography over silica gel. Compound **7** may be treated with an aqueous base, for example potassium hydroxide or tetrabutylammonium fluoride (TBAF) in MeOH to effect the removal of protecting group trimethylsilyl (TMS) to afford compound **8.** Then, reaction of compound **8** and compound **9** to form final product of formula **I** can be achieved by Sonogashira coupling of the alkyne **8** and compound **9** in a suitable inert solvent, for example CH₃CN, by adding Pd(PPH₃)₂Cl₂, Et₃N and CuI, then the reaction mixture was stirred at appropriate temperature, for example 120°C, after reaction is complete and the newly formed compound **I** can be isolated using conventional technics, for example The reaction mixture was concentrated to dryness and the residue was purified by prep-HPLC to afford the final product **I.**

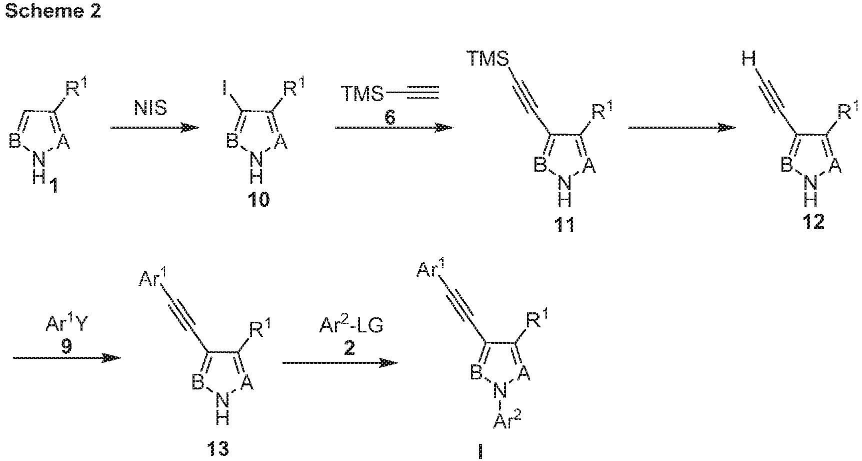

An alternative method for the preparation of compounds of formula I is shown in Scheme 2. Iodides like 3-iodopyrazole and 4-iodopyrazole are commercially available, in case it's not commercially available, iodides **10** can be achieved by nucleophilic substitution reaction, like combination of a solution of **1** to a iodination reagent, for example N-iodosuccinimide (NIS) in a suitable inert solvent, for example, chloroform at appropriate temperature, for example 60°C. Such reactions may be worked up using common procedures, for example, the reaction mixture was concentrated under vacuo and the residue was purified by silica gel chromatography, if necessary.

Reaction of compounds **10** and ethynyltrimethylsilane **6** to form compound **11** can be achieved by Sonogashira coupling of the alkyne **6** and halohydrocarbon **10** in a suitable inert solvent, for example THF, by adding Pd(PPH₃)₂Cl₂, Et₃N and CuI, then reaction at a medium temperature, for example 70°C, after reaction is complete and the newly formed compound **11** can be isolated using conventional technics, for example the reaction mixture was filtered and concentrated then purified by chromatograph column. Compound **11** may be treated with an aqueous base, for example potassium hydroxide or tetrabutylammonium fluoride (TBAF) in MeOH to effect the removal of protecting group trimethylsilyl (TMS) to afford compound **12,** which reacted with compound **9** through Sonogashira coupling in a suitable inert solvent, for example CH₃CN, by adding Pd(PPH₃)₂Cl₂, Et₃N and CuI, then the reaction mixture was stirred at appropriate temperature, for example 90°C, after reaction is complete and the newly formed compound **13** can be isolated using conventional technics, the mixture was filtered and concentrated by vacuo to give the crude product which was purified by silica gel chromatography. As described in Scheme 1, reaction condition of compound **13** and compound **2** to form the final product of formula **I** depending on the LG types, a) for example when LG is B(OH)₂, compound **13** and **2** go through Chan-Lam Coupling reaction in an suitable inert solvent, such as DCM in the presence of catalyst copper (II) like Cu(OAC)₂ at a suitable temperature, for example room temperature in air, after reaction is complete the final compound of formula **I** can be isolated using conventional technics, for example by filtering and concentrating under vacuo, the reaction residue was purified by prep-TLC; b) for example when LG is halogen like F, Cl, Br and I, compound **13** and **2** go through SNAr reaction in an suitable inert solvent, such as DMF with a base like Cs₂CO₃ at a suitable temperature, for example 110°C, after reaction is complete and the final compound of formula **I** can be isolated using conventional technics, for example by filtering and concentrating under vacuo, the reaction residue was purified by prep-HPLC.

The invention will now be further described in the Examples below, which are intended as an illustration only and do not limit the scope of the invention.

### EXAMPLES

### Example 1

### Procedure for preparation of 2-ethynyl-pyrÙnidine:

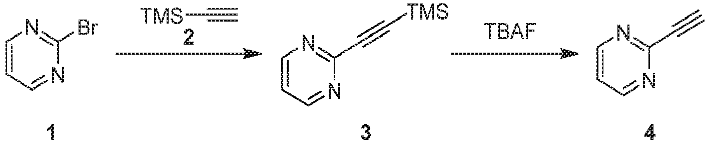

To a solution of **1**(5.0g, 31.45mmol) and 2(3.40g, 34.6mmol) in 30mL of Et₃N was added CuI (0.6g, 3.15mmol) and Pd(PPh₃)₄ (1.8g, 1.55mmol). The resulting mixture was protected with N₂ atmosphere, and then was stirred for 48 hours at room temperature. TLC showed the starting material was consumed. The reaction mixture was then concentrated in vacuo. The crude product was purified by silica gel column chromatography to give product **3**(3.5g, yield: 63.1%).

To a solution of **3**(3.0g, 17.02mmol) in 10mL THF was added 1M of TBAF over 3 minutes. TLC showed the starting material was consumed. The reaction mixture was then concentrated in vacuo. The crude product was purified by silica gel column chromatography to give product **4**(1.2 g, yield: 67.4%).
**LCMS:** *m*/*z,* 105 (M+H)⁺.

### Example 2

### Procedure for preparation of 2-chloro-4-ethynyl-pyridine:

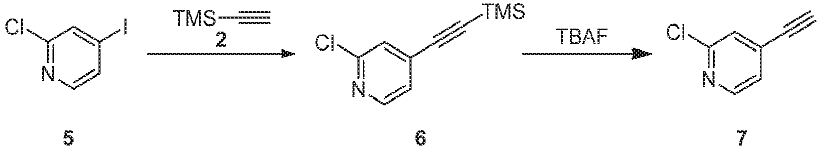

To a solution of **5**(3.3g, 13.78 mmol) and **2**(1.49g, 15.16mmol) in Et₃N was added CuI (0.26g, 1.38mmol) and Pd(PPh₃)₂Cl₂ (0.48g, 0.69mmol). The resulting mixture was protected with N₂ atmosphere, and then was heated at 70°C for 4 hours. The reaction mixture was filtered, and concentrated in vacuo. The residue was purified by flash chromatography to give product **6**(2.0g, yield: 69.2%).

To a solution of **6**(0.4g, 1.91mmol) in 10 mL THF was added 1M TBAF over 3 minutes. TLC showed the starting material was consumed. The reaction mixture was then concentrated in vacuo. The crude product was purified by silica gel column chromatography to give product 7(0.15g, yield: 57.2%).
**LCMS:** *m*/*z* 138(M+H)⁺.

### Example 3

### Procedure for preparation of 2-ethynyl-pyrazine:

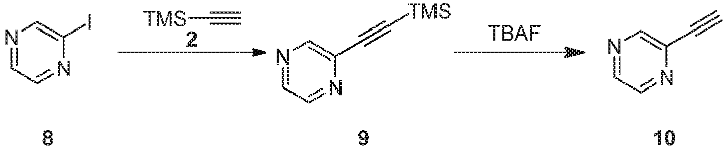

To a solution of **8**(5.0g, 24.39mmol) and **2**(2.82g, 26.8mmol) in 100 mL of Et₃N was added Pd(PPh₃)₄ (1.4g, 1.22mmol) and CuI (0.46g, 2.44mmol). The reaction mixture was protected by N₂ atmosphere, and was stirred at room temperature for 48 hours. TLC showed the starting material was consumed. The reaction mixture was then concentrated in vacuum. The resulting crude product was purified by silica gel column chromatography to give product **9**(3.2 g, yield: 74.4%).

To a solution of **9**(3.0g, 17.02mmol) in 10mL THF was added 1M of TBAF over 3 minutes. TLC analysis showed complete conversion of starting material to a major product. The reaction mixture was then concentrated in vacuo. The crude product was purified by silica gel column chromatography to give product **10**(1.0g, yield: 56.5%).
**LCMS:** *m*/*z* 105 (M+H)⁺.

### Example 4

### Procedure for preparation of 4-ethynyl-pyridine:

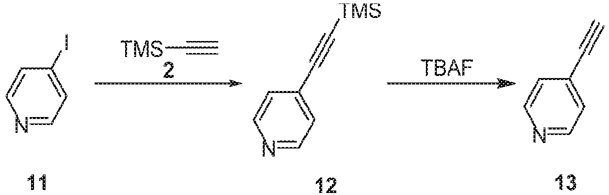

To a solution of **11**(5.0g, 24.39mmol) and 2(2.64g, 26.8mmol) in 100mL of Et₃N was added Pd(PPh₃)₄ (1.40g, 1.22mmol) and CuI (0.46g, 2.44mmol). The reaction mixture was protected by N₂ atmosphere, and was stirred at room temperature for 48 hours. TLC showed that the starting material was consumed. The reaction mixture was then concentrated in vacuo. The resulting crude product was purified by silica gel column chromatography to give the target product **12**(3.0g, yield: 70.4%).

To a solution of **12**(3.0g, 17.14mmol) in 10mL THF was added 1M of TBAF over 3 minutes. TLC analysis showed complete conversion of starting material to a major product. The reaction mixture was then concentrated in vacuo. The crude product was purified by silica gel column chromatography to give product **13**(1.0g, yield: 56.8%).
**LCMS:** *m*/*z* 104 (M+H)⁺.

### Example Compound 1

### Preparation of 2-((1-(4-fluorophenyl)-3,5-dimethyl-1H-pyrazol-4-yl)ethynyl)pyridine:

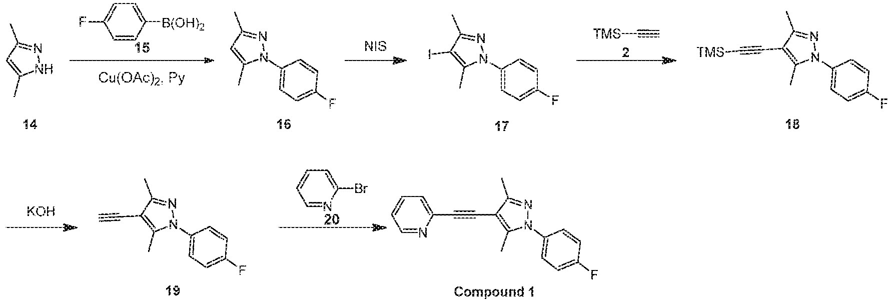

### Experimental section:

### Procedure for preparation of 16:

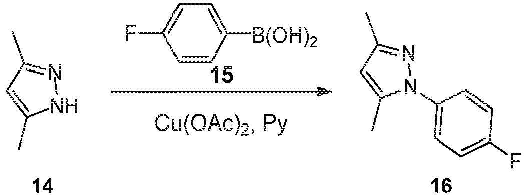

To a solution of **14**(1.00g, 10.40mmol) in 60mL of degassed DCM, **15**(2.91g, 20.81mmol), Cu(OAc)₂ (3.78mg, 20.81mmol), and pyridine (2.49g, 31.21mmol) were added successively. The mixture was then degassed for 1minute under O₂ atmosphere and stirred at room temperature overnight. The reaction mixture was filtered and the filtrate was evaporated to give crude product which was purified by chromatography column to give the title product **16**(1.7 g, yield: 86%).
**LCMS:** *m*/*z* 191 (M⁺H)⁺.

### Procedure for preparation of 17:

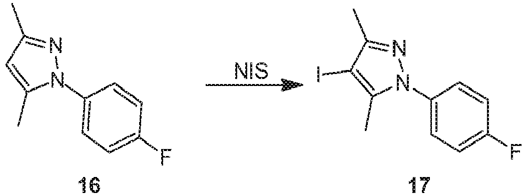

To a solution of **16**(1.00g, 5.26mmol) in CHCb (50mL) was added NIS (1.18g, 5.26mmol). Then the reaction mixture was stirred at reflux for 2 hours. LCMS showed that the reaction was complete, then the reaction mixture was concentrated and purified by chromatograph column tc give the title product **17**(1.4g, yield: 84%).
**LCMS:** *m*/*z* 317 (M+H)⁺.

### Procedure for preparation of 18:

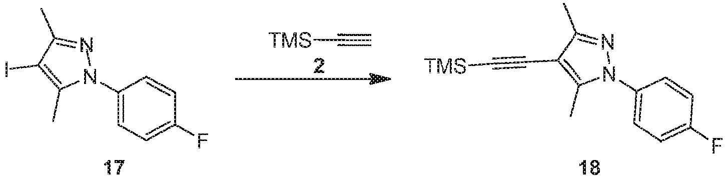

To a solution of **17**(1.00g, 3.16mmol) in 50mL of degassed CH₃CN was added successively CuI (60mg, 0.32mmol), 2 (620mg, 6.33mmol), and Pd(PPh₃)₂Cl₂ (220mg, 0.32mmol) and Et₃N (630mg, 9.49mmol). The reaction mixture was stirred at 70 °C for 18 hours. LCMS showed that the reaction was complete, then the reaction mixture was filtered and the filtrate was concentrated to give the crude product, which was purified by chromatograph column to give the title product 18(780 mg, yield 85%).
LCMS: m/z 287 (M+H)⁺.

### Procedure for preparation of 19:

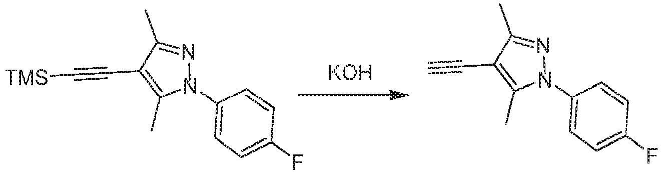

To a solution of **18**(780 mg, 2.72mmol) in MeOH (50mL) was added KOH (306 mg, 5.45 mmol). Then the reaction mixture was stirred at room temperature for 1 hour. LCMS showed that the reaction was complete, then the reaction mixture was quenched with water, extracted with DCM (3 x 50 mL). The combined organic layer was dried over Na₂SO₄, concentrated to give the title product 19(500 mg, yield: 86%).
LCMS: m/z 215 (M+H)⁺.

### Procedure for preparation of Compound 1:

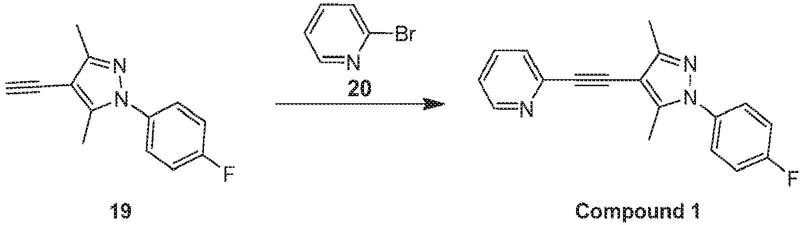

To a solution of **19**(100mg, 0.47mmol) in 3mL of degassed CH₃CN was added successively CuI (8mg, 0.046mmol), **20**(222mg, 1.40mmol), and Pd(PPh₃)₂Cl₂ (32mg, 0.046mmol) and Et₃N (142mg, 1.40mmol). The reaction vessel was sealed and heated in microwave at 120 °C for 1 hour. LCMS showed that the reaction was complete, then the reaction mixture was filtered and the filtrate was concentrated to give the crude product, which was purified by prep-TLC to give the desired product ***Compound* 1** (18 mg, yield :13%).
**LCMS:** *m*/*z* 292 (M+H)⁺ ;
**¹H NMR** (400 MHz, CDCl3) : δ 8.61 (d, *J*=4.8Hz, 1H), 7.68-7.66 (m, 1H), 7.50-7.39 (m, 3H), 7.18-7.14 (m, 3H), 2.42 (s, 3H), 2.41 (s, 3H).

### Example Compound 2

### Preparation of 2-((1-(4-fluorophenyl)-3, 5-dimethyl-1H-pyrazol-4-yl) ethynyl) pyrimidine:

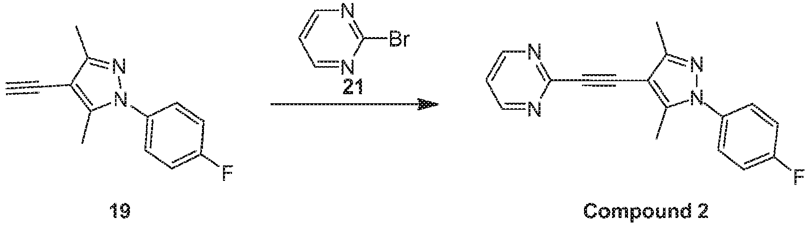

### Experimental section:

### Procedure for preparation of Compound 2:

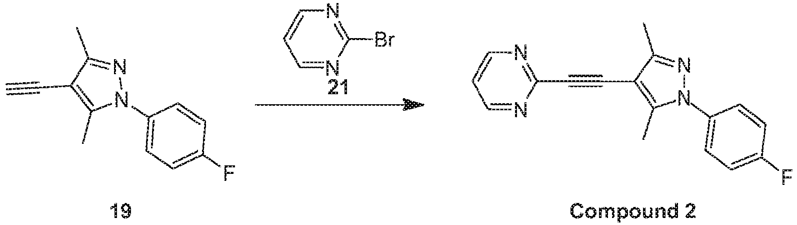

To a solution of **19**(100mg, 0.47mmol) in 3mL of degassed CH₃CN was added successively CuI (8mg, 0.046mmol), **21**(223mg, 1.40mmol), and Pd(PPh₃)₂Cl₂ (32mg, 0.046mmol) and Et₃N (142mg, 1.40mmol). The reaction vessel was sealed and heated by microwave at 120 °C for 1 hour. LCMS showed that the reaction was completed, then the reaction mixture was filtered and the filtrate was concentrated to give the crude product, which was purified by prep-TLC to give the desired product ***Compound* 2**(18 mg, yield: 13%).
**LCMS:** *m*/*z* 293 (M+H)⁺
**¹H NMR**(400 MHz, CDCl3) : δ 8.74 (d, *J*=4.8Hz, 2H), 7.43-7.39 (m, 2H), 7.20-7.14 (m, 3H), 2.45 (s, 3H), 2.44 (s, 3H).

### Example Compound 3

### Preparation of 4-((1-(4-fluorophenyl)-3, 5-dimethyl-1H-pyrazol-4-yl) ethynyl) pyrimidine:

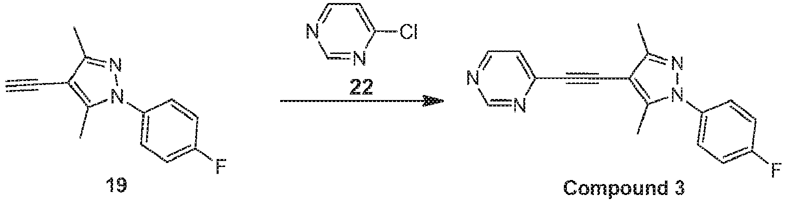

### Experimental section:

### Procedure for preparation Compound 3:

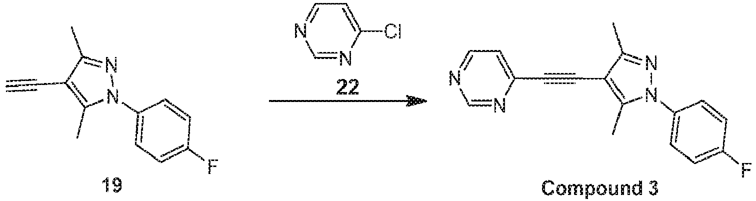

To a solution of **19**(100mg, 0.47mmol) in 3mL of degassed CH₃CN was added successively CuI (8mg, 0.0461nmol), 22(160mg, 1.40mmol), and Pd(PPh₃)₂Cl₂ (32mg, 0.046mmol) and Et₃N (142mg, 1.40mmol). The reaction vessel was sealed and heated by microwave at 120°C for 1 hour. LCMS showed that the reaction was complete. The reaction mixture was filtered and the filtrate was concentrated to give the crude product, which was purified by prep-TLC to give the desired product compound 3(9 mg, yield: 7%).
LCMS: *m*/*z* 292 (M+H)⁺ ;
**¹11 NMR** (400 MHz, CDCl3) : δ 9.19 (s, 1H), 8.69 (s, 1H), 7.43-7.39 (m, 3H), 7.19-7.15 (m, 2H), 2.43 (s, 3H), 2.42 (s, 3H).

### Example Compound 4

### Preparation of 2-((3.S-dimethyl-1-(4-(trifluoromethoxy)phenyl)-1H-pyrazol-4-yl) ethynyl)pyridine:

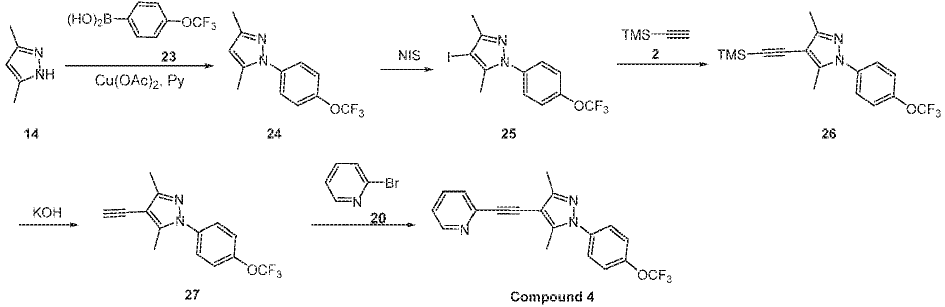

### Experimental section:

### Procedure for preparation of 24:

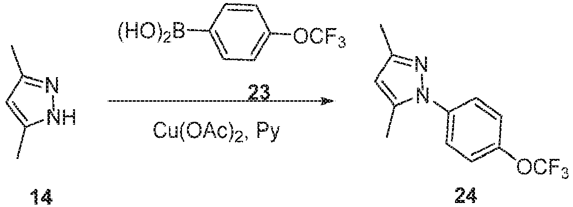

To a solution of 14(200mg, 2.08mmol) in 20mL of degassed DCM was added successively **23**(857mg, 4.16mmol), Cu(OAc)₂ (756mg, 4.16 mmol), and pyridine (493 mg, 6.24 mmol). The mixture was then degassed for 1 minute under O₂ atmosphere and stirred at room temperature overnight. The reaction mixture was filtered and the filtrate was concentrated and purified by chromatograph column to give the product 24(460mg, yield: 86%).
LCMS: *m*/*z* 257 (M+H)⁺.

### Procedure for preparation of 25:

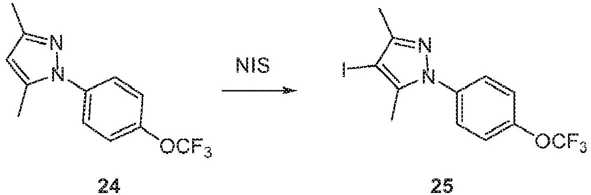

To a solution of **24**(320mg, 1.25mmol) in CHCl₃ (50mL) was added NIS (309mg, 1.37mmol). Then the reaction mixture was stirred at reflux for 2 hours. LCMS showed that the reaction was complete, then the reaction mixture was concentrated and purified by chromatograph column to give the product **25**(340mg, yield: 71%).
**LCMS:** *m*/*z* 383 (M+H)⁺.

### Procedure for preparation of 26:

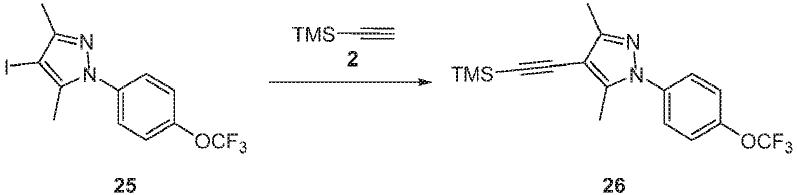

To a solution of **25**(340mg, 0.89mmol) in 50mL of degassed CH₃CN was added successively CuI (17mg, 0.089mmol), **2**(175mg, 1.78mmol), Pd(PPh₃)₂Cl₂ (62mg, 0.089mmol) and Et₃N (270mg, 2.67mmol). The mixture was stirred at 70°C for 18 hours. LCMS showed that the reaction was complete, then the reaction mixture was filtered and the filtrate was concentrated to give the crude product, which was purified by chromatograph column to give the product 26(280 mg, yield: 89%).
**LCMS:** *m*/*z* 353 (M+H)⁺.

### Procedure for preparation of 27:

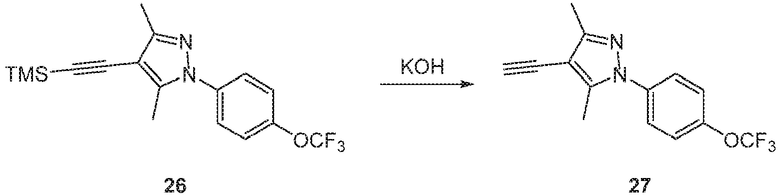

To a solution of **26**(300mg, 0.85mmol) in MeOH (10mL) was added KOH (96 mg, 1.70 mmol). Then the reaction mixture was stirred at room temperature for 1 hour. LCMS showed that the reaction was complete, then the reaction mixture was quenched with water, extracted with DCM (3 x 20 mL). The combined organic layer was dried over Na₂SO₄, concentrated to give the desired product **27**(500 mg, yield: 86%).
**LCMS**: *m*/*z* 281 (M+H)⁺.

### Procedure for preparation of Compound 4:

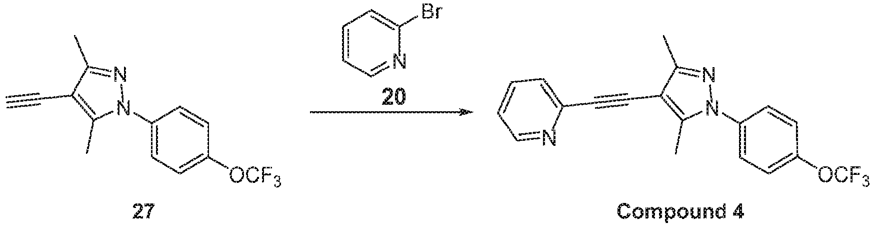

To a solution of **27** (100 mg, 0.36 mmol) in 10 mL of degassed THF (10 ml) was added successively CuI (7mg, 0.036 mmol), **20** (113 mg, 0.71 mmol), Pd(PPh₃)₂Cl₂ (25 mg, 0.036 mmol) and Et₃N (108 mg, 1.07 mmol). The mixture was stirred at 80 °C for 18 hours. LCMS showed that the reaction was completed, then the reaction mixture was filtered and the filtrate was concentrated and purified by prep-TLC to give the desired product **Compound 4**(30 mg, yield: 24%).
**LCMS:** *m*/*z* 358 (M+H)⁺;
**¹H NMR**(400 MHz, CDCl3) : δ 8.61 (d, *J*=4.8 Hz, 1H), 7.68-7.67 (m, 1H), 7.50-7.48 (m, 3H), 7.33-7.31 (m, 2H), 7.23-7.22 (m, 1H), 2.47 (s, 3H), 2.42 (s, 3H).

### Example Compound 5

### Preparation of 3-(3,5-dimethyl-4-(2-(pyridin-2-yl)ethyl)-1H-pyrazol-1-yl)-5-fluorobenzonitrile:

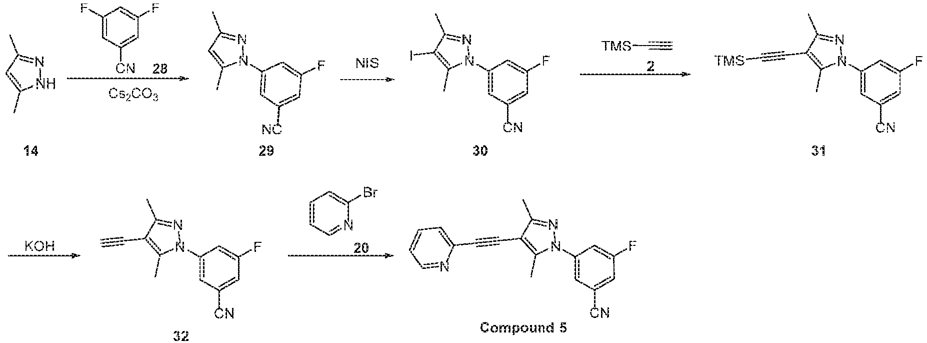

### Experimental section:

### Procedure for preparation of 29:

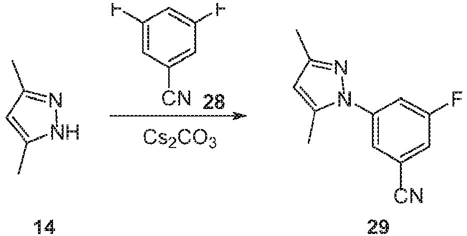

To a solution of **14**(500mg, 5.20mmol) in 20mL of degassed DMF was added successively **28**(1.09g, 7.80mmol) and Cs₂CO₃ (3.39g, 10.40mmol). The mixture was heated to 120 °C and stirred for 2 hours. LCMS showed that the reaction was completed, then the reaction mixture was filtered and the filtrate was concentrated to give the product **29,** which was purified by chromatograph column (800 mg, yield: 74%).
**LCMS:** *m*/*z* 216 (M+H)⁺.

### Procedure for preparation of 30:

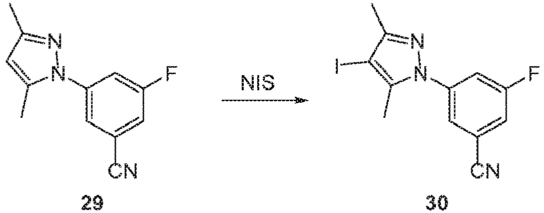

To a solution of **29**(730mg, 3.39mmol) in CHCl₃ (50mL) was added NIS (839mg, 3.73mmol). Then the reaction mixture was stirred at reflux for 2 hours. LCMS showed that the reaction was complete, then the reaction mixture was concentrated and purified by chromatograph column to give the desired product **30**(700mg, yield: 61%).
**LCMS:** *m*/*z* 342 (M+H)⁺.

### Procedure for preparation of 31:

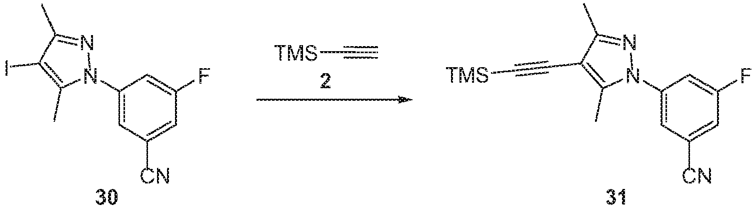

To a solution of **31** (700 mg, 2.05 mmol) in 50mL of degassed CH₃CN was added successively CuI (39 mg, 0.205 mmol), **2** (403 mg, 4.10 mmol), and Pd(PPh₃)₂Cl₂ (144 mg, 0.205 mmol) and Et₃N (623 mg, 6.16 mmol). The mixture was stirred at 70 °C for 18 hours. LCMS showed that the reaction was completed, then the reaction mixture was filtered and the filtrate was concentrated to give the crude product, which was purified by chromatograph column to give the product 31(600mg, yield 94%).
**LCMS:** *m*/*z* 312 (M+H)⁺.

### Procedure for preparation of 32:

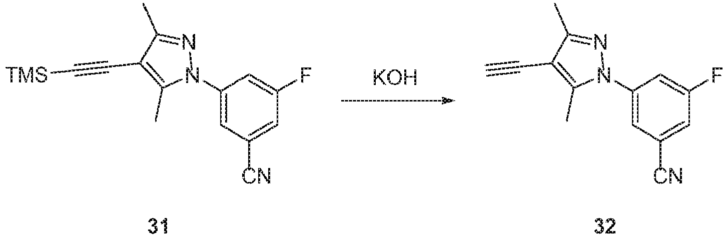

To a solution of **31** (600 mg, 1.93 mmol) in MeOH (10mL) was added KOH (216 mg, 3.85 mmol). Then the reaction mixture was stirred at room temperature for 1h. LCMS showed that the reaction was completed, then the reaction mixture was quenched with water, extracted with DCM (3 x 20 mL). The combined organic layer was dried over Na₂SO₄, concentrated to give the desired product **32**(310 mg, yield 67%).
**LCMS:** *m*/*z* 240 (M+H)⁺

### Procedure for preparation of Compound 5:

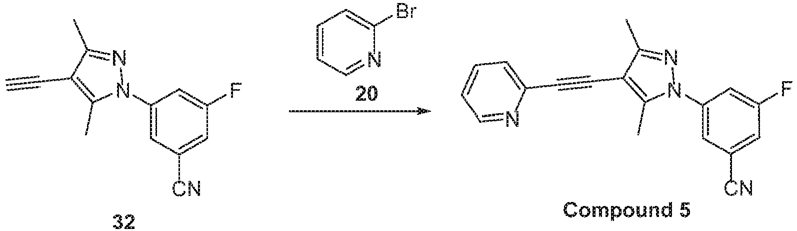

To a solution of **32** (100 mg, 0.42 mmol) in 10 mL of degassed THF (10 ml) was added successively CuI (8mg, 0.042 mmol), compound **20** (132 mg, 0.84 mmol), Pd(PPh₃)₂Cl₂ (29 mg, 0.042 mmol) and Et₃N (127 mg, 1.25 mmol). The mixture was stirred at 80 °C for 18 hours. LCMS showed that the reaction was completed, then the reaction mixture was filtered and the filtrate was concentrated and purified by prep-TLC to give the product ***Compound* 5** (35 mg, yield: 26%).
**LCMS:** *m*/*z* 317 (M+H)⁺ ;
**¹H NMR** (400 MHz, CDCl3) : δ 8.61 (d, J=4.4 Hz, 1H), 7.67-7.62 (m, 2H), 7.53-7.48 (m, 2H), 7.35-7.33 (m, 1H), 7.24-7.22 (m, 1H), 2.54 (s, 3H), 2.40(s, 3H);

### Example Compound 6

### Preparation of 3-fluoro-5-(5-methoxy-3-methyl-4-(2-(pyridin-2-yl)ethynyl)-1H-pyrazol-1-yl) benzonitrile:

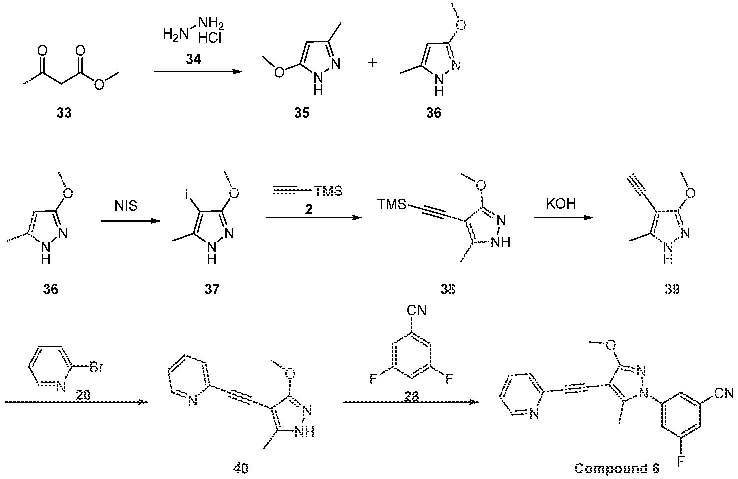

### Experimental section:

### Procedure for preparation of 36:

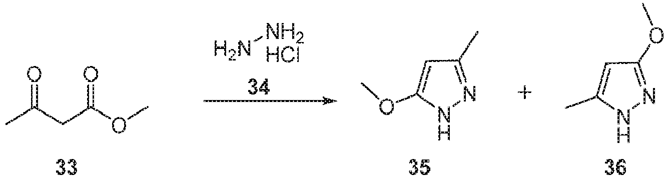

To a solution of **33** (10.00g, 86.12mmol) in MeOH (150mL) was added **34** (9.49g, 90.43mmol). Then the reaction mixture was stirred at reflux for 18 hours. The reaction mixture was concentrated and the obtained residue was treated with saturated NaHCO₃ solution (200 mL), extracted with DCM (3 x 150 mL), the combined organic layer was dried over Na₂SO₄, filtered and concentrated to give two products **35** and **36,** by separating them to get the desired product **36** (3.32 g, yield 34%).
**¹H NMR** (400 MHz, CDCl3) : δ 5.48 (s, 1H), 3.86 (s, 3H), 2.23 (s, 3H).

### Procedure for preparation of 37:

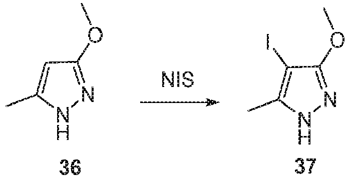

To a solution of **36** (3.32 g, 29.61 mmol) in CHCb (50 mL) was added NIS (7.33 g, 32.57 mmol). Then the reaction mixture was stirred at reflux for 2 hours. LCMS showed that the reaction was complete, then the reaction mixture was concentrated to give the desired product **37,** which was used in the next step without further purification (7.1 g, crude).
**LCMS:** *m*/*z* 239 (M+H)⁺.

### Procedure for preparation of 38:

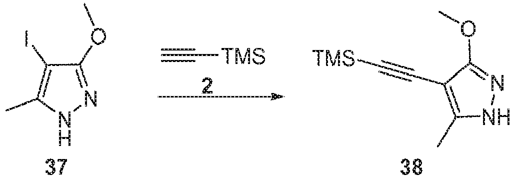

To a solution of compound **37** (3.20 g, 13.44mmol) in 60 mL of degassed CH₃CN was added successively CuI (0.26 g, 1.34mmol), **2** (2.64 g, 26.89mmol), and Pd(PPh₃)₂Cl₂ (0.94 g, 1.34mmol) and Et₃N (4.08 g, 40.33mmol). The mixture was stirred at 70 °C ***for*** 18 hours. LCMS showed that the reaction was completed, then the reaction mixture was filtered and the filtrate was concentrated to give the product **38,** which was purified by chromatograph column (910 mg, yield 32%).
**LCMS:** *m*/*z* 209 (M+H)⁺

### Procedure for preparation of 39:

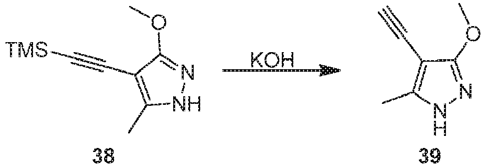

To a solution of **38** (900 mg, 4.37 mmol) in MeOH (20mL) was added KOH (490 mg, 8.74 mmol). Then the reaction mixture was stirred at room temperature for 1 hour. LCMS showed that the reaction was completed, then the reaction mixture was quenched with water, extracted with DCM (3 x 20 mL). The combined organic layer was dried over Na₂SO₄, concentrated to give the desired product **39**(380 mg, yield 64%).
**LCMS:** *m*/*z* 273 (M+H)⁺.

### Procedure for preparation of 40:

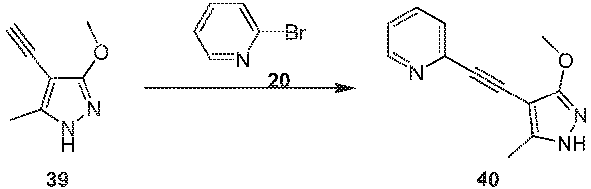

To a solution of **39** (380 mg, 2.79 mmol) in 20 mL of degassed THF was added successively CuI (53 mg, 0.28 mmol), compound **20** (882 mg, 5.58 mmol), and Pd(PPh₃)₂Cl₂ (196 mg, 0.28 mmol) and Et₃N (847 mg, 8.37 mmol). The mixture was stirred at 80 °C for 18 hours. LCMS showed that the reaction was completed, then the reaction mixture was filtered and the filtrate was concentrated and purified by prep-TLC to give the product **40**(320 mg, yield 54%).
**LCMS:** *m*/*z* 214 (M+H)⁺.

### Procedure for preparation of Compound 6:

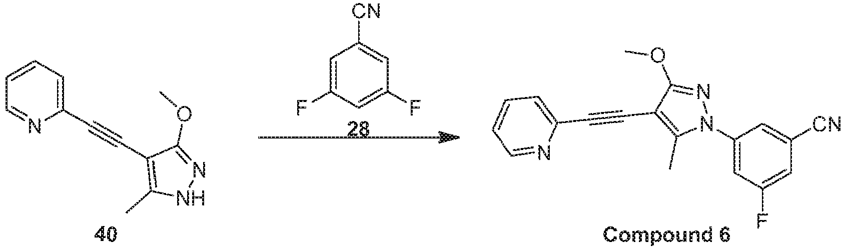

To a solution of **40** (100 mg, 0.47mmol) in 5 mL of degassed DMF was added successively compound **28** (98 mg, 0.70 mmol) and CS₂CO₃ (458 mg, 1.41 mmol). The mixture was heated to 120 °C and stirred for 2 hours. LCMS showed that the reaction was completed, then the reaction mixture was filtered and the filtrate was concentrated to give the product **Compound 6,** which was purified by prep-HPLC (36 mg, yield 23%).
**LCMS:** *m*/*z* 333 (M+H)⁺;
**¹H NMR** (400 MHz, CDCl3) : δ 8.61 (d, *J*=3.2 Hz, 1H), 7.66-7.63 (m, 2H), 7.53-7.50 (m, 2H), 7.28-7.25 (m, 1H), 7.24-7.22 (m, 1H), 4.01 (s, 3H), 2.54 (s, 3H).

### Example Compound 7

### Preparation of 2-((3-methoxy-5-methyl-1-(4-(trifluoromethoxy) phenyl)-1H-pyrazol-4-yl) ethynyl) pyridine:

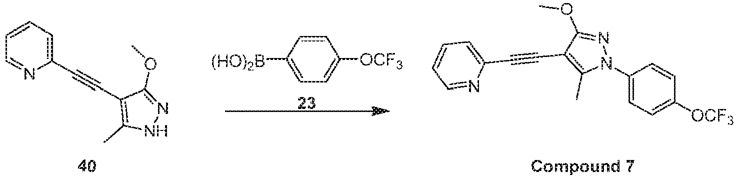

### Experimental section:

### Procedure for preparation of Compound 7:

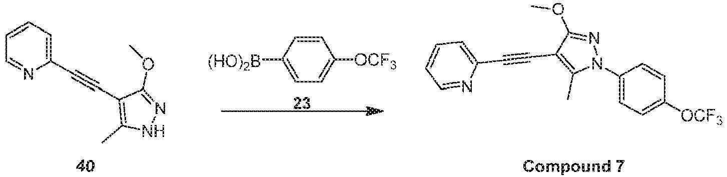

To a solution of **40** (100mg, 0.47mmol) in 10mL of DCM was added successively compound **23** (193 mg, 0.94 mmol), Cu(OAc)₂ (170 mg, 0.94 mmol), and pyridine (111 mg, 1.41 mmol). The mixture was then degassed for 1 minute under O₂ atmosphere and stirred at room temperature overnight. After the reaction was complete, the reaction mixture was filtered and the filtrate was concentrated and purified by chromatograph column to give the desired product **Compound 7** (42 mg, yield 24 %).
**LCMS:** *m*/*z* 374 (M+H)⁺
**¹H NMR** (400 MHz, CDCl3): δ 8.58 (d, *J*=4.4 Hz, 1H), 7.71-7.63 (m, 3H), 7.43-7.41 (m, 1H), 7.24-7.16 (m, 3H), 4.40 (s, 3H), 2.37 (s, 3H).

### Example Compound 8

### Preparation 2-((1-(4-fluorophenyl)-3-methoxy-5-methyl-1H-pyrazol-4-yl)ethynyl)pyridine:

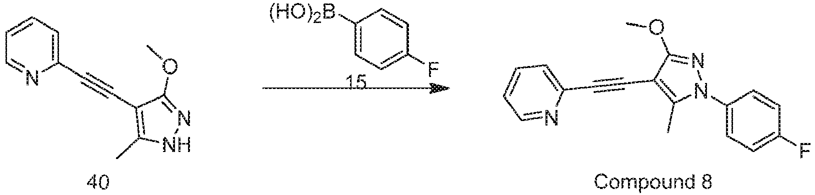

### Experimental section:

### Procedure for preparation of Compound 8:

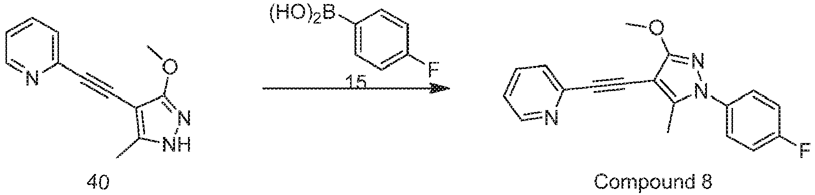

To a solution of **40** (100 mg, 0.47 mmol) in 10mL of DCM was added successively **15** (131 mg, 0.94 mmol), Cu(OAC)₂ (170 mg, 0.94 mmol), and pyridine (111 mg, 1.41 mmol). The mixture was then degassed for 1 minute under O₂ atmosphere and stirred at room temperature overnight. After the reaction was complete, the reaction mixture was filtered and the filtrate was concentrated and purified by chromatograph column to give the desired product **Compound 8** (50 mg, yield 35%).
**LCMS:** *m*/*z* 308 (M+H)⁺ ;
**¹H NMR** (400 MHz, CDCl3): δ 8.58 (s, 1H), 7.63-7.61 (m, 1H), 7.49-7.39 (m, 3H), 7.17-7.13 (m, 3H), 3.99 (s, 3H), 2.39 (s, 3H).

### Example Compound 9

### Preparation 2-((1-(4-fluorophenyl)-5-methoxy-3-methyl-1H-pyrazol-4-yl)ethynyl)pyridine:

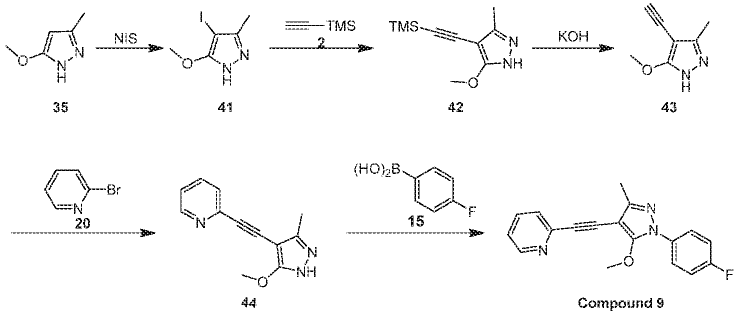

### Experimental section:

### Procedure for preparation of 41:

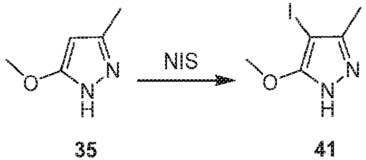

To a solution of compound **35** (3.32 g, 29.61 mmol) in CHCl₃ (50 mL) was added NIS (7.33 g, 32.57 mmol). Then the reaction mixture was stirred at reflux for 2 hours. LCMS showed that the reaction was completed. The reaction mixture was concentrated to give the desired product **41,** which was used in the next step without further purification (7.1 g, crude).
**LCMS:** *m*/*z* 239 (M+H)⁺.

### Procedure for preparation of 42:

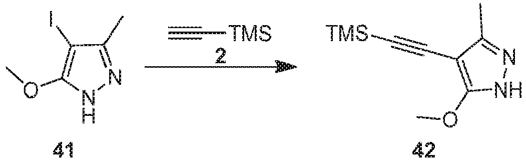

To a solution of compound **41** (3.20 g, 13.44 mmol) in 60 mL of degassed CH₃CN was added successively CuI (0.26 g, 1.34 mmol), **2** (2.64 g, 26.89 mmol), and Pd(PPh₃)₂Cl₂ (0.94 g, 1.34 mmol) and Et₃N (4.08 g, 40.33 mmol). The mixture was stirred at 70 °C for 18 hours. LCMS showed that the reaction was completed, then the reaction mixture was filtered and the filtrate was concentrated to give the product **42,** which was purified by chromatograph column (910 mg, yield 32%).
**LCMS:** *m*/*z* 209 (M+H)⁺.

### Procedure for preparation of 43:

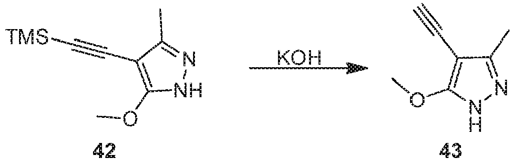

To a solution of compound **42**(900 mg, 4.37 mmol) in MeOH (20mL) was added KOH (490 mg, 8.74 mmol). Then the reaction mixture was stirred at room temperature for 1 hour. LCMS showed that the reaction was completed. The reaction mixture was quenched with water, extracted with DCM (3 x 20 mL). The combined organic layer was dried over Na₂SO₄, concentrated to give the desired product **43**(380 mg, yield 64%).
**LCMS:** *m*/*z* 273 (M+H)⁺.

### Procedure for preparation of 44:

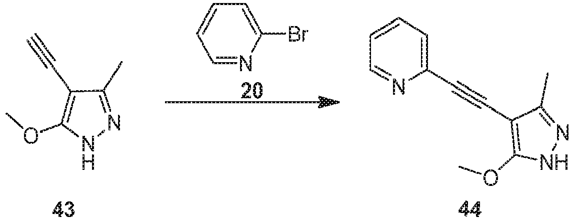

To a solution of compound **43** (380 mg, 2.79 mmol) in 20 mL of degassed THF was added successively CuI (53 mg, 0.28 mmol), **20** (882 mg, 5.58 mmol), and Pd(PPh₃)₂Cl₂ (196 mg, 0.28 mmol) and Et₃N (847 mg, 8.37 mmol). The mixture was stirred at 80 °C for 18 hours. LCMS showed that the reaction was completed, then the reaction mixture was filtered and the filtrate was concentrated and purified by prep-TLC to give the product **44**(320 mg, yield 54%).
**LCMS:** *m*/*z* 214 (M+H)⁺

### Procedure for preparation of Compound 9:

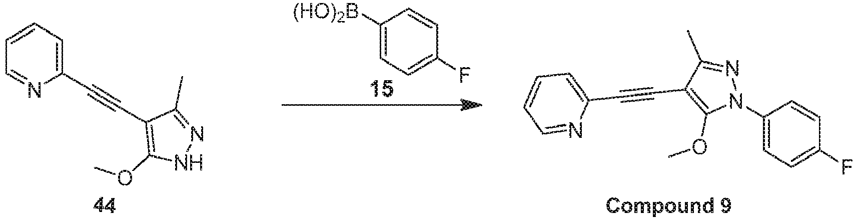

To a solution of **44** (100mg, 0.47mmol) in 10mL of DCM was added successively **15** (131mg, 0.94mmol), Cu(OAC)₂ (170mg, 0.94mmol), and pyridine (111mg, 1.41mmol). The mixture was then degassed ***for*** 1 minute under O₂ atmosphere and stirred at room temperature overnight. After the reaction was complete, the reaction mixture was filtered and the filtrate was concentrated and purified by chromatograph column to give the desired product **Compound 9** (32mg, yield 22%).
**LCMS:** *m*/*z* 308 (M+H)⁺ ;
**¹H NMR** (400 MHz, CDCl3) : δ 8.58 (s, 1H), 7.63-7.61 (m, 1H), 7.49-7.39 (m, 3H), 7.17-7.13 (m, 3H), 4.37 (s, 3H), 2.36 (s, 3H).

### Example Compound 10

### Preparation of 2-((3-methyl-5-methoxy-1-(4-(trifluoromethoxy) phenyl)-1H-pyrazol-4-yl) ethynyl) pyridine:

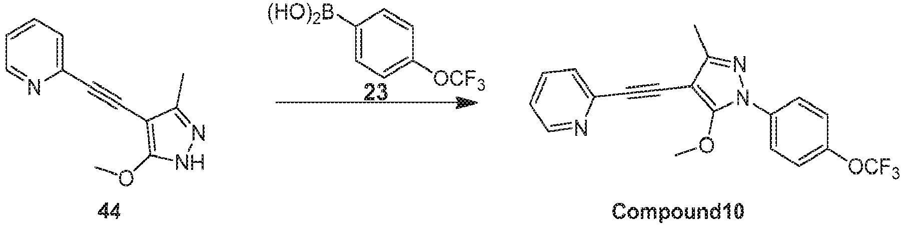

### Experimental section:

### Procedure for preparation of Compound 10:

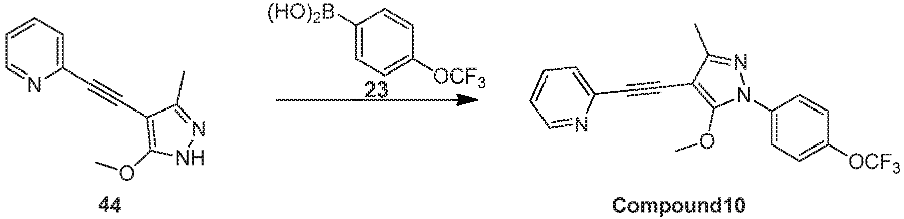

To a solution of **44**(100mg, 0.47mmol) in 10mL of DCM was added successively **23** (193mg, 0.94mmol), Cu(OAc)₂ (170mg, 0.94mmol), and pyridine (111mg, 1.41mmol). The mixture was then degassed for 1 minute under O₂ atmosphere and stirred at room temperature overnight. After the reaction was completed, the reaction mixture was filtered and the filtrate was concentrated and purified by chromatograph column to give the desired product **Compound 10** (30mg, yield 17%).
**LCMS:** *m*/*z* 374 (M+H)⁺ ;
**¹H NMR** (400 MHz, CDCl3) : δ 8.58 (d, *J*=4.4 Hz, 1H), 7.64-7.62 (m, 1H), 7.50-7.47 (m, 3H), 7.30-7.29 (m, 2H), 7.18-7.16 (m, 1H), 4.00 (s, 3H), 2.44 (s, 3H).

### Example Compound 11

### Preparation of 3-fluoro-5-(3-methyl-4-(pyridin-2-ylethynyl)-5-(trifluoromethyl)-1H-pyrazol-1-yl)benzonitrile:

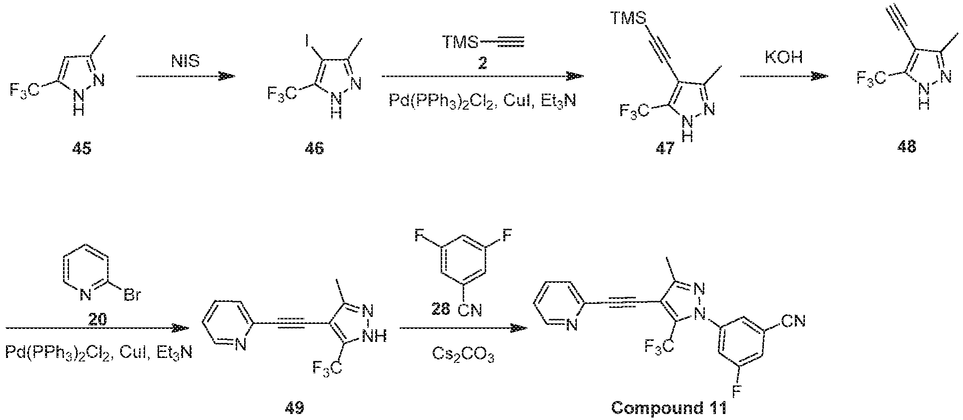

### Experimental section:

### Procedure for preparation of 46:

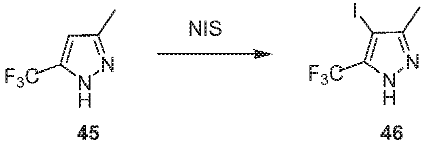

To a solution of compoud **45** (1g, 6.66mmol) in CHCl₃ (10mL) was added NIS (1.82g, 7.99mmol). The mixture was stirred at 60°C for 2 hours, then reaction mixture was quenched with water and extracted with DCM (2×20mL.). The combined organic layer was concentrated under vacuo and the residue was purified by silica gel chromatography to give the product **46**(1.55g, 84%).
**LCMS:** m/z, 277.0 (M+H)⁺.

### Procedure for preparation of 47:

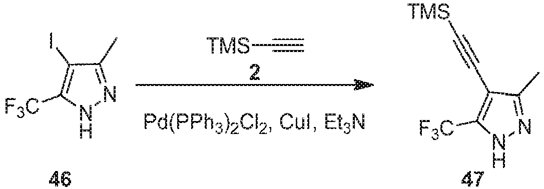

To a solution of compound **46** (1.55g, 5.62mmol) in CH₃CN (20mL) was added successively CuI (0.107g, 0.562mmol), **2** (1.10g, 11.23mmol), and Pd(PPh₃)₂Cl₂ (0.197g, 0.281mmol). The mixture was then degassed for 1 minute under N₂ atmosphere and was heated at 70 °C for 4 hours. The reaction mixture was filtered, 60mL of H₂O was added into the filtrate, and extracted with EtOAc (3×50mL). The organic layer was washed with brine (2×30mL) and dried over anhydrous Na₂SO₄, filtered and evaporated to give the product **47** (1.3g, 94%), which was used the next step without further purification.
**LCMS:** *m*/*z*, 247.1 (M+H)⁺.

### Procedure for preparation of 48:

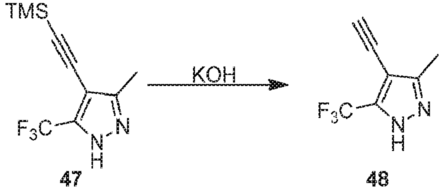

compoud **47** (1.5g, 6.09mmol) was dissolved in MeOH/CH₂Cl₂ (20mL/10mL), after cooled to 0°C, KOH (0.535g, 9.54mmol) was added. The reaction mixture stirred at room temperature for 2 hours, and then quenched with H₂O, extracted with EtOAc (3 × 30mL). The combined organic layers were dried over anhydrous Na₂SO₄, filtered and concentrated to give product **48** (1g, crude).
**LCMS:** *m*/*z*, 175.1 (M+H)⁺.

### Procedure for preparation of 49:

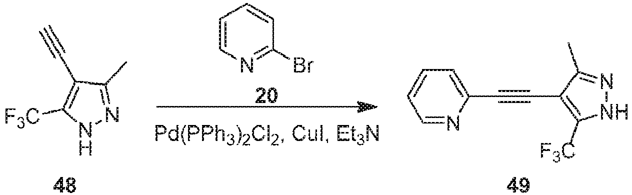

To a solution of **48**(1g, 5.74mmol), **20**(1.98g, 11.49mmol), CuI (109mg, 0.574mmol), Et₃N (1.74g, 17.23mmol) in THF (30mL) was added Pd(PPh₃)₂Cl₂ (202mg, 0.288mmol). The reaction mixture was stirred at 90°C overnight, then the mixture was filtered and concentrated by vacuo to give the crude product which was purified by silica gel chromatography to give the product**49**(700mg, 49%).
**LCMS:** *m*/*z*, 252.1 (M+H)⁺.

### Procedure for preparation of Compound 11:

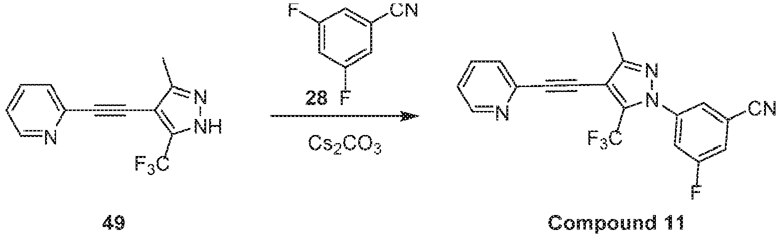

To a solution of **49** (100mg, 0.398mmol) in DMF (5mL) was added **28** (111mg, 0.796mmol) and Cs₂CO₃ (259mg, 0.796mmol). The reaction mixture was heated at 110°C for 2 hours, then filtered and concentrated under vacuo. The residue was purified by prep-TLC to give the desired product **Compound 11** (50mg, 34%).
**LCMS:** *mlz,* 371.1 (M+H)⁺;
**¹H NMR** (400 MHz, CDCl3): δ 8.65-8.64 (s, 1H), 7.72 (m, 1H), 7.67 (m, 1H), 7.59-7.57 (m, 2H), 7.56 (m, 1H), 7.26 (m, 1H), 2.60 (s, 3H).

### Example Compound 12

### Preparation of 2-((1-(4-fluorophenyl)-3-methyl-5-(trifluoromethyl)-1H-pyrazol-4-yl) ethyny) pyridine:

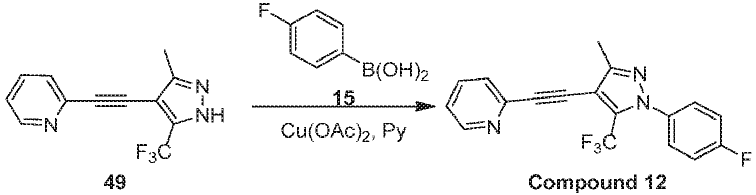

### Experimental section:

### Procedure for preparation of Compound 12:

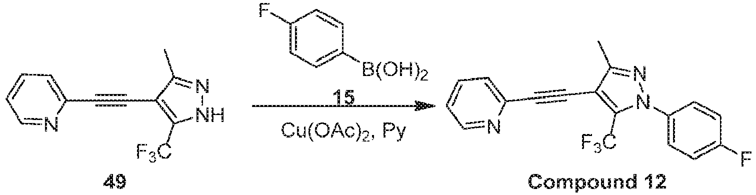

To a solution of compoud **49** (100mg, 0.398mmol) in DCM (10mL) was added **15** (111mg, 0.796mmol), Cu(OAC)₂ (145mg, 0.796mmol), pyridine (94mg, 19mmol). The reaction mixture was stirred at room temperature overnight under O₂ atmosphere. The mixture was filtered and concentrated under vacuo. The residue was purified by prep-TLC to give the desired product **Compound 12** (50mg, 36%).
**LCMS:** *m*/*z*, 346.1 (M+H)⁺;
**¹H NMR** (400 MHz, CDCl3): δ 8.57-8.56 (m, 1H), 7.63 (m, 1H), 7.47 (m, 1H), 7.39-7.37 (m, 2H), 7.19-7.13 (m, 3H), 2.41 (s, 3H).

### Example Compound 13

### Preparation of 2-((3-methyl-1-(4-(trifluoromethoxy)phenyl)-5-(trifluoromethyl)-1H-pyrazol-4-yl) ethynyl) pyridine:

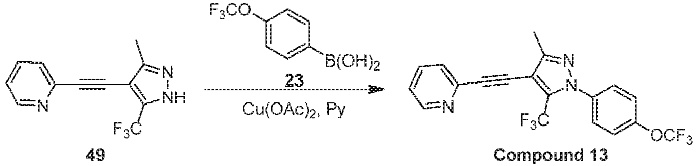

### Experimental section:

### Procedure for preparation of Compound 13:

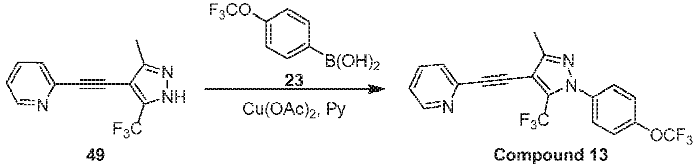

To a solution of **49** (100mg, 0.398mmol) in DCM (10mL) was added **23** (164mg, 0.796mmol), Cu(OAc)₂ (145mg, 0.796mmol), pyridine (94mg,1.19mmol). The reaction mixture was stirred at room temperature overnight under O₂, then the mixture was filtered and concentrated under vacuo, residue was purified by prep-TLC to give the desired product **Compound 13** (50mg, 31%).
**LCMS:** *m*/*z*, 412.0 (M+H)⁺;
**¹H NMR** (400 MHz, CDCl3): δ 8.57-8.56 (m, 1H), 7.65-7.62 (m, 1H), 7.48-7.45 (m, 3H), 7.32-7.30 (m, 2H), 7.20-7.19 (m, 1H), 2.45 (s, 3H).

### Example Compound 14

### Preparation of 2-((3, 5-dimethyl-1-(4-(trifluoromethoxy) phenyl)-1H-pyrazol-4-yl) ethynyl) pyrimidine:

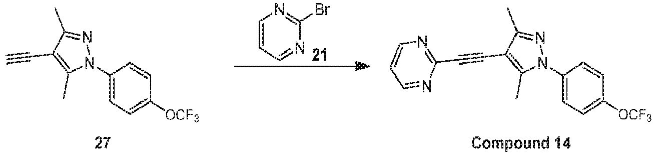

### Experimental section:

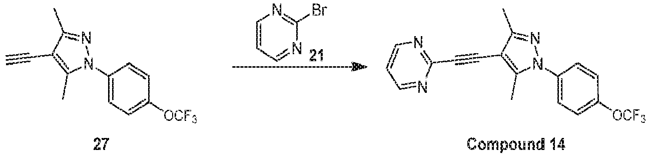

To a solution of **27** (100 mg, 0.36 mmol) in 10 mL of degassed THF (10 ml) was added successively CuI (7mg, 0.036 mmol), **21** (113 mg, 0.71 mmol), and Pd(PPh₃)₂Cl₂ (25 mg, 0.036 mmol) and Et₃N (108 mg, 1.07 mmol). The mixture was stirred at 80 °C for 18 hours. LCMS showed that the reaction was completed, then the reaction mixture was filtered and the filtrate was concentrated and purified by prep-TLC to give the product **Compound 14** (6 mg, yield 5%). **LCMS:** *m*/*z* 359 (M+H⁺) ;
**¹H NMR** (400 MHz, CDCl3): δ 8.74 (d, J=4.8 Hz, 2H), 7.50-7.48 (m, 2H), 7.34-7.32 (m, 2H), 7.23-7.21 (m, 1H). 2.50 (s, 3H), 2.45 (s, 3H).

### Example Compound 15

### Preparation of 3-(3-(dimethylaminuteso)-5-methyl-4-(2-(pyridin-2-yl)ethynyl)-1H-pyrazol-1-yl)-5-fluorobenzonitrile:

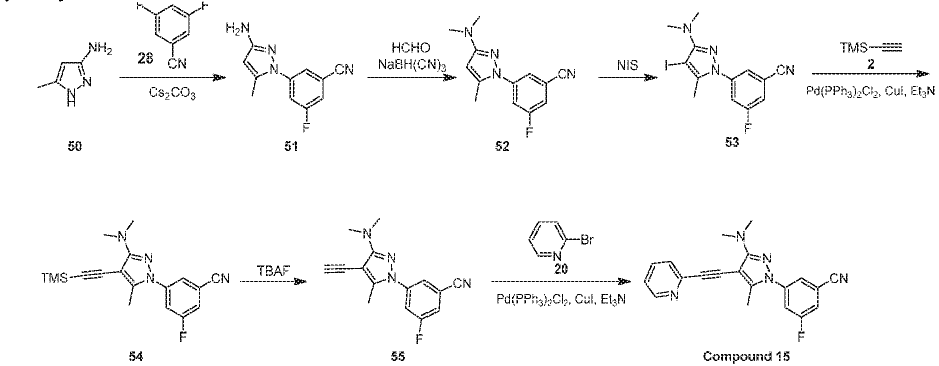

### Experimental section:

### Procedure for preparation of 50:

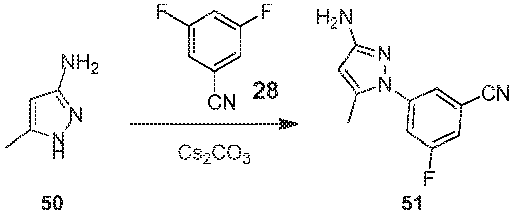

To a solution of **50** (500mg, 5.15mmol) in DMF (10mL) was added **28** (1430mg, 10.30mmol) and Cs₂CO₃ (3350g, 10.30mmol). The reaction mixture was heated at 110°C for 2 hours, then the mixture was filtered and concentrated under vacuo. The residue was purified by silica gel chromatography to give product **51** (350mg, 31%).
LCMS: *m*/*z,* 217.1 (M+H)⁺.

### Procedure for preparation of 52:

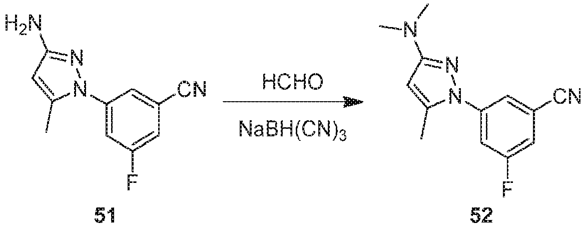

To a solution of **51** (500mg, 2.31 mmol) in MeOH (15mL) was added HCHO (37% in H₂O) (15mL) and AcOH (1mL). The mixture was stirred at room temperature overnight, then NaBH(CN)₃ (783mg, 6.94mmol) was added to the mixture which was stirred for another 1 hour. The mixture was diluted with DCM, the organic layer was separated, concentrated and purified by silica gel chromatography to give product **52** (150mg, 27%).
**¹H NMR** (400 MHz, CDCl3): δ 7.62 (s, 1H), 7.54-7.51 (m, 1H), 7.19-7.17 (m, 1H), 5.72 (s, 1H), 2.90 (s, 6H), 2.40 (s, 3H).

### Procedure for preparation of 53:

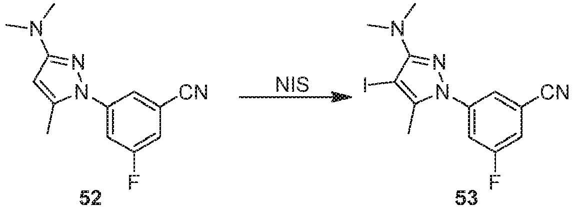

To a solution of **52** (100mg, 0.410mmol) in CHCl₃ (10mL) was added NIS (110mg, 0.492mmol). The reaction mixture was stirred at 60°C for 2 hours, then the mixture was quenched with water and extracted with DCM (2x20mL). The combined organic layer was concentrated under vacuo and the residue was purified by prep-TLC to give product **53** (120mg, 79%).
**LCMS:** *m*/*z,* 371.0 (M+H)⁺.

### Procedure for preparation of 54:

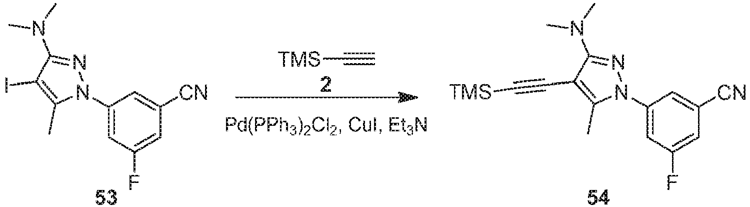

To a solution of **53** (120mg, 0.324mmol) in CH₃CN (10mL) was added successively CuI (6mg, 0.032mmol), **2** (64mg, 0.648mmol), Pd(PPh₃)₂Cl₂ (11mg, 0.016mmol) and Et₃N (98mg, 0.972mmol). The mixture was then degassed for 1 minute under N₂ atmosphere and was heated att 70°C for 7 hours. The reaction mixture was filtered and concentrated by vacuo and the residue was purified prep-TLC to give product **54** (110mg, 100%).
LCMS: *m*/*z,* 341.1 (M+H)⁺.

### Procedure for preparation of 55:

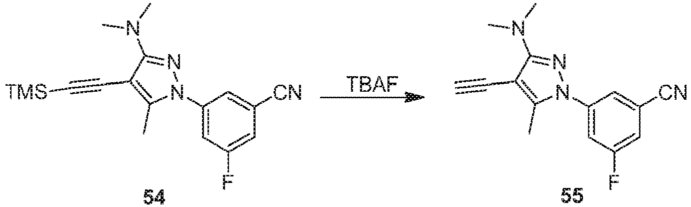

Compoud **54(100mg,** 0.294mmol) was dissolved in THF (8mL). TBAF (1M in THF) (0.44mL, 0.44mmol) was added to the solution dropwise. The reaction mixture stirred at room temperature for 1 hour. The mixture was extracted with EA. The combined organic layer was concentrated by vacuo and purified with prep-TLC to give product **55**(70mg, 89%).

### Procedure for preparation of Compound 15:

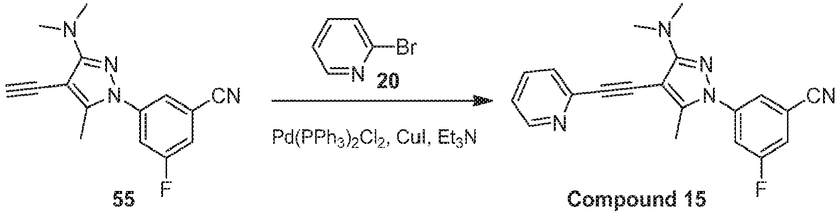

To a solution of **55** (70mg, 0.261 mmol), **20** (82mg, 0.522mmol), CuI (5mg, 0.026mmol), Et₃N (79mg, 0.783mmol) in THF (5mL) Pd(PPh₃)₂Cl₂(9mg, 0.013mmol)was added. The mixture was microwaved at 90°C for 1 hour, then the mixture was filtered and concentrated under vacuo. The residue was purified by prep-TLC to give the desired product **Compound 15** (20mg, 22%).
**LCMS:** *m*/*z,* 346.2 (M+H)⁺;
**¹H NMR** (400 MHz, CDCl3): δ 8.61-8.60 (m, 1H), 7.66-7.64 (m, 2H), 7.54-7.52 (m, 1H), 7.45-7.43 (m, 1H), 7.24-7.21 (m, 2H), 3.12 (s, 6H), 2.54 (s, 3H).

### Example Compound 16

### Preparation of 2-(3-(dimethylaminuteso)-5-methyl-1-(4-(trifluoromethoxy)phenyl)-1H-pyrazol-4-yl)ethynyl)pyridine:

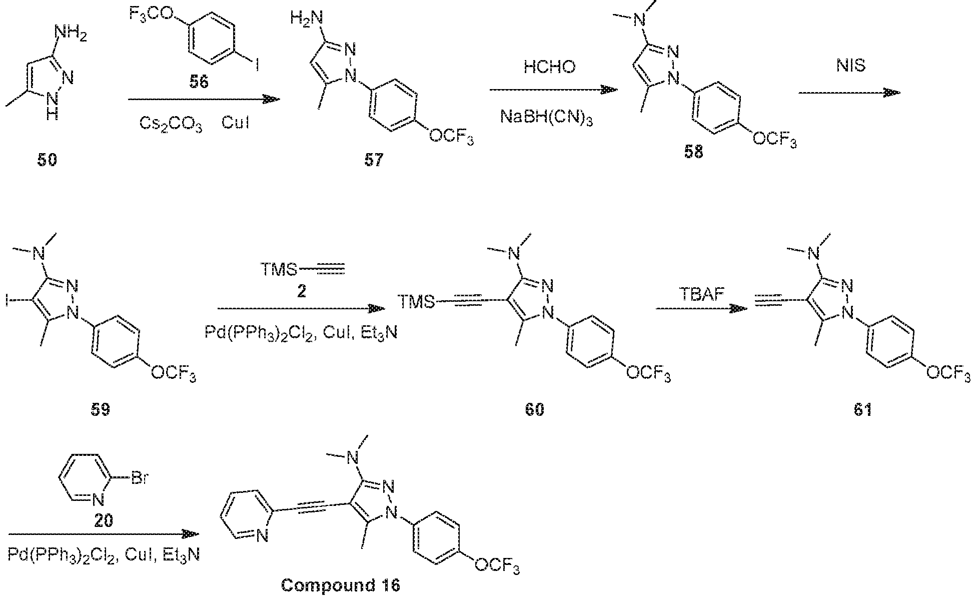

### Experimental section:

### Procedure for preparation of 57:

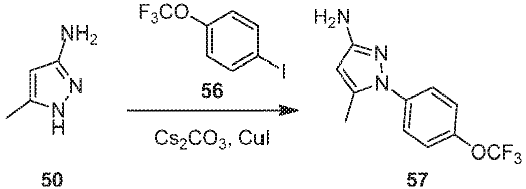

To a solution **of 50** (1.56g, 16.06mmol) in DMF (50mL) was added **56** (5.09g, 17.67mmol), CuI (306mg, 1.61mmol), Cs₂CO₃ (10.47g, 32.13mmol). The mixture was stirred at 110°C for 5 hours under N₂ atmosphere, then the mixture was filtered and concentrated to give the crude product which was purified by silica gel chromatography to give product **57**(800mg, 19%).
**LCMS:** *m*/*z,* 259.1 (M+H)⁺.

### Procedure for preparation of 58:

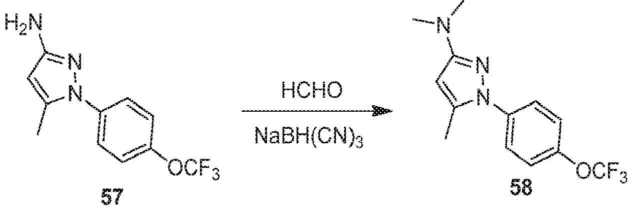

To a solution of **57** (800mg, 3.11mmol) in MeOH (20mL) was added 37% HCHO (aq.) (20mL) and AcOH(0.5mL). The mixture was stirred at room temperature overnight, then NaBH(CN)₃ (1050mg, 9.33mmol) was added to the mixture which was stirred for another 1 hours. The mixture was diluted with DCM, the organic layer was separated, concentrated and purified by silica gel chromatography to give product **58**(130mg, 15%).
**LCMS:** *m*/*z,* 286.1 (M+H)⁺.

### Procedure for preparation of 59:

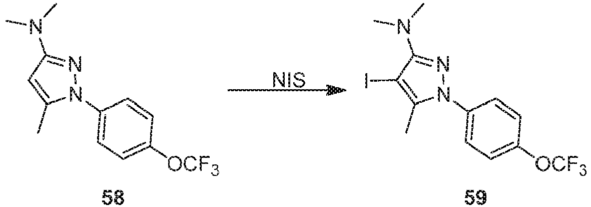

To a solution of **58** (130mg, 0.456mmol) in CHCl3 (10mL) was added NIS (123mg, 0.547mmol). The mixture was stirred at 60°C for 2 hours, then the mixture was quenched with water and extracted with DCM (2x20mL). The combined organic layer was concentrated under vacuo and the residue was purified by prep-TLC to give the product **59** (130mg, 69%).
**LCMS:** *m*/*z,* 412.0 (M+H)⁺.

### Procedure for preparation of 60:

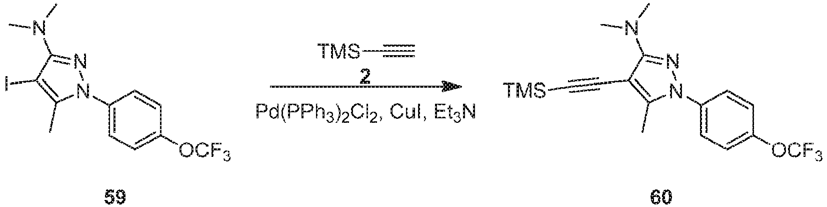

To a solution of **59**(130mg, 0.316mmo!) in CH₃CN (5mL) was added successively CuI (6mg, 0.032mmol), **2** (62mg, 0.632mmol), Pd(PPh₃)₂Cl₂ (11mg, 0.016mmol) and Et₃N (96mg, 0.949mmol). The mixture was degassed for 1 minute under N₂ atmosphere and was heated at 95°C for 1 hour under microwave. Then the reaction mixture was filtered and concentrated by vacuo and the residue was purified by prep-TLC to give product **60** (50mg, 42%).
**LCMS:** *m*/*z,* 382.1 (M+H)⁺.

### Procedure for preparation of 61:

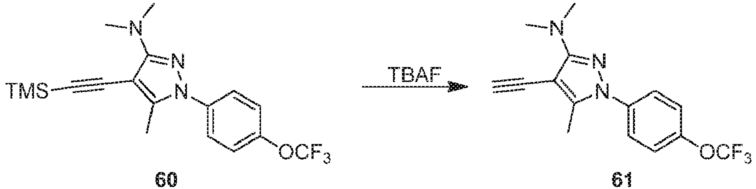

A solution of **60** (50mg, 0.2131mmol) in THF (5mL), TBAF(1M in THF) (0.197mL, 0.197mmol) was added to the solution dropwise. The reaction mixture was stirred at room temperature for 1 hour, then the mixture was extracted with EA. The combined organic layer was concentrated by vacuo and purified with prep-TLC to give product **61** (30mg, 74%). **LCMS:** *m*/*z,* 310.1 (M+H)⁺.

### Procedure for preparation of Compound 16:

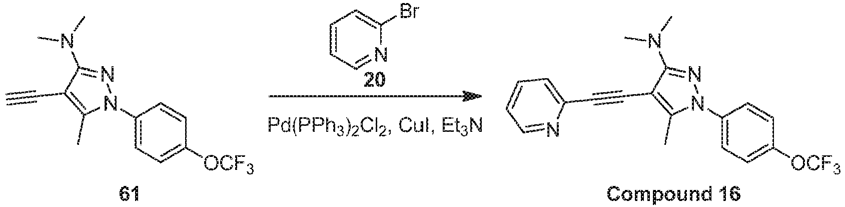

To a solution of **61** (30mg, 0.097mmol), **20** (31mg, 0.194mmol), CuI (2mg, 0.010mmol), and Et₃N (29mg, 0.291mmol) in THF (3mL) Pd(PPh₃)₂Cl₂ (3mg, 0.004mmol)was added. The mixture was stirred at 90°C under microwave for 1 hour, then the mixture was filtered and concentrated under vacuo. The residue was purified by prep-HPLC to give the desired product **Compound 16**(5mg, 14%).
**LCMS:** *m*/*z*, 387.1 (M+H)⁺;
**¹H NMR** (400 MHz, CDCl3): δ 8.62 (s, 1H), 7.65-7.63 (m, 1H), 7.49-7.48 (m, 2H), 7.47-7.46 (m, 1H), 7.28-7.25 (m, 2H), 7.24-7.23 (m, 1H), 3.10 (s, 6H), 2.48 (s, 3H).

### Example Compound 17

### Preparation of 2-((5-chloro-1-(4-fluorobenzyl)-1H-pyrazol-4-yl) ethynyl) pyrimidine:

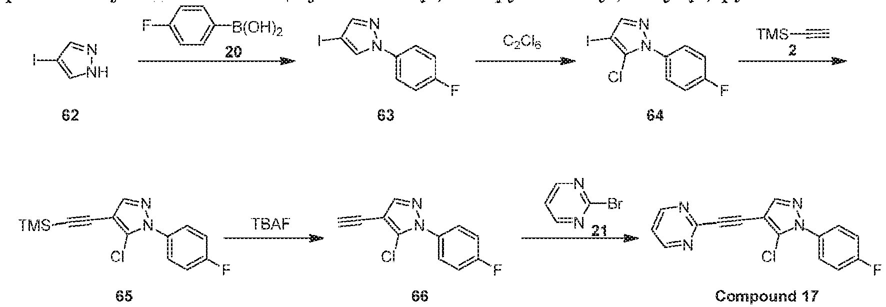

### Experimental section:

### Procedure for preparation of 63:

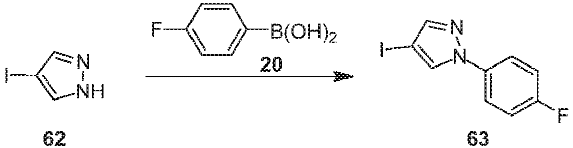

To a solution of **62**(1g, 5.16mmol) and **20**(1.08g, 7.73mmol) in DCM(20ml) was added Cu(OAc)₂ (1.87g, 10.3mmol) and pyridine(1.22g, 15.5mmol) at room temperature under O₂ atmosphere. The reaction mixture was stirred at room temperature overnight, then the mixture was filtered and filter cake was washed with DCM(20ml), The combined organic layers were washed with brine and dried over Na₂SO₄. The crude product was purified by silica gel chromatography to give product **63**(1.1g, yield:74%).
**¹HNMR** (400 MHz, CDCl₃): 67.06∼7.10 (m, 2 H), 7.49∼7.51 (m, 2 H), 7.68 (s, 1 H), 7.83(s, 1 H).

### Procedure for preparation of 64:

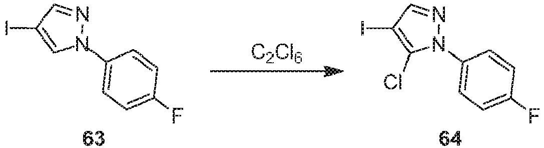

To a solution of **63**(1g, 3.47mmol) in 30mL of degassed THF was added successively, LDA (2.1mL, 4.17mmol) at -78°C. After stirring 30 minutes, perchloroethane (0.99mg, 4.17mmol) was added. The reaction mixture was stirred at -78°C for 30 minutes. An aqueous solution of NH₄Cl (5mL) was added drop-wise into the reaction mixture once the reaction mixture was warmed to room temperature. The mixture was extracted with ethyl acetate (30mL), organic layer was washed with water, dried over (Na₂SO₄) and evaporated to dryness, which was purified by Prep-TLC to give title product **64**(700mg, yield:63%).
**¹HNMR** (400 MHz, CDCl₃): 57.06∼7. 11 (m, 2 H), 7.35∼7. 47 (m, 2 H), 7.63 (s, 1 H).

### Procedure for preparation of 65:

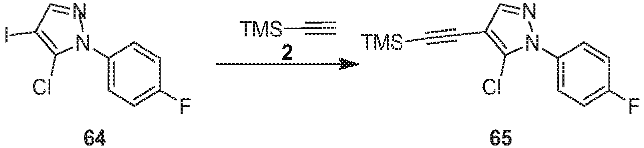

To a solution of **64**(0.7g, 2.17mmol), **2**(1.28g, 13.0mmol), CuI(41mg, 0.217mmol), Et₃N (0.66g, 6.54mmol) in THF(20mL) was added Pd(PPh₃)₂Cl₂(152mg, 0.217mmol). The suspension was degassed under vacuum and purged with N₂ several times. After stirring at 90°C for 6 hours under N₂ atmosphere, the mixture was filtered and concentrated under vacuo. The residue was purified by prep-TLC to give the product **65**(300mg, yield: 47%).
**¹HNMR** (400 MHz, CDCl₃): δ 0.21(s, 9H), 7.12∼7.17 (m, 2 H), 7.45∼7.51 (m, 2 H), 7.67 (s, 1 H).

### Procedure for preparation of 66:

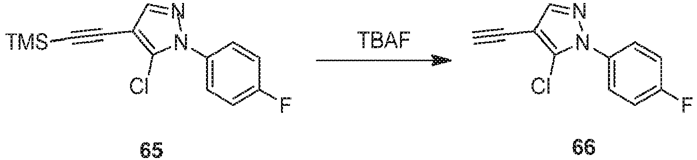

To a solution of **65**(300mg, 1.02mmol) in 10mL of degassed THF was added TBAF (1.54mL, 1.54mmol) at room temperature. After stirring 2 hours, the solvent was removed. It was extracted with DCM (20mL) and washed with brine. The organic layer was dried over Na₂SO₄ and purified by Prep-TLC to give title product **66**(200mg, yield: 88%).

### Procedure for preparation of Compound 17:

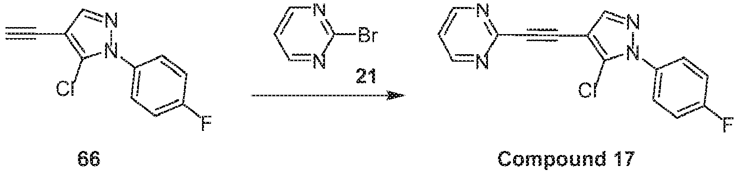

To a solution of **66**(100mg, 0.45mmol) and **21**(1 08mg, 0.68mmol), CuI (9mg, 0.045mmol), Et₃N (136mg, 1.35mmol) in degassed THF(5mL) was added Pd(PPh₃)₂Cl₂ (31mg, 0.045mmol). The suspension was degassed under vacuo and purged with N₂ several times. The mixture was stirred at 90°C for 6 hours under N₂ atmosphere. The mixture was filtered and concentrated under vacuo to afford crude product, which was purified by prep-HPLC to give the desired product **Compound 17**(20mg, yield: 15%).
**LCMS:** *m*/*z*, 299.1(M+H)⁺;
**¹HNMR** (400 MHz, CDCl₃): δ7.17∼7.25 (m, 3 H), 7.51∼7.53 (m, 2 H), 7.72 (s, 1 H), 8.71∼8.75(m, 2 H).

### Example Compound 18

### Preparation of 5-(5-chloro-4-(pyridin-2-ylethynyl-1H-pyrazol-1-yl)-2-fluoropyridine:

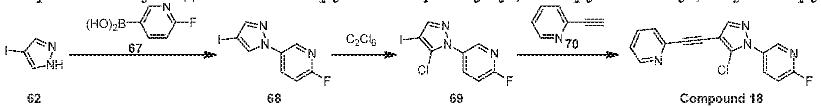

### Experimental section:

### Procedure for preparation of 68:

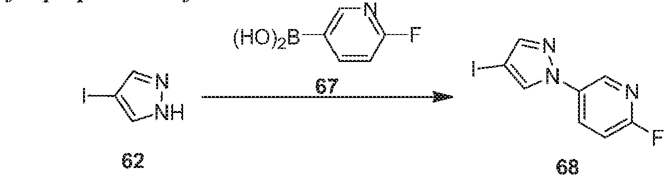

To a solution of compound **62** (1g, 5.16mmol) and compound **67** (1.09g, 7.73mmol) in DCM (20ml) was added Cu(OAc)₂ (1.87g, 10.3mmol) and pyridine(1.22g, 15.5mmol) at room temperature under O₂ atmosphere, the mixture was stirred at room temperature overnight. The suspension was filtered and filter cake was washed with DCM (20ml). The combined organic layers were washed with water and dried over Na₂SO₄. The crude product was purified by silica gel chromatography to give product **68** (0.6g, 40%).
**LCMS:** *m*/*z,* 290.9(M+H)⁺.

### Procedure for preparation of 69:

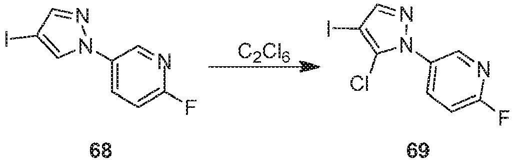

To a solution of **68** (500mg, 1.75mmol) in 20mL of degassed THF was added a solution of LDA (1.05mL, 2.1mmol) at -78°C. After stirring 30 minutes, a solution of perchloroethane (500mg, 2.1mmol) in THF (2mL) was added, and the reaction mixture was stirred at the same condition for another 30 minutess. The reaction mixture was quenched with sat.NH₄Cl, extracted with DCM and water, the organic layer was dried and purified by prep-TLC to give the title product **69** (400mg, 35%).
**LCMS:** *m*/*z,* 323.9(M+H)⁺.

### Procedure for preparation of Compound 18:

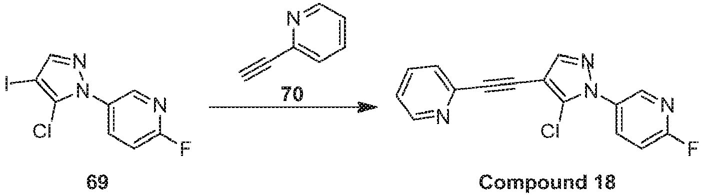

To a solution of **69** (50mg, 0.15mmol) in 4mL of degassed THF was added solid CuI (1.5mg, 0.008mmol), **70** (24mg, 0.24mmol), Et₃N (45.5mg, 0.45mmol) and Pd(PPh₃)₂Cl₂ (5.6mg, 0.008mmol). The mixture was then degassed for 2 minutes under N₂ atmosphere and stirred at 90°C for 1 hour under microwave. Most of the solvent was removed, the residue was dissolved in EtOAc (30mL), then washed with brine (2×20mL) and dried over anhydrous Na₂SO₄, filtered and evaporated to give the residue which was purified by prep-HPLC to afford the desired product **Compound 18** (5mg, 11%).
**LCMS:** *m*/*z,* 298.9(M+H)⁺;
**¹HNMR** (400 MHz, CDCl₃): δ7.11-7.14 (m, 1 H), 7.27 - 7.31 (m, 1 H), 7.54-7.56 (m, 1 H), 7.70-7.72 (m, 1 H), 7.94(s, 1H), 7.95-8.05 (m, 1 H), 8.54(s, 1 H), 8.65(d, *J*=4.0Hz, 1H).

### Example Compound 19

### Preparation of 2-((5-chloro-1-(6-fluoropyridin-3-yl)-1H-pyrazol-4-yl)ethynil)pyrimidine:

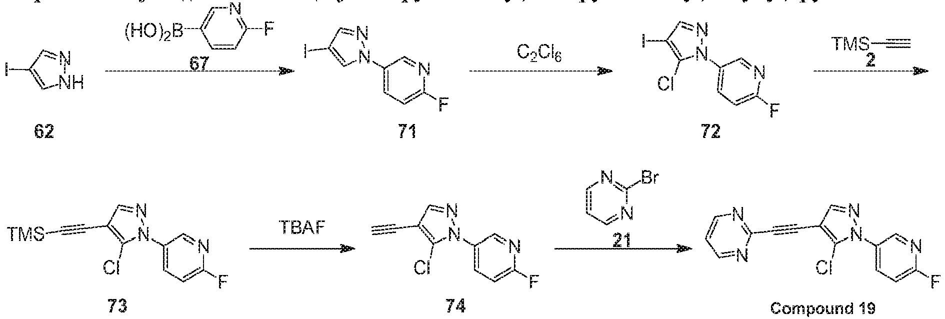

### Experimental section:

### Procedure for preparation of 71:

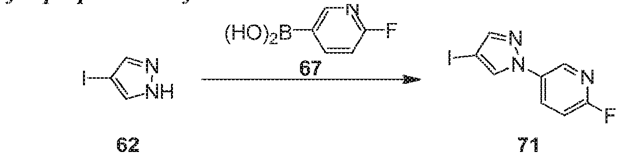

To a solution of **62** (1g, 5.16mmol) and **67** (1.09g, 7.73mmol) in DCM (20ml) was added Cu(OAc)₂ (1.87g, 10.3mmol) and pyridine(1.22g, 15.5mmol) at room temperature under O₂ atmosphere, the mixture was stirred at room temperature overnight. The suspension was filtered and the filter cake was washed with DCM (20ml). The combined organic layers were washed with water and dried over Na₂S0₄. The crude product was purified by silica gel chromatography to give product **71** (0.6g, 40%).
**LCMS:** *m*/*z,* 290.9(M+H)⁺.

### Procedure for preparation of 72:

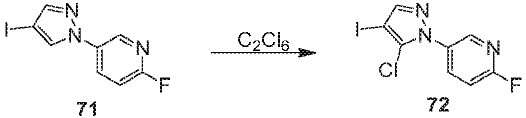

To a solution of **71** (500mg, 1.75mmol) in 20mL of degassed THF was added a solution of LDA (1.05mL, 2.1mmol) at -78°C. After stirring 30 minutes, a solution of perchloroethane (500mg, 2.1mmol) in THF (2mL) was added, and stirred at the same condition for another 30 minutes. The reaction mixture was quenched with sat.NH₄Cl. After solvent was removed, it was extracted with DCM and water, the organic layer was dried and purified by prep-TLC to give the title product **72** (400mg, 35%).
**LCMS:** *m*/*z,* 323 .

### Procedure for preparation of 73:

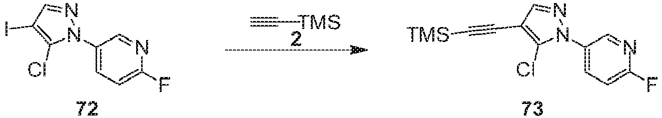

To a solution of **72** (320mg, 1.0mmol) in 4mL of degassed THF was added successively CuI (1mg, 0.02mmol), **2** (855mg, 1.0mmol), Et₃N (120mg, 1.2mmol) and Pd(PPh₃)₂Cl₂ (3mg, 0.005mmol). The mixture was then degassed for 2minutess under N₂ atmosphere and stirred at 90°C for 1h under microwave. Most of the solvent was removed. The residue was dissolved in EtOAc (30mL). The organic layer was washed with brine (2×20mL) and dried over anhydrous Na₂SO₄, filtered and evaporated to dryness on a rotary evaporator, which was purified by TLC plate to give of the product **73** (280mg,95.9%).

### Procedure for preparation of 74:

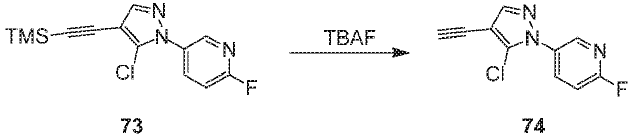

To a solution of **73** (240mg, 0.82mmol) in THF (4mL) was added a solution of TBAF-THF (1.2mL, 1.2mmol). The mixture was stirred at rt. for 1h. TLC showed the reaction was reacted complete, most of the solvent was removed. The residue was dissolved in EtOAc (20mL). The organic layer was washed with brine (2×10mL) and dried over anhydrous Na₂SO₄, filtered and evaporated to give the product **74** (150mg, 83%), which was directly used for next step.

### Procedure for preparation of Compound 19:

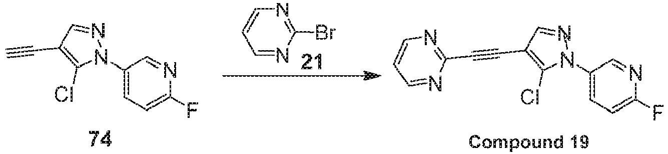

To a solution of **74** (80mg, 0.36mmol) in 4mL of degassed THF was added solid CuI (9.5mg, 0,05mmol), **21** (63mg, 0.4mmol), Et₃N (111mg, 1.1mmol) and Pd(PPh₃)₂Cl₂ (14mg, 0.02mmol). The mixture was then degassed for 2 minutes under N₂ atmosphere and stirred at 90°C for 1h under microwave. Most of the solvent was removed. The residue was dissolved in EtOAc (30mL). The organic layer was washed with brine (2×20mL) and dried over anhydrous Na₂SO₄, filtered and evaporated to give the residue which was purified by prep-HPLC to afford the desired product **Compound 19** (23mg, 21.3%).
**LCMS:** *m*/*z,* 300.0(M+H)⁺;
**¹H NMR** (400 MHz, CDCl₃): δ7.13 (dd, *J₁*=3.30 Hz, *J*₂=8.68 Hz, 1 H), 7.27 - 7.31 (m, 1H), 7.99 (s, 1H), 8.07 (m, 1 H), 8.54 (d, *J*=1.71 Hz, 1 H), 8.79 (d, *J*=4.89 Hz, 2 H).

### Example Compound 20

### Preparation of Preparation of 2-((5-chloro-1-(4-fluorobenzyl)-1H-pyrazol-4-yl) ethynyl) pyridine:

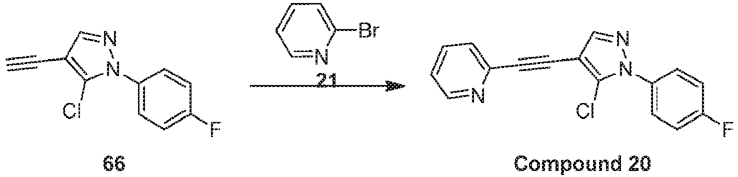

### Experimental section:

### Procedure for preparation of Compound 20:

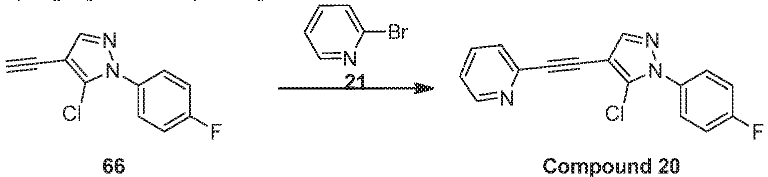

To a solution of **66**(100mg, 0.45mmol), **21**(108mg, 0.68mmol), CuI(9mg, 0.045mmol), Et₃N (136mg, 1.35mmol) in THF(5mL) was added Pd(PPh₃)₂Cl₂ (31mg, 0.045mmol). The suspension was degassed under vacuo and purged with N₂ several times. The mixture was stirred at 90°C for 6 hours under N₂ atmosphere, then the mixture was filtered and concentrated under vacuo. The residue was purified by prep-HPLC to give the desired product **Compound 20** (40mg, yield: 30%).
**LCMS:** *m*/*z,* 298.0(M+H)⁺;
**¹H NMR** (400 MHz, CHLOROFORM-d): δ 7.17-7.30 (m, 3 H), 7.51-7.56(m, 3 H), 7.65 -7.74 (m, 1 H), 7.88 (s, 1 H), 8.55 -8.72 (m, 1 H).

### Example Compound 21

### Preparation of 2-(5-chloro-1-(4-(trifluoromethoxy)phenyl)-1H-pyrazol-4-yl) pyridine:

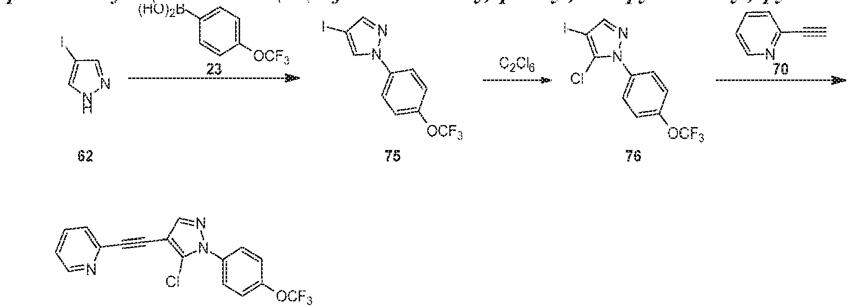

### Compound 21

### Experimental section:

### Procedure for preparation of 75:

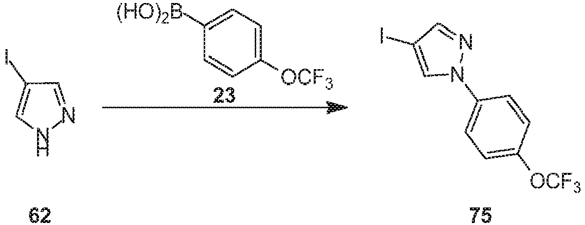

To a solution of **62**(5 g, 25.78mmol), **23**(7.96g, 38.67mmol) and Cu(AcO)₂ (7.96g, 38.67mmol) in 3mL of degassed DCM was added successively Pyridine (6.12g, 77.33mmol) at room temperature. After the reaction mixture was stirred at room temperature for 12 hours, it was filtrated and most of the solvent was removed. The residue was dissolved in EtOAc (60mL). The organic layer was washed with brine (2×30mL) and dried over anhydrous Na₂SO₄, filtered and evaporated to dryness on a rotary evaporator, which was purified by chromatography to give the product **75**(3.7g, 40.5%).
**¹H NMR** (400 MHz, CDCl₃): δ7.24 (d, *J*=8.4 Hz, 2 H), 7.60 (d, *J*=8.8 Hz, 2H), 7.66 (s, 1 H).7.87 (s, 1H).

### Procedure for preparation of 76:

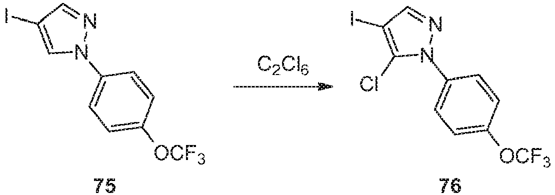

To a solution of **75** (177mg, 0.5mmol) in 3mL of degassed THF, LDA (0.3mL, 0.6mmol)was added at -78°C. After stirring 30 minutes, perchloroethane (119mg, 0.6mmol) was added, then stirred at the same condition for another 30 minutes. It was quenched with sat.NH₄Cl. The solvent was removed and extracted with DCM and water, the organic layer was dried and purified by prep-HPLC to give product **76**(50mg, 25.8%).
**¹H NMR** (400 MHz, CDCl₃): δ7.36 (d, *J*=8.4 Hz, 2 H), 7.60 (d, *J*=8.8 Hz, 2H), 7.73 (s, 1 H).

### Procedure for preparation of Compound 21:

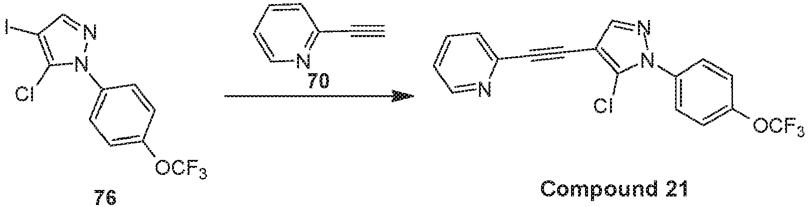

To a solution of **76** (40mg, 0.1mmol) in 3mL of degassed dioxane was added successively CuI(1 mg, 0.005mmol), **70**(16mg, 0.15mmol), Et₃N(21mg, 0,2mmol) and Pd(PPh₃)₂Cl₂ (15mg, 0.005mmol). The mixture was then degassed for 2 minutess under N₂ atmosphere and heated to 80°C overnight. Then most of the solvent was removed, the residue was dissolved in EtOAc-(30mL) and the organic layer was washed with brine (2 × 20mL) and dried over anhydrous Na₂SO₄, filtered and evaporated to give a residue which was purified by prep-HPLC to give of the desired product **Compound 21**( 25mg, 66.7%).
**LCMS:** *m*/*z,* 364.0(M+H)⁺ ;
**¹H NMR** (400 MHz, CDCl₃): δ7.25 (s, 1 H), 7.36 (d, *J*=8.56 Hz, 2 H), 7.53 (d, *J*=7.83 Hz, 1 H), 7.60-7.74 (m, 3 H), 7.89 (s, 1 H), 8.63 (d, *J*=4.40 Hz, 1 H).

### Example Compound 22

### Preparation of 2-(5-chloro-1-(4-(trifluoromethoxy) phenyl)-1H-pyrazol-4-yl) pyrimidine:

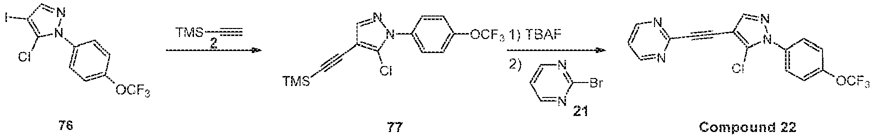

### Experimental section:

### Procedure for preparation of 77:

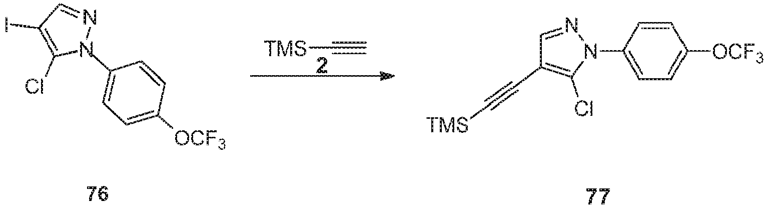

To a solution of **76** (350mg, 0.87mmol) in 4mL of degassed THF was added successively CuI (8mg, 0.04mmol), **2** (171mg, 1.7mmol), Et₃N (263mg, 2.6mmol) and Pd(PPh₃)₂Cl₂ (28mg, 0.04mmol). The mixture was then degassed for 2 minutes under N₂ atmosphere and stirred at 90°C for 1h under microwave. Then most of the solvent was removed, and the residue was dissolved in EtOAc (30mL). The organic layer was washed with brine (2×20mL) and dried over anhydrous Na₂SO₄, filtered and evaporated to afford the residue which was purified by flash chromatography to give product 77 (295mg, 91.3%)
**LCMS:** *m*/*z,* 359.0(M+H)⁺

### Procedure for preparation of Compound 22:

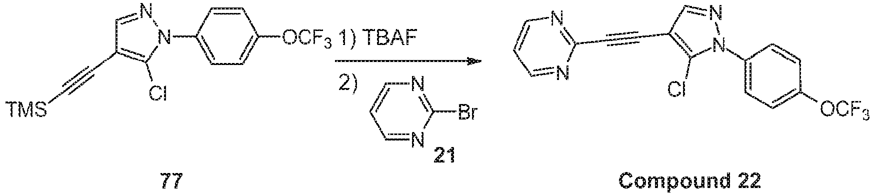

To a solution of 77 (370mg, 1mmol) in 4mL of degassed THF was added a solution of TBAF (1.5mL, 1.5mmol). It was stirred at room temperature for 1 h. Then solid CuI (9.5mg, 0.05mmol), **21** (240mg, 1.5mmol), Et₃N (303mg, 3mmol) and Pd(PPh₃)₂Cl₂ (35mg, 0.05mmol) was added into it. The mixture was then degassed for 2 minutes under N₂ atmosphere and stirred at 90°C for 1h under microwave. Most of the solvent was removed. The residue was dissolved in EtOAc (30mL). The organic layer was washed with brine (2×20mL) and dried over anhydrous Na₂SO₄, filtered and evaporated to dryness on a rotary evaporator, which was purified by flash chromatography to give the desired product **Compound 22** (196mg, 45.9%).
**LCMS:** *m*/*z,* 365.0(M+H)⁺;
**¹H NMR** (400 MHz, CDCl₃): δ7.27 (s, 1 H), 7.37 (d, *J*=8.38 Hz, 2 H), 7.66 (d, *J*=8.82 Hz, 2 H), 7.97 (s, 1 H), 8.78 (d, *J*=4.85 Hz, 2 H).

### Example Compound 23

### Preparation of 2-fluoro-5-(4-(pyridin-2-yl)-1H-pyrazol-1-yl) pyridine:

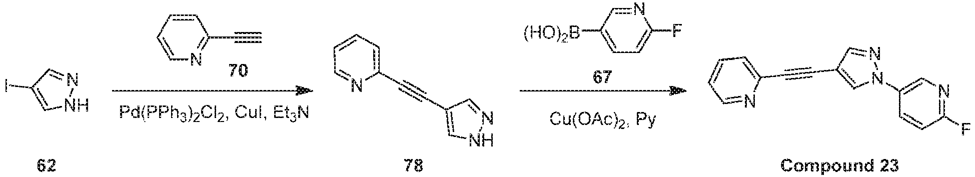

### Experimental section:

### Procedure for preparation of 78:

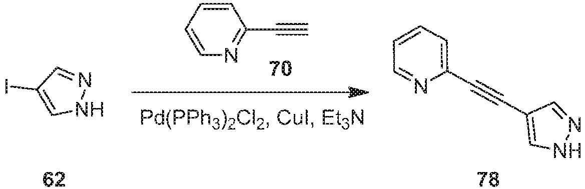

To a solution of compoud **62**(1.06g, 10.31mmol), **70** (1g, 5.16mmol), CuI (98mg, 0.516mmol), Et3N (1.57g, 15.47mmol) in Toluene (40mL) was added Pd(PPh₃)₂Cl₂ (181mg, 0.258mmol). The mixture was stirred at 100 °C overnight. The mixture was filtered and concentrated under vacuo. The residue was purified by silica gel chromatography (eluting with 0-80% EA in PE) to give product **78** (350mg, 40%).
**LCMS:** *m*/*z,* 170.1 (M+H)⁺.

### Procedure for preparation of Compound 23:

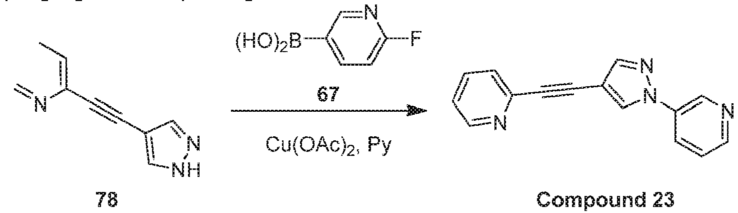

To a solution of **78** (50mg, 0.296mmol) in DCM (8mL) was added **67** (83mg, 0.591mmol), Cu(OAc)₂ (107mg, 0.591mmol), pyridine (70mg, 0.887mmol). The mixture was stirred at room temperature overnight under O₂ atmosphere, then the mixture was filtered and concentrated under vacuo. The residue was purified by prep-TLC to give the desired product **Compound 23** (20mg, 26%).
**LCMS:** *m*/*z,* 265.1 (M+H)⁺;
**¹H NMR** (400 MHz, CDCl3): δ 8.63 (s, 1H), 8.57-8.55 (m, 1H), 8.17-8.15 (m, 2H), 7.95 (s, 1H), 7.70 (m, 1H), 7.52-7.50 (m, 1H), 7.26 (m, 1H), 7.10-7.08 (m, 1H).

### Example Compound 24

### Preparation of 5-(3,5-dimethyl-4-(pyridin-2-yl)-1H-pyrazol-1-yl)-2-fluoropyridine:

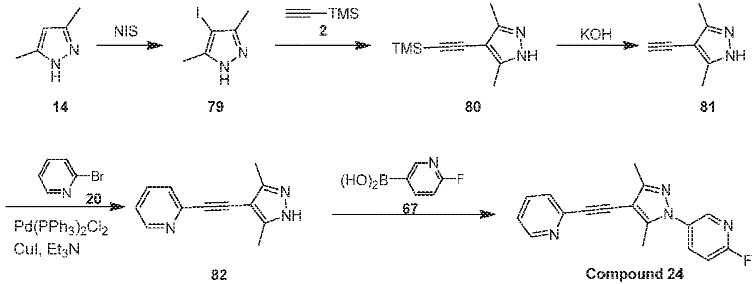

### Experimental section:

### Procedure for preparation of 79:

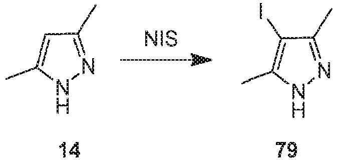

To a solution of **79** (3.00 g, 31.21 mmol) in 50 mL of degassed CHCl₃ was added NIS (7.02 g, 31.21mmol). The mixture was heated to reflux and stirred for 18 hours. LCMS showed that starting material was consumed, then the reaction mixture was cooled to room temperature and filtered. The filtrate was concentrated to give the product **79** (5.5 g, 79%).
**LCMS:** *m*/*z* 223 (M+H)⁺.

### Procedure for preparation of 80:

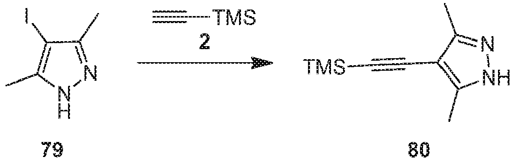

To a solution of **79** (3.00g, 13.51mmol) in 100 mL of degassed THF was added successively CuI (257mg, 1.35mmol), **2** (2.65g, 27.02mmol), Pd(PPh₃)₂Cl₂ (948mg, 1.35mmol) and Et₃N (4.10g, 40.54mmol). The mixture was stirred at 80 °C for 18 hours. LCMS showed that the reaction was completed. The reaction mixture was filtered and the filtrate was concentrated and purified by chromatograph column to give the product **80** (2.2 g, yield 85%).
**LCMS:** *m*/*z* 193 (M+H)⁺.

### Procedure for preparation of 81:

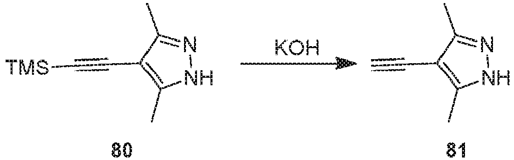

To a solution of **80** (2.20g, 11.44 mmol) in MeOH (30mL) was added KOH (1.28g, 22.88mmol). Then the reaction mixture was stirred at rt. for 1h. LCMS showed that the reaction was complete, then the reaction mixture was quenched with 50mL water, extracted with DCM (3 x 50mL). The combined organic layer was washed with brine, dried over Na₂SO₄, concentrated to give the 80 (1.1 g, yield 80%).

### Procedure for preparation of 82:

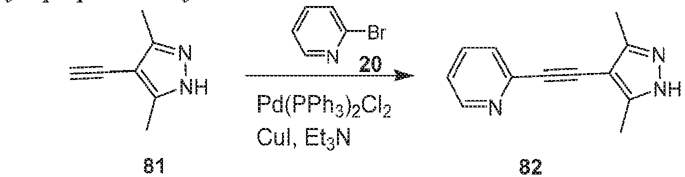

To a solution of **81** (1.00g, 8.32mmol) in 50 mL of degassed THF was added successively CuI (159mg, 0.83mmol), **20** (2.63g, 16.65mmol), Pd(PPh₃)₂Cl₂ (584mg, 0.83mmol) and Et₃N (2.53g, 24.97mmol). The mixture was stirred at 80 °C for 18 hours. LCMS showed that the reaction was completed, then the reaction mixture was filtered and the filtrate was concentrated and purified by prep-TLC to give the product **82** (1.1 g, yield 67%).
**LCMS:** *m*/*z* 198 (M+H)⁺.

### Procedure for preparation of Compound 24:

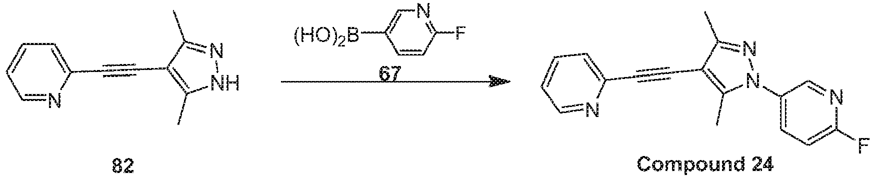

To a solution of **82** (100mg, 0.51 mmol) in 5 mL of degassed DCM was added successively 67 (142 mg, 1.01 mmol), Cu(OAc)₂ (184 mg, 1.01 mmol), and pyridine (120mg, 1.52mmol). The reaction mixture was then degassed for 1 minute under O₂ atmosphere and stirred at room temperature overnight. The reaction mixture was filtered and the filtrate was concentrated. The obtained residue was purified by prep-TLC give the product **Compound 24**(22 mg, yield 15%).
**LCMS:** *m*/*z* 293 (M+H)⁺;
**¹H NMR** (400 MHz, CDCl3) : δ 8.62 (d, *J*=4.4 Hz, 1H), 8.33 (s, 1H), 7.96-7.93 (m, 1H), 7.68-7.66 (m, 1H), 7.50 (d, *J*=8.0 Hz, 1H), 7.25-7.23 (m, 1H), 7.08-7.05 (m, 1H), 2.47 (s, 3H), 2.42 (s, 3H).

### Example Compound 25

### Preparation of 2-(3,5-dimethyl-4-(pyridin-2-ylethynyl)-1H-pyrazol-1-yl)--5-fluoropyridine:

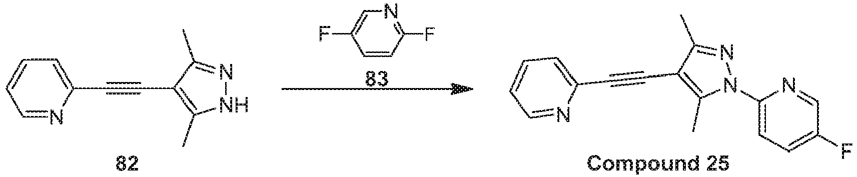

### Experimental section:

### Procedure for preparation of Compound 25:

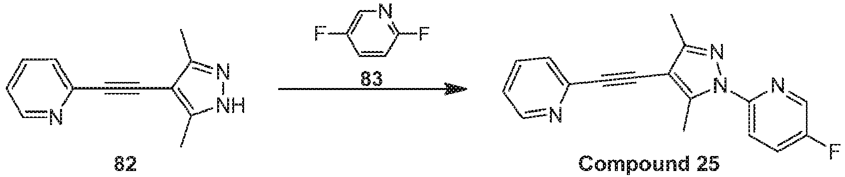

To a solution of **82** (50mg, 0.25 mmol) in 5 mL of degassed DMF was added successively **83** (44mg, 0.38mmol), Cs₂CO₃ (248mg, 0.76mmol). The reaction mixture was stirred at 110°C for 2 hours., then was filtered and the filtrate was concentrated and purified by prep-TLC to give the desired product **Compound 25** (14 mg, yield 19%).
**LCMS:** *m*/*z* 293 (M+H)⁺ ;
**¹H NMR** (400 MHz, CDCl3) : δ 8.61 (d, *J*=4.4 Hz, 1H), 8.28 (d, *J*=2.8 Hz, 1H), 7.89-7.85 (m, 1H), 7.67-7.64 (m, 1H), 7.53-7.49 (m, 2H), 7.24-7.22 (m, 1H), 2.76 (s, 3H), 2.42 (s, 3H) ;

### Example Compound 26

### Preparation of 2-((1-(4-(2-methoxyethoxy)phenyl)-1H-pyrazol-4-yl)ethynyl) pyridine:

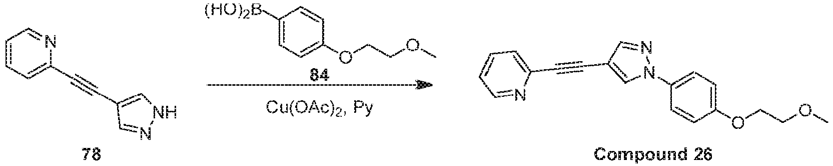

### Experimental section:

### Procedure for preparation of Compound 26:

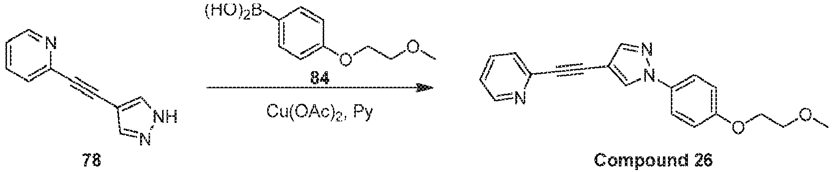

To a solution of **78** (50mg, 0.296mmol) in DCM (20mL) was added **84** (116mg, 0.591mmol), Cu(OAC)₂ (107mg, 0.591mmol), pyridine (70mg, 0.887mmol). The mixture was stirred at room temperature overnight under O₂ atmosphere. The mixture was filtered and concentrated under vacuo. The residue was purified by prep-TLC to give the desired product **Compound 26** (30mg, 32%).
**LCMS:** *m*/*z,* 320.2 (M+H)⁺;
**¹H NMR** (400 MHz, CDC13): δ 8.55 (s, 1H), 8.00 (s, 1H), 7.81 (s, 1H), 7.61 (m, 1H), 7.52-7.49 (m, 2H), 7.43-7.41 (m, 1H), 7.19-7.18 (m, 1H), 6.96-6.94 (m, 2H), 4.11-4.08 (m, 2H), 3.72-3.70 (m, 2H), 3.40 (s, 3H).

### Example Compound 27

### Preparation of 5-fluoro-2-(4-(pyridin-2-ylethynyl)-1H-pyrazol-1-yl)pyridine:

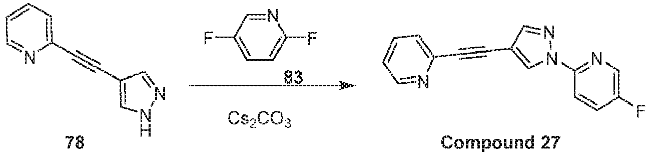

### Experimental section:

### Procedure for preparation of Compound 27:

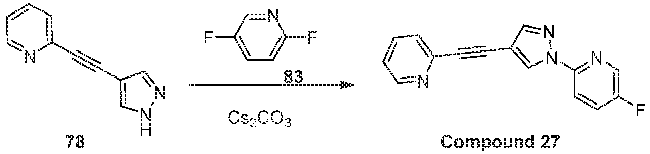

To a solution of 78 (50mg, 0.296mmol) in DMF (5mL) was added **83** (102mg, 0.887mmol), Cs₂CO₃ (289mg, 0.887mmol). The reaction mixture was stirred at 100 °C for 2 hours, then it was filtered and concentrated under vacuo. The residue was purified by prep-TLC to give the product Compound 27 (25mg, 32%).
**LCMS:** *m*/*z,* 265.1 (M+H)⁺;
**¹H NMR** (400 MHz, CDCl3): δ 8.70 (s, 1H), 8.62-8.61 (s, 1H), 8.27 (s, 1H), 7.99-7.97 (m, 1H), 7.89 (s, 1H), 7.68-7.66 (m, 1H), 7.56-7.54 (m, 1H), 7.51-7.49 (m, 1H), 7.24 (m, 1H)

### Example Compound 28

### Preparation of 2-(2-methoxyethoxy)-5-(4-(pyridin-2-ylethynyl)-1H-pyrazol-1-yl) pyridine:

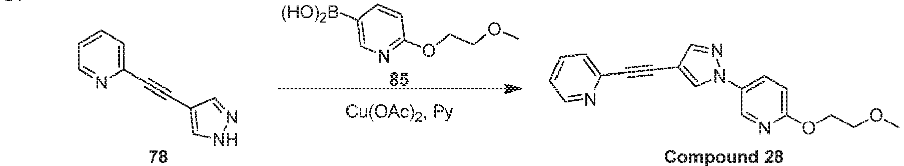

### Experimental section:

### Procedure for preparation of Compound 28:

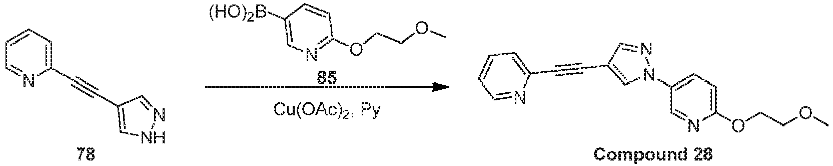

To a solution of **78** (40mg, 0.236mmol) in DCM (15mL) was added **85** (93mg, 0.473mmol), Cu(OAC)₂ (86mg, 0.473mmol), pyridine (56mg, 0.709mmol). The reaction mixture was stirred at room temperature overnight under O₂ atmosphere, then it was filtered and concentrated under vacuo. The residue was purified by prep-TLC to give the desired product **Compound 28** (15mg, 20%).
**LCMS:** *m*/*z,* 321.1 (M+H)⁺;
**¹H NMR** (400 MHz, CDCl3): δ 8.62 (s, 1H), 8.36 (s, 1H), 8.01 (s, 1H), 7.86-7.81 (m, 2H), 7.64 (m, 1H), 7.45-7.43 (m, 1H), 7.20-7.18 (m, 1H), 6.87-6.85 (m, 1H), 4.46-4.44 (m, 2H), 3.71-3.69 (m, 2H), 3.39-3.36 (m, 3H).

### Example Compound 29

### Preparation of 3-fluoro-5-(3-(2-methoxyethoxy)-5-methyl-4-(pyridin-2-ylethynyl)-1H-pyrazol-1-yl)benzonitrile:

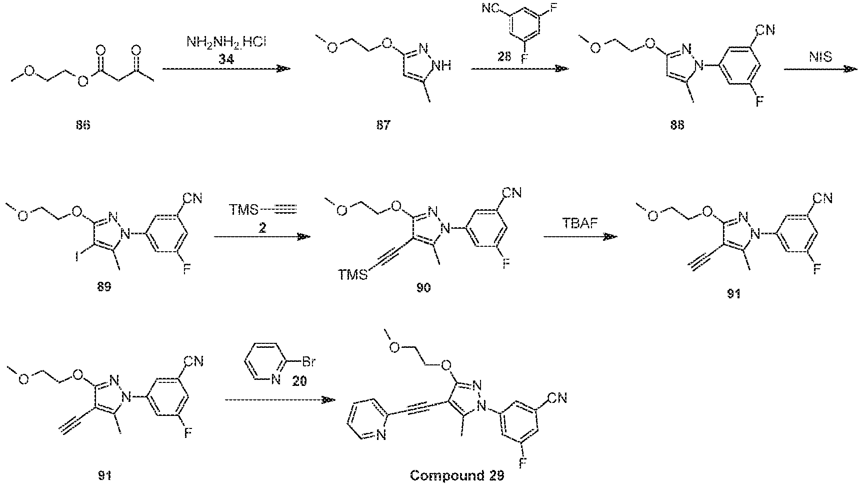

### Procedure for preparation of 87:

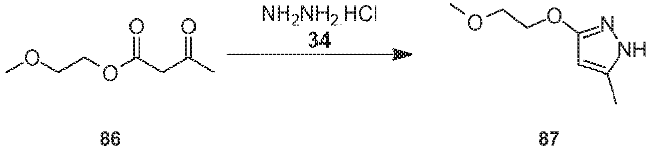

To a solution of **86** (3.2g, 20mmol) in EtOH (30mL) was added a solid of NH₂NH₂HCl (1.4 g, 20mmol) at room temperature. It was stirred at 60°C for 6 hours. The reaction mixture was concentrated and the obtained residue was treated with saturated NaHCO3 solution (200mL), extracted with DCM (3 x 150mL). The combined organic layer was dried over Na₂SO₄, filtered and concentrated to give the desired product **87** (1g, 32.3%)
**LCMS:** *m*/*z*, 157.1(M+H)⁺;
**¹H NMR** (400 MHz, DMSO-*d*₆): δ2.13 (s, 3 H), 3.26 - 3.30 (m, 3 H), 3.53 - 3.61 (m, 2 H), 4.05 - 4.14 (m, 2 H), 5.37 - 5.44 (m, 1 H).

### Procedure for preparation of 88:

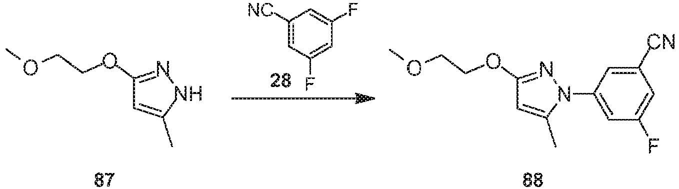

To a solution of **87**(156mg, 1mmol), **28**(224mg, 1.1mmol) in DMF (3mL) was added solid Cs₂CO₃ (664mg, 2mmol) at room temperature. Then it was stirred at 100°C for 3 hours, after cooling to room temperature, water (6mL) was added into the mixture with stirring at ice bath slowly. Gradually, solid was formed, it was filtrated. The residue was purified by TLC to afford the product **88** (80 mg, 29%).
**¹H NMR** (400 MHz, CDCl₃): δ2.35 - 2.49 (m, 3 H) 3.37 - 3.50 (m, 3 H) 3.67 - 3.80 (m, 2 H) 4.24 - 4.43 (m, 2 H) 5.76 (s, 1 H) 7.20 - 7.24 (m, 1 H) 7.48 (dd, *J*₁= 2.09 Hz, *J*₂=9.70 Hz, 1 H) 7.58 (s, 1 H).

### Procedure for preparation of 89:

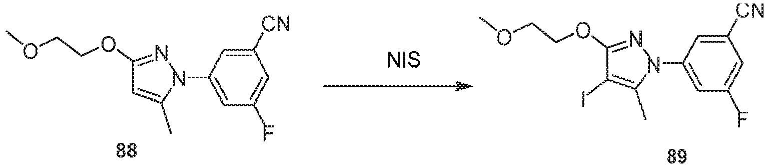

To a solution of **88** (250 mg, 0.91mmol) in CHCl₃ (3mL) was added NIS (225 mg, 1mmol). Then the reaction mixture was stirred at reflux for 2 hours. LCMS showed that the reaction was complete. The reaction mixture was concentrated to give the desired product, which was purified by TLC to afford the product **89** (300 mg, 82.4%).
**LCMS:** *m*/*z,* 402.0 (M+H)⁺.

### Procedure for preparation of 90:

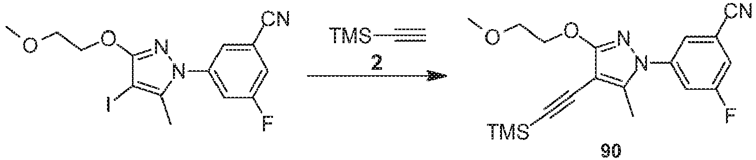

To a solution of **89** (350mg, 0.87mmol) in 4mL of degassed THF was added successively CuI (8mg, 0.04mmol), **2** (171mg, 1.7mmol), Et₃N (263mg, 2.6mmol) and Pd(PPh₃)₂Cl₂ (28mg, 0.04mmol). The mixture was then degassed for 2 minutes under N₂ atmosphere and stirred at 90°C for 1 hour under microwave. Most of the solvent was removed. The residue was dissolved in EtOAc (30mL). The organic layer was washed with brine (2×20mL) and dried over anhydrous Na₂SO₄, filtered and evaporated to give a residue which was purified by TLC to give product **90** (300mg,92.5%)
**LCMS:** *m*/*z,* 372.1 (M+H)⁺.

### Procedure for preparation of 91:

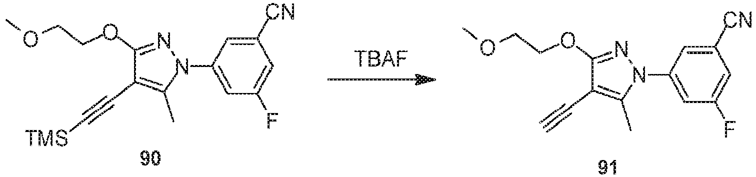

To a solution of **90** (300mg, 0.81mmol) in THF (4mL) was added a solution of TBAF-THF (1.2mL, 1.2mmol). The mixture was stirred at room temperature for 1 hour. Most of the solvent was removed. The residue was dissolved in EtOAc (20mL). The organic layer was washed with brine (2×10mL) and dried over anhydrous Na₂SO₄, filtered and evaporated to dryness on a rotary evaporator, which was purified by TLC to give product **91**(200mg, 82.6%).
**LCMS:** *m*/*z,* 300.0(M+H)⁺;
**¹H NMR** (400 MHz, CDCl₃): δ2.40 (s, 3 H), 3.21 (s, 1 H), 3.38 (s, 3 H), 3.66 - 3.75 (m, 2 H), 4.33 - 4.41 (m, 2 H), 7.23 (d, *J*=7.50 Hz, 1 H), 7.37 - 7.45 (m, 1 H), 7.52 (s, 1 H).

### Procedure for preparation of Compound 29:

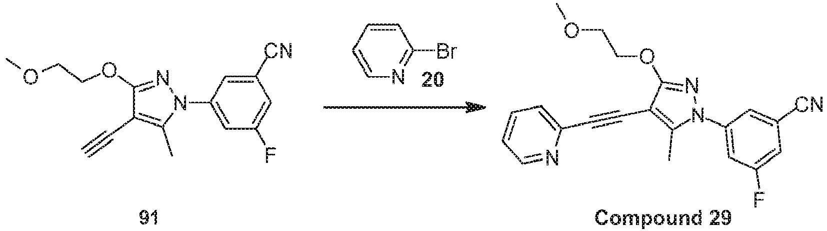

To a solution of **91** (50mg, 0.17mmol) in 3mL of degassed THF was added successively CuI (1.8 mg, 0.009mmol), **20** (29mg, 0.18mmol), Et₃N (34mg, 0.34mmol) and Pd(PPh₃)₂Cl₂ (0.7mg, 0.009mmol). The mixture was then degassed for 2 minutes under N₂ atmosphere and stirred at 90°C for 1h under microwave. Most of the solvent was removed. The residue was dissolved in EtOAc (30mL). The organic layer was washed with brine (2 × 20mL) and dried over anhydrous Na₂SO₄, filtered and evaporated to give the residue which was purified by flash chromatography to give the desired product **Compound 29** (25 mg, 33.3%).
**LCMS:** *m*/*z,* 377.1(M+H)⁺ ;
**¹H NMR** (400 MHz, CDCl₃): δ2.55 (s, 3 H), 3.45 (s, 3 H), 3.79 (t, *J*=4.80 Hz, 2 H), 4.46 (t, *J*=4.80 Hz, 2 H), 7.21 (m, 1 H), 7.29 (m, 1 H), 7.50(m, 2 H), 7 .66 (m, 2 H), 8.76 (d, *J*=4.40Hz, 1 H).

### Example Compound 30

### Preparation of 3-fluoro-5-(3-(2-methoxyethoxy)-5-methyl-4-(pyrimidin-2-ylethynyl) -1H-pyrazol-1-yl)benzonitrile:

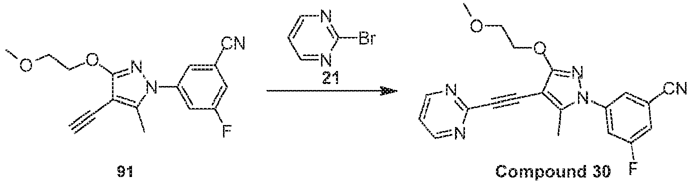

### Procedure for preparation of Compound 30:

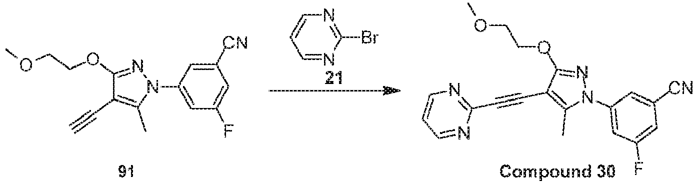

To a solution of **91** (40mg, 0.2mmol) in 3mL of degassed dioxane was added successively CuI (1 mg, 0.01mmol), 9 (32mg, 0.3mmol), Et₃N (42mg, 0.4mmol) and Pd(PPh₃)₂Cl₂ (30mg, 0.01mmol). The mixture was then degassed for 2 minutes under N₂ atmosphere and heated to 120°C overnight. Most of the solvent was removed. The residue was dissolved in EtOAc (30mL). The organic layer was washed with brine (2×20mL) and dried over anhydrous Na₂SO₄, filtered and evaporated to give a residue which was purified by prep-HPLC to give the desired product **Compound 30** (25 mg, 66.7 %).
**LCMS:** *m*/*z*, 378. 1(M+H)⁺ ;
**¹H NMR** (400 MHz, CDCl₃): δ2.57 (s, 3 H), 3.44 (s, 3 H), 3.78 (t, *J*=4.80 Hz, 2 H), 4.44 (t, *J*=4.80 Hz, 2 H), 7.20 (m, 1 H), 7.23 (d, *J*=12.0Hz, 1H), 7.50(d, *J*=9.6Hz, 1H), 7 .61 (s, 1H), 8.73 (d, *J*=5.20Hz, 2H).

### Example Compound 31

### Preparation of 2-((3-(2-methoxyethoxy)-1-(4-(trifluoromethoxy)phenyl)-1H-pyrazol-4-yl)ethynyl)pyridine:

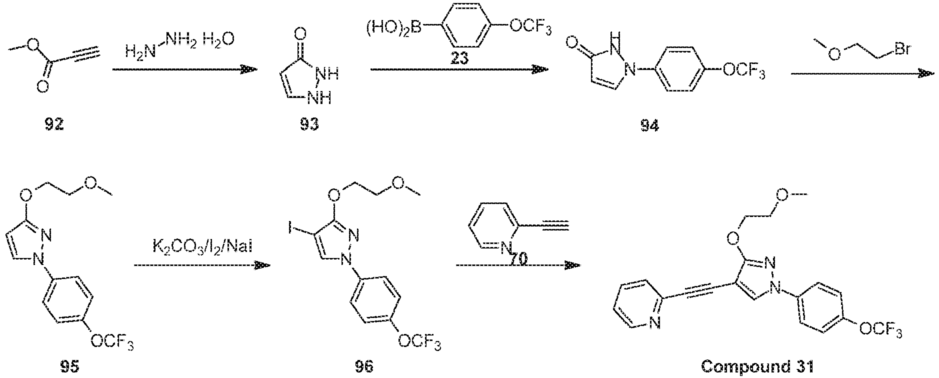

### Experimental section:

### Procedure for preparation of 93:

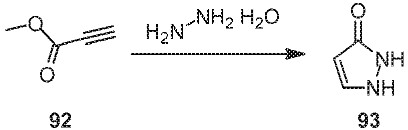

The compound of hydrazine monohydrate (85%, 4.81g, 95.2mmol) was added drop-wise to a cooled (ice bath) solution of **92** (8g, 96.2mmol) in methanol (50mL). The reaction was allowed to stir for 30 minutes at room temperature, then, the solvent was removed under vacuo. The remaining aqueous layer was extracted with EtOAc(75mL×5) and the combined organic layers were dried (MgSO₄), filtered and concentrated in vacuo to yield the title product **93** (5g, yield: 62%)
**¹HNMR** (400 MHz, DMSO-d₆): δ5.40 (s, 2H), 7.32 (s, 2 H).

### Procedure for preparation of 94:

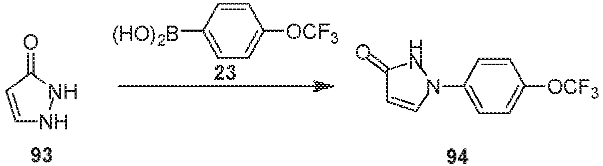

To a solution of **93** (1g, 11.9mmol) and **23**(3.67g, 17.8mmol) in DCM(20ml) was added Cu(OAc)₂ (4.32g, 23.8mmol) and pyridine(2.82g, 35.7mmol) at room temperature under O₂, The mixture was stirred at room temperature overnight, The suspension was filtered through a pad of Celite and filter cake was washed with DCM(30ml). The combined organic layers were washed with water and dried over Na₂SO₄. The crude product was purified by silica gel chromatography to give product **94** (50mg, yield: 1.7%).
**LCMS:** *m*/*z,* 245.1(M+H)⁺

### Procedure for preparation of 95:

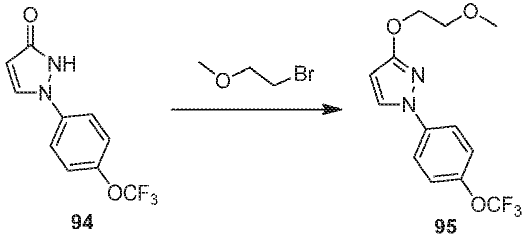

To a solution of **94** (250mg, 1.02mmol) and 1-bromo-2-methoxyethane (284mg, 2.05mmol) in DMF (15ml) was added NaI(183mg, 1.02mmol) and K₂CO₃(424mg, 3.07mmol). The mixture was heated at 60 °C overnight. The mixture was extracted with EtOAc(75mL×2) and the combined organic layers were dried (MgSO₄), filtered and concentrated in vacuo, which was purified by silica gel chromatography to give product **95** (200mg,yield: 64%).
**LCMS:** *m*/*z,* 303. 1(M+H)⁺.

### Procedure for preparation of 96:

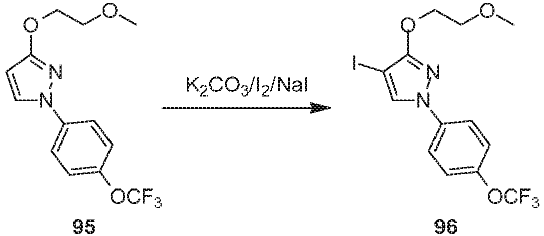

To a solution of **95** (20mg, 66.2ummol) in CHCl₃ (5ml) was added NIS(22mg, 99.3umol). the mixture was heated at 60°C for 1 hour, then, The reaction mixture was concentrated to dryness, which was purified by prep-TLC to afford the title product **96** (20mg, yield: 70%).
**¹HNMR** (400 MHz, CDCl₃): 83.43(s, 3H), 3.71 ∼7.73 (m, 2H), 4.38∼4.41 (m, 2H), 7.17∼7.21 (m, 2H), 7.49∼7.52 (m, 2H), 7.67(s, 1H).

### Procedure for preparation of Compound 31:

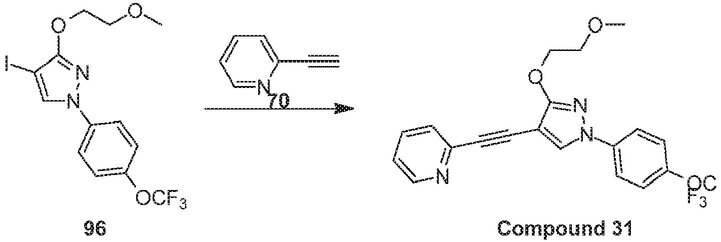

To a solution of **96** (100mg, 0.23mmol), **70** (50mg, 0.47mol), CuI (5mg, 0.023mol), Et₃N (70mg, 0.39mol) in THF (5mL) was added Pd(PPh₃)₂Cl₂ (15mg, 0.0023mol). The suspension was degassed under vacuo and purged with N₂ several times. The mixture was stirred at 90°C for 6 hours under N₂ atmosphere. The mixture was filtered and concentrated under vacuo. The residue was purified by prep-TLC to give desired product **Compound 31** (20mg, yield: 20%).
**LCMS:** *m*/*z,* 404.1 (M+H)⁺;
**¹HNMR** (400 MHz, CDCl₃): δ 3.46(s, 3H), 3.61-3.63 (m, 2 H), 4.44-4.49(m, 2 H), 7.15-7.26 (m, 3H), 7.45-7.51(m, 1H), 7.57-7.621(m, 3H), 7.82 (s, 1H), 8.57(s, 1H).

### Example Compound 32

### Preparation of 3-fluoro-5-(3-(2-methoxyethoxy)-4-(pyridin-2-ylethynyl)-1H-pyrazol-1-yl)benzonitrile:

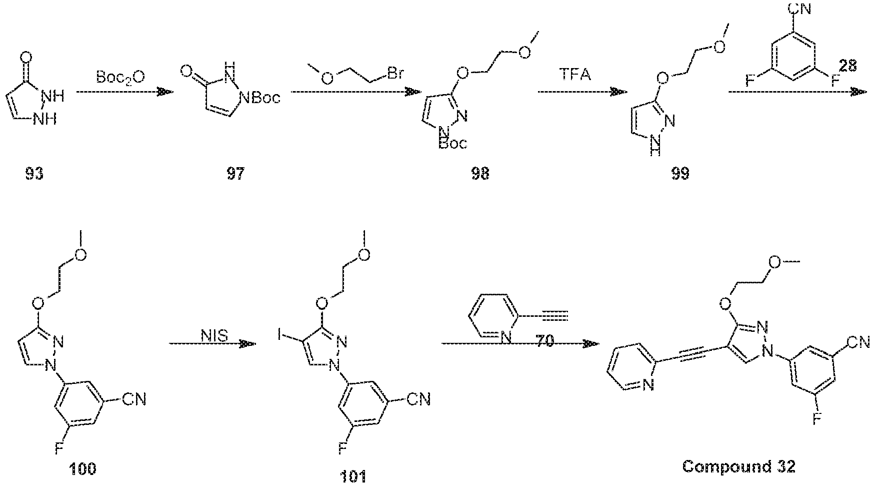

### Experimental section:

### Procedure for preparation of 85:

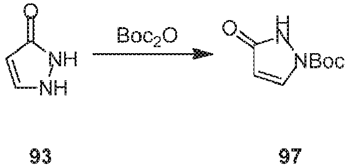

To a solution of **93**(2g, 23.8mmol) in DCM (20ml) was added Boc₂O (5.71g, 26.2mmol) and Et₃N (2.65g, 26.2mmol). The mixture was stirred at room temperature for 6 hours. The mixture was extracted with EtOAc (100mL×2) and the combined organic layers were dried (MgSO₄), filtered and concentrated in vacuo to yield the title product **97** (4g, yield: 91%)
**¹HNMR** (400 MHz, CDCl₃): δ1.54(s, 9H), 5.71 (s, 2 H), 7.46(s, 1H), 7.73(s, 1 H).

### Procedure for preparation of 98:

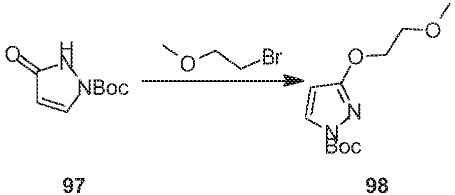

To a solution of **97** (2g, 11mmol) and 1-bromo-2-methoxyethane (3g, 22mmol) in DMF (25ml) was added NaI (1.6g, 11mmol) and K₂CO₃ (4.5mg, 32.6mmol). the mixture was heated at 60°C overnight, then the mixture was cool to room temperature and extracted with EtOAc (75mL) The combined organic layers were dried (MgSO4), filtered and concentrated in vacuo to yield the title product **98** (1.5g, yield: 57%)
**LCMS:** *m*/*z,* 242.3(M+H)⁺

### Procedure for preparation of 99:

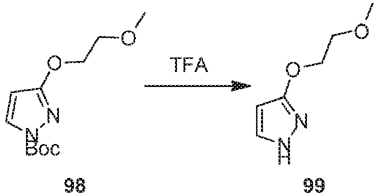

To a solution of **98** (2g, 11mmol) in DCM (15ml) was added TFA (5ml), the mixture was stirred at room temperature for 3hours, the solvent was removed under vacuo. The remaining residue was extracted with EtOAc (100mL×3) and the combined organic layers were dried (MgSO₄), filtered and concentrated in vacuo to yield the title product **99** (1.1g, yield: 93%)

### Procedure for preparation of 100:

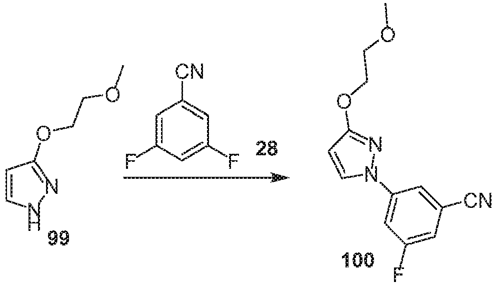

To a solution of **99** (170mg, 1.2mmol) and **28** (166mg, 1.2mmol) in DMF (5ml) was added Cs₂CO₃ (467mg, 1.44mmol). The mixture was heated at 60 °C overnight, the mixture was extracted with EtOAc (50mL×2) and the combined organic layers were dried (MgSO₄), filtered and concentrated in vacuo to afford the crude product, which was purified by silica gel chromatography to afford the title product **100** (250mg, yield: 80%).
**¹HNMR** (400 MHz, CDCl₃): δ 3.45(s, 3H), 3.72∼3.75(m, 2 H), 4.59∼4.62 (m, 2 H), 6.09(s, 1H), 7.15 - 7.24 (m, 1 H), 7.59~7.64(m, 1H), 7.59∼7.66 (m, 2H).

### Procedure for preparation of 101:

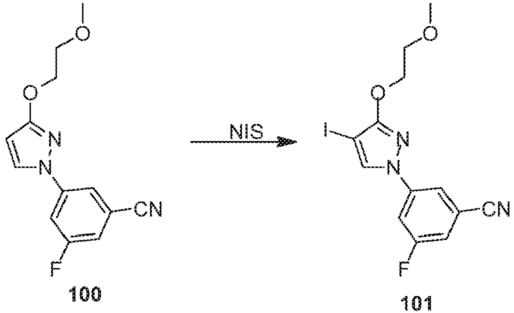

To a solution of **100** (250mg, 0.96mmol) in CHCl₃ (15ml) was added NIS (323mg, 1.44mmol). The mixture was heated at 60°C for 3 hours, then was extracted with EtOAc (50mL) and the combined organic layers were dried (MgSO₄), filtered and concentrated in vacuo, which was purified by silica gel chromatography to give product **101** (300mg, yield: 81%).
**¹HNMR** (400 MHz, CDCl₃): δ 3.46(s, 3H), 3.71~3.78(m, 2 H), 4.47~4. 53 (m, 2 H), 7.1∼7.22 (m, 1 H), 7.55~7,63(m, 1H), 7.64(s, 1H), 7.77 (s, 2H).

### Procedure for preparation of Compound 32:

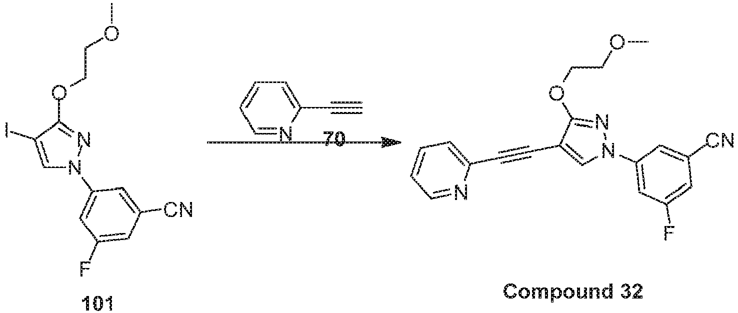

To a solution of **101**(300mg, 0.77mmol), **70**(160mg, 1.55mmol), CuI (15mg, 0.077mmol), Et₃N (235mg, 2.32mmol) in THF (5mL) was added Pd(PPh₃)₂Cl₂ (53mg, 0.077mmol). The suspension was degassed under vacuum and purged with N₂ several times. The mixture was stirred at 90°C for 6 hours under N₂ atmosphere. The mixture was filtered and concentrated under vacuo. The residue was purified by prep-TLC to give the product **Compound 32**(30mg, yield: 10%).
**LCMS:** *m*/*z,* 363.1(M⁺H)⁺;
**¹HNMR** (400 MHz, CDCl₃): δ 3.47(s, 3H), 3.81-3.84 (m, 2 H), 4.46-4.54(m, 2 H), 7.15-7.21 (m, 2 H), 7.53-7.57(m, 2H), 7.65-7.71 (m, 2H), 7.98 (s, 1H), 8.65(s, 1H).

### Example Compound 33

### Preparation of 3-fluoro-5-(4-(pyridin-2-ylethynyl)-1H-pyrazol-1-yl)pyridine:

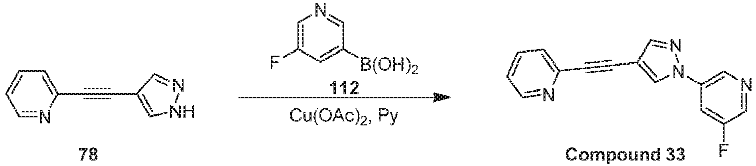

### Experimental section:

### Procedure for preparation of Compound 33:

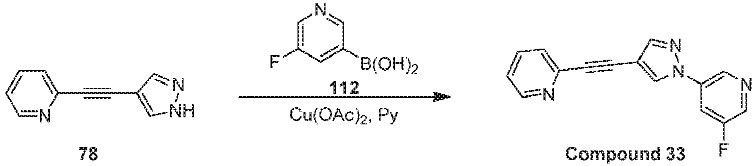

To a solution of **78** (50mg, 0.296mmol) in DCM (8mL) was added **112** (83mg, 0.591mmol), Cu(OAc)₂ (107mg, 0.591mmol), pyridine (70mg, 0.887mmol). The mixture was stirred at room temperature overnight under O₂ atmosphere. The mixture was filtered and concentrated under vacuo. The residue was purified by prep-TLC to give product **Compound 33**(6mg, 8%).
**LCMS:** *m*/*z,* 265.1 (M+H)⁺;
**¹H NMR** (400 MHz, CDCl3): δ 8,81 (s, 1H), 8.64-8.62 (m, 1H), 8.46 (s, 1H), 8.22 (s, 1H), 7.97 (s, 1H), 7.89-7.86 (m, 1H), 7.71-7.69 (m, 1H), 7.52-7.50 (m, 1H), 7.27-7.26 (m, 1H).

### Example Compound 34

### Preparation of 2-((1-(6-fluoropyridin-3-yl)-1H-pyrazol-4-yl)ethynyl)pyrimidine:

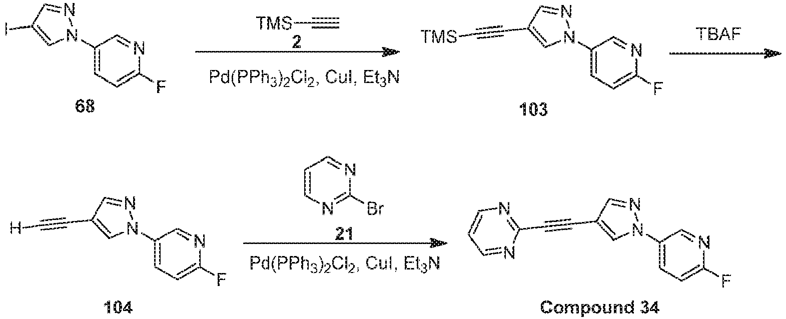

### Experimental section:

### Procedure for preparation of 103:

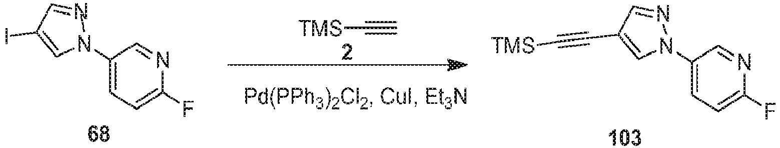

To a solution of **68** (400mg, 1.38mmol) in CH₃CN (20mL) was added successively CuI (26mg, 0.138mmol), **2** (272mg, 2.77mmol), Et₃N (420mg, 4.15mmol) and Pd(PPh₃)₂Cl₂ (49mg, 0.069mmol). The mixture was then degassed for 1 minute under N₂ atmosphere and stirred at 80°C overnight. The reaction mixture was filtered and concentrated to give the crude product which was purified by silica gel chromatography to give product **103** (300mg, 84%).
**LCMS:** *m*/*z,* 260.1 (M+H)⁺.

### Procedure for preparation of 104:

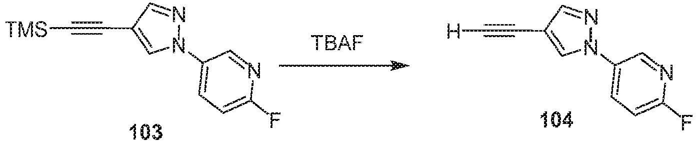

To a solution of compoud **103** (300mg, 1. 1 6mmol) in THF (20mL) was added TBAF (1.74mL, 1.74mmol) dropwise. The mixture was stirred at room temperature for 2 hours. The reaction mixture was quenched with water and extracted with EtOAc (3x20mL). The combined organics was concentrated by vacuo to give product **104** (200mg, crude).
**LCMS:** *m*/*z,* 188.2 (M+H)⁺.

### Procedure for preparation of Compound 34:

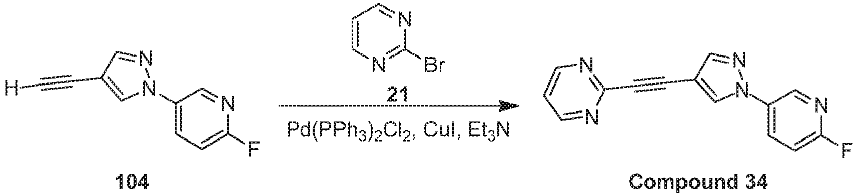

To a solution of **104** (80mg, 0.427mmol) in THF (3mL) was added successively CuI (8mg, O.043mmol), **21** (136mg, 0.854mmol), Et₃N (130mg, 1.28mmol) and Pd(PPh3)₂Cl₂ (15mg, 0.021mmol). The mixture was then degassed for 1 minute under N₂ atmosphere and stirred at 90°C for 1h under microwave. The reaction mixture was filtered and concentrated to give the crude product which was purified by prep-TLC to give product **Compound 34** (20mg, 18%).
**LCMS:** *m*/*z*, 266.0 (M+H)⁺;
**¹H NMR** (400 MHz, CDCl₃): δ 8.77 (s, 1H), 8.56 (s, 1H), 8.21-8.15 (m, 2H), 8.01 (s, 1H), 7.26-7.24 (m, 1H), 7.11-7.08 (m, 1H).

### Example Compound 35

### Preparation of ((5-chloro-10-(4-(trifluoromethyl)phenyl)-1H-pyrazol-4-yl) ethynyl)pyridine:

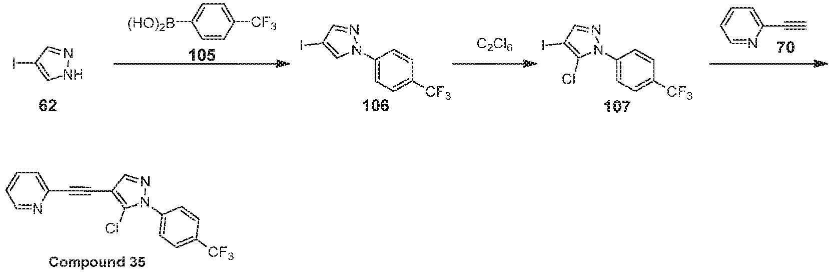

### Experimental section:

### Procedure for preparation of 106:

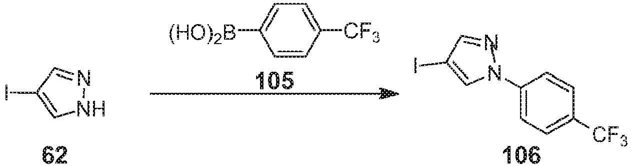

To a solution of **62** (1.3 g, 6.5mmol), **105** (1.5g, 7.8mmol) and Cu(AcO)₂ (2.4g, 13mmol) in 50mL of degassed DCM was added successively Pyridine (1.5g, 19.4mmol) at room temperature. The mixture was stirred at room temperature for 12 hours. It was filtrated and most of the solvent was removed. The residue was dissolved in EtOAc (60mL). The organic layer was washed with brine (2×30mL) and dried over anhydrous Na₂SO₄, filtered and evaporated to dryness on a rotary evaporator, which was purified by chromatography to give of the desired product **106**(1g, 43.5%).
**¹H NMR** (400 MHz, CDCl₃): δ7.77-7.80 (m, 5 H), 8.03 (s, 1H)

### Procedure for preparation of 107:

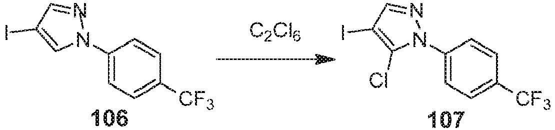

To a solution of **106** (100mg, 0.3mmol) in 10mL of degassed THF was added LDA (0.21mL, 0.42mmol) at -78 °C. After stirring 30 minutes, perchloroethane (98mg, 0.42mmol) was added. It was stirred at the same condition for another 30 minutes. Then the reaction mixture was quenched with sat.NH₄Cl. The solvent was removed, the residue was extracted with DCM and water, the organic layer was dried and purified by prep-TLC to give title product **107** (50mg, 45%).
**¹H NMR** (400 MHz, CDCl₃): 7.64-7.77(m, 5 H).

### Procedure for preparation of Compound 35:

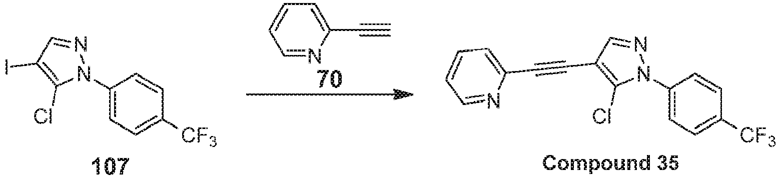

To a solution of **107** (50mg, 0.131mmol) in 4mL of degassed THF was added solid CuI (1.3mg, 0.007mmol), **70** (21mg, 0.20mmol), Et₃N (39mg, 0.39mmol) and Pd(PPh₃)₂Cl₂ (5mg, 0.007mmol). The mixture was then degassed for 2 minutes under N₂ atmosphere and stirred at 90°C for 1h under microwave. Most of the solvent was removed. The residue was dissolved in EtOAc (30mL). The organic layer was washed with brine (2×20mL) and dried over anhydrous Na₂SO₄, filtered and evaporated to give the residue which was purified by prep-HPLC to afford the product **Compound 35** (5mg, 11%).
**LCMS:** *m*/*z,* 348.0(M+H)⁺,
**¹H NMR** (400 MHz, CDCl₃): δ7.30 (m, 1 H), 7.56(m, 1 H), 7.70(m, 1 H), 7.79 (s, 4H), 7.94 (s, 1H), 8.65 (s, 1H).

### Example Compound 36

### Preparation of 2-((5-chloro-1-(4-(trifluoromethyl)phenyl)-1H-pyrazol-4-yl)ethynyl) pyrimidine:

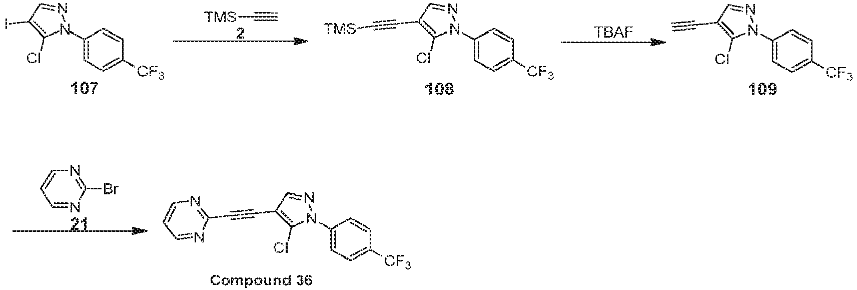

### Experimental section:

### Procedure for preparation of 108:

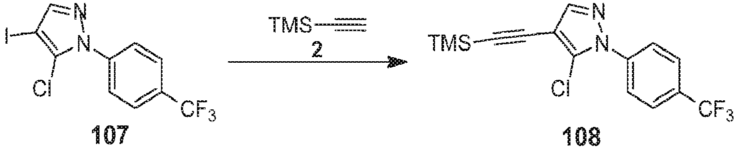

To a solution of **107** (80mg, 0.2mmol) in 4mL of degassed THF was added successively CuI (1mg, 0.005mmol), **2** (171mg, 0.2mmol), Et₃N (30mg, 0.3mmol) and Pd(PPh₃)₂Cl₂ (3mg, 0.005mmol). The mixture was then degassed for 2 minutes under N₂ atmosphere and stirred at 90°C for 1 hour under microwave. Then most of the solvent was removed. The residue was dissolved in EtOAc (30mL). The organic layer was washed with brine (2×20mL) and dried over anhydrous Na₂SO₄, filtered and evaporated to dryness on a rotary evaporator, which was purified by TLC plate to give the product 108 (70mg,95.9%)
LCMS: *m*/*z,* 343.1 (M+H)⁺.

### Procedure for preparation of 109:

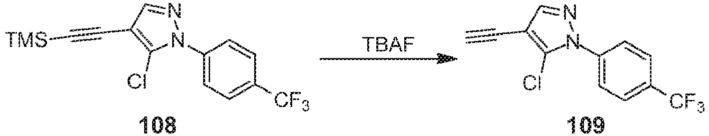

To a solution of **108** (70mg, 0.2mmol) in THF (4mL) was added a solution of TBAF-THF (1.2mL, 0.3mmol). The mixture was stirred at room temperature for 1 hour. TLC showed the reaction was complete, then most of the solvent was removed. The residue was dissolved in EtOAc(20mL). The organic layer was washed with brine (2×10mL) and dried over anhydrous Na₂SO₄, filtered and evaporated to give the product **109** (50mg,90.4%), which was directly used for next step
LCMS: m/z, 271.3(M+H)⁺.

### Procedure for preparation of Compound 36:

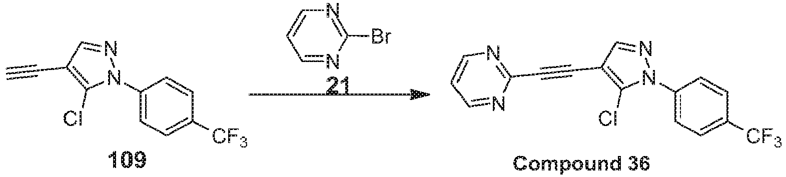

To a solution of **109** (50mg, 0.19mmol) in 4mL of degassed THF was added solid CuI (2mg, 0.01mmol), **21** (35mg, 0.22mmol), Et₃N (58mg, 0.57mmol) and Pd(PPh₃)₂Cl₂(7mg, 0.01mmol). The mixture was then degassed for 2 minutes under N₂ atmosphere and stirred at 90°C for 1 hour under microwave. Then most of the solvent was removed. The residue was dissolved in EtOAc (30mL). The organic layer was washed with brine (2×20mL) and dried over anhydrous Na₂SO₄, filtered and evaporated to give a residue which was purified by prep-HPLC to give of the product **Compound 36** (17mg, 26.6%).
**LCMS:** *m*/*z,* 349.0(M+H)⁺
**¹H NMR** (400 MHz, CDCl₃): δ7.27 (m, 1 H), 7.79 (s, 4H), 7.98 (s, 1H), 8.78 (d, *J*=4.8Hz, 2H).

### Example Compound 37

### Preparation of 3-(3-(difluoromethoxy)-5-methyl-4-(pyridin-2-ylethynyl)-1H-pyrazol-1-yl)-5-fluorobenzonitrile:

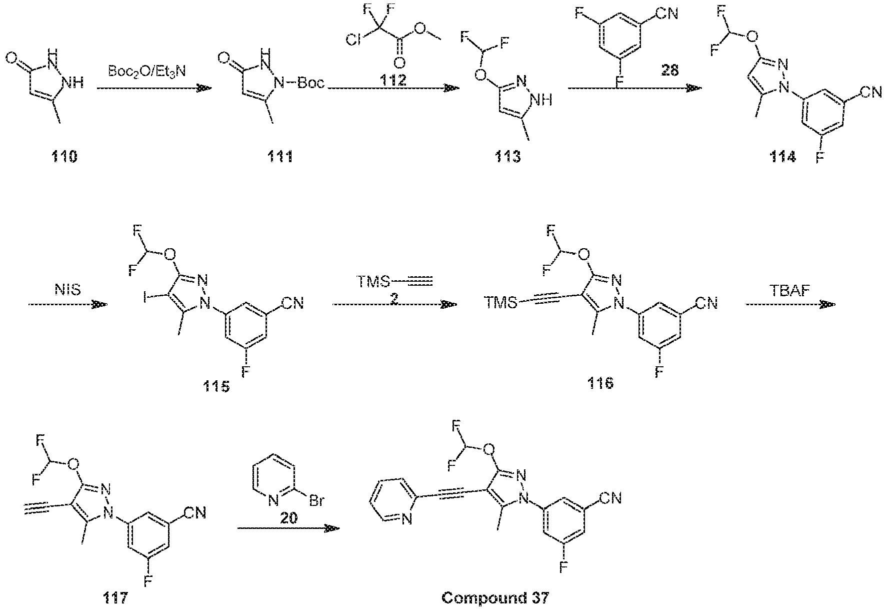

### Experimental section:

### Procedure for preparation of 111:

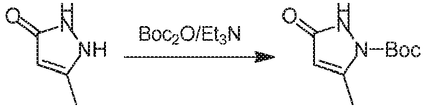

A solution of **110** (981mg, 10mmol) in DCM (20mL) was added Et₃N (1113mg, 11mmol) and Boc₂O (2180mg, 10mmol). The mixture was stirred at room temperature overnight. The reaction mixture was diluted with H₂O (30mL). The separated organic layer was dried over anhydrous Na₂SO4, filtered and concentrated to give product **111** (800mg, 40%).
**LCMS:** *m*/*z,* 143.1(M-57)⁺.

### Procedure for preparation of 123:

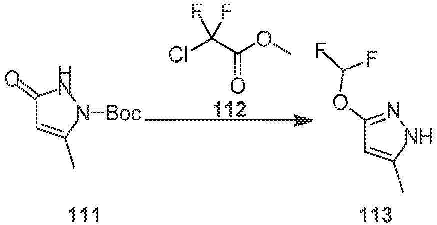

A solution of **111** (750mg, 3.79mmol) in DMF (20mL) was added **112** (547mg, 3.79mmol) and Cs₂CO₃ (1480mg, 4.54mmol). The mixture was stirred at 100 °C for 6 hours. After cooling, the mixture was diluted with H₂O (60mL), and extracted with EtOAc (15mL). The organic layer was dried over anhydrous Na₂SO₄, filtered and concentrated to give product **113** (350mg, 60%).
**LCMS:** *m*/*z,* 149.1(M+H)⁺.

### Procedure for preparation of 114:

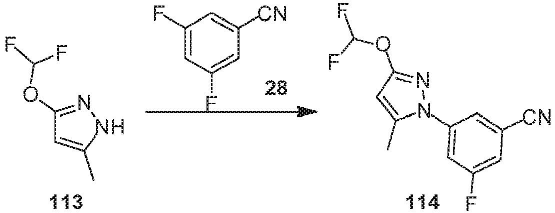

A solution of **113** (320mg, 2.16mmol) in DMF (15mL) was added **28** (300mg, 2.16mmol) and Cs₂CO₃ (845mg, 2.59mmol). The mixture was stirred at 100°C for 4 hours. After cooling, the reaction mixture was diluted with H₂O (30mL) and extracted with EtOAc (15mL). The separated organic layer was dried over anhydrous Na₂SO₄, filtered and concentrated to residue which was purified by prep-TLC to give product **114.**

### Procedure for preparation of 115:

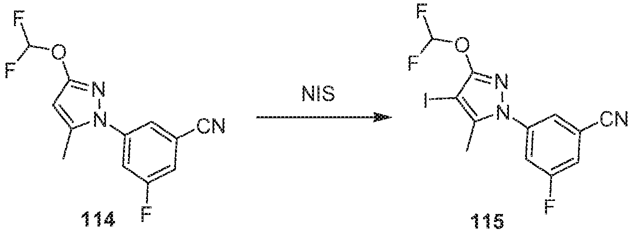

To a solution **of 114** (120mg, 0.449mmol) in CHCl₃ (15mL) was added NIS (123mg, 0.539mmol). The mixture was stirred at 60°C for 6 hours. The mixture was quenched with water and extracted with DCM (2×10mL). The combined organics was concentrated under vacuo and the residue was purified by prep-TLC to give product **115** (120mg, 68%).

### Procedure for preparation of 6:

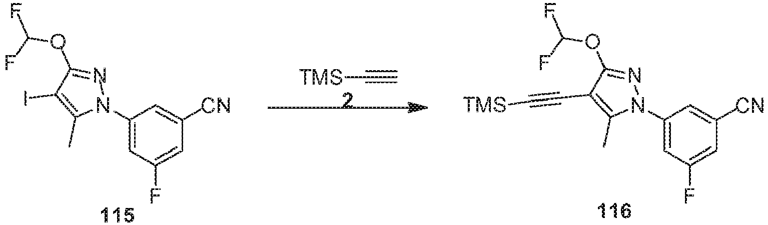

To a solution of **115** (120mg, 0.305mmol) in CH₃CN (3mL) was added successively CuI (6mg, 0.031mmol), **2** (60mg, 0.611mmol), Et₃N (93mg, 0.916mmol) and Pd(PPh₃)₂Cl₂ (11mg, 0.015mmol). The mixture was then degassed for 1 minute under N₂ atmosphere and was heated at 90°C under microwave for 1 hour. The reaction mixture was filtered and evaporated to give crude product, which was purified by prep-TLC to give product **116** (100mg, 90%).
**LCMS:** *m*/*z,* 364.1 (M+H)⁺.

### Procedure for preparation of 117:

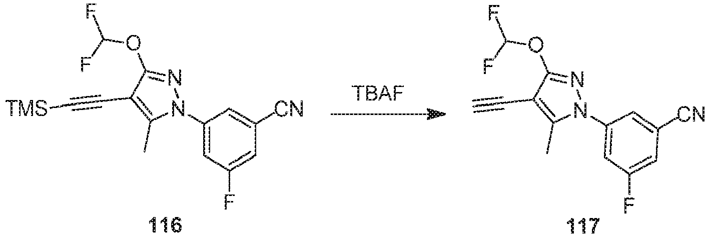

A solution of **116** (100mg, 0.275mmol) in THF (5mL). The solution was cooled to 0°C and TBAF (0.413mL, 0.413mmol) was added. The reaction mixture stirred at room temperature for 2 hours. The mixture was quenched with water and extracted with EA (3x10mL). The combined organics was concentrated by vacuo to give the crude product **117** (80mg, crude).

### Procedure for preparation of Compound 37:

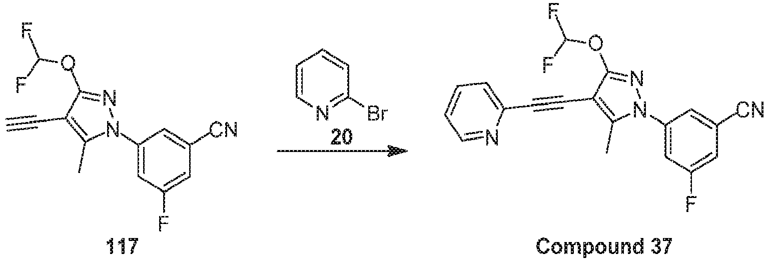

To a solution of **117** (80mg, 0.275mmol) in THF (3mL) was added successively CuI (5mg, 0.027mmol), **20** (87mg, 0.549mmol), Et₃N (83mg, 0.824mmol) and Pd(PPh₃)₂Cl₂ (10mg, 0.014mmol). The mixture was then degassed for 1 minute under N₂ atmosphere and stirred at 90°C for 1 hour under microwave. The reaction mixture was filtered and concentrated to give the crude product which was purified by prep-HPLC to give product **Compound 37** (20mg, 18%).
**LCMS:** *m*/*z,* 369.1 (M+H)⁺;
**¹H NMR** (400 MHz, CDCl3): δ 8.63-8.62 (m, 1H), 7.71-7.69 (m, 1H), 7.61 (s, 1H), 7.55-7.50 (m, 2H), 7.48-7.46 (m, 1H), 7.27-7.25 (m, 1H), 7.08-6.90 (m, 1H), 2.57 (s, 3H).

### Example Compound 38

### Preparation of 3-(3-chloro-4-(pyridin-2-ylethynyl)-1H-pyrazol-1-yl)-5-fluorobenzonitrile:

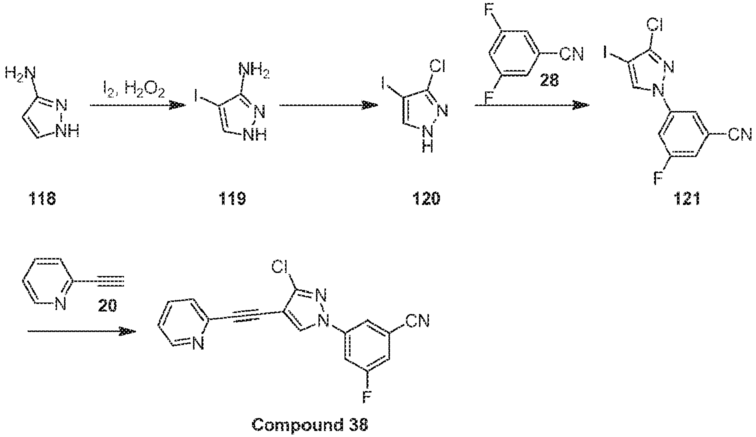

### Experimental section:

### Procedure for preparation of 119:

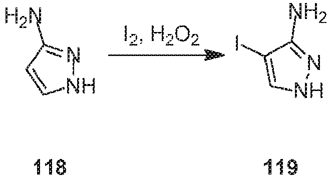

To a solution of **118** (1.0g, 12.0mmol) in 10mL of H₂O was added 30% of H₂O₂ (0.82g, 7.2mmol) and I₂ (1.5g, 6.0mmol), the mixture was stirred at room temperature for 1 hour. The mixture was treated with 20mL of saturated Na₂S₂O₃ solution and filtered. The filter cake was washed by water and dried in air overnight to afford crude product **119** which was used for the next step directly (2.0g, 79.5% yield).
**¹HNMR** (400 MHz, DMSO): δ4.55 (br, 2 H), 7.38 (s, 1 H), 12.0 (br, 1 H).

### Procedure forpreparation of 120:

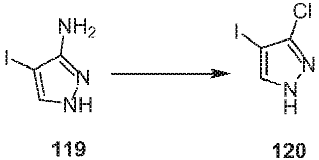

A solution of **119** (20.0g, 100.0mmol) in 60.0mL of water, was added 100.0mL of concentrated HCl and 30.0 mL of 85% H₃PO₄ the mixture was cooled to -5°C. A solution of NaNO₂ (7.0g, 100.0mmol) in 30.0mL of H₂O was added over 30minutes, the temperature was kept at -2°C. After stirred for 1 hour, the above mixture was added to a solution of CuCl (15g, 150.0mmol) in 100.0mL of concentrated HCl. The mixture was heated to 60°C until the mixture was no gas goes off, then extracted with chloroform. The organic phase was washed with water, brine, dried over Na₂SO₄, filtered and concentrated to dryness. The residue was purified by flash chromatography column on silicone gel to afford product **120** (1.6g, yield: 6.9%).
**LCMS:** *m*/*z,* 228.9(M+H)⁺.

### Procedure for preparation of 121:

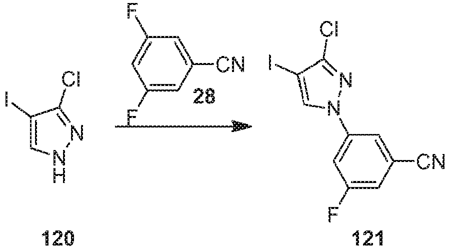

To a solution of **120** (73.0mg, 0.5mmol) and **28** (100.0mg, 0.4mmol) in DMF (2.0mL) was added Cs₂CO₃ (260.6mg, 0.8mmol at room temperature, the mixture was stirred at 80°C for 30 minutes. TLC showed the reaction was completed. The reaction mixture was treated with water, extracted with 15mL of EA. The organic phase was separated, washed by water, brine, dried over Na₂SO₄, filtered and concentrated to dryness. The residue was purified by pre-TLC to afford product **121** (100.0mg, yield: 66.7%).
**¹HNMR** (400 MHz, CDCl3): δ7.31 (d, J=2 Hz, 1 H), 7.65-7.68 (m, 1 H), 7.74 (s, 1 H), 7.95 (s, 1H).

### Procedure for preparation of Compound 38:

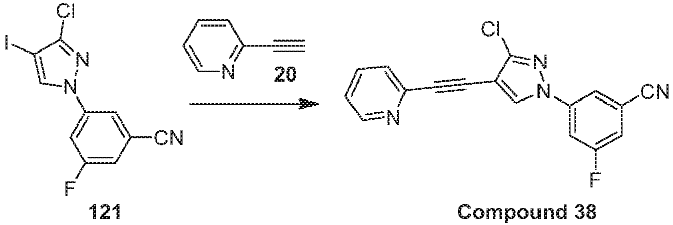

To a solution of **121** (60.0mg, 0.17mmol) and **20** (26.7mg, 0.26mmol) in THF (3.0mL) was added Pd(PPh₃)₂Cl₂ (1.4mg, 0.002mmol), CuI (0,7mg, 0.005mmol) and Et₃N (34.4mg, 0.34mmol) at room temperature, the mixture was heated at 90°C by microwave for 1 hour under N₂ atmosphere. The reaction mixture was concentrated to dryness and the residue was purified by prep-HPLC to afford product **Compound 38** (9.0mg, yield: 16.7%).
**LCMS:** *m*/*z,* 323.0 (M+H)⁺,
**¹HNMR** (400 MHz, CDCl3): δ7.28-7.31 (m, 2 H), 7.55 (d, H=8.0 Hz, 1 H), 7.67-7.76 (m, 3 H), 8.11 (s, 1 H), 8.64 (d, J=4.4 Hz, 1 H).

### Example Compound 39

### Preparation of 3-(-3-chloro-4-(pyrimidin-2-ylethynyl)-1H-pyrazol-1-yl)-5-fluorobenzonitrile:

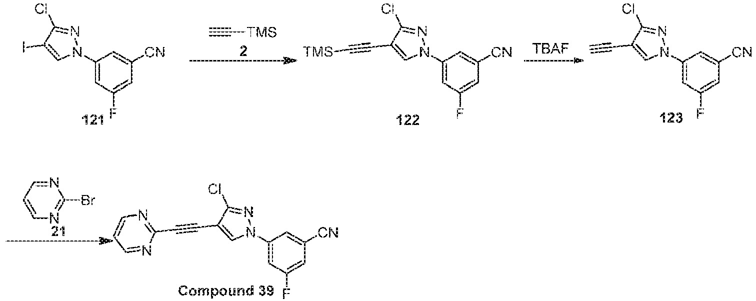

### Experimental section:

### Procedure for preparation of 122:

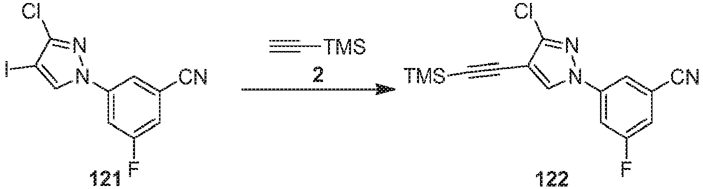

To a solution of **121** (100.0mg, 0.3mmol) and 2 (57.0mg, 0.6mmol) in THF (3.0mL) was added Pd(PPh₃)₂Cl₂ (2.1mg, 0.003mmol), CuI (1.1mg, 0.006mmol) and Et₃N (60.0mg, 0.6mmol) at room temperature, the mixture was heated at 90°C by microwave for 1 hour under N₂ atmosphere. The reaction mixture was concentrated to dryness, and the crude product was purified by prep-TLC to afford title product **122**(50.0mg, 55.0% yield).

### Procedure for preparation of 123:

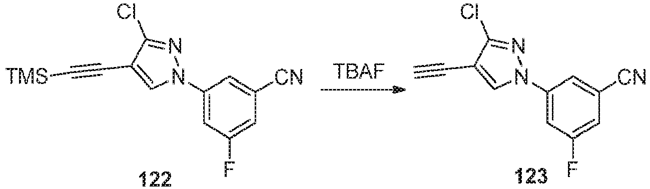

To a solution of **122** (50.0mg, 0.16mmol) in THF (3.0mL) was added 0.2mL of TBAF in THF solution (0.2mmol, 1M), the reaction mixture was stirred at room temperature for 1 hour and then concentrated to dryness. The crude product **123** was used for the next step directly (38.6mg, 100% yield).

### Procedure for preparation of Compound 39:

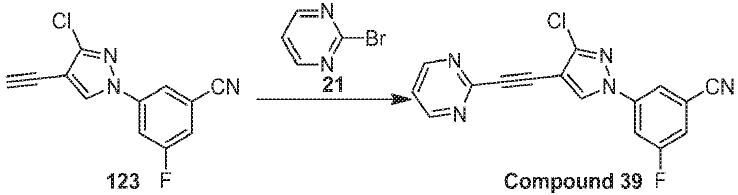

To a solution of **123** (38.6mg, 0.16mmol) and **21** (37.5mg, 0.24mmol) in THF (3.0mL) was added Pd(PPh₃)₂Cl₂ (1.4mg, 0.002mmol), CuI (0.7mg, 0.004mmol) and Et₃N (60.0mg, 0.6mmol) at room temperature, the mixture was heated at 90°C by microwave for 1 hour under N₂ atmosphere. The reaction mixture was concentrated to dryness, and the crude product was purified by prep-HPLC to afford product **Compound 39** (9.0mg, yield: 17.7%).
**LCMS:** *m*/*z,* 323.9 (M+H)⁺;
**¹HNMR** (400 MHz, CDCl3): δ7.27-7.31 (m, 1 H), 7.34 (d, J=8.0 Hz, 1 H), 7.72 (d, J=8.0 Hz, 1H), 7.75 (s, 1 H), 8.17 (s, 1 H), 8.78 (d, J=4.4 Hz, 2H).

### Example Compound 40

### Preparation of 2-((3-chloro-1-(4-(trifluoromethoxy)phenyl)-1H-pyrazol-4-yl) ethynyl)pyridine:

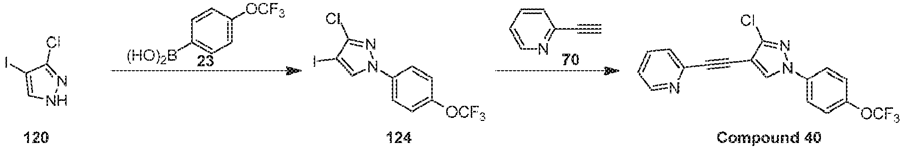

### Experimental section:

### Procedure for preparation of 124:

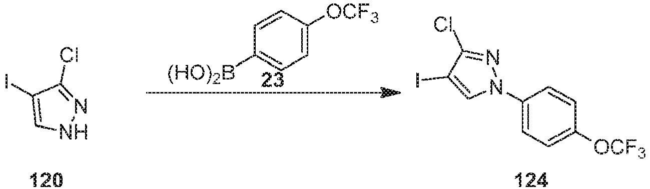

To a solution of **120** (100.0mg, 0,4mmol) and **23** (135.2mg, 0.6mmol) in DCM (10.0mL) was added pyridine (95mg, 1.2mmol) and Cu(OAC)₂ (218mg, 1.2mmol), the mixture was stirred at room temperature under O₂ balloon overnight. The reaction mixture was filtered and the filtrate was concentrated to dryness. The residue was purified by pre-TLC to afford product **124** (70.0mg, yield: 41.1%).
**LCMS:** *m*/*z,* 388.8 (M+H)⁺.

### Procedure for preparation of Compound 40:

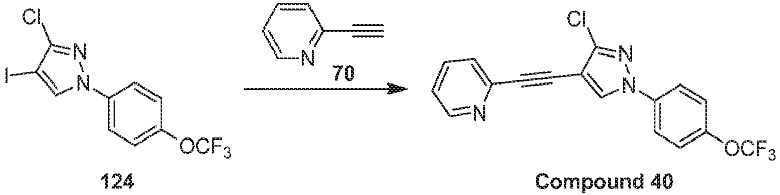

To a solution of **124** (70.0mg, 0. 1 8mmol) and **70** (27.8mg, 0.27mmol) in THF (3.0mL) was added Pd(PPh₃)₂Cl₂ (1.4mg, 0.002mmol), CuI (0.7mg, 0.005mmol) and Et₃N (36.4mg, 0.36mmol) at room temperature, the mixture was heated at 90°C by microwave for 1 hour under N₂ atmosphere. The reaction mixture was concentrated to dryness, and the crude product was purified by prep-HPLC to afford **Compound 40** (7.0mg, yield: 10.8%).
**LCMS:** *m*/*z,* 363.9 (M+H)⁺.
**¹HNMR** (400 MHz, CDCl3): δ7.27-7.29 (m, 1 H), 7.34 (d, J=8.0 Hz, 2 H), 7.55 (d, J=8.0 Hz, 1 H), 7.67-7.71 (m, 3 H), 8.08 (s, 1 H), 8.63 (d, J-4.0 Hz, 1H).

### Example Compound 41

### Preparation of 2-((3-chloro-1-(4-(trifluoromethoxy)phenyl)-1H-pyrazol-4-yl) ethynyl)pyrimidine:

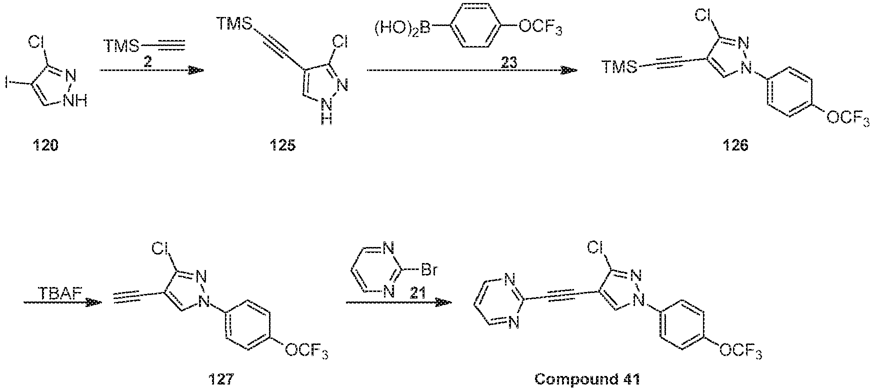

### Experimental section:

### Procedure for preparation of 125:

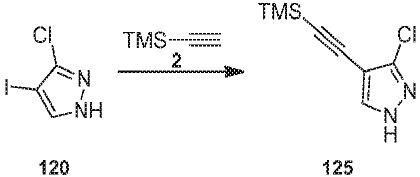

To a solution of **120** (228.0mg, 1.0mmol) and **2** (147.0mg, 1.5mmol) in THF (3.0mL) was added Pd(PPh₃)₂Cl₂ (7.0mg, 0.01mmol), CuI (3.8mg, 0.02mmol) and Et₃N (202.4mg, 2.0mmol) at room temperature, the mixture was heated at 90°C by microwave for 1 hour under N₂ atmosphere. The reaction mixture was purified by prep-TLC to afford product **125** (150.0mg, yield: 75.4%). **LCMS:** *m*/*z,* 199.1 (M+H)⁺.

### Procedure for preparation of 126:

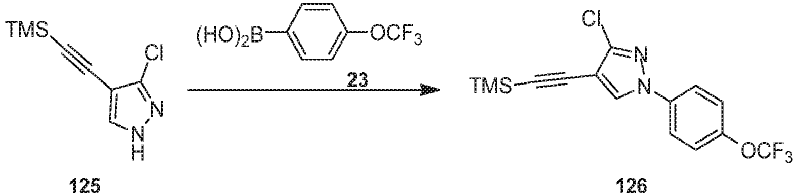

To a solution of **125** (75.0mg, 0.38mmol) and **23** (155.4mg, 0.75mmol) in DCM (10.0mL) was added pyridine (90.1mg, 1.14mmol) and Cu(OAc)₂ (207.0mg, 1.14mmol), the mixture was stirred at room temperature under O₂ balloon overnight. The reaction mixture was filtered and the filtrate was concentrated to dryness. The residue was purified by pre-TLC to afford product **126** (80.0mg, yield: 59.1%).
LCMS: *m*/*z,* 359.0 (M+H)⁺.

### Procedure for preparation of 127:

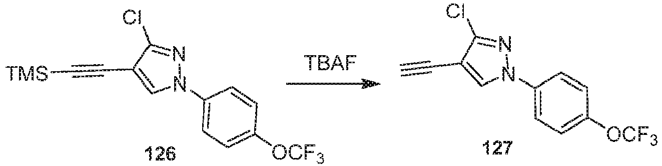

To a solution of **126** (80.0mg, 0.22mmol) in THF (3.0mL) was added 0.22mL of TBAF in THF solution (0.22mmol, 1M), the reaction mixture was stirred at room temperature for 1 hour and then concentrated to dryness. The crude product **127** was used for the next step directly (63.9mg, yield: 100%).

### Procedure for preparation of Compound 41:

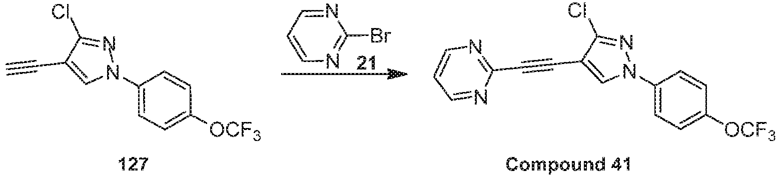

To a solution of **127** (63.9mg, 0.22mmol) and **21** (53.2mg, 0.33mmol) in THF (3.0mL) was added Pd(PPh₃)₂Cl₂ (1.4mg, 0.002mmol), CuI (0.7mg, 0.004mmol) and Et₃N (44.5mg, 0.44mmol) at room temperatuer, the mixture was heated at 90°C by microwave for 1 hour under N₂ atmosphere. The reaction mixture was concentrated to dryness and the residue was purified by prep-HPLC to afford product **Compound 41** (15.0 mg, yield: 18.7%).
**LCMS:** *m*/*z,* 365.0 (M+H)⁺;
**¹HNMR** (400 MHz, CDCl3): 87.27 (d, J=4.0 Hz, 1 H), 7.34 (d, J=8.0 Hz, 2 H), 7.69 (d, J=8.0 Hz, 2 H), 8.15 (s, 1 H), 8.77 (d, J=4.0 Hz, 2 H).

### Example Compound 42

### Preparation of 2-((3-chloro-1-(4-fluorophenyl)-1H-pyrazol-4-yl)ethynyl)pyridine:

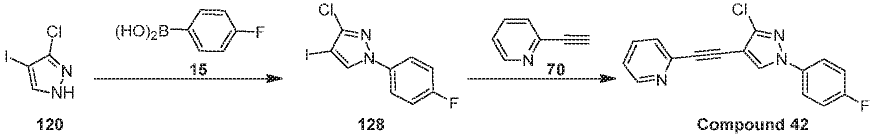

### Experimental section:

### Procedure for preparation of 128:

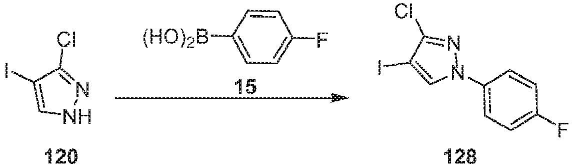

To a solution of **120** (100.0mg, 0.4mmol) and **15** (92.0mg, 0.6mmol) in DCM (10.0mL) was added pyridine (95mg, 1.2mmol) and Cu(OAc)₂ (218mg, 1.2mmol), the mixture was stirred at room temperature under O₂ balloon overnight. The reaction mixture was filtered and the filtrate was concentrated to dryness. The residue was purified by prep-TLC to afford product **128** (120.0mg, yield: 85.0%).
**LCMS:** *m*/*z,* 322.9(M+H)⁺.

### Procedure for preparation of Compound 42:

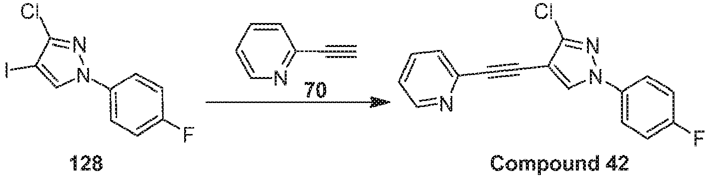

To a solution of **128** (60.0mg, 0.18mmol) and **70** (28.7mg, 0.28mmol) in THF (3.0mL) was added Pd(PPh₃)₂Cl₂ (1.4mg, 0.002mmol), CuI (0.7mg, 0.005mmol) and Et₃N (36.4mg, 0.36mmol) at room temperatuer, the mixture was heated at 90°C by microwave for 1 hour under N₂ atmosphere. The reaction mixture was concentrated to dryness and the crude product was purified by prep-HPLC to afford **Compound 42** (8.0mg, 14.4% yield).
**LCMS:** *m*/*z,* 298.0 (M+H)⁺;
**¹HNMR** (400 MHz, CDCl3): δ7.13-7.18 (m, 2 H), 7.27-7.29 (m, 1 H), 7.51-7.64 (m, 3 H), 7.66-7.69 (m, 1 H), 8.03 (s, 1 H), 8.61 (d, J=4.4 Hz, 1 H).

### Example Compound 43

### Preparation of 2-((3-chloro-1-(4-fluorophenyl)-1H-pyrazol-4-yl)ethynyl)pyrimidine:

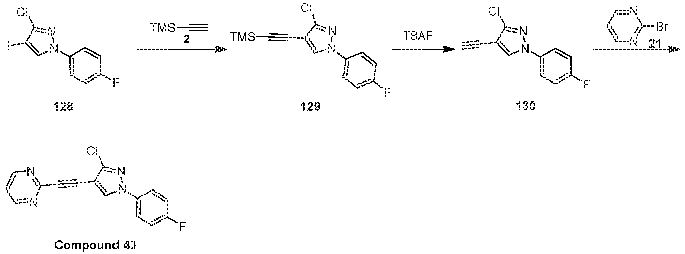

### Experimental section:

### Procedure for preparation of 129:

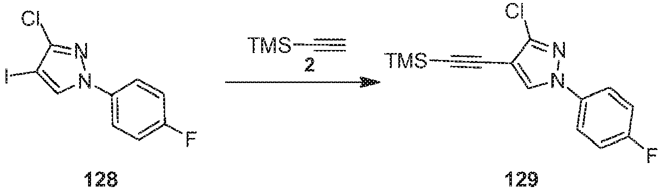

To a solution of **128** (50.0mg, 0.15mmol) and **2** (30.5mg, 0.3mmol) in THF (3.0mL) was added Pd(PPh₃)₂Cl₂ (1.4mg, 0.002mmol), CuI (0.7mg, 0.004mmol) and Et₃N (30.0mg, 0.30mmol) at room temperature, the mixture was heated at 90°C by microwave for 1 hour under N₂ atmosphere. The reaction mixture was concentrated to dryness and the crude product **129** was used for the next step directly without purification (45.4mg, yield: 100.0%).

### Procedure for preparation of 130:

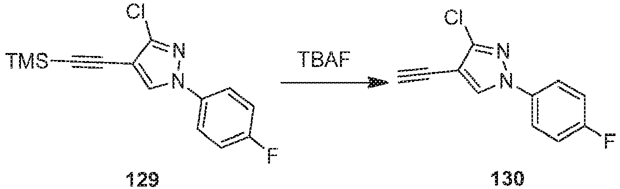

To a solution of **129** (45.4mg, 0.15mmol) in THF (3.0mL) was added 0.15mL of TBAF (0.15mmol, 1M in THF), the reaction mixture was stirred at room temperature for 1 hour and then concentrated to dryness. The crude product **130** was used for the next step directly (34.2mg, yield: 100%).

### Procedure for preparation of Compound 43:

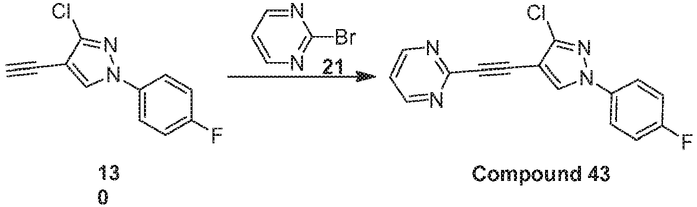

To a solution of **130** (34.2mg, 0.15mmol) and **21** (49.3mg, 0.3mmol) in THF (3.0mL) was added Pd(PPhs)₂Cl₂ (1.4mg, 0.002mmol), CuI (0.7mg, 0.004mmol) and Et₃N (30.0mg, 0.3mmol) at room temperature, the mixture was heated at 90°C by microwave for 1 hour under N₂ atmosphere. The reaction mixture was concentrated to dryness and the residue was purified by prep-HPLC to afford product **Compound 43**(2.5mg, yield: 5.4%).
**LCMS:** *m*/*z,* 299.0 (M+H)⁺;
**¹HNMR** (400 MHz, CDCl3): δ7.18 (t, J=8.0 Hz, 2 H), 7.27 (d, J=4.0 Hz, 1 H), 7.60-7.63 (m, 2 H), 8.10 (s, 1 H), 8.77 (d, J=4.0 Hz, 2H).

### Example Compound 44

### Preparation of 3-(3-chloro-4-(pyridin-2-ylethynyl)-1H-pyrazol-1-yl)-5-fluoropyridine:

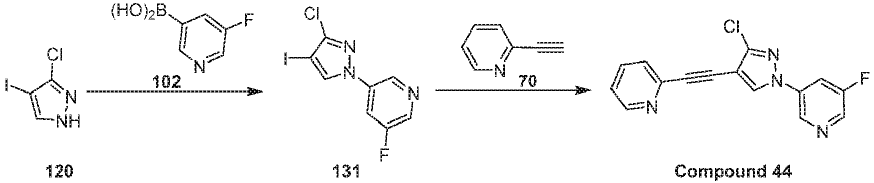

### Experimental section:

### Procedure for preparation of 131:

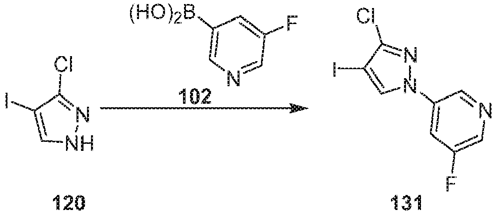

To a solution of **120** (100.0mg, 0.4mmol) and **102** (123.4mg, 0.8mmol) in DCM (10.0mL) was added pyridine (95mg, 1.2mmol) and Cu(OAc)₂ (218mg, 1.2mmol), the mixture was stirred at room temperature under O₂ balloon overnight. The reaction mixture was filtered and the filtrate was concentrated to dryness. The residue was purified by flashing chromatography column on silicon gel to afford product **131** (70.0mg, yield: 50%).
**¹HNMR** (400 MHz, CDCl3): δ7.76-7.84 (m, 1 H), 7.98 (s, 1 H), 8.46 (s, 1 H), 8.73 (s, 1 H).

### Procedure for preparation of Compound 44:

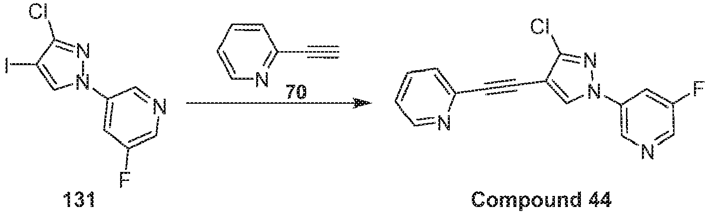

To a solution **of 131** (70.0mg, 0.21mmol) and **70** (33.5mg, 0.32mmol) in THF (3.0mL) was added Pd(PPh₃)₂Cl₂ (1.4mg, 0.002mmol), CuI (0.7mg, 0.005mmol) and Et₃N (34.4mg, 0.34mmol) at room temperature, the mixture was heated at 90°C by microwave for 1 hour under N₂ atmosphere. The reaction mixture was concentrated to dryness, and the crude product was purified by prep-HPLC to give product **Compound 44** (7.0mg, yield: 10.8%).
**LCMS:** *m*/*z,* 299.0 (M+H)⁺;
**¹HNMR** (400 MHz, CDCl3): δ7.30 (d, *J*=5.6 Hz, 1 H), 7.56 (d, *J*=7.6 Hz, 1 H), 7.72 (t, *J*=7.6 Hz, 1 H), 7.84 (d, *J*=8.8 Hz, 1 H), 8.17 (s, 1 H), 8.48 (s, 1 H), 8.64 (d, *J*=3.6 Hz, 1 H), 8.76 (s, 1 H).

### Example Compound 45

### Preparation of 2-((3-chloro-1-(5-fluoropyriditz-3-yl)-1H-pyrazol-4-yl)ethynyl) pyrimidine:

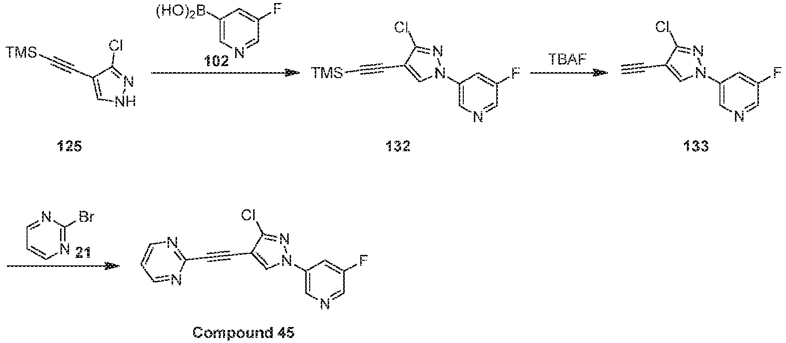

### Experimental section:

### Procedure for preparation of 132:

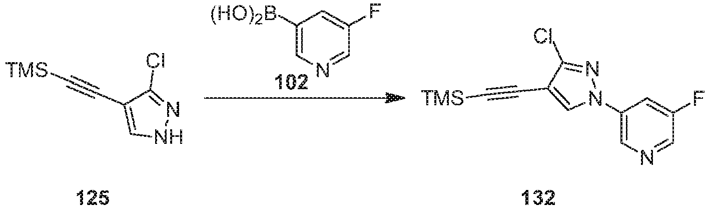

To a solution of **125** (75.0mg, 0.38mmol) and **102** (106.4mg, 0.75mmol) in DCM (10.0mL) was added pyridine (90.1mg, 1.14mmol) and Cu(OAc)₂ (207.0mg, 1.14mmol), the mixture was stirred at room temperature under O₂ balloon overnight. The reaction mixture was filtered and the filtrate was concentrated to dryness. The residue was purified by prep-TLC to afford product **132** (70.0mg, yield: 63.1%).
**LCMS:** *m*/*z,* 294.0 (M+H)⁺

### Procedure for preparation of 133:

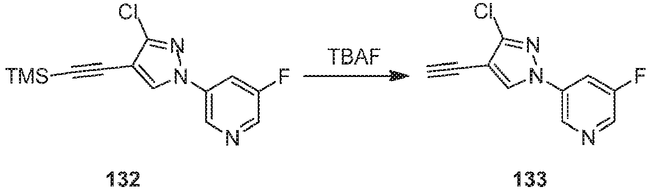

To a solution of **132** (70.0mg, 0.24mmol) in THF (3.0mL) was added 0.24mL of TBAF (0.24mmol, 1M in THF), the reaction mixture was stirred at room temperature for 1 hour and then concentrated to dryness. The crude product **133** was used for the next step directly (52.8mg, yield: 100%).

### Procedure for preparation of Compound 45:

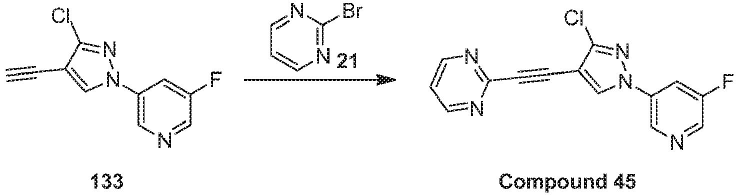

To a solution of **133** (52.8mg, 0.24mmol) and **21** (56.8mg, 0.36mmol) in THF (3.0mL) was added Pd(PPh₃)₂Cl₂ (1.4mg, 0.002mmol), CuI (0.7mg, 0.004mmol) and Et₃N (48.6mg, 0.48mmol) at room temperature, the mixture was heated at 90°C by microwave for 1 hour under N₂ atmosphere. The reaction mixture was concentrated to dryness and the residue was purified by prep-HPLC to afford product **Compound 45** (5.0 mg, yield: 7.0%).
**LCMS:** *m*/*z,* 300.0 (M+H)⁺;
**¹HNMR** (400 MHz, CDCl3): δ7.29 (t, *J*=4.0 Hz, 1 H), 7.84 (dt, *J*=8.0, 4.0 Hz, 1 H), 8.23 (s, 1 H), 8.49 (d, *J*=2.0 Hz, 1 H), 8.76 (d, *J*=2.0 Hz, 1 H), 7.78 (d, *J*=4.0 Hz, 2 H).

### Example Compound 46

### Preparation of 2-((1-(4-(methylsulfonyl)phenyl)-1H-pyrazol-4-yl)ethynyl)pyridine:

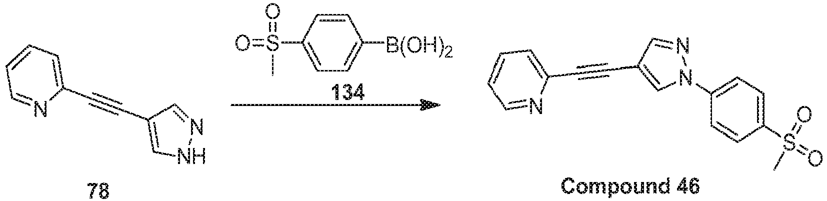

### Experimental section:

***Procedure for preparation of Compound 46:***

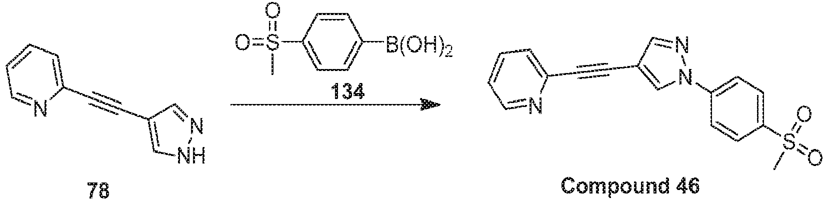

To a solution of **78** (70mg, 0.414mmol) in DCM (20mL) was added compoud **134** (166mg, 0.828mmol), Cu(OAc)₂ (150mg, 0.828mmol), pyridine (98mg, 1.24mmol). The mixture was stirred at room temperature overnight under O₂ atmosphere. The mixture was filtered and concentrated under vacuo. The residue was purified by prep-HPLC to give product **Compound 46**(6mg, yield: 4%).
**LCMS:** *m*/*z,* 324.0 (M+H)⁺
**¹H NMR** (400 MHz, CDCl3) δ 8.63 (s, 1H), 8.26 (s, 1H), 8.09-8.06 (m, 2H), 7.97 (s, 1H), 7.93-7.91 (m, 2H), 7.71 (m, 1H), 7.52 (m, 1H), 7.28 (m, 1H), 3.10 (s, 3H).

### Example Compound 47

### Preparation of 3-(3-(difluoromethoxy)-4-(pyridin-2-ylethynyl)-1H-pyrazol-1-yl)-5-fluorobenzonitrile:

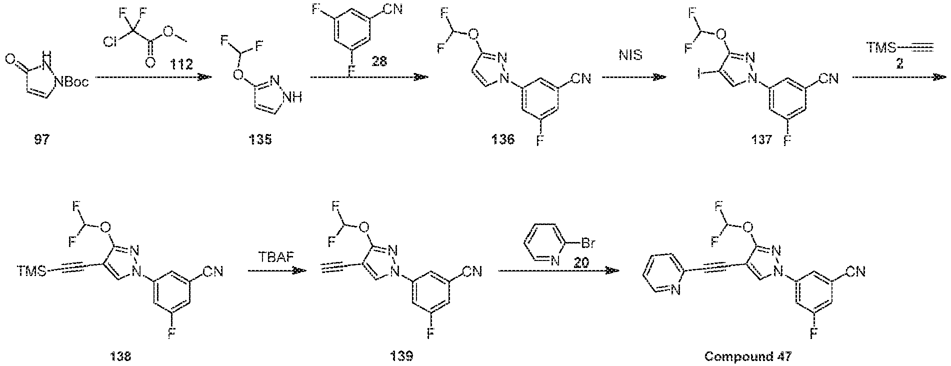

### Experimental section:

### Procedure for preparation of 135:

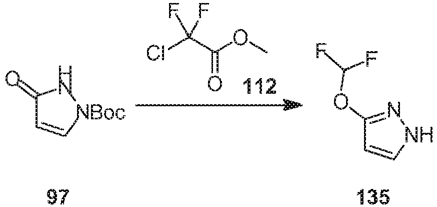

To a solution **of 97** (280mg, 1.52mmol) in DMF (20mL) was added **112** (145mg, 1.82mmol) and CS₂CO₃ (991mg, 3.04mmol). The mixture was stirred at 60°C for 4 hours. The mixture was filtered and concentrated by vacuo to give product **135** (200mg, crude).

### Procedure for preparation of 136:

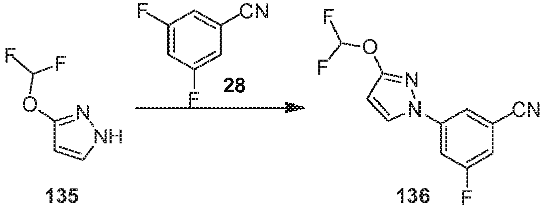

To a solution of **135** (100mg, 0.746mmol) in CH₃CN (10mL) was added **28** (124mg, 0.895mmol) and Cs₂CO₃ (486mg, 1.49mmol). The mixture was stirred at 70°C for 2 hours. The mixture was filtered and concentrated by vacuo to give the crude product which was purified by prep-TLC to give product **136** (70mg, 37%).
**LCMS:** *m*/*z,* 254.0 (M+H)⁺.

### Procedure for preparation of 137:

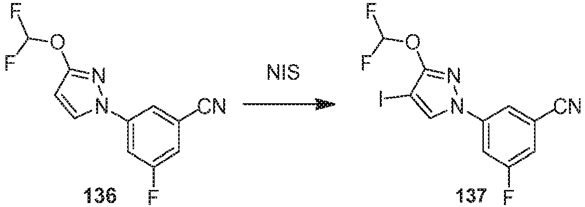

To a solution of compoud **136** (130mg, 0.513mmol) in CHCl₃ (15mL) was added NIS (139mg, 0.616mmol). The mixture was stirred at 60°C for 6 hours. The mixture was quenched with water and extracted with DCM (2x10mL). The combined organics was concentrated under vacuo and the residue was purified by prep-TLC to give product **137** (170mg, 87%). **LCMS:** *m*/*z,* 379.9 (M+H)⁺.

### Procedure for preparation of 138:

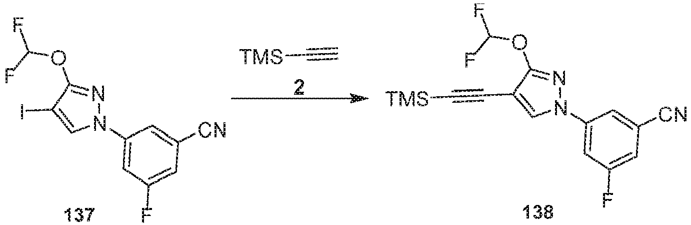

To a solution of **137** (170mg, 0.448mmol) in CH₃CN (3mL) was added successively CuI (9mg, 0.045mmol), **2** (88mg, 0.897mmol), Et₃N (136mg, 1.35mmol) and Pd(PPh₃)₂Cl₂ (16mg, 0.022mmol). The mixture was then degassed for 1 minute under N₂ atmosphere and was heated at 90°C under microwave for 1 hour. The reaction mixture was filtered and evaporated to give crude product, which was purified by prep-TLC to give product **138** (1 30mg, yield: 83%). **LCMS:** *m*/*z,* 350.1 (M+H)⁺.

### Procedure for preparation of 139:

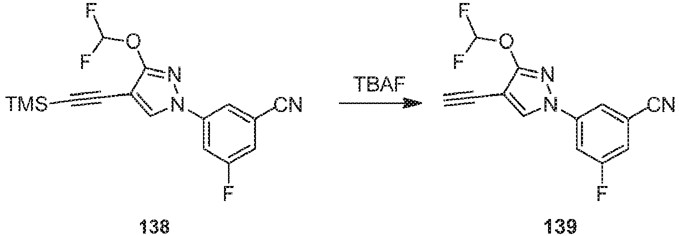

A solution of **126** (130mg, 0.372mmol) in THF (5mL) was cooled to 0°C and TBAF (0.558mL, 0.558mmol) was added. The reaction mixture stirred at room temperature for 1 hour. The mixture was quenched with water and extracted with EtOAc (3x10mL). The combined organics was concentrated by vacuo to give the crude product **139** (90mg, yield: 87%).

### Procedure for preparation of Compound 47:

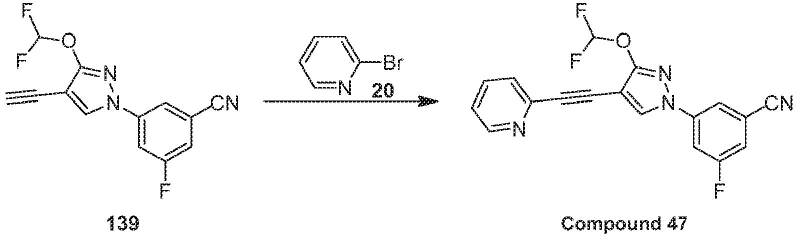

To a solution of **139** (90mg, 0.325mmol) in THF (3mL) was added successively CuI (6mg, 0.032mmol), **20** (103mg, 0.649mmol), Et₃N (99mg, 0.974mmol) and Pd(PPh₃)₂Cl₂ (11mg, 0.016mmol). The mixture was then degassed for 1 minute under N₂ atmosphere and stirred at 90°C for 1 hour under microwave. The reaction mixture was filtered and concentrated to give the crude product which was purified by prep-HPLC to give **Compound 47** (30mg, yield: 26%). **LCMS:** *m*/*z,* 355.1 (M+H)⁺;
**¹H NMR** (400 MHz, CDCl3): δ 8.64 (s, 1H), 8.06 (s, 1H), 7.73-7.72 (m, 2H), 7.72-7.71 (m, 1H), 7.61-7.56 (m, 1H), 7.32-7.26 (m, 2H), 7.14-6.96 (m, 1H).

### Example Compound 48

### Preparation of 3-fluoro-5-(3-(2-hydroxyethoxy)-4-(pyridin-2-ylethynyl)-1H-pyrazol-1-yl)henzonitrile:

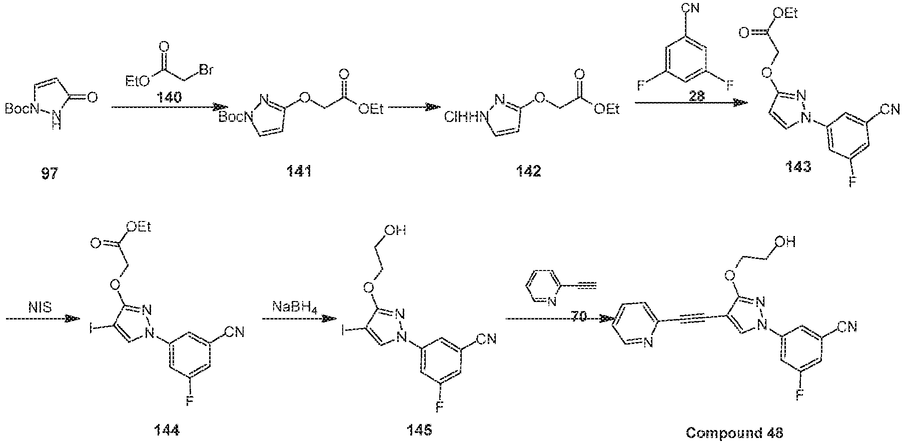

### Experimental section:

### Procedure for preparation of 141:

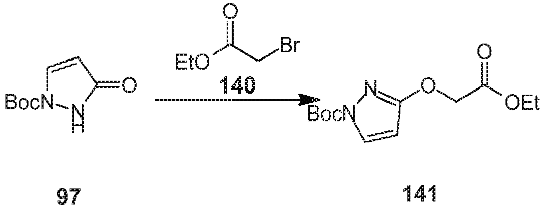

To a solution of **97** (500 mg, 2.71 mmol) in 30 mL of dioxane was added successively compound **140** (680 mg, 4.07 mmol) and Cs₂CO₃ (2.65g, 8.14 mmol). The reaction vessel was sealed and stirred at room temperature for 18 hours. LCMS showed that the reaction was complete. The reaction mixture was filtered and the filtrate was concentrated to give the product **141**(650 mg, crude).
**LCMS:** *m*/*z* 215 *(M*+*H)*⁺*.*

### Procedure for preparation of 142:

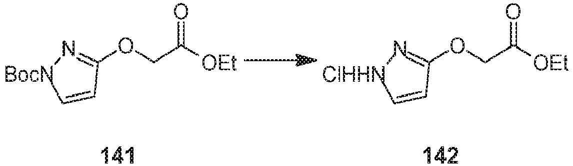

A solution of **141** (650mg, 1.21mmol) in HCl/EA (20mL) was stirred at room temperature for 1 hour. LCMS showed that starting material was consumed. The reaction mixture was concentrated to give the product **142** as HCl salt (480 mg, crude).
**LCMS:** *m*/*z* 171 (M+H)⁺.

### Procedure for preparation of 143:

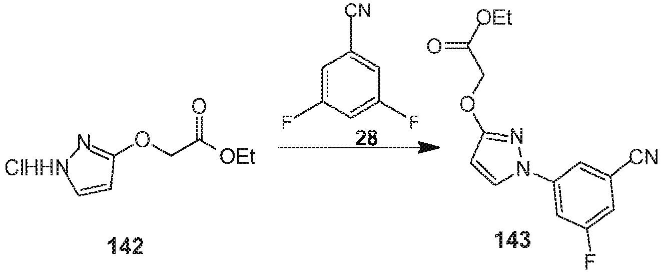

To a solution of compound **142** (400 mg, 1.94 mmol) in 20 mL of degassed DMF was added successively compound **28** (539 mg, 3.87 mmol) and CS₂CO₃ (1.89 g, 5.87 mmol). The mixture was heated to 110°C and stirred for 1 hour. The reaction mixture was treated with water (50ml), extracted with DCM (2 x 50ml). The combined organic layer was dried over Na₂SO₄, concentrated and purified by prep-TLC to give the product **143** (130 mg, yield: 23%).
**LCMS:** *m*/*z* 290 (M+H)⁺.

### Procedure for preparation of 144:

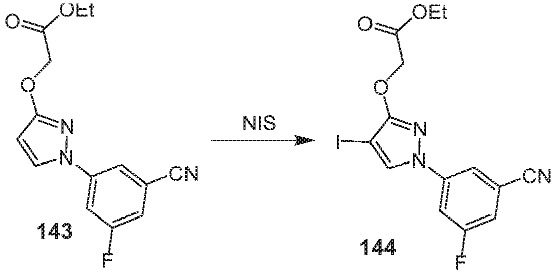

To a solution of compound **143** (130mg, 0.45mmol) in 30 mL of degassed CHCl₃ was added NIS (152mg, 0.67mmol). The mixture was heated to reflux and stirred for 18 hours. LCMS showed that compound **143** was consumed. The reaction mixture was cooled to room temperature and filtered. The filtrate was concentrated and purified by prep-TLC to give the product **144** (150 mg, yield: 80%).
**LCMS:** *m*/*z* 416 (M+H)⁺.

### Procedure for preparation of 145:

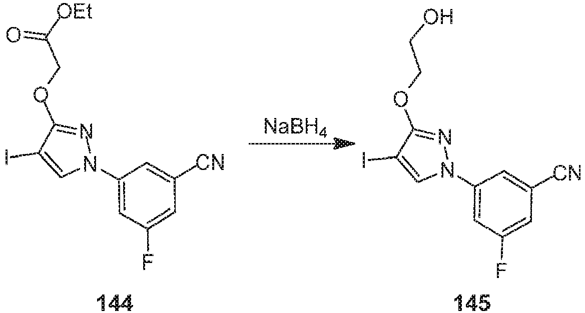

To a solution of compound **144** (150mg, 0.36mmol) in 20 mL of CH₃OH was added NaBH₄ (68mg, 1.81mmol). The mixture was stirred at room temperature for 2 hours. LCMS showed that compound **144** was consumed. The reaction mixture was quenched with water (20ml), extracted with DCM (3 x 20ml). The combined organic layer was dried over Na₂SO₄, concentrated to give the product **145** (110 mg, yield: 82%).
**LCMS:** *m*/*z* 374 (M+H)⁺.

### Procedure for preparation of Compound 48:

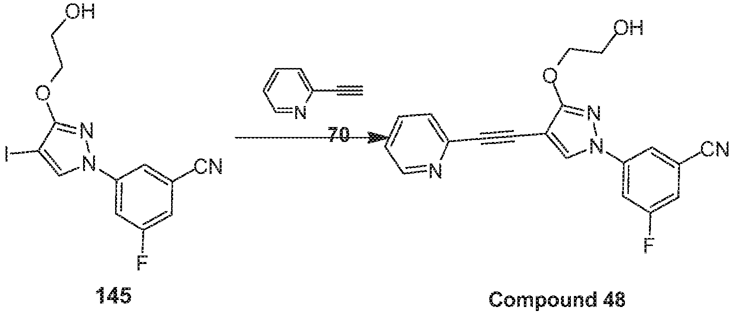

To a solution of compound **145** (90mg, 0.24mmol) in 10 mL of degassed THF was added successively CuI (5mg, 0.024 mmol), compound **70** (50mg, 0.48mmol), and Pd(PPh₃)₂Cl₂ (17mg, 0.024mmol) and Et₃N (73mg, 0.72mmol). The mixture was stirred at 80°C for 18 hours. The reaction mixture was filtered and the filtrate was concentrated to give the crude product, which was purified by prep-HPLC to give the product **Compound 48**(22 mg, yield: 26%).
**LCMS:** *m*/*z* 349 (M+H)⁺;
**¹H NMR** (400 MHz, CDCl3): δ 8.61 (d, *J*=4.8 Hz, 1H), 7.99 (s, 1H), 7.71-7.67 (m, 2H), 7.26-7.21 (m, 2H), 4.52-4.50 (m, 2H), 4.04-4.03 (m, 2H), 2.67-2.65 (m, 1H).

### Example Compound 49

### Preparation of 2-(3-chloro-4-(pyridin-2-ylethynyl)-1H-pyrazol-1-yl)-5-fluoropyridine:

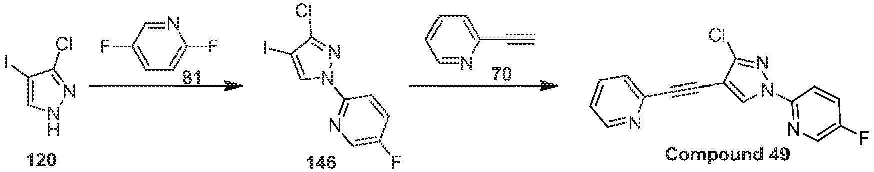

### Experimental section:

### Procedure for preparation of 146:

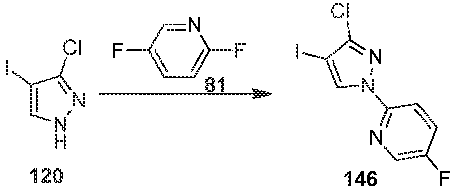

To a solution of **120** (150.0mg, 0.66mmol) and **81** (113.4mg, 0.96mmol) in DMF (5.0mL) was added CS₂CO₃ (430.0mg, 1.32mmol) at room temperature, the mixture was stirred at 80°C for 30 minutes. TLC showed the reaction was completed. The reaction was treated with water, extracted with 15mL of EA. The organic phase was separated, washed by water, brine, dried over Na₂SO₄ and concentrated to dryness. The residue was purified by prep-TLC to afford product **146** (80.0mg, yield: 37.6%).
**LCMS:** *m*/*z,* 323.9 (M+H)⁺.

### Procedure for preparation of Compound 49:

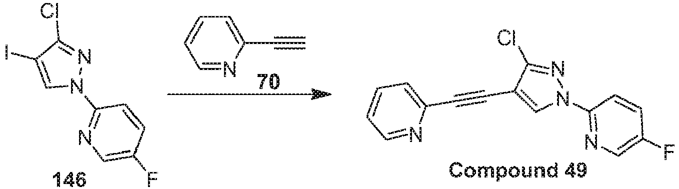

To a solution of **146** (80.0mg, 0.25mmol) and **70** (38.2mg, 0.37mmol) in THF (3.0mL) was added Pd(PPh₃)₂Cl₂ (1.4mg, 0.002mmol), CuI (0.7mg, 0.005mmol) and Et₃N (36.4mg, 0.36mmol) at room temperature, the mixture was heated at 90°C by microwave for 1 hour under N₂ atmosphere. The reaction mixture was concentrated to dryness, and the crude product was purified by prep-HPLC to afford title product **Compound 49** (13.0mg, yield: 17.6%).
**LCMS:** *m*/*z,* 299.0 (M+H)⁺;
**¹HNMR** (400 MHz, CDCl3): δ7.21-7.22 (m, 1 H), 7.48-7.53 (m, 2 H), 7.64 (dt, J=8.0, 4.0 Hz, 1 H), 7.88 (dd, J=8.0, 4.0 Hz, 1 H), 8.21 (d, J=4.0 Hz, 1 H), 8.57 (d, J=8.0 Hz, 1 H), 8.59 (s, 1 H).

### Example Compound 50

### Preparation of 3-fluoro-5-(5-methyl-4-(pyridin-2-ylethynyl)-1H-pyrazol-1-yl) benzonitrile:

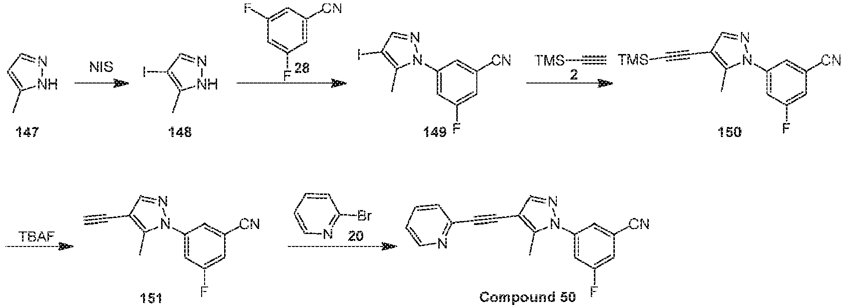

### Experimental section:

### Procedure for preparation of 148:

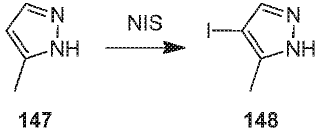

To a solution of 147 (300mg, 3.65mmmol) in CHCl₃(20ml) was added NIS (1.07g, 4.75mmol). The mixture was stirred at 70 °C for 2 hours, cooled to room temperature. The mixture was diluted with EtOAc (70mL), washed with brine (20mL) and the organic layers was dried (MgSO4), filtered and concentrated in vacuum, which was purified by silica gel chromatography to give title product **148** (600mg, yield: 79%).
**LCMS:** *m*/*z,* 208.9 (M+H)⁺.

### Procedure for preparation of 149:

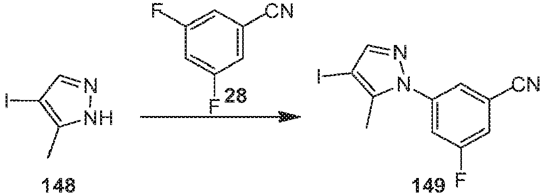

To a solution of **148** (300mg, 1.44mmol) and **28** (241mg, 1.73mmol) in DMF(10ml) was added CS₂CO₃ (940mg, 2.88mmol). The mixture was heated at 60°C for 2 hours, then, the reaction mixture was concentrated to dryness, which was purified by prep-TLC to afford the title product **149** (300mg, yield: 64%).
**¹H NMR** (400 MHz, CDCl₃): δ7.91-7.89(m, 1 H), 7.83-7.81 (m, 1 H), 7.73 (s, 1 H), 7.61-7.57 (m, 1 H), 7.25-4.23 (m, 1 H), 2.33(s, 3 H).

### Procedure for preparation of 150:

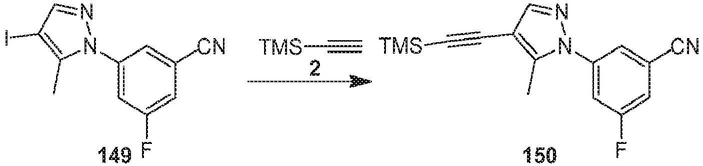

To a solution of **149** (200mg, 0.6mmol) and **2** (120mg, 1.2mmol), CuI (12mg, 0.06mmol), Et₃N (186mg, 1.8mmol) in THF(5mL) was added Pd(PPh₃)₂Cl₂(21mg, 0.03mmol). The mixture was stirred at 90°C for 6 hours under N₂ atmosphere. The mixture was filtered and concentrated under vacuo. The residue was purified by prep-TLC to give the product **150** (120mg, yield: 67%). **LCMS:** *m*/*z,* 298. 1(M+H)⁺.

### Procedure for preparation of 151:

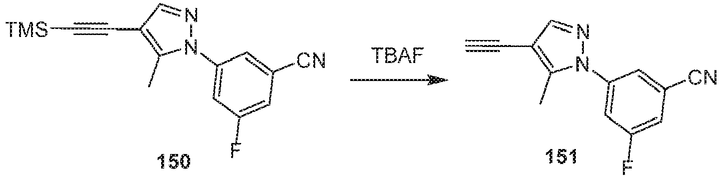

To a solution of **150** (160mg, 0.54mmol) in THF (4mL) was added TBAF (0.81mL, 0.81mmol, 1.0M in THF). The mixture was stirred at 20°C for 2 hours. The mixture was diluted with EtOAc (30mL) and washed with brine (20mL), dried over Na₂SO₄, concentrated under vacuum. The residue was purified by prep-TLC to give the product **151** (100mg, yield: 82%).
**LCMS:** *m*/*z,* 226.1 (M+H)⁺.

### Procedure for preparation of Compound 50:

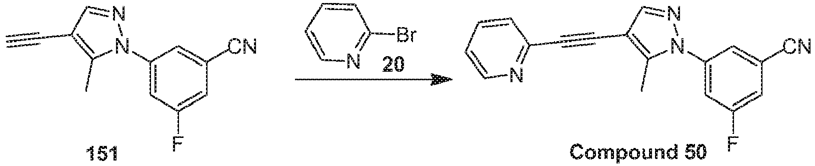

To a solution of compound **152** (90mg, 0.4mmol) and **20** (81mg, 0.82mmol), CuI(9mg, 0.04mmol), Et₃N(117mg, 1.2mmol) in THF(5mL) was added Pd(PPh₃)₂Cl₂ (27mg, 0.04mmol). The mixture was stirred at 90°C for 6 hours under N₂ atmosphere. The mixture was filtered and concentrated under vacuum. The residue was purified by prep-TLC to give the product **Compound 50** (20mg, yield: 16%).
**LCMS:** *m*/*z,* 303.1(M+H)⁺;
**¹H NMR** (400 MHz, CDCl3): δ8.59 ∼8.68 (m, 1 H), 8.07 (s, 1 H), 7.78 (s, 1 H), 7.69 (s, 2 H), 7.52 (s, 1 H), 7.26 ∼7.29 (m, 2 H), 2.48 (s, 3 H).

### Example Compound 51

### Preparation of 2-((1-(4-fluorophenyl)-5-methoxy-1H-pyrazol-4-yl)ethynyl)pyridine:

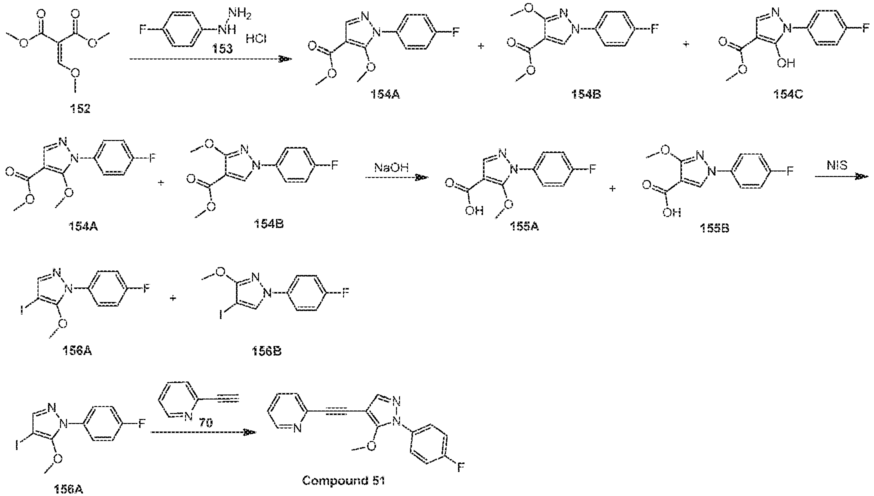

### Experimental section:

### Procedure for preparation of 154:

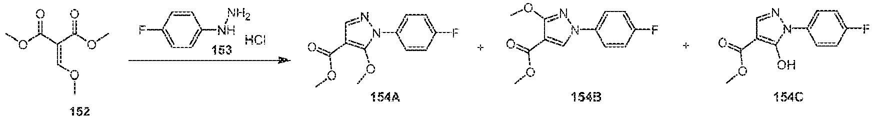

To a solution of **152** (5.00g, 28.71mmol) in 250 mL ofMeOH was added compound 141 (4.90g, 30.15mmol). The mixture was stirred at 70 °C for 18 hours. The reaction mixture was concentrated and the obtained residue was treated with saturated NaHCO₃ solution, extracted with DCM (2 x 300mL). The combined organic layer was dried over Na₂SO₄, concentrated and purified by chromatograph column to give a mixture of compound **154A** and compound **154B** (1.1 g, yield 15%) and 1.6 g of compound **154C**.
**¹H NMR** (400 MHz, CDCl3): δ 8.19 (s, 1H), 7.91 (s, 1H), 7.63-7.58 (m, 3H), 7.17-7.12 (m, 3H), 4.14 (s, 3H), 4.08 (s, 1.4H), 3.86 (s, 4H), 3.77(s, 1.3H).

### Procedure for preparation of 155A and 155B:

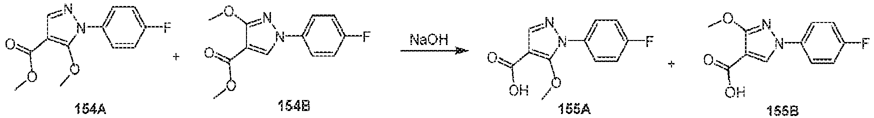

To a solution of compound **154A** and compound **154B** (900mg, 3.60mmol) in 30 mL/30 mL of EtOH/H₂O was added NaOH (432mg, 10.79mmol). The mixture was stirred at 80 °C for 2 hours. The reaction mixture was concentrated and the obtained residue was acidified with 1 N HCl solution to pH=5∼6, extracted with DCM (2 x 30mL). The combined organic layer was dried over Na₂SO₄, concentrated to give a mixture of compound **155A** and compound **155B** (700 mg, yield 82%).
**LCMS:** *m*/*z* 237 (M+H)⁺;
**¹H NMR** (400 MHz, CDCl3): δ 8.28 (s, 0.45H), 8.00 (s, 1H), 7.64-7.61 (m, 3H), 7.19-7.15(m, 3H), 4.17 (s, 3H), 4.12 (s, 1.3H).

### Procedure for preparation of 156A and 156B:

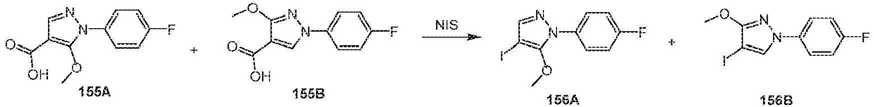

To a solution of a mixture of compound **155A** and compound **155B** (700mg, 2.96mmol) in 30 mL of DMF was added NIS (1.00g, 4.45mmol) and NaHCO₃ (996mg, 11.85mmol). The mixture was stirred at 60°C for 48 hours. LCMS showed that the reaction was complete. The reaction mixture was concentrated and purified by prep-TLC to give the products compound **154A** (260 mg, yield 27%) and compound **156B** (110 mg, yield 11%).
**¹H NMR** (400 MHz, CDCl3): δ 7.62-7.58 (m, 2H), 7.51 (s, 1H), 7.16-7.12 (m, 2H), 3.97 (s, 3H).

### Procedure for preparation of Compound 51:

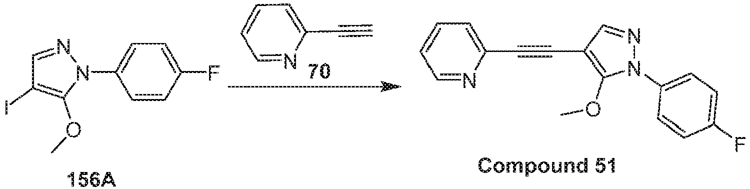

To a solution of compound **156A** (120mg, 0.38mmol) in 10 mL of degassed THF was added successively CuI (8mg, 0.038 mmol), compound **70** (79mg, 0.75mmol), and Pd(PPh₃)₂Cl₂ (26mg, 0.038mmol) and Et₃N (115mg, 1.13mmol). The mixture was stirred at 80 °C for 18 hours. The reaction mixture was filtered and the filtrate was concentrated to give the crude product, which was purified by prep-HPLC to give the product **Compound 51** (15 mg, yield 14%).
**LCMS:** *m*/*z* 294 (M+H)⁺;
**¹H NMR** (400 MHz, CDCl3): 88.60 (s, 1H), 7.68-7.61 (m, 4H), 7.44 (d, J= 7.6 Hz, 1H), 7.25-7.23 (m, 1H), 7.15-7.11 (m, 2H), 4.41(s, 3H).

### Example Compound 52

### Preparation of 2-((1-(4-fluorophenyl)-3-methoxy-1H-pyrazol-4-yl)ethynyl)pyridine:

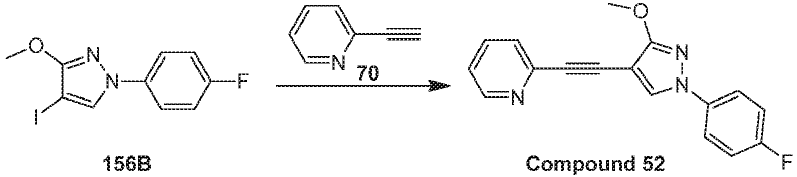

### Experimental section:

### Procedure for preparation of Compound 52:

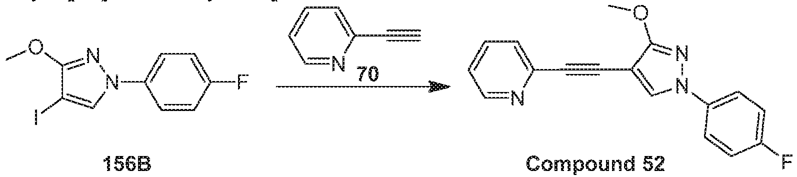

To a solution of **156B** (11 0mg, 0.35mmol) in 5 mL of degassed THF was added successively CuI (7mg, 0.035mmol), **70** (71mg, 0.71mmol), and Pd(PPh₃)₂Cl₂ (24mg, 0.035mmol) and Et₃N (105mg, 1.04mmol). The mixture was stirred at 80 °C for 18 hours. The reaction mixture was filtered and the filtrate was concentrated to give the crude product, which was purified by prep-HPLC to give the product **Compound 52** (10 mg, yield 10%).
**LCMS:** *m*/*z* 294 (M+H)⁺;
**¹H NMR** (400 MHz, CDCl3): δ8.59 (s, 1H), 7.90 (s, 1H), 7.65-7.54 (m, 4H), 7.19-7.10(m, 3H), 4.06(s, 3H).

### Example Compound 53

### Preparation of 3-(3-(2-(dimethylamino)ethoxy)-4-(pyridin-2-ylethynyl)-1H-pyrazol-1-yl)-5-fluorobenzonitrile:

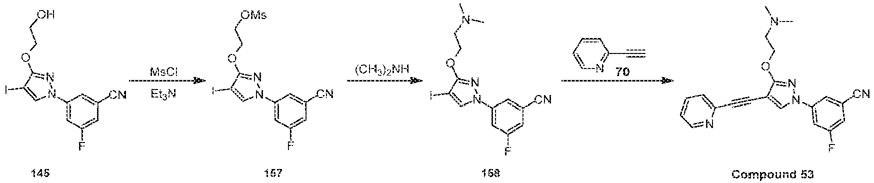

### Experimental section:

### Procedure for preparation of 157:

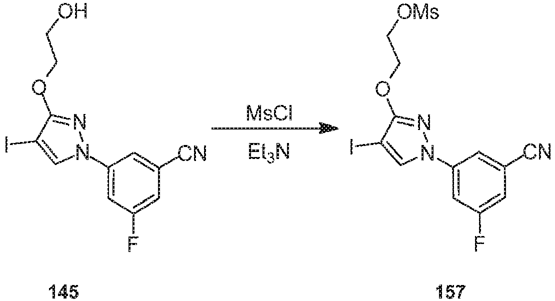

To a solution of **145** (150mg, 0.402mmol) in DCM (20mL) was added TEA (69mg, 0.683mmol). The mixture was cooled to 0°C and added MsCl (69mg, 0.603mmol) dropwise. The mixture was allowed to warm to room temperature and stirred for 1hour. The mixture was quenched with water and extracted with EA (2x20mL). The combined organics was concentrated by vacuo to give the crude product 145 (180mg, crude).
**LCMS:** *m*/*z,* 451.8 (M+H)⁺.

### Procedure for preparation of 158:

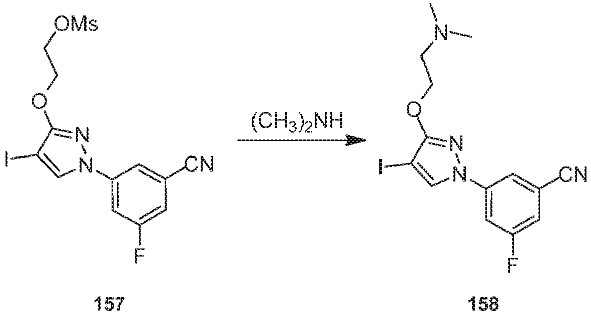

To a solution of **157** (100mg, 0.222mmol) in THF (5mL) was added compoud dimethyl amine (in MeOH) (1. 11mL, 1.11mmol) dropwise. The mixture was stirred at 60 °C for 5 hours. The mixture was quenched with water and extracted with EA (2x20mL). The combined organics was concentrated by vacuo to give the crude product which was purified by prep-TLC to give product **158** (80mg, 90%) .
**LCMS:** *m*/*z,* 401.0 (M+H)⁺.

### Procedure for preparation of Compound 53:

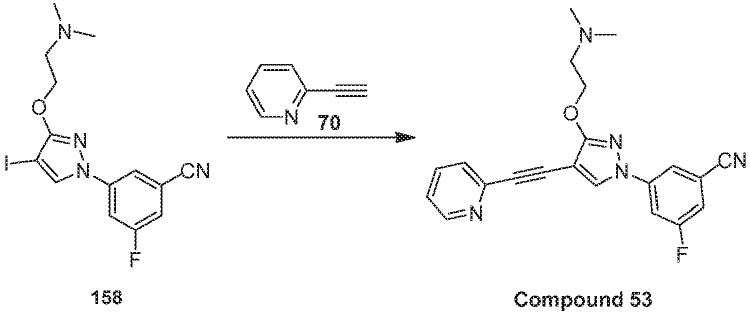

To a solution of **158** (65mg, 0.162mmol) in THF (3mL) was added successively CuI (3mg, 0.016mmol), **70** (34mg, 0.325mmol), Et₃N (49mg, 0.487mmol) and Pd(PPh₃)₂Cl₂ (6mg, 0.008mmol). The mixture was then degassed for 1 minute under N₂ atmosphere and stirred at 90 °C for 1 hour under microwave. The reaction mixture was filtered and concentrated to give the crude product which was purified by prep-HPLC to give product **Compound 53** (4mg, 7%).
**LCMS:** *m*/*z,* 376.1 (M+H)⁺;
**¹H NMR** (400 MHz, CDCl3): δ 8.61 (s, 1H), 7.98 (s, 1H), 7.70-7.66 (m, 2H), 7.58-7.56 (m, 1H), 7.50-7.48 (m, 1H), 7.22-7.20 (m, 2H), 4.51-4.48 (m, 2H), 2.86-2.83(m, 2H), 2.40 (s, 6H).

### Example Compound 54

### Preparation of 3-(3-cyclopropyl-4-(pyridin-2-ylethynyl)-1H-pyrazol-1-yl)-5-fluorobenzonitrile:

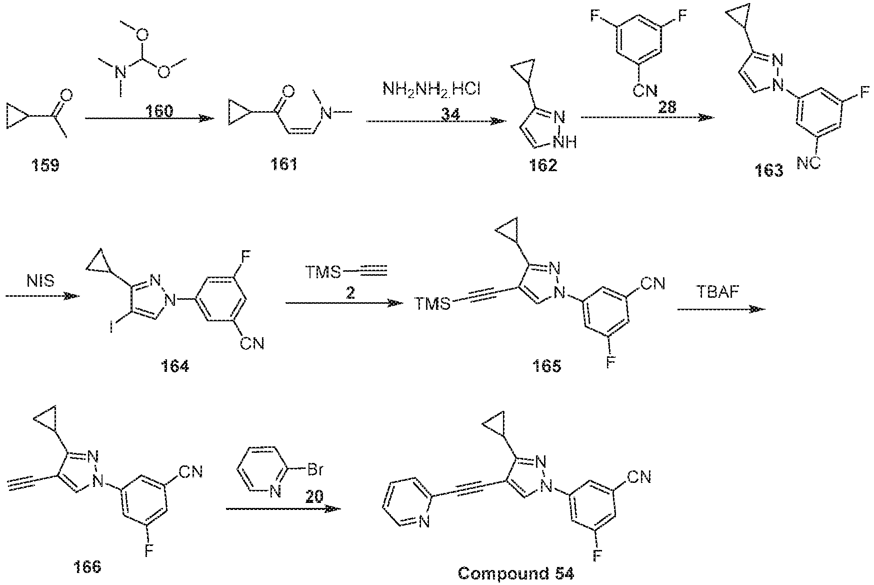

### Experimental section:

### Procedure for preparation of 161:

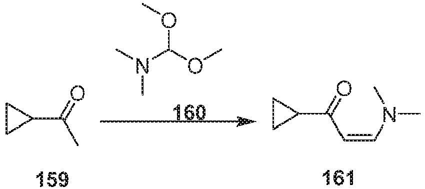

To a solution of **159** (700mg, 8.32mmol) in DMF (10mL) was added **160** (4.96g, 41.61mmol) dropwi se. The mixture was stirred at 110°C for 12 hours. The mixture was concentrated by vacuo to give product **161** (1.1g, crude).

### Procedure for preparation of 162:

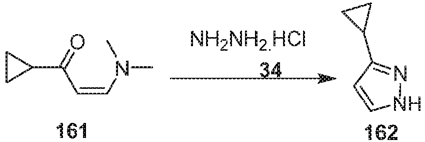

To a solution of **161** (600mg, 4.31mmol) in EtOH (30mL) was added **34** (2360mg, 34.48mmol). The mixture was stirred at refluxing temperature for 12 hours. The mixture was filtered and residue was concentrated by vacuo to give crude product **162** (700mg, crude).

### Procedure for preparation of 163:

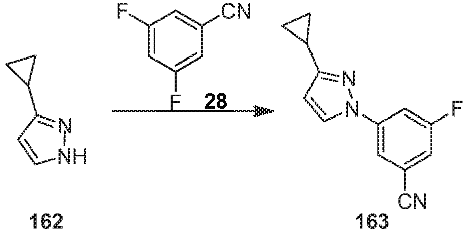

To a solution of **162** (1700mg, 6.47mmol) in DMF (20mL) was added 34 (1080mg, 7.77mmol) and Cs₂CO₃ (6330mg, 19.42mmol). The mixture was stirred at 70°C for 4 hours. The mixture was filtered and concentrated by vacuo to give the crude product which was purified by prep-TLC to give product **163** (350mg, 24%).
**LCMS:** *m*/*z,* 228.0 (M+H)⁺.

### Procedure for preparation of 164:

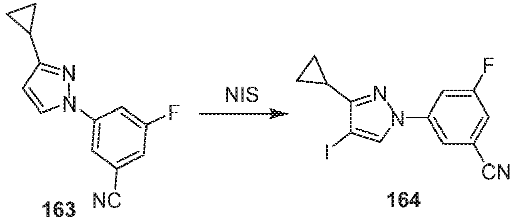

To a solution of **163** (350mg, 1.54mmol) in CHCl₃ (25mL) was added NIS (520mg, 2.31mmol). The mixture was stirred at 60°C for 5 hours. The mixture was quenched with water and extracted with DCM (2x10mL). The combined organic phase was concentrated under vacuo and the residue was purified by prep-TLC to give product **164** (400mg, 74%).

### Procedure for preparation of 165:

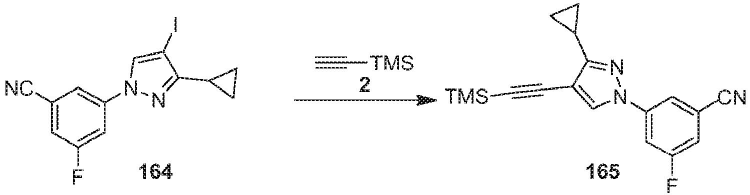

To a solution of **164** (450mg, 1.27mmol) in CH₃CN (10mL) was added successively CuI (24mg, 0.127mmol), **2** (250mg, 2.55mmol), Et₃N (387mg, 3.82mmol) and Pd(PPh₃)₂Cl₂ (45mg, 0.064mmol). The mixture was then degassed for 1 minute under N₂ atmosphere and stirred at 90°C for 1 hour under microwave. The reaction mixture was filtered and concentrated to give the crude product which was purified by prep-TLC to give **165** (400mg, 97%).
**LCMS:** *m*/*z,* 324.0 (M+H)⁺.

### Procedure for preparation of 166:

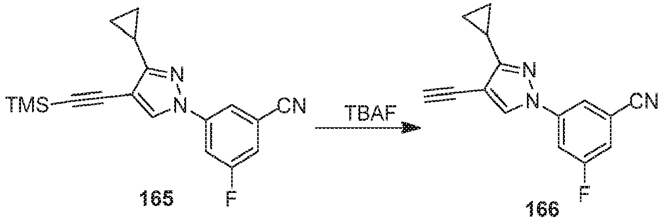

A solution of **165** (400mg, 1.24mmol) in THF (10mL). The solution was cooled to 0°C and TBAF (1.86mL, 1.86mmol) was added. The reaction mixture stirred at room temperature for 1 hour. The mixture was quenched with water and extracted with EA (3x10mL). The combined organic phase was concentrated by vacuo to give the crude product **166** (210mg, 68%).

### Procedure for preparation of Compound 54:

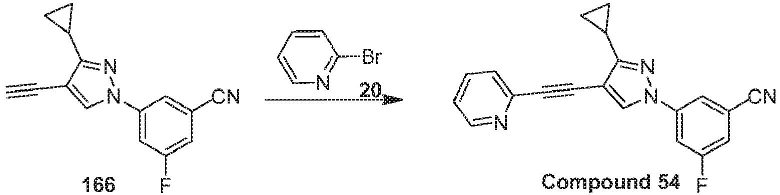

To a solution of compoud **166** (70mg, 0.279mmol) in THF (3mL) was added successively CuI (5mg, 0.028mmol), **20** (88mg, 0.557mmol), Et₃N (85mg, 0.836mmol) and Pd(PPh₃)₂Cl₂ (10mg, 0.014mmol). The mixture was then degassed for 1 minute under N₂ atmosphere and stirred at 90°C for 1 hour under microwave. The reaction mixture was filtered and concentrated to give the crude product which was purified by prep-HPLC to give the product **Compound 54** (6mg, 7%).
**LCMS:** *m*/*z,* 329.1 (M+H)⁺;
**¹H NMR** (400 MHz, CDCl3): δ 8.62 (s, 1H), 8.03 (s, 1H), 7.73-7.61 (m, 3H), 7.51-7.49 (m, 1H), 7.25-7.24 (m, 2H), 2.19-2.16 (m, 1H), 1.08-1.05 (m, 4H).

### Example Compound 55

### Preparation of 3-(3-cyclopropyl-4-(pyrimidin-2-ylethynyl)-1H-pyrazol-1-yl)-5-fluorobenzonitrile:

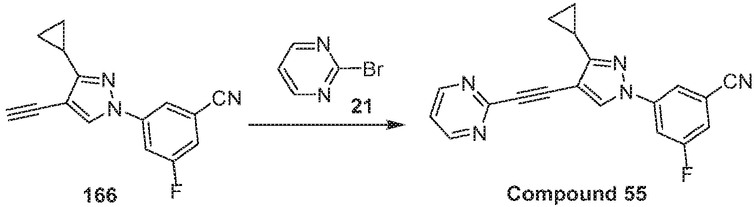

### Experimental section:

### Procedure for preparation of Compound 55:

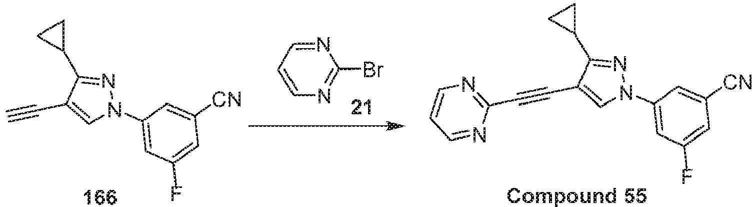

To a solution of **166** (70mg, 0.279mmol) in THF (3mL) was added successively CuI (5mg, 0.028mmol), **21** (89mg, 0.557mmol), Et₃N (85mg, 0.836mmol) and Pd(PPh₃)₂Cl₂ (10mg, 0.014mmol). The mixture was then degassed for 1 minute under N₂ atmosphere and stirred at 90°C for 1 hour under microwave. The reaction mixture was filtered and concentrated to give the crude product which was purified by prep-HPLC to give product **Compound 55** (3mg, 3%). **LCMS:** *m*/*z,* 330.1 (M+H)⁺;
**¹H NMR** (400 MHz, CDCl3): δ 8.76 (s, 1H), 8.75(s, 1H), 8.09 (s, 1H), 7.73 (s, 1H), 7.63-7.61 (m, 1H), 7.25-7.23 (m, 2H), 2.21 (m, 1H), 1.14-1.13 (m, 2H), 1.09-1.07(m, 2H).

### Example Compound 56

### Preparation of 2-((1-(-4-fluorophenyl)-5-(2-methoxyethoxy)-1H-pyrazol-4-yl) ethynyl)pyridine:

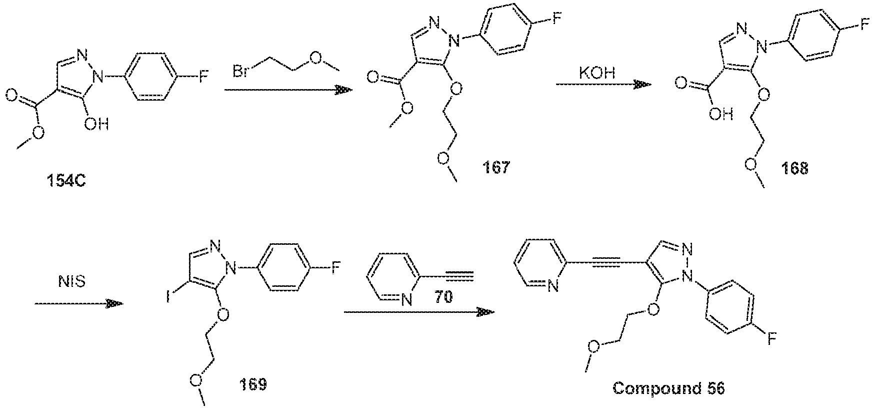

### Experimental section:

### Procedure for preparation of 167:

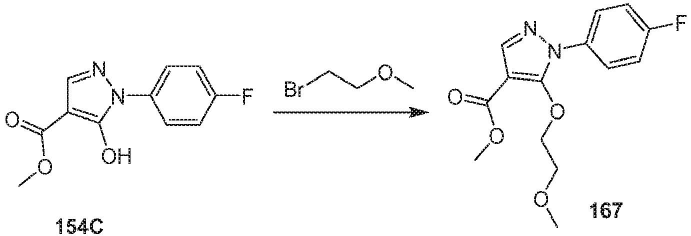

To a solution of **154C** (800mg,3.4mmol) and 1-bromo-2-methoxy-ethane(942mg, 6.8mmol) in DMF(15ml) was added NaI (508mg, 3.4mmol) and K₂CO₃(1400mg, 10.2mmol), the mixture was heated at 60°C for 3 hours. After cooling to room temperature, water (30ml) was added into above mixture with stirring at ice bath. Gradually, white solid was formed. It was filtrated and the residue was the product **167** (900mg, 90.3%).
**LCMS:** *m*/*z,* 295.0(M+H)⁺.

### Procedure for preparation of 168:

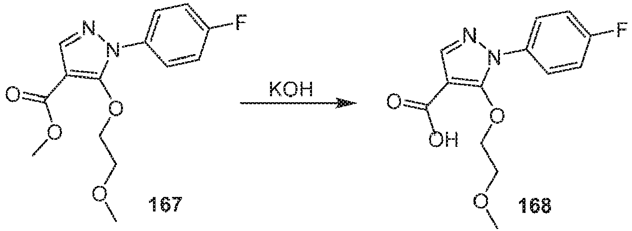

To a solution of **167** (0.9g, 3.1mmol) in MeOH (10mL) was added a solution of 35%KOH (5mL). Then it was heated to reflux for 2 hours. Most of the solvent was removed. It was adjust pH to 2 by a solution of HCl (6mol/L) with stirring at ice bath. Then white solid was gradually formed. It was filtrated, the residue was washed with water and evaporated to give the product **168** (750mg, 87.5%).
**LCMS:** *m*/*z,* 343.1 (M+H)⁺.

### Procedure for preparation of 169:

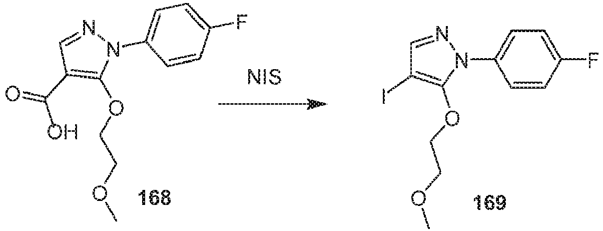

To a solution of **168** (350mg, 1.25mmol) in dry DMF (6mL) was added solid NIS(422mg, 1.87mmol) and NaHCO₃ (525mg, 6.24mmol). Then it was heated to 80°C for 24 hours. After cooling to room temperature, water (20mL) as added into it. It was extracted with EtOAc (20mL x3). The organics was collected, washed with brine (20mL), evaporated to give the crude product which was purified by chromatography to give the product **169** (200mg, 44.2%).
**LCMS:** *m*/*z,* 362.9(M+H)⁺;
**¹H NMR** (400 MHz, CDCl₃): δ3.48 (s, 3H), 3.79-3.82 (m, 2H), 7.46-7.48 (m, 2H), 7.09-7.14(m, 3H), 7.50-7.54(m, 2H), 7.72(s, 1H).

### Procedure for preparation of Compound 56:

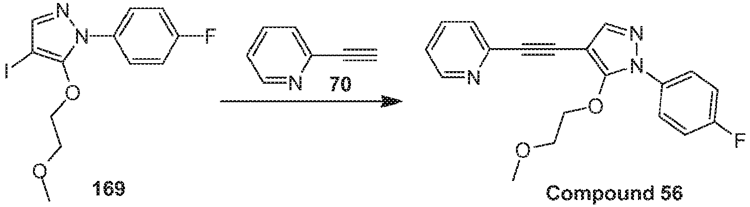

To a solution of **169** (60mg, 0.16mmol) in 4mL of degassed THF was added solid CuI (0.2mg, 0.008mmol), **70** (20mg, 020mmol), Et₃N (48mg, 0.48mmol) and Pd(PPh₃)₂Cl₂ (1.4mg, 0.008mmol) was added into it. The mixture was then degassed for 2 minutes under N₂ atmosphere and stirred at 90°C for 1 hour under microwave. Most of the solvent was removed. The residue was dissolved in EtOAc (30mL). The organic layer was washed with brine (2×20mL) and dried over anhydrous Na₂SO₄, filtered and evaporated to dryness on a rotary evaporator, which was purified by pre-HPLC to give the product **Compound 56**(15mg, 26.8%).
**LCMS:** *m*/*z,* 338.1(M+H)⁺;
**¹H NMR** (400 MHz, CDCl₃): δ3.47(s,3H), 3.82 (t, *J*=4.8Hz, 2H), 4.52 (t, *J*=4.8Hz, 2H), 7.11-7.13 (m, 2H), 7.13-7.15 (m, 1H), 7.49-7.57 (m, 3H), 7.65-7.67(m, 1H), 7.92 (s, 1H), 8.60 (s, 1H).

### Example Compound 57

### Preparation of 2-((1-(4-fluorophenyl)-5-(2-methoxyethoxy)-1H-pyrazol-4-yl) ethynyl)pyrimidine:

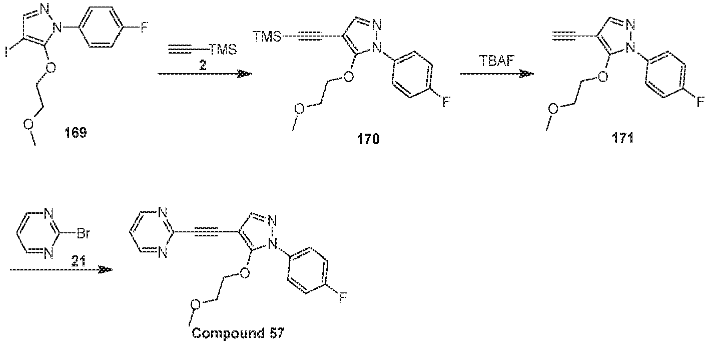

### Experimental section:

### Procedure for preparation of 170:

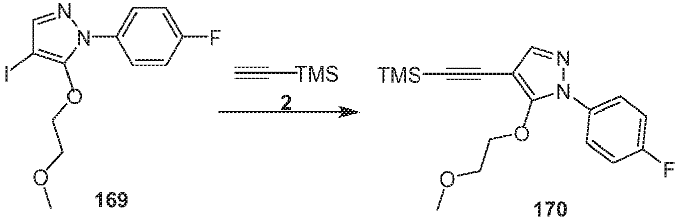

To a solution of compound **169** (108mg, 0.3mmol) in 4mL of degassed THF was added successively CuI (3mg, 0.015mmol), **2** (58.9mg, 0.6mmol), Et₃N (90mg, 0.9mmol) and Pd(PPh₃)₂Cl₂ (9mg, 0.015mmol). The mixture was then degassed for 2 minutes under N₂ atmosphere and stirred at 90°C for 1 hour under microwave. Most of the solvent was removed. The residue was dissolved in EtOAc (30mL). The organic layer was washed with brine (2×20mL) and dried over anhydrous Na₂SO₄, filtered and evaporated to dryness on a rotary evaporator, which was purified by flash chromatography to give product **170** (80mg, 80.2%).
**LCMS:** *m*/*z,* 333.0(M+H)⁺.

### Procedure for preparation of 171:

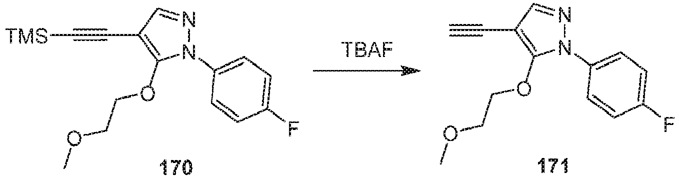

To a solution of **170** (80mg, 0.24mmol) in THF (4mL) was added a solution of TBAF-THF (0.36mL, 0.36mmol). The mixture was stirred at room temperature for 1 hour. TLC showed the reaction was complete. Most of the solvent was removed. The residue was dissolved in EtOAc (20mL). The organic layer was washed with brine (2×10mL) and dried over anhydrous Na₂SO₄, filtered and evaporated to give a crude product **171** (60mg, crude), which was directly used for next step.

### Procedure for preparation of Compound 57:

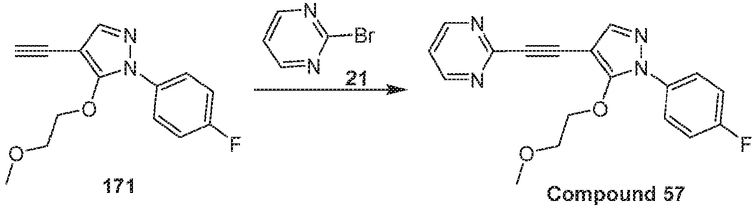

To a solution of **171** (60mg, 0.23mmol) in 4mL of degassed THF was added solid CuI (2mg, 0.01mmol), **21** (43.2mg, 0.27mmol), Et₃N (70mg, 0.69mmol) and Pd(PPh₃)₂Cl₂ (7mg, 0.01mmol). The mixture was then degassed for 2 minutes under N₂ atmosphere and stirred at 90°C for 1 hour under microwave. Most of the solvent was removed. The residue was dissolved in EtOAc (30mL). The organic layer was washed with brine (2×20mL) and dried over anhydrous Na₂SO₄, filtered and evaporated to dryness on a rotary evaporator, which was purified by pre-HPLC to give the product **Compound 57** (15mg, 19.2%).
LCMS: *m*/*z,* 339.0(M+H)⁺;
**¹H NMR** (400 MHz, CDCl₃): δ3.48(s,3H), 3.82-3.84 (m, 2H), 4.52-4.54 (m, 2H), 7.12-7.23 (m, 3H), 7.55-7.58 (m, 2H), 7.99-7.57 (s, 1H), 8.74 (d, *J*=5.2Hz, 2H).

### Example Compound 58

### Preparation of 2-((3-chloro-1-(4-(methylsulfonyl)phenyl)-1H-pyrazol-4-yl) ethynyl)pyridine:

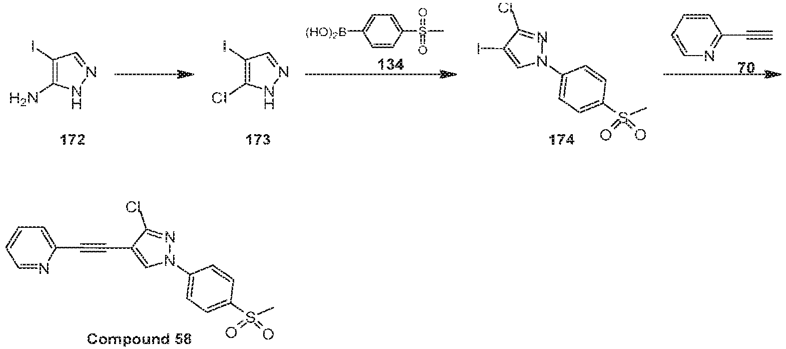

### Experimental section:

### Procedure forpreparation of 173:

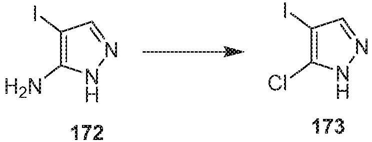

To a solution of **172** (2.0g, 10.0mmol) in 6.0mL of water, 10.0mL of concentrated HCl and 3.0 mL of 85% H₃PO₄ were added, the mixture was cooled to -5°C. A solution of NaNO₂ (0.7g, 10.0mmol) in 3.0mL of H₂O was added over 30minutes, the temperature was kept at -2°C. After stirred for 1 hour, the above mixture was added to a solution of CuCl (1.5g, 15.0mmol) in 10.0mL of concentrated HCl. The resultant mixture was heated to 60°C until the mixture was no gas goes off, extracted with chloroform. The organic phase was washed by water, brine, dried over sodium sulfate and concentrated to dryness. The residue was purified by flash chromatography column on silica gel to afford product **173** (160mg, 6.9%)

### Procedure for preparation of 174:

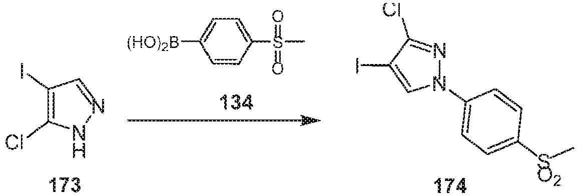

To a solution of **173** (500mg, 2.2mmol), **134** (525mg, 2.6mmol) and Cu(AcO)₂ (795mg, 4.4mmol)in dry DCM(5mL) was added pyridine (520mg, 6.6mmol) and pyridine 1-oxide (625 mg, 6.6mmol) at room temperature. After adding, it was stirred at room temperature under O₂ protected for 60 hours. It was filtrated and most of the solvent was removed. The residue was dissolved in EtOAc (60mL). The organic layer was washed with brine (2×30mL) and dried over anhydrous Na₂SO₄, filtered and evaporated to give a residue, which was purified by chromatography column to give the desired product **174** (120mg, 14.3%).
**LCMS:** *m*/*z,* 474.8(M+H)⁺.

### Procedure for preparation of Compound 58:

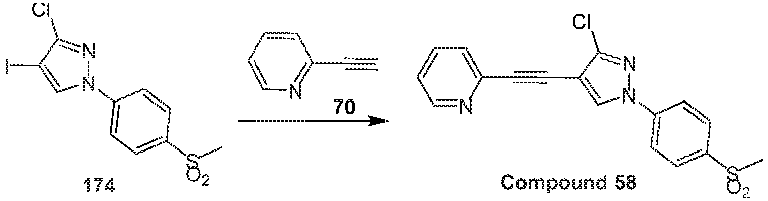

To a solution of **174** (60mg, 0.15mmol) in 4mL of degassed THF was added solid CuI (1.6mg, 0.008mmol), **70** (20mg, 0.2mmol), Et₃N (50mg, 0.18mmol) and Pd(PPh₃)₂Cl₂ (5.6mg, 0.008mmol) was added into it. The mixture was then degassed for 2 minutes under N₂ atmosphere and stirred at 90°C for 1 hour under microwave. Most of the solvent was removed. The residue was dissolved in EtOAc (30mL). The organic layer was washed with brine (2×20mL) and dried over anhydrous Na₂SO₄, filtered and evaporated to dryness on a rotary evaporator, which was purified by HPLC to give of the product **Compound 58** (10mg, 17.9%).
**LCMS:** *m*/*z,* 357.9(M+H)⁺;
**¹H NMR** (400 MHz, CDCl₃): δ3.09 (s, 3H), 7.27-7.29 (m, 1 H), 7.55-7.56 (m, 1 H),7.69-7.71 (m,1H), 7.86-7.88 (m,1H), 8.05 (m,1H), 8.07 (m,1H),8.20 (s, 2H), 8.63 (d, *J*=4.8Hz, 2H).

### Example Compound 59

### Preparation of 3-fluoro-5-(4-methyl-3-(pyridin-2-ylethynyl)-1H-pyrazol-1-yl) benzonitrile:

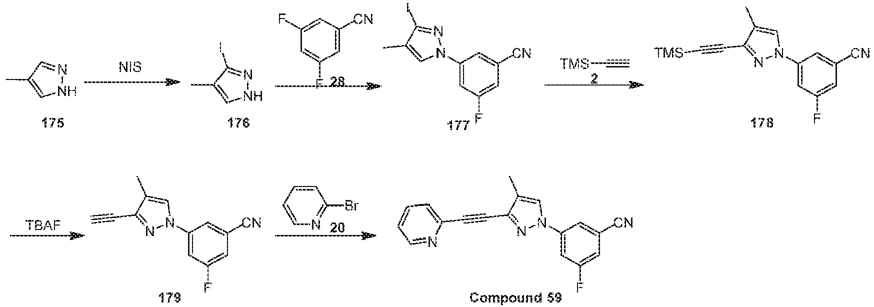

### Experimental section:

### Procedure for preparation of 176:

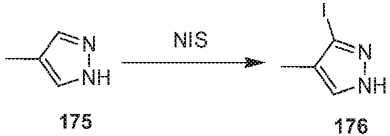

To a solution of **175** (0.5g, 6.1mmmol) in CHCl₃(20ml) was added NIS(1.64g, 7.31mmol). The mixture was stirred at 70°C for 2 hours, cooled to room temperature. The remaining aqueous layer was extracted with EtOAc(75mL) and the combined organic layers were dried (MgSO4), filtered and concentrated in vacuum to yield the title product **176** (0.6g, yield: 47%).
**LCMS:** *m*/*z,* 208.9(M+H)⁺.

### Procedure for preparation of 177:

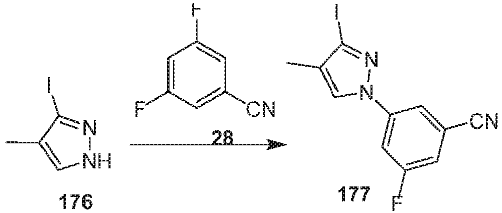

To a solution of **176** (0.5g, 2.4mmmol) in DMF(15ml) was added **28** (0.4g, 2.88mmol) and Cs₂CO₃(1.57, 4.81mmol). The mixture was stirred at 70°C for 2 hours, cooled to room temperature. The remaining aqueous layer was extracted with EtOAc(75mL) and the combined organic layers were dried (MgSO₄), filtered and concentrated in vacuum to yield the title product **177** (100mg, yield: 13%).
**LCMS:** *m*/*z,* 327.9(M+H)⁺.

### Procedure for preparation of 178:

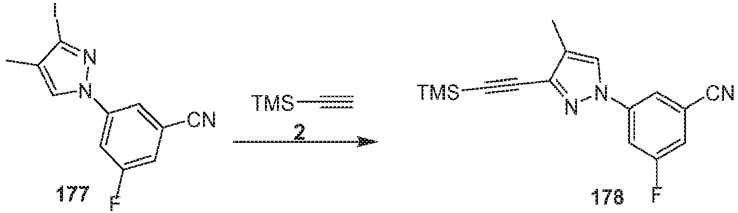

To a solution of **177** (250mg, 0.76mmol), **2** (150mg, 1,52mmol), CuI(15mg, 0.076mmol), Et₃N (230mg, 2.28mmol) in THF(15mL) was added Pd(PPh₃)₂Cl₂(53mg, 0.076mmol). The suspension was degassed under vacuum and purged with N₂ several times. The mixture was stirred at 90°C for 6 hours under N₂ atmosphere. The mixture was filtered and concentrated under vacuo. The residue was purified by prep-TLC to give the product **178** (180mg, yield: 79%). **1H NMR** (400 MHz, CDCl₃): δ7.49-7.43 (m, 3H),6.87-6.84 (m, 1 H), 1.89 (s, 3 H).

### Procedure for preparation of 179:

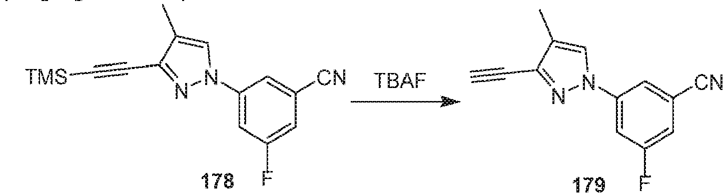

To a solution of **178** (180mg, 0.605mmol) in 5mL of degassed THF was added TBAF (0.91mL, 0.907mmol) at room temperature. After stirring 2 hours, the solvent was removed. It was extracted with DCM (20mL) and washed with brine (10mL). The organic layer was dried over Na₂SO₄ and purified by Prep-TLC to give title product **179** (136mg, yield: 100%).
**LCMS:** *m*/*z,* 226. 1(M+H)⁺.

### Procedure for preparation of Compound 59:

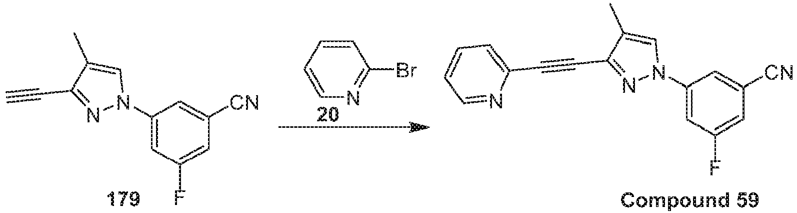

To a solution of **179** (70mg, 0.31mmol) and **20** (74mg, 0.47mmol), CuI (6mg, 0.031mmol), Et₃N (94mg, 0.93mmol) in degassed THF (5mL) was added Pd(PPh₃)₂Cl₂ (22mg, 0.031mmol). The suspension was degassed under vacuum and purged with N₂ several times. The mixture was stirred at 90°C for 6 hours under N₂ atmosphere. The mixture was filtered and concentrated under vacuum to afford crude product, which was purified by Prep-HPLC to give the product **Compound 59** (7mg, yield: 16%).
**LCMS:** *m*/*z,* 303.1(M+H)⁺;
**1H NMR** (400 MHz, CDCl3): δ8.53-8.61 (m, 1 H), 8.28 (s, 1 H), 8.05 (s, 1 H), 7.86-7.99 (m, 2 H), 7.65-7.74 (m, 1 H) 7.41-7.54 (m, 2 H),2.28 (s, 3 H).

### Example Compound 60

### Preparation of 3-fluoro-5-(4-methyl-3-(pyrimidin-2-ylethynyl)-1H-pyrazol-1-yl) benzonitrile:

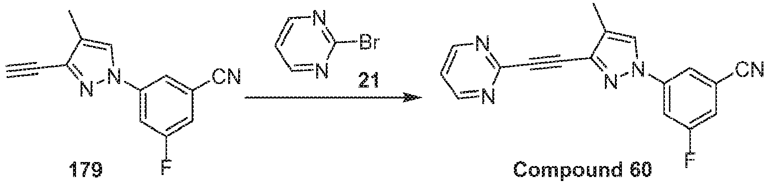

### Experimental section:

### Procedure for preparation of Compound 60:

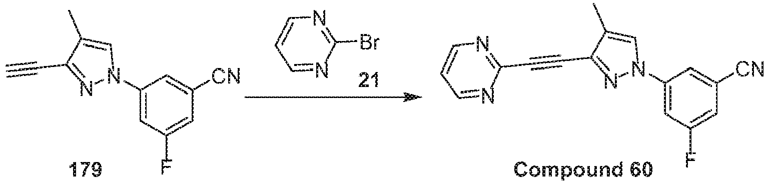

To a solution of **179** (70mg, 0.31mmol) and **21** (74mg, 0.47mmol), CuI (6mg, 0.031mmol), Et₃N (94mg, 0.93mmol) in degassed THF (5mL) was added Pd(PPh₃)₂Cl₂ (22mg, 0.031mmol). The suspension was degassed under vacuum and purged with N₂ several times. The mixture was stirred at 90°C for 6 hours under N₂ atmosphere. The mixture was filtered and concentrated under vacuum to afford crude product, which was purified by Prep-HPLC to give the product **Compound 60** (8mg, yield: 21%).
**LCMS:** *m*/*z,* 304.1(M+H)⁺;
**¹H NMR** (400 MHz, CDCl3): δ8.79-8.78(m, 1 H), 7.76-7.73 (m, 3H), 7.28-7.2 (m, 2 H), 2.29 (s, 3 H).

### Example Compound 61

### Preparation of 3-(4-chloro-3-(pyridin-2-ylethynyl)-1H-pyrazol-1-yl)-5-fluorobenzonitrile:

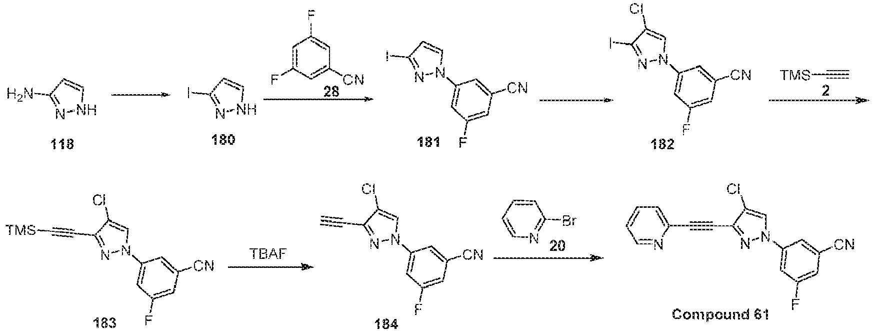

### Experimental section:

### Procedure for preparation of 180:

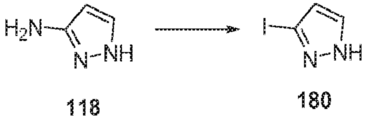

To a stirred suspension of **118** (2.00g, 24.07mmol) in concentrated HCl (32mL) was added a solution of NaNO₂ (3.32g, 48.14mmol) in water (5mL) over 5 minutes at 0°C. To the resulting orange reaction mixture was added a solution of KI (9.99g, 60.18mmol) in water (10mL) over 10 minutes. The reaction mixture was stirred at 0°C for 30 minutes and then kept at 28°C for another 2 hours. TLC showed the reaction was complete, then, solvent THF (30mL) was added, followed by water (30mL). The aqueous mixture was extracted with EtOAc (3 x80mL) and the combined organic extracts were washed with Na₂S₂O₃ (2 x40mL), dried over Na₂SO₄, filtered and concentrated in vacuum to afford product **180** (2.00g, crude), the crude product was used directly for the next step without purification.
LCMS: *m*/*z,* 194.9(M+H)⁺.

### Procedure for preparation of 181:

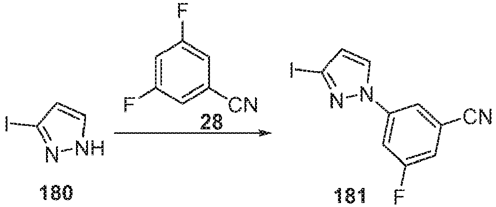

To a mixture of compound 168(2.00g, 10.31mmol) and 16 (1.72g, 12.37mmol) in DMF (20mL), was added Cs₂CO₃(6.72g, 20.62mmol) in one portion at 29°C. The mixture was heated to 70°C and stirred for 2 hours. LCMS showed the reaction was completed. The mixture was cooled to 29°C and concentrated in reduced pressure at 40°C. The residue was poured into water (40mL) and stirred for 5 minutes. The aqueous phase was extracted with EtOAc(80mLx2). The combined organic phase was washed with saturated brine (20mL), dried with anhydrous Na₂SO₄, filtered and concentrated in vacuum. The residue was purified by silica gel chromatography to afford product 169 (1.50g, yield: 46.47%).
**¹H NMR** (400 MHz, CDCl₃): δ7.62-7.84 (m, 3H), 7.28∼7.26 (m, 1 H), 6.68∼6.67 (m, 1 H).

### Procedure for preparation of 182:

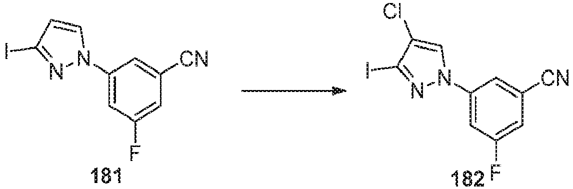

The mixture of **181** (500.00mg, 1.60mmol) and NCS (319.89mg, 2.40mmol) in a 5mL single-necked round bottom flask, was stirred at 120°C for 1 hour. Then cooled down to 29°C. LCMS showed the starting material was consumed completely and the desired compound was detected. The residue was partitioned between ethyl acetate (100mL) and H₂O (50mL). The organic phase was washed with saturated brine (20mL), dried with anhydrous Na₂SO₄, filtered and concentrated in vacuum to afford product **182** (200.00mg, crude), which was used directly for the next step without purification
**LCMS:** *m*/*z,* 347.9(M+H)⁺.

### Procedure for preparation of 183:

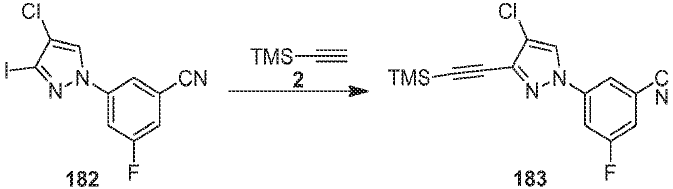

Mixture of **182** (600.00mg, 1.73mmol), **2** (254.38mg, 2.59mmol), CuI (16.47mg, 86.50umol) and Et₃N (524.13mg, 5.18mmol) and Pd(PPh₃)₂Cl₂ (60.71mg, 86.50umol, 0.05Eq) were taken up into a microwave tube. The sealed tube was heated at 95°C for 1 hour under microwave. LCMS showed the starting material was consumed completely and the desired compound was detected. Ethyl acetate (100mL) and H₂O (20mL) were added into the mixture, the separated organic layer was washed with brine (30mL), dried over Na₂SO₄ and evaporated to dryness, which was purified by silica gel chromatography to afford product **183** (300.00 mg, yield: 54.67%).
**LCMS:** *m*/*z,* 318.0(M+H)⁺.

### Procedure for preparation of 184:

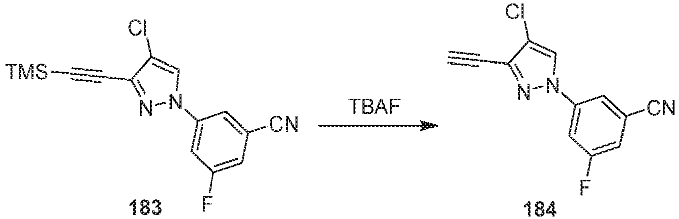

To a solution of **183** (300.00mg, 943.93umol) in THF (5mL) was added TBAF (1 M, 1.42mL) at 0°C. The reaction mixture was stirred at 0°C for 0.5 hour and then kept at room temperature (28°C) for another 1 hour. LCMS showed the starting material was consumed completely and the title compound was detected. The reaction mixture was concentrated to dryness, which was partitioned between ethyl acetate (100mL) and H₂O (50mL). The separated organic layer was washed with saturated brine(20mL), dried over Na₂SO₄ and evaporated in vacuo to afford **184** (200.00 mg, crude), which was used directly for the next step without purification.
**LCMS:** *m*/*z,* 245.9(M+H)⁺.

### Procedure for preparation of Compound 61:

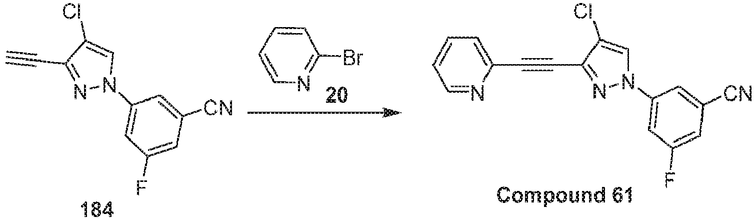

A mixture of **184** (200.00mg, 732.78umol), **20** (150.51mg, 952.61umol), CuI (6.98mg, 36.64umol), Et₃N (222.45mg, 2.20mmol) and Pd(PPh₃)₂Cl₂ (25.72mg, 36.64umol) were taken up into a microwave tube in THF (8mL). The sealed tube was heated at 95°C for 1 hour under microwave. LCMS showed the starting material was consumed completely and the title compound was detected. After cooling to 28°C, ethyl acetate (80mL) and saturated aqueous of -Na₂CO₃ (20mL) were added. The aqueous layer was extracted with ethyl acetate (40mLx2). The combined organic layers were washed with brine (30mL), dried over Na₂SO₄, concentrated in vacuum to give the crude product, which was pre-purified by column chromatography followed by prep-HPLC purification to afford product **Compound 61**(50.00mg, yield: 21.14%).
**LCMS:** *m*/*z,* 322.9(M+H)⁺;
**¹H NMR** (400 MHz, CDCl₃): δ8.65-8.72 (m, 1 H), 7.99 (s, 1 H), 7.79-7.82 (m, 1 H), 7.70-7.77 (m, 2 H), 7.61-7.66 (m, 1 H), 7.29-7.36 (m, 2 H).

### Example Compound 62

### Preparation of 5-fluoro-2-(5-methoxy-4-(pyridin-2-ylethynyl)-1H-pyrazol-1-yl) pyridine:

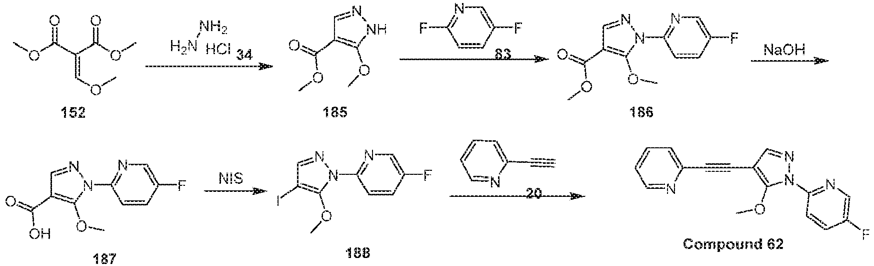

### Experimental section:

### Procedure for preparation of 185:

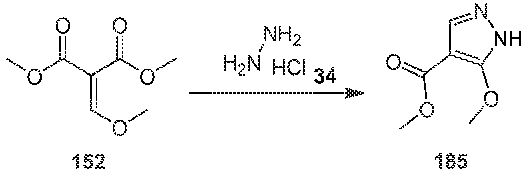

To a sloution of **152** (5.00g, 28.71 mmol) in MeOH (50 mL) was added **34** (1.97 g, 28.71 mmol). The mixture was stirred at 70 °C for 18 hours. Then the reaction mixture was concentrated and the obtained residue was treated with saturated NaHCO₃ solution (100ml), extracted with DCM (3 x 50ml). The combined organic layer was dried over Na₂SO₄, concentrated to give the product **185** (2.0 g, yield: 44%).
**LCMS:** *m*/*z* 157(M+H)⁺;
**¹H NMR** (400 MHz, CDCl3): δ 1.02-9.68 (br, 1H), 7.89 (s, 1H), 4.00 (s, 3H), 3.83 (s, 3H).

### Procedure for preparation of 186:

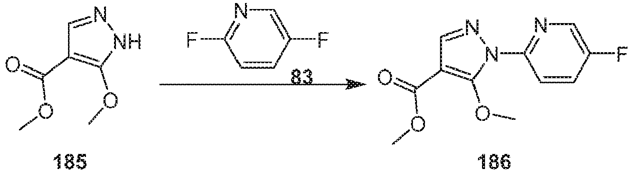

To a mixture of **185** (600.00 mg, 3.80 mmol) and **83** (656.03 mg, 5.70 mmol) in DMF (20mL) was added Cs₂CO₃ (2.48 g, 7.60 mmol) in one portion. The mixture was stirred at 110 °C for 1 hour. LCMS showed the reaction was completed. The mixture was cooled to 25°C and concentrated in reduced pressure at 70°C. The residue was poured into water (50mL) and stirred for 2 minutes. The aqueous phase was extracted with ethyl acetate (30mLx3). The combined organic phase was washed with saturated brine (30mLx2), dried with anhydrous Na₂SO₄, filtered and concentrated in vacuum. The residue was purified by silica gel chromatography to afford **186** (550.00 mg, yield: 57.62%).
**¹H NMR** (400 MHz, CDCl3): δ 8.79 (s, 1H), 8.23 (d, *J*=2.8Hz, 1H), 7.86-7.83 (m, 1H), 7.55-7.50 (m, 1H), 4.07 (s, 3H), 3.84 (s, 1.3H).

### Procedure for preparation of 187:

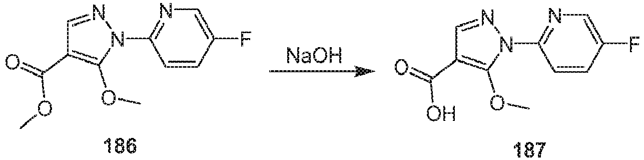

To a solution of 186 (300.00mg, 1.19mmol) in MeOH (20mL) was added NaOH (238.00mg, 5.95mmol) and H₂O (5mL). The mixture was stirred at 28°C for 18 hours. LCMS showed the reaction was completed. The mixture was concentrated in reduced pressure at 50°C. The residue was poured into water (10mL) and adjusted to pH= 5~6 with 1 N HCl solution. The aqueous phase was extracted with ethyl acetate (20mLx3). The combined organic phase was washed with saturated brine (20mLx2), dried with anhydrous Na₂SO₄, filtered and concentrated in vacuum to afford **187** (240.00mg, 85.03% yield).
**LCMS:** *m*/*z* 238 (M+H)⁺.

### Procedure for preparation of 188:

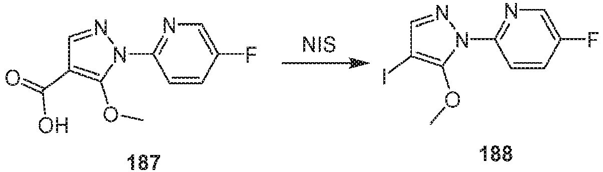

To a mixture of 187 (300.00mg, 1.26mmol) and NIS (569.11mg, 2.53mmol) in DMF (10 mL) was added NaHCO₃ (850.05mg, 10.12mmol) in one portion at 25°C under N₂ atmosphere. The mixture was stirred at 80 °C for 24 hours. LCMS showed the reaction was completed. The mixture was cooled to 25°C and concentrated in reduced pressure at 60°C. The residue was poured into water (30 mL) and stirred for 10 minutes. The aqueous phase was extracted with ethyl acetate (30 mLx3). The combined organic phase was washed with saturated brine (20mLx2), dried with anhydrous Na₂SO₄, filtered and concentrated in vacuo. The residue was purified by silica prep-TLC to afford product **188** (80.00mg, 19.90% yield).
**LCMS:** *m*/*z* 320 (M+H)⁺.

### Procedure for preparation of Compound 62:

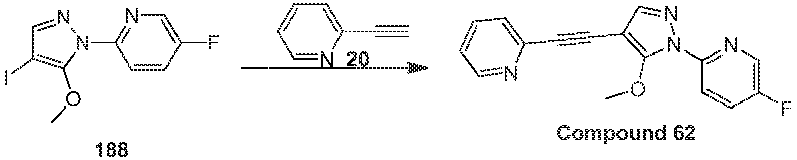

Amixture of **188** (80.00mg, 250.73umol), 20 (51.71mg, 501.46umol), CuI (4.78mg, 25.07umol), TEA (50.74mg, 501.46umol) and Pd(PPh₃)₂Cl₂ (17.60mg, 25.07 umol) were taken up into a microwave tube in THF (5mL). The sealed tube was heated at 90°C for 1hour under microwave. LCMS showed the starting material was consumed. After cooling to 25 °C, ethyl acetate (20mL) and water (20mL) were added. The aqueous layer was extracted with ethyl acetate (20mLx2). The combined organic layers were washed with brine (20mL), dried over Na₂SO₄, concentrated in vacuo to give the crude product, which was purified by prep-HPLC to give product **Compound 62** (22.00mg, 29.82% yield).
**LCMS:** *m*/*z* 295 (M+H)⁺;
**¹H NMR:** (400 MHz, CDCl3); δ 8.60 (s, 1H), 8.51 (s, 1H), 8.21 (d, *J*=2.8Hz, 1H), 7.83-7.80 (m, 1H), 7.68-7.65 (m, 1H), 7.53-7.48 (m, 2H), 7.25-7.24 (m, 1H), 4.06 (s, 3H);

### Example Compound 63

### Preparation of 5-fluoro-2-(3-methyl-4-(pyridin-2-ylethynyl)-1H-pyrazol-1-yl) pyridine:

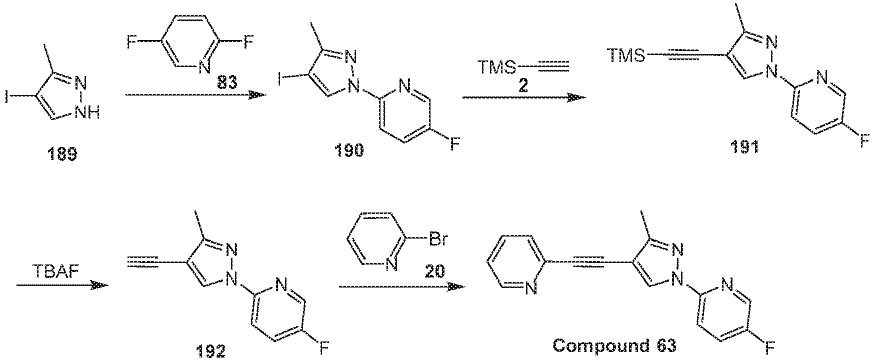

### Experimental section:

### Procedure for preparation of 190:

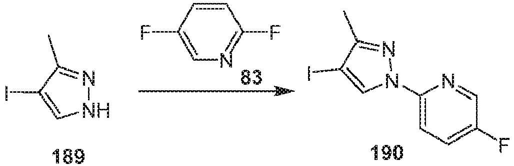

To a solution of **189** (100.00mg, 384.62umol) and 71(44.26mg, 384.62umol) in DMF (3mL) was added solid Cs₂CO₃ (125.32mg, 384.6umol) at 25°C. It was stirred at 80-90°C for 6 hours. After cooling, water (6mL) was added into the mixture with stirring at ice bath slowly. Gradually, white solid was formed. It was filtrated and the residue was purified by TLC to afford the product **190** (50.00mg, yield: 42.89%).
**LCMS:** *m*/*z,* 304.0(M+H)⁺;
**¹H NMR** (400 MHz, CDCl₃): δ2.35 (s, 3 H), 7.52- 7.53(m, 1 H), 7.87- 7.90 (m, 2 H), 8.21(d, *J*= 2.8Hz, 1 H), 8.47 (s, 2 H).

### Procedure for preparation of 191:

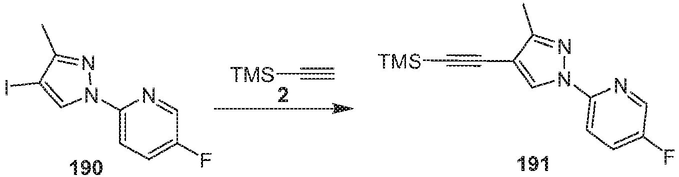

To a solution of **190** (50.00mg, 141.88umol) and 2 (30.81 mg, 313.64umol) in 4mL of degassed THF was added a solution of Et₃N (47.61mg, 470.46umol), solid CuI (1.35mg, 7.09umol)and Pd(PPh3)2Cl2 (4.98mg, 7.09umol) at 25°C.The mixture was then degassed for 2 minutes under N₂ atmosphere and stirred at 90°C for 1 hou under microwave. Most of the solvent was removed. The residue was dissolved in EtOAc (30mL) and filtrated. The organic layer was washed with brine (2×20mL),dried over anhydrous Na₂SO₄, filtered and evaporated to give the product **191** (43.00 mg, crude).
**LCMS:** *m*/*z,* 274.0(M+H)⁺.

### Procedure for preparation of 180:

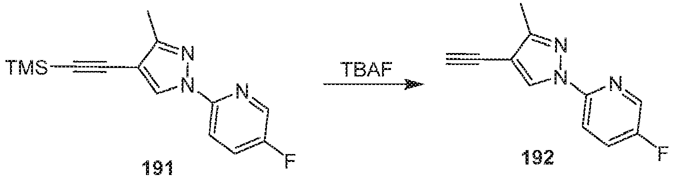

To a solution of **191** (43.00mg, 157.29umol) in THF (2mL) was added a solution of TBAF (1M in THF) in one portion at 20°C. It was stirred for 1 hour at the same condition. TLC showed the reaction was complete. Most of the solvent was removed. The residue was dissolved in EtOAc (20mL). The organic layer was washed with brine (2×10mL) and dried over anhydrous Na₂SO₄, filtered and evaporated to give the crude product **192** (30.00 mg, crude) which was directly used for next step.

### Procedure for preparation of Compound 63:

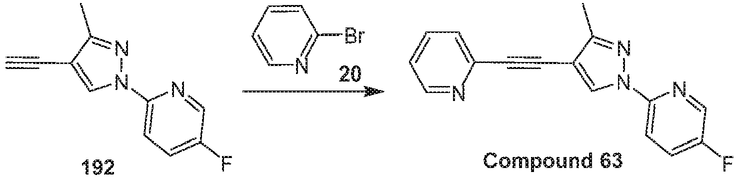

To a solution **192** (30.00mg, 149.11umol) and **20** (28.27mg, 178.93umol) in 4mL of degassed THF was added solid CuI (1.42mg, 7.46umol), Pd(PPh3)2Cl2 (5.23mg, 7.46umol) and Et₃N (45.26mg, 447.32umol) into it at 25°C. The mixture was then degassed for 2 minutes under N₂ atmosphere and stirred at 90°C for 1 hour under microwave. LCMS showed the reaction was complete. Most of the solvent was removed. The residue was dissolved in EtOAc(30mL). The organic layer was washed with brine (2×20mL) and dried over anhydrous Na₂SO₄, filtered and evaporated to give a residue, which was purified by prep-HPLC to give product **Compound 63**(10.00mg, yield: 23.93%).
**LCMS:** *m*/*z,* 279.0(M+H)⁺;
**¹H NMR** (400 MHz, CDCl₃): δ2.48 (S, 3 H), 7.21-7.27 (m, 1 H), 7.48- 7.59 (m, 2 H), 7.69 (m, 1 H) 7.93 (dd, *J₁*=3.79Hz, *J₂*=8.93 Hz, 1 H), 8.26 (d, *J*=2.93 Hz, 1 H), 8.57-8.67 (m, 2 H).

### Example Compound 64

### Preparation of 5-fluoro-2-(3-(2-methoxyethoxy)-4-(pyridin-2-ylethynyl)-1H-pyrazol-1-yl)pyridine:

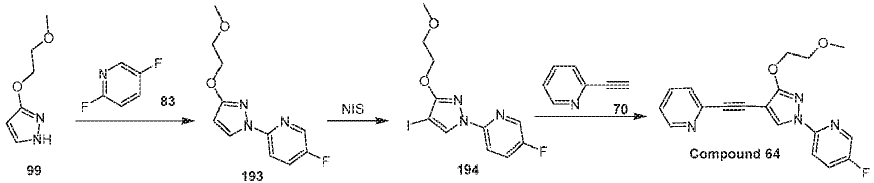

### Experimental section:

### Procedure for preparation of 193:

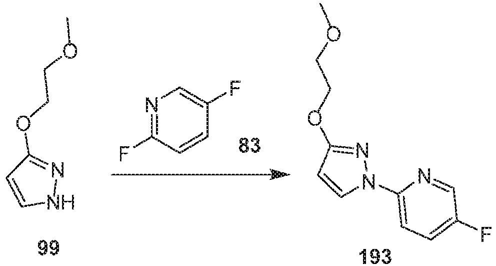

To a mixture of **99** (500.00 mg, 3.52mmol) and **83** (486.10 mg, 4.22mmol) in DMF (10mL) was added Cs₂CO₃ (3.44g, 10.56mmol) in one portion at room temperature under N₂ atmosphere. The mixture was heated to 70°C and stirred for 2 hours. LCMS showed the starting material was consumed completely and the title compound was detected. The mixture was cooled to room temperature and concentrated in reduced pressure at 40°C. The residue was poured into water (15mL) and stirred for 5 minutes. The aqueous phase was extracted with ethyl acetate (30mLx2). The combined organic phase was washed with saturated brine (20mL), dried with anhydrous Na₂SO₄, filtered and concentrated in vacuum. The residue was purified by silica gel chromatography to afford product **193** (400.00mg, yield: 47.90%).
**¹H NMR** (400 MHz, CDCl₃): δ8.16-8.24 (m, 1 H), 8.02-8.14 (m, 1 H), 7.64-7.76 (m, 1 H), 7.34-7.48 (m, 1 H), 5.88 (d, *J*=2.65 Hz, 1 H), 4.30-4.43 (m, 2 H), 3.65-3.76 (m, 2 H), 3.39 (s, 3 H).

### Procedure for preparation of 194:

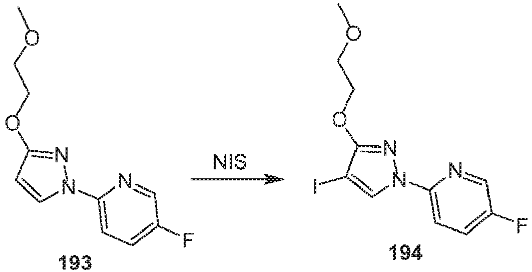

To a solution of **193** (400.00mg, 1.69mmol) in CHCl₃ (20mL) was added NIS (493.15mg, 2.19mmol) in one portion at room temperature. The mixture was heated to 70°C and stirred for 5 hours. TLC showed the starting material was consumed completely. The mixture was cooled to room temperature and concentrated in reduced pressure to afford dryness, which was partitioned between ethyl acetate (100mL) and H₂O (50mL). The separated organic layer was washed with saturated brine (20mL), dried over Na₂SO₄ and evaporated in vacuum to afford the crude product, which was purified by silica gel chromatography to afford product **194** (400.00mg, yield: 65.18%).
**¹H NMR** (400 MHz, CDCl₃): δ8.36 (s, 1 H), 8.14-8.21 (m, 1 H), 7.69-7.80 (m, 1 H), 7.44-7.55 (m, 1 H), 4.42-4.53 (m, 2 H), 3.75-3.88 (m, 2 H), 3.48 (s, 3 H).

### Procedure for preparation of Compound 64:

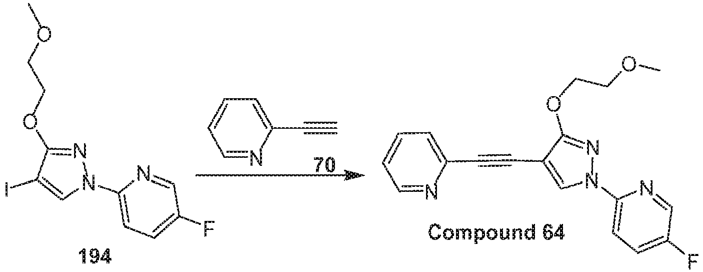

A mixture of **194** (200.00mg, 550.77umol), 70 (85.19 mg, 826.16umol), Pd(PPh₃)₂Cl₂ (19.33mg, 27.54umol), CuI (5.24mg, 27.54umol) and Et₃N (167.20mg, 1.65mmol) in THF (8mL) was de-gassed and were taken up into a microwave tube. The sealed tube was heated at 95°C for 1 hour under microwave. LCMS showed the starting material was consumed completely and the desired compound was detected. The mixture was partitioned between ethyl acetate (100mL) and H₂O (20mL), the separated organic layer was washed with brine (30mL), dried over Na₂SO₄ and evaporated to dryness, which was purified by prep-HPLC to afford product **Compound 64** (20.00mg, yield: 10.61%).
**LCMS:** *m*/*z,* 339.1(M+H)⁺;
**¹H NMR** (400 MHz, CDCl₃): 58.53-.67 (m, 1 H), 8.43-.53 (m, 1 H), 8.15-.24 (m, 1 H), 7.74-.83 (m, 1 H), 7.59-.70 (m, 1 H), 7.43-7.54 (m, 2 H), 7.16-.23 (m, 1 H), 4.47-.54 (m, 2 H), 3.82 (d, *J*=4.89 Hz, 2 H), 3.46 (s, 3 H).

### Example Compound 65

### Preparation of 2-((1-(4-(trifluoromethoxy)phenyl)-1H-pyrazol-4-yl)ethynyl)pyrazine:

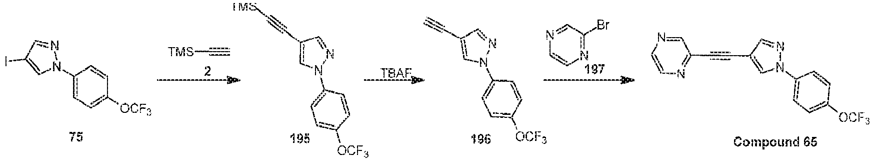

### Experimental section:

### Procedure for preparation of 195

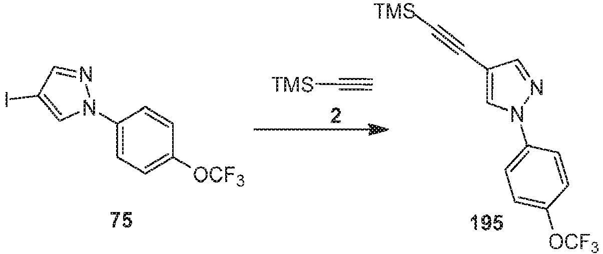

To a mixture of **75** (600.00mg, 1.75mmol) and 2 (344.46mg, 3.51mmol) in THF (10mL), was added solid CuI (16.70mg, 87.68umol), Et₃N (532.31mg, 5.26mmol) and Pd(PPh₃)₂Cl₂ (61.54mg, 87.68umol) in one portion at room temperature under N₂ atmosphere. The mixture was stirred at 90°C for 1 hour in microwave. LCMS showed the reaction was completed. The mixture was cooled to room temperature and concentrated in reduced pressure. The residue was dissolved in ethyl acetate (40mL), and washed with sattirated brine (20mLx2), dried with anhydrous Na₂SO₄, filtered and concentrated in vacuo to afford product **195** (500.00mg, crude).
**LCMS:** *m*/*z,* 325.0(M+H)⁺.

### Procedure for preparation of 196:

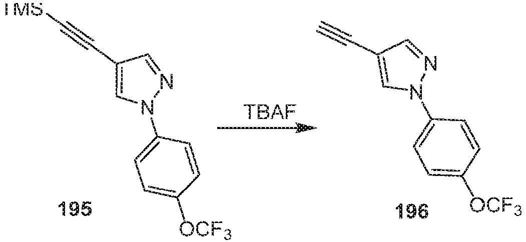

To a solution of **195** (500.00 mg, 1.54mmol) in THF (10mL), was added TBAF (THF) (1M, 2.31mL) dropwise at room temperature under N₂ atmosphere. The mixture was stirred at room temperature for 1 hour. TLC showed the reaction was completed. The mixture was concentrated in reduced pressure. The residue was dissolved in ethyl acetate (40mL). It was washed with saturated brine (20mLx2), dried with anhydrous Na₂SO₄, filtered and concentrated in vacuum. The residue was purified by silica gel chromatography to afford product **196** (350.00mg, yield: 90.12%).
**¹H NMR** (400 MHz, CDCl₃): δ3.12 (s, 1 H), 7.33 (d, *J*=8.8 Hz, 2 H), 7.70 (d, *J*=8.8 Hz, 2 H), 7.83 (s, 1 H) 8.06 (s, 1 H).

### Procedure for preparation of Compound 65:

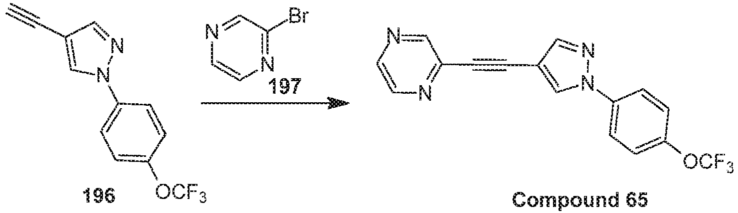

A mixture of **196** (70.00mg, 277.57umol), **197** (44.13mg, 277.57umol), CuI (2.64mg, 13.88umol), Et₃N (84.26mg, 832.71umol) and Pd(PPh₃)₂Cl₂ (9.74mg, 13.88umol) were taken up into a microwave tube in THF (4mL). The sealed tube was heated at 90°C for 1 hour under microwave. TLC showed the starting material was consumed. After cooling to room temperature, H₂O (10mL) were added. The aqueous layer was extracted with ethyl acetate (15mLx3). The combined organic layers were washed with brine (15mL), dried over Na₂SO₄, concentrated in vacuo to give the crude product which was purified by prep-HPLC to afford product **Compound 65** (18.00mg, yield: 19.48%).
**LCMS:** *m*/*z,* 330.9(M+H)⁺;
**¹H NMR** (400 MHz, CDCl₃): δ7.36 (d, *J*=8.4 Hz, 2 H), 7.74 (d, *J*=9.2 Hz, 2 H), 7.96 (s, 1 H), 8.20 (s, 1 H), 8.50 (d, *J*=2.8 Hz, 1 H), 8.59(s, 1 H), 8.75 (s, 1 H).

### Example Compound 66

### Preparation of 3-fluoro-5-(4-(pyridin-2-ylethynyl)-1H-pyrazol-1-yl)benzonitrile:

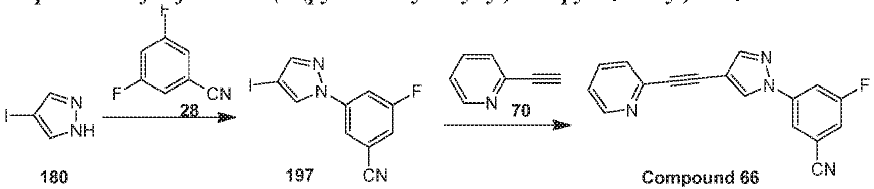

### Experimental section:

### Procedure for preparation of 197:

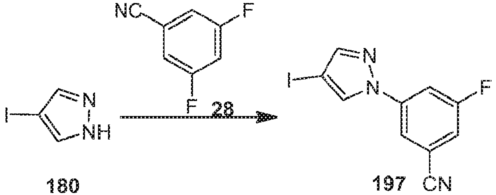

To a mixture of **180** (193.97mg, 1.00mmol) and **28** (139.10mg, 1.00mmol) in DMF (5mL), was added Cs₂CO₃ (325.82mg, 1.00mmol) in one portion at room temperature. The mixture was then heated to 80°C for 1.5 hours under N₂ atmosphere. TLC showed the reaction was completed. The mixture was cooled to room temperature and was then poured into water (15mL) slowly. A white solid precipitated out from the mixture, filtered off. The solid was dried to afford product 197 (300.00 mg, yield: 67.08%).
**LCMS:** *m*/*z,* 313.9 (M+H)⁺.

### Procedure for preparation of Compound 66:

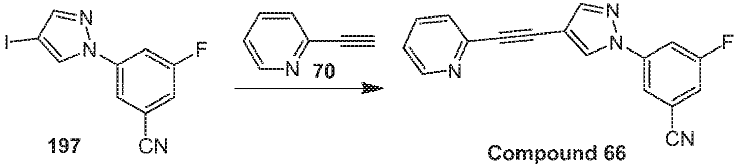

Mixtuer of **197** (100.00mg, 319.42umol), **70** (32.94mg, 319.42umol), CuI (3.04mg, 15.97umol), Et₃N (96.97mg, 958.26umol) and Pd(PPh₃)₂Cl₂ (11.21mg, 15.97umol) were taken up into a microwave tube in THF (3mL). The sealed tube was heated at 90°C for 1 hour under microwave. LCMS showed only the desired product. After cooling to room temperature, H₂O (10mL) were added. The aqueous layer was extracted with ethyl acetate (15mLx3). The combined organic layers were washed with brine (15mL), dried over Na₂SO₄, concentrated in vacuo to give the crude product which was purified by prep-HPLC to afford product **Compound 66** (34.00mg, yield: 35.70%).
**LCMS:** *m*/*z,* 289.0 (M+H)⁺;
**¹H NMR** (400 MHz, CDCl₃): δ7.30-7.49 (m, 2 H), 7.51-7.68 (m, 1H), 7.70-7.74 (m, 1H), 7.81(s, 1 H), 7.91(s, 1 H), 8.16(s, 1 H), 8.61 (d, *J*=4.4. Hz, 1 H).

### Example Compound 67

### Preparation of 5-fluoro-2-(3-(pyridin-2-ylethynyl)-1H-pyrazol-1-yl)pyridine:

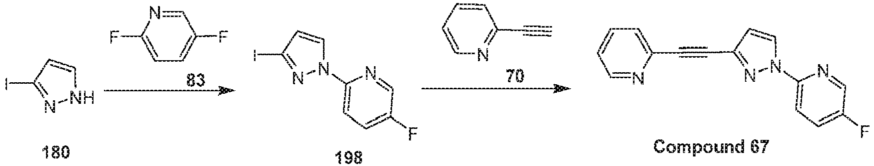

### Experimental section:

### Procedure for preparation of 198:

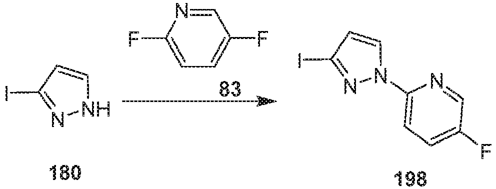

To a solution of **180** (100.00mg, 515.54 umol) and **83** (71.19mg, 618.65umol) in DMF (3mL) was added solid Cs₂CO₃ (251.96mg, 773.31umol) in one charge at room temperature. It was stirred at 80-90°C for 6 hours. After cooling, water (10mL) was added into the mixture with stirring at ice bath slowly. Gradually, solid was formed. It was filtrated. The residue was the product **198** (70.00 mg, crude) which was used for next step directly.
**LCMS:** *m*/*z,* 290.0 (M+H)⁺;
**¹HNMR** (400 MHz, CDCl3): δ6.62 (d, *J*=2.8 Hz, 1 H), 7.53-7.58 (m, 1 H), 7.97-8.00 (m, 1 H), 8.24 (d, *J*=2.4 Hz, 1 H), 8.31 (d, *J*=2.4 Hz, 1 H).

### Procedure for preparation of Compound 67:

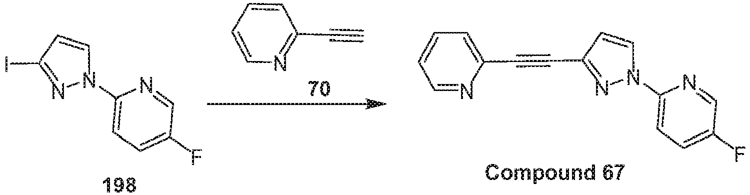

Mixture of **198** (60.00mg, 207.58umol), **70** (32.11mg, 311.36umol), CuI (3.95mg, 20.76 umol), TEA (63.01mg, 622.73umol), and Pd(PPh₃)₂Cl₂ (7.28mg, 10.38umol) were taken up into a microwave tube in THF (3mL). The sealed tube was heated at 90°C for 1 hour under microwave. LCMS showed the starting material was consumed. After cooling to room temperature, ethyl acetate (5mL) and H₂O (3mL) were added. The aqueous layer was extracted with ethyl acetate (5 mLx2). The combined organic layers were washed with brine (5mL), dried over Na₂SO₄, concentrated in vacuo to give the crude product, which was purified by prep-HPLC to afford product **Compound 67** (16.00 mg, yield: 28.91%).
**LCMS:** *m*/*z,* 265.0 (M+H)⁺;
**¹HNMR** (400MHz, CDCl₃): δ6.71 (d, *J*=2.4 Hz, 1 H), 7.25-7.27 (m, 1 H), 7.56-7.60 (m, 2 H), 7.67-7.72 (m, 1 H), 8.03 (dd, *J*=40, 8.8 Hz, 1 H), 8.26 (d, *J*=2.8 Hz, 1 H), 8.4 (d, *J*=2.8 Hz, 1 H), 8.63 (d, *J*=4.4 Hz, 1 H).

### Example Compound 68

### Preparation of 2-(3-(difluoromethoxy)-4-(pyridin-2-ylethynyl)-1H-pyrazol-1-yl)-5-fluoropyridine:

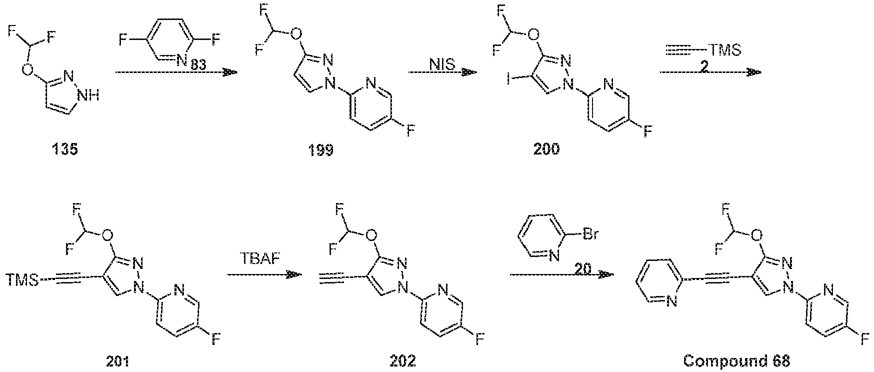

### Experimental section:

### Procedure for preparation of 199:

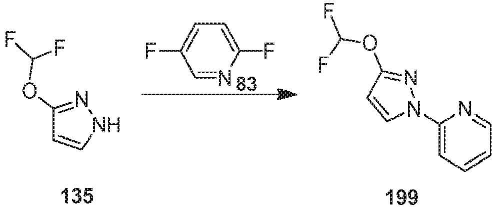

To a mixture of compoud **135** (370.00mg, 2.76mmol) and **199** (635.24mg, 5.52 mmol) in DMF (10mL), was added Cs₂CO₃ (2.70g, 8.28mmol) in one portion at room temperature. The mixture was heated at 70°C for 4 hours. LCMS showed the reaction was completed. The mixture was cooled to room temperature and filtered. The filtrate concentrated in reduced pressure. The residue was purified by silica gel chromatography to afford product **199** (200.00mg, yield: 31.62%).
**¹H NMR** (400 MHz, CDCl3): δ 8.36 (s, 1H), 8.21 (s, 1H), 7.83-7.80 (m, 1H), 7.54-7.51 (m, 1H), 7.15-6.78 (m, 1H), 6.09 (s, 1H).

### Procedure for preparation of 200:

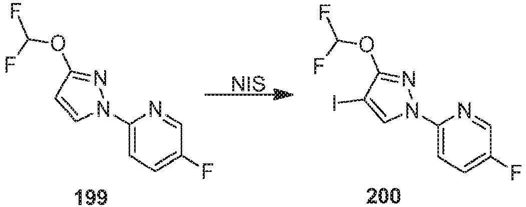

To a mixture of compoud **199** (200.00mg, 872.75umol) in CHCl3 (10mL), was added NIS (294.53mg, 1.31mmol) in one portion at room temperature. Then the mixture was heated at 60°C for 5 hours. LCMS showed the reaction was completed. The mixture was cooled to room temperatuer and concentrated in reduced pressure . The residue was poured into water (10mL). The aqueous phase was extracted with DCM (20mLx2). The combined organic phase was dried with anhydrous Na₂SO₄, filtered and concentrated in vacuum. The residue was purified by silica gel chromatography to afford product **200** (260.00mg, yield: 83.91%).
**LCMS:** *m*/*z,* 355.8 (M+H)⁺.

### Procedure for preparation of 201:

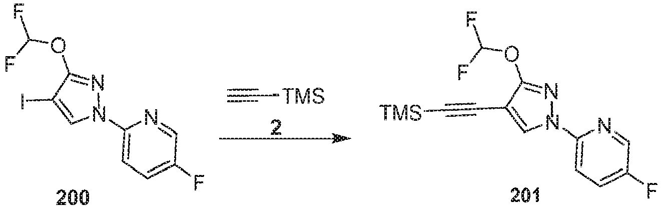

Mixture of **200** (260.00mg, 732.29umol), 2 (215.78mg, 2.20mmol), CuI (13.95 mg, 73.23umol), TEA (222.30mg, 2.20mmol) and Pd(PPh₃)₂Cl₂ (25.70mg, 36.61umol) were taken up into a microwave tube in THF (5mL) .The sealed tube was heated at 90°C for 1 hour under microwave. LCMS showed the starting material was consumed and only the desired product. After cooling to room temperature, the mixture was concentrated in reduced pressure. The residue was purified by silica gel chromatography to afford product **201** (200.00mg, yield: 83.94%).
**LCMS:** *m*/*z,* 325.9 (M+H)⁺.

### Procedure for preparation of 202:

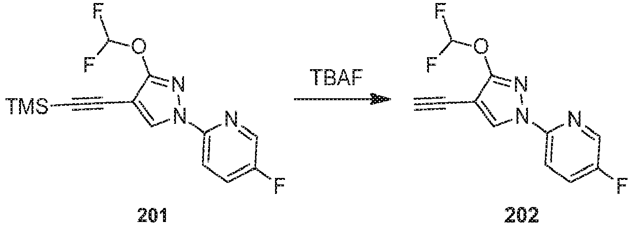

To a mixture of **201** (200.00mg, 614.70umol) in THF (15mL), was added TBAF(in THF) (1 M, 922.05uL) dropwise at room temperature. The mixture was stirred at room temperature for 3 hours. LCMS showed the reaction was completed. The mixture was poured into water(10mL). The aqueous phase was extracted with ethyl acetate (20mLx2). The combined organic phase was washed with saturated brine (10 mL), dried with anhydrous Na₂SO₄, filtered and concentrated in vacuum to afford product **202** (150.00mg, crude) which was used directly for next step.
**LCMS:** *m*/*z,* 254.0 (M+H)⁺.

### Procedure for preparation of Compound 68:

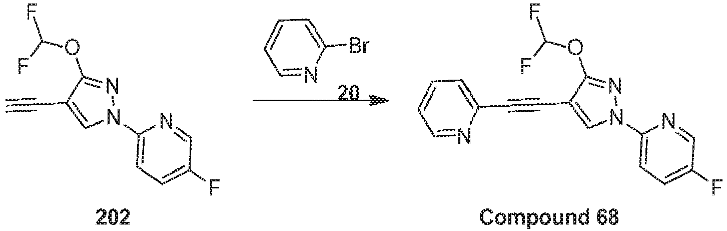

Mixture of **202** (200.00mg, 789.95umol), **20** (249.62mg, 1.58mmol), CuI (15.04mg, 79.00umol), TEA (239.81mg, 2.37mmol) and Pd(PPh₃)₂Cl₂ (27.72mg, 39.50umol) were taken up into a microwave tube in THF (4mL). The sealed tube was heated at 90°C for 1 hour under microwave. TLC showed the starting material was consumed. After cooling to room temperature, the mixture was concentrated in reduced pressure. The residue was purified by prep-HPLC to afford product **Compound 68** (15.00mg, yield: 5.73%).
**LCMS:** *m*/*z,* 331.0 (M+H)⁺;
**¹H NMR** (400 MHz, CDCl3): δ 8.62-8.59 (m, 2H), 8.25 (s, 1H), 7.84-7.81 (m, 1H), 7.70-7.69 (m, 1H), 7.54-7.53 (m, 2H), 7.31-7.95 (m, 2H).

### Example Compound 69

### Preparation of 2-(4-chloro-3-(pyridin-2-ylethynyl)-1H-pyrazol-1-yl)-5-fluoropyridine:

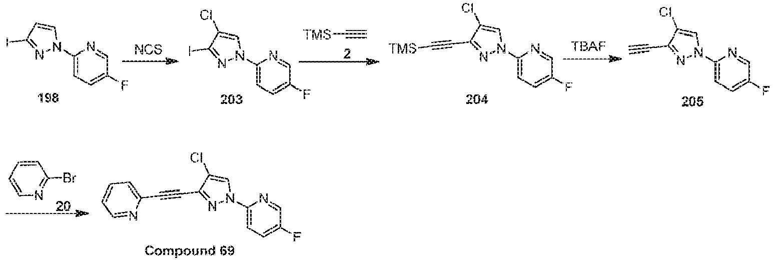

### Experimental section:

### Procedure for preparation of 203:

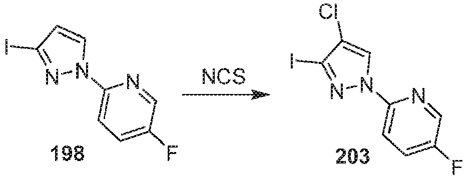

The mixture of compound **198** (200.00mg, 691.92umol) and NCS (138.59mg, 1.04 mmol) in a 5mL single-necked round bottom flask. The mixture was stirred at 120°C for 1 hour. Then cooled down to room temperature. LCMS showed the starting material was consumed completely and the desired compound was detected. The residue was partitioned between ethyl acetate (100mL) and H₂O (50mL). The organic phase was washed with saturated brine (20mL), dried with anhydrous Na₂SO₄, filtered and concentrated in vacuum to afford product **203** (220.00mg, crude), which was used directly for the next step without purification.
**LCMS:** *m*/*z,* 323.9(M+H)⁺.

### Procedure for preparation of 204:

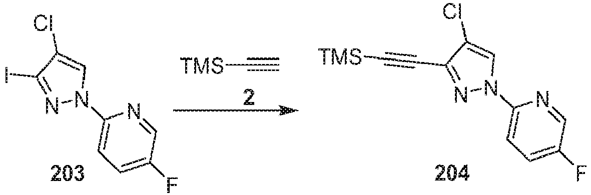

Mixture of compound **203** (220.00mg, 536.59umol), **2** (79.06mg, 804.89umol), Et₃N (162.89mg, 1.61mmol) and CuI (5.11mg, 26.83umol) and Pd(PPh₃)₂Cl₂ (18.83mg, 26.83umol) in THF (8mL) was de-gassed and were taken up into a microwave tube. The sealed tube was heated at 95°C for 1 hour under microwave. LCMS showed the starting material was consumed completely and the desired compound was detected. The mixture was partitioned between ethyl acetate (100mL) and H₂O (20mL), the separated organic layer was washed with brine (30mL), dried over Na₂SO₄ and evaporated to dryness, which was purified by silica gel chromatography to afford product **204** (120.00mg, yield: 76.12%).
**LCMS:** *m*/*z,* 294.0(M+H)⁺.

### Procedure for preparation of 205:

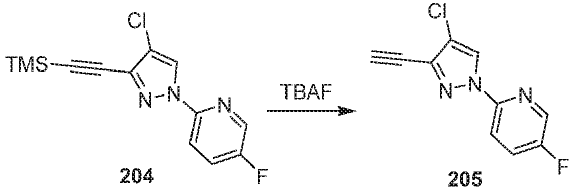

To a solution of compound **204** (170.00mg, 578.62umol) in THF (5mL) was added TBAF (1M, 867.94uL) at 0°C. The reaction mixture was stirred at 0°C for 0.5 hour and then kept at room temperature for 1 hour. LCMS showed the starting material was consumed completely and the title compound was detected. The reaction mixture was concentrated to dryness, which was partitioned between ethyl acetate (100mL) and H₂O (50mL). The separated organic layer was washed saturated brine(20mL), dried over Na₂SO₄ and evaporated in vacuo to afford product **205** (120.00mg, crude), which was used directly for the next step without purification.
**LCMS:** *m*/*z,* 222.0(M+H)⁺.

### Procedure for preparation of Compound 69:

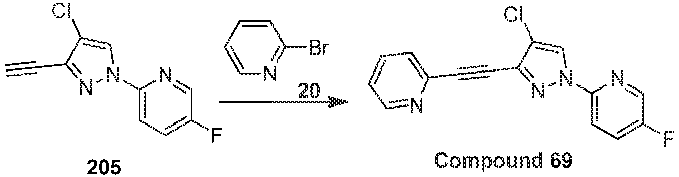

Mixture of compound **205** (150.00mg, 676.83umol), **20** (160.41mg, 1.02mmol), CuI (6.45mg, 33.84umol), Et₃N (205.47mg, 2.03 mmol) and Pd(PPh₃)₂Cl₂ (23.75mg, 33.84 umol) were taken up into a microwave tube in THF (8mL) The sealed tube was heated at 95°C for 1 hour under microwave. LCMS showed the starting material was consumed completely and the title compound was detected. After cooling to room temperature, ethyl acetate (80mL) and saturated aqueous of Na₂CO₃ (20mL) were added. The aqueous layer was extracted with ethyl acetate (40mLx2). The combined organic layers were washed with brine (30mL), dried over Na₂SO₄, concentrated in vacuum to give the crude product, which was purified prep-HPLC to afford product **Compound 69**(15.00mg, yield: 7.32%).
**LCMS:** *m*/*z*, 299.0(M+H)⁺;
**¹H NMR** (400MHz, CDCl₃): δ8.66 (d, *J*=4.85 Hz, 1 H), 8.51 (s, 1 H), 8.25 (d, *J*=2.65 Hz, 1H), 8.00 (dd, *J*=8.93, 3.86 Hz, 1 H), 7.68-7.75 (m, 1 H), 7.54-7.65 (m, 2 H), 7.26-7.34 (m, 1 H).

### Example Compound 70

### Preparation of 3-fluoro-5-(3-(pyridin-2-ylethynyl)-1H-pyrazol-1-yl)pyridine:

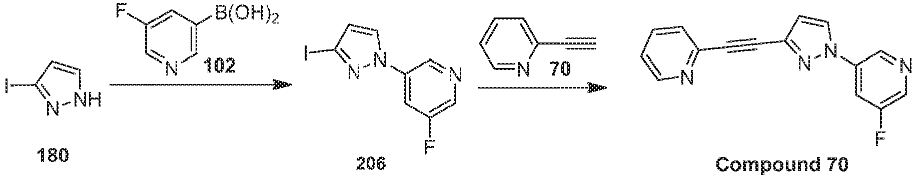

### Experimental section:

### Procedure for preparation of 206:

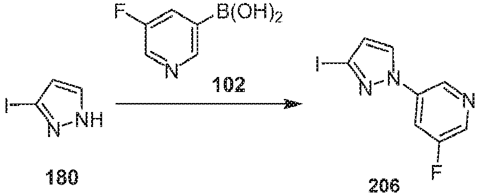

To a mixture of compound **180**(300.00mg, 1.55mmol) and **102**(327.62mg, 2.32mmol) in DCM (20mL), was added pyridine (367.02mg, 4.64mmol), Cu(OAc)₂(561.83mg, 3.09mmol) and 1-oxidopyridin-1-ium (442.21 mg, 4.65mmol, 3.00Eq) in one portion at room temperature. The mixture was stirred at room temperature for 48 hours under O₂ protected. LCMS showed the reaction was completed. The mixture was filtrated and the filtrate was concentrated in reduced pressure. The residue was dissolved in ethyl acetate (40mL). The organic phase was washed with water (30mLx2), saturated brine (30mLx1), dried over anhydrous Na₂SO₄, filtered and concentrated in vacuum. The residue was purified by silica gel chromatography to afford product **206**(380.00mg, yield: 84.82%).
LCMS: *m*/*z*, 289.9(M+H)⁺.

### Procedure for preparation of Compound 70:

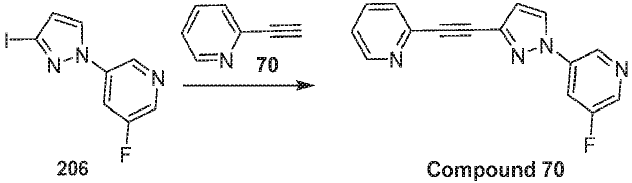

A mixture of **206** (190.00mg, 657.33umol), **70** (101.68mg, 985.99umol), CuI (6.26 mg, 32.87umol), Et₃N (199.54mg, 1.97mmol) and Pd(PPh₃)₂Cl₂ (23.07mg, 32.87 umol) were taken up into a microwave tube in THF (3mL). The sealed tube was heated at 90°C for 1 hour under microwave. TLC showed the starting material was consumed completely. After cooling to room temperature, H₂O (10mL) were added. The aqueous layer was extracted with ethyl acetate (15mLx3). The combined organic layers were washed with brine (15mL), dried over Na₂SO₄, concentrated in vacuo to give the crude product which was purified by prep-HPLC to afford product **Compound 70** (20.00mg, yield: 11.40%).
**LCMS:** *m*/*z*, 265.1(M+H)⁺;
**¹H NMR** (400MHz, CDCl₃): δ6.79 (d, *J*=2.4Hz, 1H), 7.25-7.29 (m, 1H), 7.59-7.61 (m, 1 H), 7.69-7.72 (m, 1H), 7.91-7.93 (m, 1H), 7.98-7.99 (m, 1H), 8.44 (d, *J*=2.4Hz, 1H), 8.65 (m, 1H), 8.82 (s, 1H).

### Example Compound 71

### yPreparation of 2-((1-(pyridin-2-yl)-1H-pyrazol-3-yl)ethynyl)pyridine:

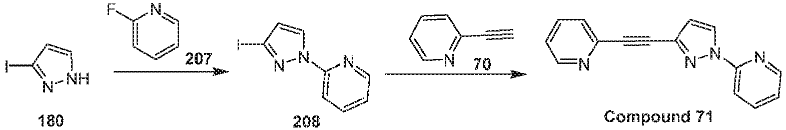

### Experimental section:

### Procedure for preparation of 208:

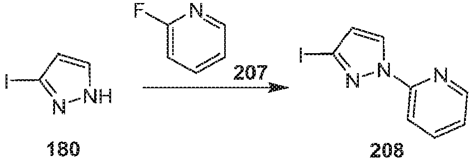

To a solution of **180** (100.00mg, 515.54umol) and **207**(60.06mg, 618.65umol) in DMF (3mL) was added solid Cs₂CO₃ (251.96mg, 773.31 umol) in one portion at room temperature. It was stirred at 90°C for 4 hours. After cooling, water (20mL) was added into the mixture with stirring at ice bath slowly. Gradually, solid was formed. It was filtrated to afford the product **208**(80.00mg, crude), which was used for next step directly.
LCMS: *m*/*z,* 272.0 (M+H)⁺.

### Procedure for preparation of Compound 71:

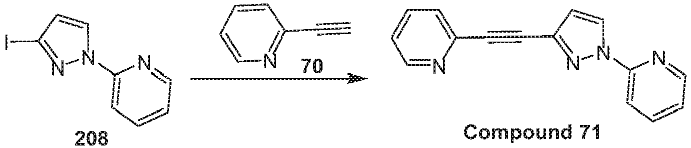

Mixture of compound **208**(50.00mg, 184.46umol), **70**(22.83mg, 221.35umol), CuI (3.51mg, 18.45umol), TEA (56.00mg, 553.38umol), and Pd(PPh₃)₂Cl₂ (6.47mg, 9.22 umol) were taken up into a microwave tube in THF (3 mL). The sealed tube was heated at 90°C for 1 hour under microwave. LCMS showed the starting material was consumed. After cooling to room temperature, ethyl acetate (5mL) and H₂O (3mL) were added. The aqueous layer was extracted with ethyl acetate (5mLx2). The combined organic layers were washed with brine (5mL), dried over Na₂SO₄, concentrated in vacuo to give the crude product, which was purified by pre-HPLC (neutral) to afford product **Compound 71**(10.20mg, yield: 22.30%).
**LCMS:** *m*/*z*, 247.0 (M+H)⁺;
**¹HNMR** (400MHz, CDCl₃): δ6.66 (d, *J*=2.4 Hz, 1 H), 7.15-7.18 (m, 1 H), 7.21-7.23 (m, 1H), 7.54 (d, *J*=80 Hz, 1 H), 7.64 (t, *J*=8.0 Hz, 1H), 7.77 (d, *J*=8.0 Hz, 1H), 7.97 (d, *J*=8.0 Hz, 1H), 8.36 (d, *J*=8.0 Hz, 1 H), 8.51 (d, *J*=4.0 Hz, 1 H), 8.57 (d, *J*=4.0 Hz, 1H).

### Example Compound 72

### Preparation of -5-fluoro-2-(4-methyl-3-(pyridin-2-ylethynyl)-1H-pyrazol-1-yl) pyridine:

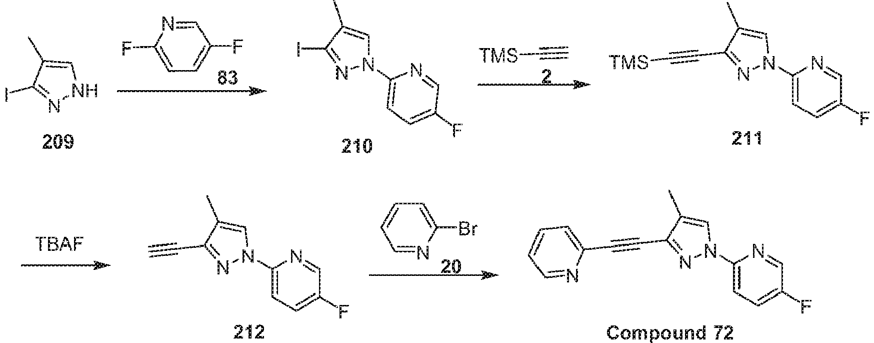

### Experimental section:

### Procedure for preparation of 210:

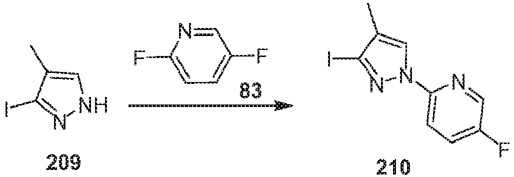

To a mixture of **209**(300.00mg, 1.44mmol) and **83**(331.43mg, 2.88 mmol) in DMF (10mL), was added Cs₂CO₃ (1.41g, 4.33mmol) in one portion at room temperature. The mixture was heated at 70°C for 4 hours. LCMS showed the reaction was completed. The mixture was cooled to room temperature and filtered. The filtrate was poured into water (25mL), filtered and the filter cake was washed with 5mL of water, dried in vacuum to afford product 210 (260.00mg, yield: 59.58%).
LCMS: *m*/*z*, 303.9 (M+H)⁺.

### Procedure forpreparation of intermediate 211:

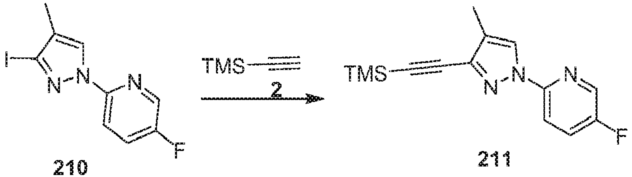

Mixture of compoud **210**(260.00mg, 857.89umol), **2**(252.79mg, 2.57mmol), CuI (16.34mg, 85.79umol), TEA (260.43mg, 2.57mmol) and Pd(PPh₃)₂Cl₂ (30.11mg, 42.89umol) were taken up into a microwave tube in THF (4mL). The sealed tube was heated at 90°C for 1 hour under microwave. LCMS showed the starting material was consumed and only the desired product. After cooling to room temperature, the mixture was filtered and concentrated in vacuo. The residue was purified by prep-TLC to give the crude product **211**(200.00mg, yield: 85.28%).
**LCMS:** *m*/*z*, 274.1 (M+H)⁺.

### Procedure for preparation of 212:

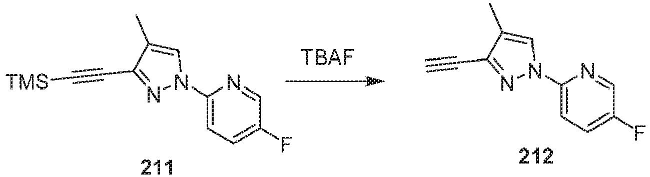

To a mixture of **211** (200.00mg, 731.58umol) in THF (15mL), was added TBAF(in THF) (1M, 1.10mL) dropwise at room temperautre. The mixture was stirred at room temperautre for 3 hours. LCMS showed the reaction was completed. The mixture was poured into water (10mL) The aqueous phase was extracted with ethyl acetate (20 mLx2). The combined organic phase was washed with saturated brine (10 mL), dried with anhydrous Na₂SO₄,filtered and concentrated in vacuo to afford product **212**(140.00tng, crude) which was used directly for next step.
**LCMS:** *m*/*z*, 202.0 (M+H)⁺.

### Procedure for preparation of Compound 72:

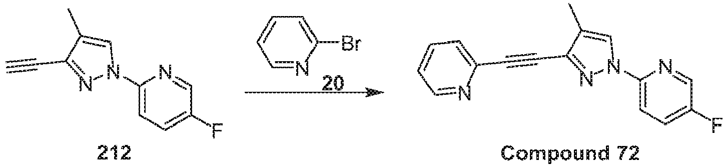

Mixture of **212**(140.00mg, 695.83umol), **20** (219.88mg, 1.39mmol), CuI (13.25mg, 69.58umol), TEA (211.23mg, 2.09mmol) and Pd(PPh₃)₂Cl₂ (24.42mg, 34.79umol) were taken up into a microwave tube in THF (4mL) .The sealed tube was heated at 90°C for 1 hour under microwave. TLC showed the starting material was consumed. After cooling to room temperature, the mixture was concentrated in reduced pressure. The residue was purified by prep-HPLC to afford product **Compound 72**(20.00mg, yield: 10.18%).
**LCMS:** *m*/*z*, 279.1 (M+H)⁺;
**¹H NMR** (400MHz, CDCl₃): δ 8.64-8.63 (m, 1H), 8.29 (s, 1H), 8.23 (s, 1H), 8.01-7.97 (m, 1H), 7.98-7.97 (m, 1H), 7.69-7.68 (m, 1H), 7.60-7.58 (m, 1H), 7.26-7.25 (m, 1H), 2.27 (s, 3H).

### Example Compound 73

### Preparation of 5-fluoro-2-(5-methyl-3-(pyridin-2-ylethynyl)-1H-pyrazol-1-yl) pyridine:

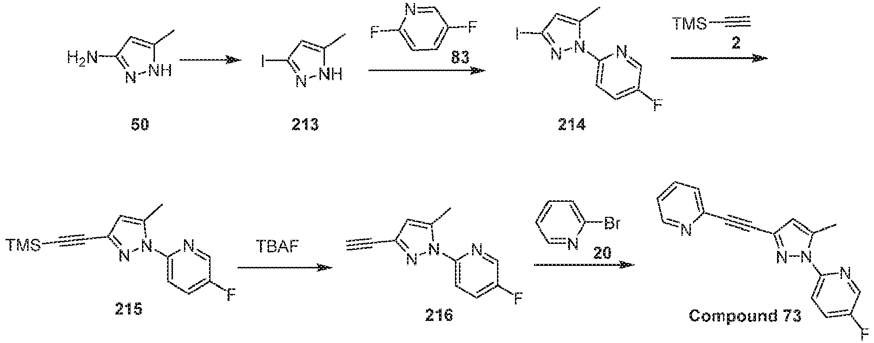

### Experimental section:

### Procedure for preparation of 213:

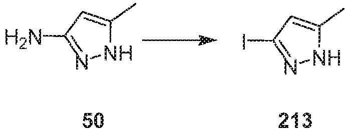

To a mixture of **50** (1.00g, 10.30mmol) in concentrated HCl (20mL) was added NaNO₂ (888.08mg, 12.87mmol) in water (2mL) over 4 minutes at 0°C. To the resulting yellow reaction mixture was added a solution of KI (2.56g, 15.44mmol) in water (4mL) over 5 minutes at 0°C, resulting in nitrogen evolution. The reaction mixture was stirred for 3 hours at 0°C and warmed to room temperature, upon which nitrogen evolution ceased. LCMS showed the reaction was completed. EtOAc (20mL) was added, followed by adding water (10mL). The aqueous phase was neutralized with Na₂CO₃ and the pH of aqueous phase was 8-9, then extracted with EtOAc (30mLx3). The combined organic phase was washed with Na₂S₂O₃(10mLx2), dried over anhydrous Na₂SO₄, filtered and concentrated in vacuum. The residue was purified by silica gel chromatography to afford product **213** (200.00 mg, yield: 8.40%) .
**LCMS:** *m*/*z*, 208.9 (M+H)⁺.

### Procedure for preparation of 214:

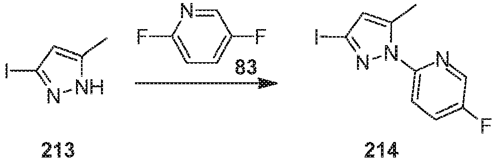

To a mixture of **213**(100.00mg, 480.77umol) and **83**(82.99mg, 721.15umol) in DMF (5 mL), was added Cs₂CO₃ (469.93mg, 1.44mmol) in one portion at 25°C .The mixture was heated to 100°C and stirred for 6 hours. LCMS showed about 28% of desired product was detected. The mixture was cooled to 25°C and filtered. The filtrate was poured into water (25mL), and extracted with EtOAc(10mLx3). The combined organic phase was washed with saturated brine (5mLx2), dried over anhydrous Na₂SO₄, filtered and concentrated in vacuum to afford product **214**(80.00mg, crude).
**LCMS:** *m*/*z*, 304.0 (M+H)⁺.

### Procedure for preparation of 215:

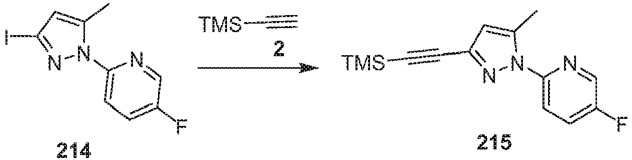

A mixture of **214**(80.00mg, 263.97umol), **2**(77.278mg, 791.90umol), CuI (5.03mg, 26.40umol), TEA (80.13mg, 791.90umol) and Pd(PPh₃)₂Cl₂ (9.26mg, 13.20umol) in THF (4 mL) was taken up into a microwave tube. The sealed tube was heated at 90°C for 1 hour under microwave. LCMS showed the starting material was consumed and only the desired product. After cooling to 25°C, the mixture was filtered and concentrated in vacuo. The residue was purified by prep-TLC to give the crude product **215**(40.00mg, yield: 55.43%).
**LCMS:** *m*/*z*, 274.1 (M+H)⁺.

### Procedure for preparation of 216:

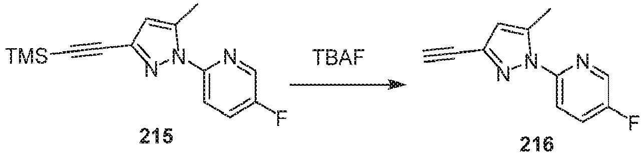

To a mixture of **215**(140.00mg, 512.11umol) in THF (15mL), was added TBAF(in THF) (1M, 768.16 uL) dropwise at 25°C.The mixture was stirred at 25°C for 3 hours. TLC showed the reaction was completed. The mixture was poured into water (10mL) .The aqueous phase was extracted with ethyl acetate (20mLx2). The combined organic phase was washed with saturated brine (10mL), dried over anhydrous Na₂SO₄, filtered and concentrated in vacuum to afford product **216**(90.00mg, crude) and used directly for next step.
**LCMS:** *m*/*z*, 202.1 (M+H)⁺.

### Procedure for preparation of Compound 73:

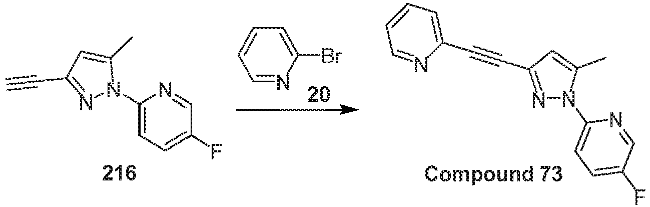

Amixture of **216**(90.00mg, 447.32umol), **20**(141.35mg, 894.63umol), CuI (8.52mg, 44.73umol), TEA(135.79mg, 1.34mmol) and Pd(PPh₃)₂Cl₂ (15.70mg, 22.37umol) in THF (4 mL) were taken up into a microwave tube .The sealed tube was heated at 90 °C for 1 hour under microwave. TLC showed the starting material was consumed. After cooling to 25°C, the mixture was concentrated in reduced pressure. The residue was purified by prep-HPLC purification to afford desired product **Compound 73**(20.00mg, yield: 16.07%).
**LCMS:** *m*/*z,* 279.1 (M+H)⁺;
**¹H NMR** (400 MHz, CDCl₃): δ 8.63-8.62 (m, 1H), 8.28 (s, 1H), 7.96-7.93 (m, 1H), 7.68-7.67 (m, 1H), 7.58-7.55 (m, 2H), 7.25-7.24 (m, 1H), 6.44 (s, 1H), 2.65 (s, 3H).

### Example Compound 74

### Preparation of 3-fluoro-5-(4-(phenylethynyl)-1H-pyrazol-1-yl)benzonitrile:

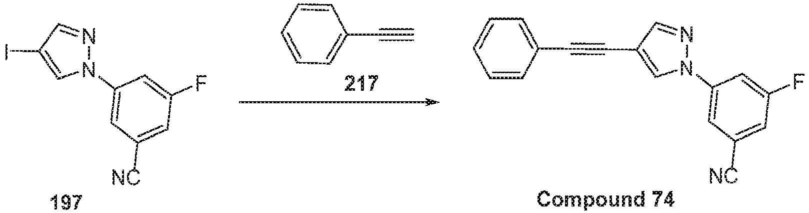

### Experimental section:

### Procedure for preparation of Compound 74:

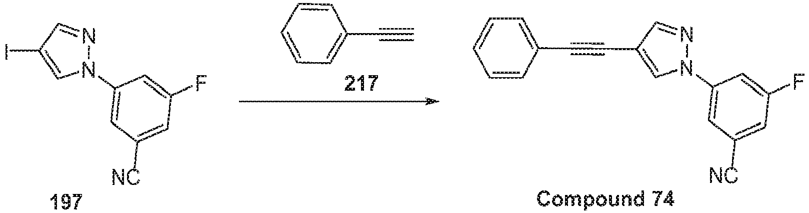

To a solution of **197**(100mg, 0.319mmol) and **217**(0.039ml, 0.351mmol) in 20 mL of Et₃N was added Pd(PPh₃)₂Cl₂ (11.21mg, 0.016mmol) and CuI (6.08mg, 0.032 mmol). The mixture was protected with N₂ atmosphere, then was heated at 70°C for 4 hours. TLC analysis showed complete conversion of starting material to a major product. The reaction mixture was then concentrated in vacuo. The crude product was purified by prep-HPLC to yield product **Compound 74** (28mg, yield: 30.5%).
**LCMS:** *m*/*z* 288 (M+H)⁺;
**¹H NMR** (400 MHz, CDCl₃): δ 8.11 (s, 1H), 7.89 (s, 1H), 7.83-7.80 (m, 1H), 7.73 (dt, *J*=9.5, 2.2 Hz, 1H), 7.53-7.49 (m, 2H), 7.38-7.33 (m, 3H), 7.32-7.28 (m, 1H).

### Example Compound 75

### Preparation of 3-fluoro-5-(4-(pyridin-2-ylethynyl)-1H-pyrazol-1-yl)benzonitrile:

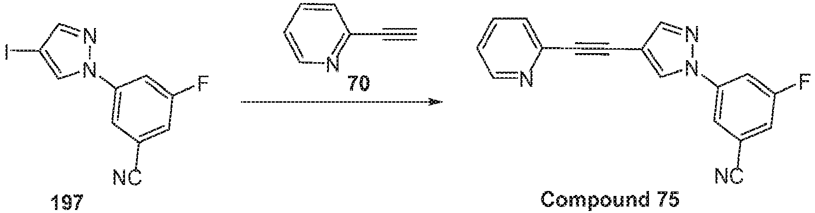

### Experimental section:

### Procedure for preparation of Compound 75:

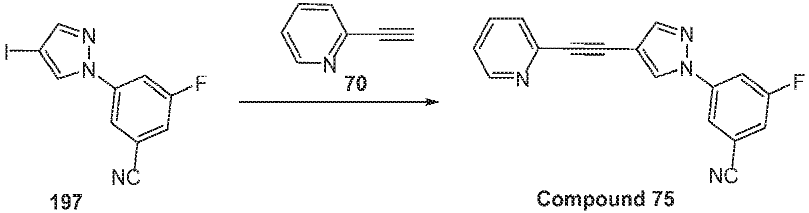

To a solution of **196**(100mg, 0.319mmol) and **70**(0.036mL, 0.351mmol) in 20mL of Et₃N was added Pd(PPh₃)₂Cl₂ (11.21mg, 0.016mmol) and CuI (6.08mg, 0.032mmol). The mixture was protected with N₂ atmosphere, then was heated at 70°C for 4 hours. TLC analysis showed complete conversion of starting material to a major product. The reaction mixture was then concentrated in vacuo. The crude product was purified by prep-HPLC to give the target product **Compound 75**(22 mg, yield: 23.89 %).
LCMS: *m*/*z* 289 (M+H)⁺;
**¹H NMR** (400 MHz, CDCl₃): δ 8.65-8.61 (m, 1H), 8.17 (s, 1H), 7.94 (s, 1H), 7.81 (s, 1H), 7.75-7.68 (m, 2H), 7.54-7.49 (m, 1H), 7.34-7.27 (m, 2H).

### Example Compound 76

### Preparation of 3-fluoro-5-(4-(pyridin-3-ylethynyl)-1H-pyrazol-1-yl)benzonitrile:

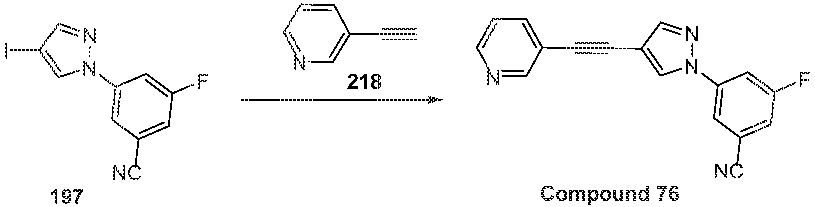

### Experimental section:

### Procedure for preparation of Compound 76:

To a solution of **197**(100mg, 0.319mmol) and **218**(36.2mg, 0.351mmol) in 20mL of Et₃N was added Pd(PPh₃)₂Cl₂ (11.21mg, O.016mmol) and CuI (6.08mg, 0.032mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 76**(20mg, yield: 21.72%).
**LCMS:** *m*/*z* 289 (M+H)⁺;
**¹H NMR** (400 MHz, CDCl₃): δ 8.75 (d, *J*=2.1, 1.0 Hz, 1H), 8.57 (dd, *J*=5.0, 1.7 Hz, 1H), 8.15 (s, 1H), 7.91 (s, 1H), 7.85-7.78 (m, 2H), 7.77-7.72 (m, 1H), 7.35-7.28 (m, 2H).

### Example Compound 77

### Preparation of 3-fluoro-5-(4-(3-chloro-phenylethynyl)-1H-pyrazol-1-yl) benzonitrile:

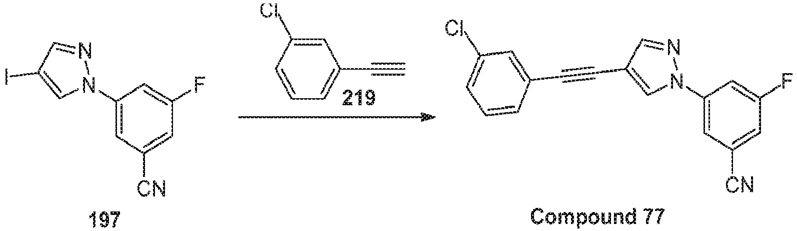

### Experimental section:

### Procedure for preparation of Compound 77:

To a solution of **197**(100mg, 0.319mmol) and **219**(0.044mL, 0.351mmol) in 20mL of Et₃N was added Pd(PPh₃)₂Cl₂ (11.21mg, 0.016mmol) and CuI (6.08mg, 0.032mmol). The mixture was protected with N₂ atmosphere, then was heated at 70°C for 4 hours. TLC analysis showed complete conversion of starting material to a major product. The reaction mixture was then concentrated in vacuo. The crude product was purified by pep-HPLC to give the target product **Compound** 77(23mg, yield: 22.38 %).
**LCMS:** *m*/*z* 322 (M+H)⁺;
**¹H NMR** (400 MHz, CDCl₃): δ 8.12 (s, 1H), 7.89 (s, 1H), 7.81 (s, 1H), 7.73 (dt, *J* = 9.4, 2.2 Hz, 1H), 7.50 (t, *J*=2.3, 1.5, 0.7 Hz, 1H), 7.39 (dt, *J*= 7.2, 1.6 Hz, 1H), 7.36-7.27 (m, 3H).

### Example Compound 78

### Preparation of 3-fluoro-5-(4-(pyrimidin-2-ylethynyl)-1H-pyrazol-1-yl) benzonitrile:

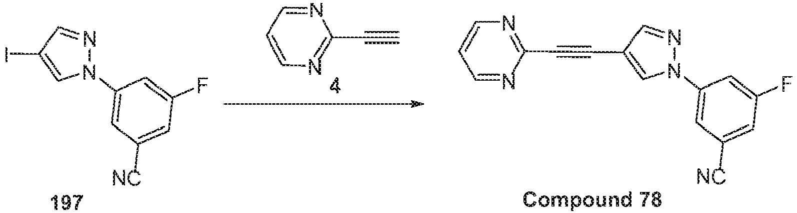

To a solution of **197**(100mg, 0.319mmol) and **4**(36.6mg, 0.351mmol) in 20mL of Et₃N was added Pd(PPh₃)₂Cl₂ (11.21mg, 0.016mmol) and CuI (6.08mg, 0.032mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 78**(45mg, yield: 48.7 %).
**LCMS:** *m*/*z* 290 (M+H)⁺;
**¹H NMR** (400 MHz, CDCl₃): δ 8.77 (d, *J*= 4.8 Hz, 2H), 8.23 (s, 1H), 7.99 (s, 1H), 7.82 (s, 1H), 7.73 (d, *J* = 9.2 Hz, 1H), 7.34-7.27 (m, 2H).

### Example Compound 79

### Preparation of 3-fluoro-5-(4-(pyridin-4-ylethynyl)-1H-pyrazol-1-yl)benzonitrile:

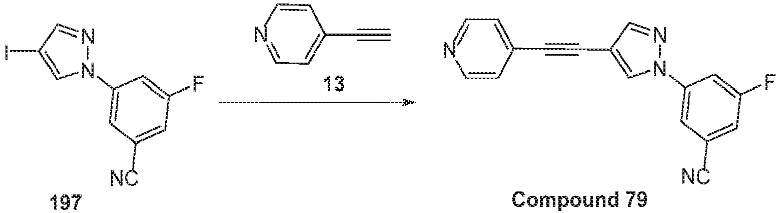

To a solution of **197**(100mg, 0.319mmol)) and **13**(32.9mg, 0.319mmol) in 20mL of Et₃N was added Pd(PPh₃)₂Cl₂ (11.21mg, 0.016mmol) and CuI (6.08mg, 0.032mmol)). The mixture was protected under N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 79**(25mg, yield: 27.1 %).
**LCMS:** *m*/*z* 289 (M+H)⁺;
**¹H NMR** (400 MHz, CDCl₃): δ 8.67 (br, 2H), 8.17 (s, 1H), 7.92 (s, 1H), 7.82 (s, 1H), 7.76 - 7.73 (m, 1H), 7.40 (br, 2H), 7.34-7.32 (m, 1H).

### Example Compound 80

### Preparation of 3-fluoro-5-(4-(pyrazin-2-ylethynyl)-1H-pyrazol-1-yl)benzonitrile:

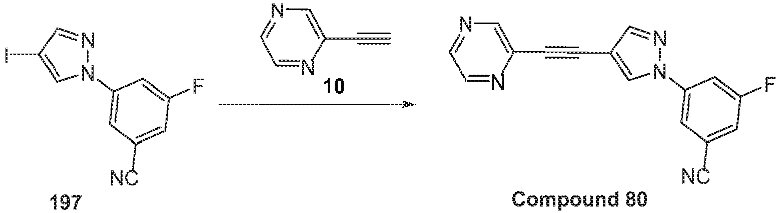

To a solution of **197**(100mg, 0.319mmol)) and **10**(36.6mg, 0.351mmol) in 20mL of Et₃N was added Pd(PPh₃)₂Cl₂ (11.21mg, 0.016mmol) and CuI (6.08mg, 0.032mmol)). The mixture was protected under N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 80**(25mg, yield: 27.1 %).
LCMS: *m*/*z* 290 (M+H)⁺;
**¹H NMR** (400 MHz, CDCl₃): δ 8.78 (br, 1H), 8.62-8.55 (m, 2H), 8.21 (s, 1H), 7.97 (s, 1H), 7.83 (s, 1H), 7.76-7.73 (m, 1H), 7.35-7.32 (m, 1H).

### Example Compound 81

### Preparation of 1-(4-fluorophenyl)-4-(phenylethynyl)-1H-pyrazole:

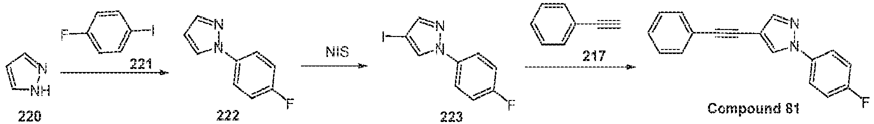

### Experimental section:

### Procedure for preparation of 222:

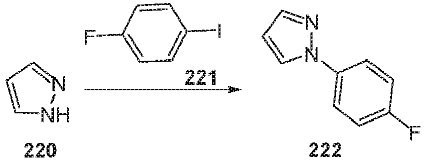

A mixture of **220**(0.613g, 9.01mmol), Cs₂CO₃ (4.40g, 13.51mmol), **221**(2.0g, 9.01mmol) and Cu(OAc)₂ (0.16g) in 20mL DMF, was heated at 120°C for the appropriate time and subsequent cooling, the reaction mixture was diluted with saturated aqueous ammonium chloride. Products were isolated by extraction with ethyl acetate. The organic layer was dried over anhydrous Na₂SO₄, filtered, and concentrated. Product **222** was purified by silica gel column chromatography (1.0g, yield: 68%).

### Procedure for preparation of 223:

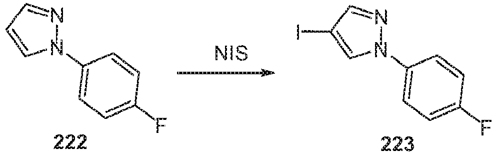

A mixture of **222**(1.0g) and NIS (1.66g) in 10ml AcOH, was heated at 120 °C via MW for 30 minutes. The reaction mixture was diluted with EA, washed with brine, then dried over Na₂SO₄, filtered, and concentrated. The residue was purified by column chromatography on silica gel to afford product **223** (0.8g, yield: 45%).
**LCMS:** *m*/*z* 289 (M+H)⁺.

### Procedure for preparation of Compound 81:

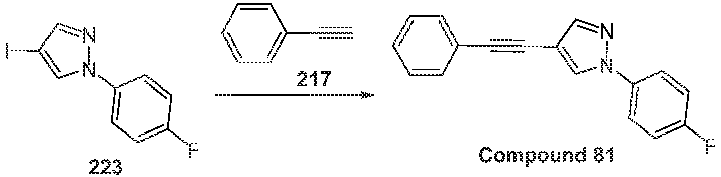

To a solution of **223**(100mg, 0.347mmol) and **217**(0.042mL, 0.382mmol) in 20 mL of Et₃N was added Pd(PPh₃)₂Cl₂ (12.18mg, 0.017mmol) and CuI (6.61mg, 0.035mmol). The mixture was protected under N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 81**(36mg, yield: 39.5%).
**LCMS:** *m*/*z* 263 (M+H)⁺;
**¹H NMR** (400 MHz, CDCl₃): δ 8.04 (s, 1H), 7.86 (s, 1H), 7.71-7.60 (m, 2H), 7.54-7.47 (m, 2H), 7.38-7.31 (m, 3H), 7.23-7.13 (m, 2H).

### Example Compound 82

### Preparation of 2-((1-(4-fluorophenyl)-1H-pyrazol-4-yl)ethynyl)pyridine:

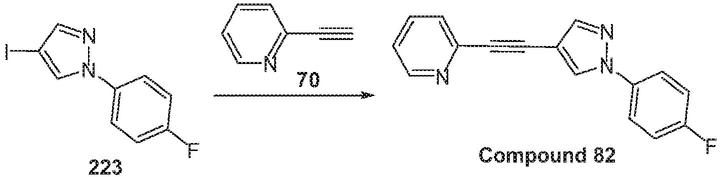

To a solution of **223**(100mg, 0.347mmol) and **70**(0.039mL, 0.382mmol) in 20mL of Et₃N was added Pd(PPh₃)₂Cl₂ (12.18mg, 0.017mmol) and CuI (6.61mg, 0.035mmol). The mixture was protected under N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 82**(24mg, yield: 26.3%).
**LCMS:** *m*/*z* 264 (M+H)⁺;
**¹H NMR** (400 MHz, CDCl₃): δ 8.65-8.59 (m, 1H), 8.09 (s, 1H), 7.89 (s, 1H), 7.72-7.59 (m, 3H), 7.49 (m, 1H), 7.25-7.21 (m, 1H), 7.20-7.13 (m, 2H).

### Example Compound 83

### Preparation of 3-((1-(4-fluorophenyl)-1H-pyrazol-4-yl)ethynyl)pyridine:

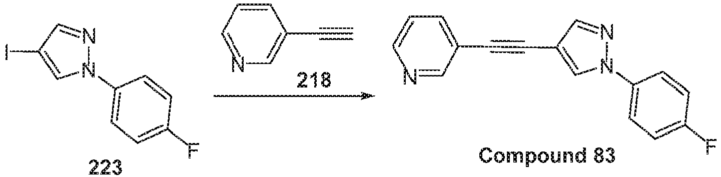

To a solution of **223**(100mg, 0.347mmol) and **218**(39.4mg, 0.382mmol) in 20mL of Et₃N was added Pd(PPh₃)₂Cl₂ (12.18mg, 0.017mmol) and CuI (6.61mg, 0.035mmol). The mixture was protected under N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 83**(28mg, yield: 30.6%).
**LCMS:** *m*/*z* 264 (M+H)⁺;
**¹H NMR** (400 MHz, CDCl₃) δ 8.75 (s, 1H), 8.56 (d, 1H), 8.08 (s, 1H), 7.87 (s, 1H), 7.80 (dt, *J* = 7.9, 1.9 Hz, 1H), 7.71-7.60 (m, 2H), 7.35-7.27 (m, 1H). 7.22-7.13 (m, 2H).

### Example Compound 84

### Preparation of 4-((3-chlorophenyl)ethynyl)-1-(4-fluorophenyl)-1H-pyrazole:

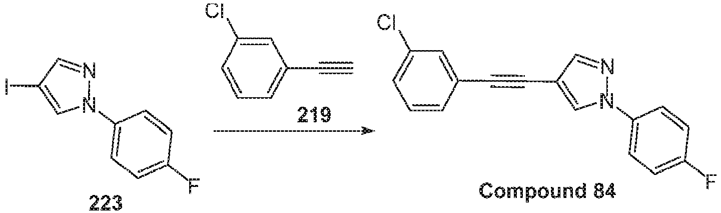

To a solution of **223**(100mg, 0.347mmol) and **219**(39.4mg, 0.382mmol) in 20mL of Et₃N was added Pd(PPh₃)₂Cl₂ (12.18mg, 0.017mmol) and CuI (6.61mg, 0.035mmol). The mixture was protected under N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 84** (21mg, yield: 20.39%).
**LCMS:** *m*/*z* 297 (M+H)⁺:
**¹H NMR** (400 MHz, CDCl₃): δ 8.04 (s, 1H), 7.85 (s, 1H), 7.69-7.62 (m, 2H), 7.49 (s, 1H), 7.40-7.36 (m, 1H), 7.33-7.27 (m, 2H), 7.21-7.14 (m, 2H).

### Example Compound 85

### Preparation of 2-chloro-4-((1-(4-fluorophenyl)-1H-pyrazol-4-yl)ethynyl)pyridine:

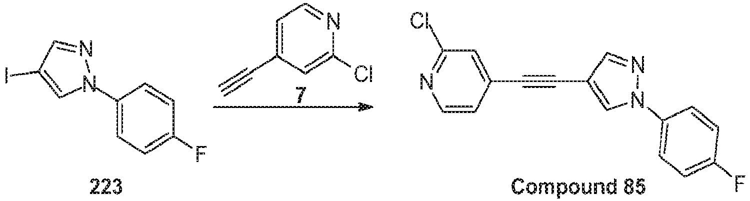

To a solution of **223**(100mg, 0.347mmol) and **7**(39.4mg, 0.382mmol) in 20mL of Et₃N was added Pd(PPh₃)₂Cl₂ (12.18mg, 0.017mmol) and CuI (6.61mg, 0.035mmol). The mixture was protected under N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 85**(25mg, yield: 12.09%).
**LCMS:** *m*/*z* 298 (M+H)⁺;
**¹H NMR** (400 MHz, CDCl₃) δ 8.37 (d, *J*= 5.1, 0.8 Hz, 1H), 8.09 (s, 1H), 7.88 (s, 1H), 7.69-7.62 (m, 2H), 7.40 (s, 1 H), 7.29-7.26 (m, 1H), 7.22-7.14 (m, 2H).

### Example Compound 86

### Preparation of 2-((1-(4-fluorophenyl)-1H-pyrazol-4-yl)ethynyl)pyrimidine:

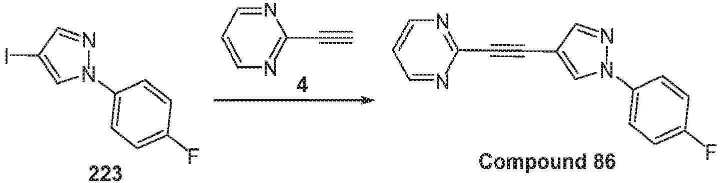

To a solution of **223**(200mg, 0.694mmol) and **4**(80mg, 0.764mmol) in 20mL of Et₃N was added Pd(PPh₃)₂Cl₂ (24.37mg, 0.035mmol) and CuI (13.22mg, 0.069mmol). The mixture was protected under N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 86(18mg,** yield: 9.80 %).
**LCMS:** *m*/*z* 265 (M+H)⁺;
**¹H NMR** (400 MHz, CDCl₃); δ 8.75 (d, *J* = 4.9 Hz, 2H), 8.16 (s, 1H), 7.96 (s, 1H), 7.69-7.61 (m, 2H), 7.25-7.22 (m, 1H), 7.22-7.11 (m, 2H).

### Example Compound 87

### Preparation of 4-((1-(4-fluorophenyl)-1H-pyrazol-4-yl)ethynyl)pyridine:

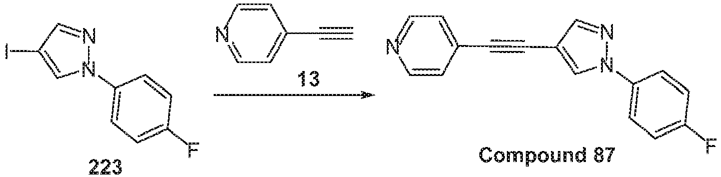

To a solution of **223**(100mg, 0.347mniol), **13**(39.4mg, 0.382mmol) in 20mL of Et₃N was added Pd(PPh₃)₂Cl₂ (12.18mg, 0.017mmol) and CuI (6.61mg, 0.035mmol). The mixture was protected under N₂ atmosphere, then was heated at 70°C for 4 hours. TLC analysis showed complete conversion of starting material to a major product. The reaction mixture was then concentrated in vacuo. The crude product was purified by Prep-HPLC to give the target product Compound **87**(20mg, yield: 21.88%).
LCMS: *m*/*z* 264 (M+H)⁺;
**¹H NMR** (400 MHz, CDCl₃): δ 8.61 (br, 2H), 8.10 (s, 1H), 7.79 (s, 1H), 7.68-7.64 (m, 2H), 7.38 (d, *J* = 5.6 Hz, 2H), 7.21-7.16 (m, 2H).

### Example Compound 88

### Preparation of 2-((1-(4-fluorophenyl)-1H-pyrazol-4-yl)ethynyl)pyrazine:

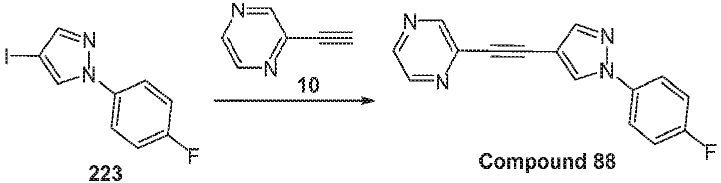

To a solution of **223**(100mg, 0.347mmol), **10**(36. 1mg, 0.347mmol) in 20 mL of Et₃N was added Pd(PPh₃)₂Cl₂ (244mg, 0.347mmol) and CuI (66.1mg, 0.347mmol). The mixture was protected under N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 88**(20mg, yield: 21.80%).
**LCMS:** *m*/*z* 265 (M+H)⁺;
**¹H NMR** (400 MHz, CDCl₃): δ 8.74 (d, J= 1.2 Hz, 1H), 8.58-8.57 (m, 1H), 8.49 (d, *J*= 2.4 Hz, 1H), 8.13 (s, 1H), 7.93 (s. 1H), 7.68-7.64 (m, 2H), 7.18 (t, *J*= 8.6 Hz, 2H).

### Example Compound 89

### Preparation of 4-(phenylethynyl)-1-(4-(trifluoromethoxy)phenyl)-1H-pyrazole:

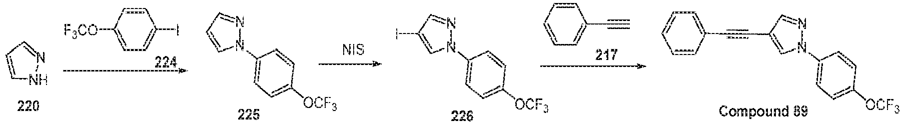

### Experimental section:

### Procedure for preparation of 225:

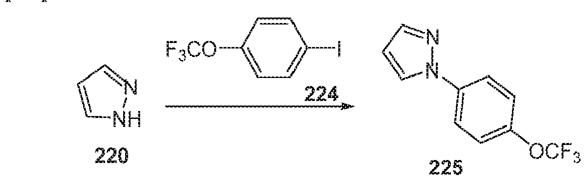

A mixture of **220**(0.613g, 9.01mmol), Cs₂CO₃ (4.40g, 13.51mmol), **224**(2.0g, 9.01mmol) and Cu(OAc)₂ (0.16g) in 20mL DMF. The mixture was heated at 120°C for the appropriate time and subsequent cooling, the reaction mixture was diluted with saturated aqueous ammonium chloride. Products were isolated by extraction with EA. The organic layer was dried over anhydrous Na₂SO_{4;} filtered, and concentrated. Products **225** were purified by silica gel column chromatography (1.40g, yield: 68%).

### Procedure for preparation of 226:

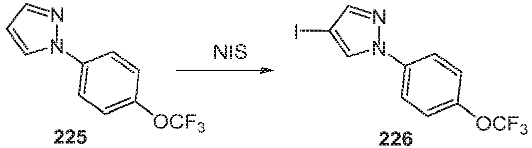

225(1.0 g) and NIS (1.66g) were added to AcOH (10ml). The reaction mixture was heated at 120°C via MW for 30 minutes. The reaction mixture was diluted with EA, washed with brine, then dried over Na₂SO₄, filtered, and concentrated. The residue was purified by column chromatography on silica gel to afford product **226**(0.70g, yield: 45%).

### Procedure for preparation of Compound 89:

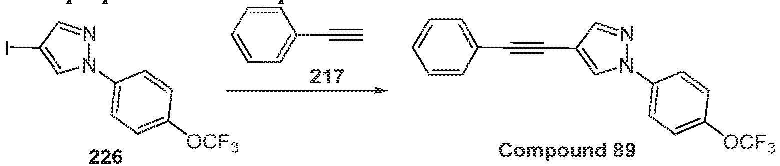

The solution of **226**(100mg, 0.282mmol), **217**(57.7mg, 0.565mmol) in 20mL of Et₃N was added Pd(PPh₃)₂Cl₂ (9.91mg, 0.014mmol) and CuI (5.38mg, 0.028mmol). The mixture was protected with N₂ atmosphere, then was heated at 70°C for 4h. TLC analysis showed complete conversion of starting material to a major product. The reaction mixture was then concentrated in vacuo. The crude product was purified by Prep-HPLC to give the target product **Compound 89**(25mg, yield: 27.0%).
**LCMS:** *m*/*z* 329 (M+H)⁺;
**¹H NMR** (400 MHz, CDCl₃); δ 8.09 (s, 1H), 7.87 (s, 1H), 7.74-7.72 (m, 2H), 7.53-7.50 (m, 2H), 7.36-7.32 (m, 5H).

### Example Compound 90

### Preparation of 2-((1-(4-(trifluoromethoxy)phenyl)-1H-pyrazol-4-yl)ethynyl)pyridine:

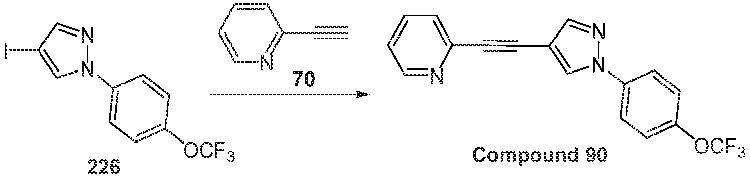

To a solution of **26**(100mg, 0.282mmol) and 70(58.2mg, 0.565mmol) in 20mL of Et₃N was added Pd(PPh₃)₂Cl₂ (9.91mg, 0.014mmol)and CuI (5.38mg, 0.028mmol). The mixture was protected under N₂ atmosphere, then was heated at 70 °C for 4 hours. TLC analysis showed complete conversion of starting material to a major product. The reaction mixture was then concentrated in vacuo. The crude product was purified by Prep-HPLC to give the target product **Compound 90**(20mg, yield: 21.6%).
**LCMS:** *m*/*z* 330 (M+H)⁺;
**¹H NMR** (400 MHz, CDCl₃): δ8.65 (br, 1H), 8.15 (s, 1H), 7.92 (s, 1H), 7.72 (m, 3H), 7.52 (br, 1H), 7.34 (dd, *J*= 9.0, 1.0 Hz, 2H), 7.27 (br, 1H).

### Example Compound 91

### Preparation of 3-((1-(4-(trifluoromethoxy)phenyl)-1H-pyrazol-4-yl)ethynyl)pyridine:

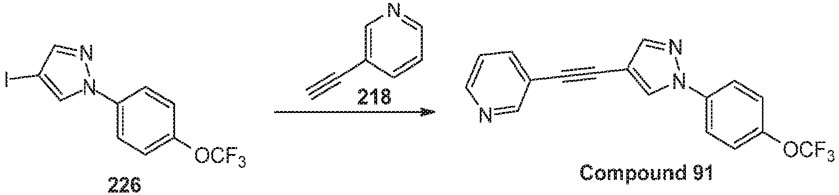

To a solution of **226**(100mg, 0.282mmol) and **218**(58.2mg, 0.565mmol) in 20mL of Et₃N was added Pd(PPh₃)₂Cl₂ (9.91mg, 0.014mmol)and CuI (5.38mg, 0.028mmol). The mixture was protected under N₂ atmosphere, then was heated at 70 °C for 4 hours. TLC analysis showed complete conversion of starting material to a major product. The reaction mixture was then concentrated in vacuo. The crude product was purified by Prep-HPLC to give the target product **Compound 91**(23mg, yield: 24.73%).
**LCMS:** *m*/*z* 330 (M+H)⁺;
**¹H NMR** (400 MHz, CDCl₃); δ 8.75 (d, *J*= 1.2 Hz, 1H), 8.57-8.55 (m, 1H), 8.12 (d, *J* = 0.4 Hz, 1H), 7.89 (s, 1H), 7.81 (d, *J* = 8 Hz, 1H), 7.74-7.72 (m, 2H), 7.36-7.33 (m, 3H).

### Example Compound 92

### Preparation of 4-((3-chlorophenyl)ethynyl)-1-(4-(trifluoromethoxy)phenyl)-1H-pyrazole :

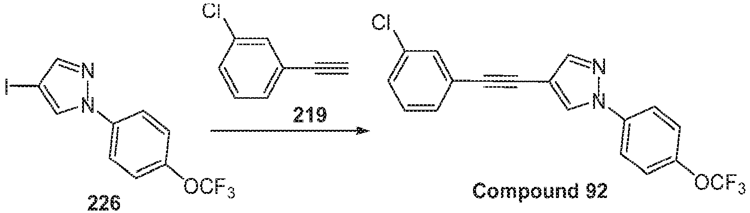

To a solution of **226**(100mg, 0.282mmol) and **219**(38.6mg, 0.282mmol) in 20mL of Et₃N was added Pd(PPh₃)₂Cl₂ (9.91mg, 0.014mmol) and CuI (5.38mg, 0.028mmol). The mixture was protected under N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 92**(23mg, yield: 22.45%).
**LCMS:** *m*/*z* 363 (M+H)⁺;
**¹H NMR** (400 MHz, CDCl₃): δ 8.09 (d, *J* = 0.4 Hz, 1H), 7.87 (s, 1H), 7.73 (d, *J*= 9.6 Hz, 2H), 7.50 (d, *J*= 1.6 Hz, 1H), 7.39-7.28 (m, 5H).

### Example Compound 93

### Preparation of 2-((1-(4-(trifluoromethoxy)phenyl)-1H-pyrazol-4-yl)ethynyl) pyrimidine:

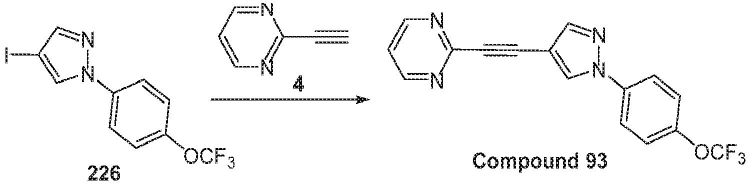

To a solution of **226**(100mg, 0.282mmol) and **4**(58.8mg, 0.565mmol) in 20mL of Et₃N was added Pd(PPh₃)₂Cl₂ (9.91mg, 0.014-mmol) and CuI (5.38mg, 0.028mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 93**(20mg, yield: 21.44%).
**LCMS:** *m*/*z* 331 (M+H)⁺;
**¹H NMR** (400 MHz, CDCl₃): δ 8.76 (d, *J*= 5.2 Hz, 2H), 8.21 (d, *J* = 0.8 Hz, 1H), 7.98 (s, 1H), 7.73 (d, *J* = 9.2 Hz, 2H), 7.36-7.34 (m, 2H), 7.26 (d, *J* = 9.6 Hz, 1H).

### Example Compound 94

### Preparation of 4-((1-(4-(triflluoromethoxy)phenyl)-1H-pyrazol-4-yl)ethynyl)pyridine:

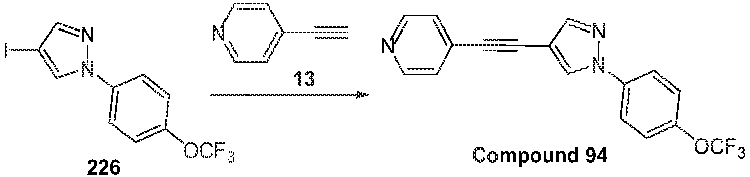

To a solution **226**(100mg, 0.282mmol) and **13**(58.2mg, 0.565mmol) in 20mL of Et₃N was added Pd(PPh₃)₂Cl₂ (9.91mg, 0.014mmol) and CuI (5.38mg, 0.028mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 94**(19mg, yield: 20.43%).
**LCMS:** *m*/*z* 330 (M+H)⁺;
**¹H NMR** (400 MHz, DMSO-*d₆*): δ 9.06 (s, 1H), 8.63-8.62 (m, 2H), 8.13 (s, 1H), 8.02-7.99 (m, 2H), 7.56 (d, *J* = 8.0 Hz, 2H), 7.49-7.48 (m, 2H).

### Example Compound 95

### Preparation of 2-((1-(4-(trifluoromethoxy)phenyl)-1H-pyrazol-4-yl)ethynyl) pyrazine:

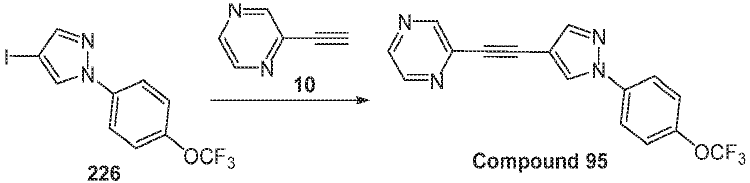

To a solution of **226**(100mg, 0.282mmol) and **10**(58.8mg, 0.565mmol) in 20mL of Et₃N was added Pd(PPh₃)₂Cl₂ (9.91mg, 0.014mmol) and CuI (5.38mg, 0.028mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 95**(20mg, yield: 21.44%).
**LCMS:** *m*/*z* 331 (M+H)⁺;
**¹H NMR** (400 MHz, CDCl₃); δ 8.78 (br, 1H), 8.60-8.51 (m, 2H), 8.18 (s. 1H), 7.95 (s. 1H), 7.76-7.72 (m, 2H), 7.35 (d, *J*= 8.0 Hz, 2H).

### Example Compound 96

### Preparation of 3-fluoro-5-(5-methyl-3-(phenylethynyl)-1H-pyrazol-1-yl) benzonitrile:

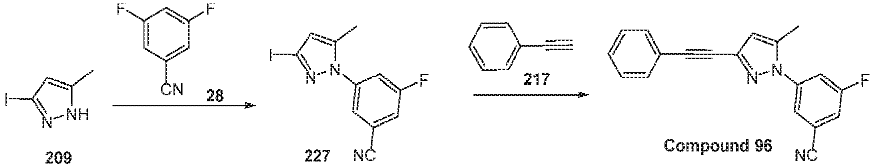

### Experimental section :

### Procedure of preparation of 227:

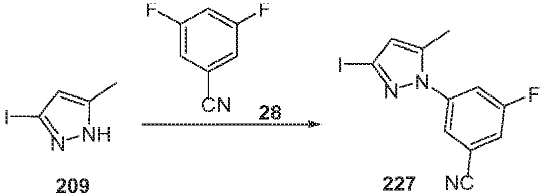

To a solution of compound **209**(2 g, 9.62 mmol) in DMF (20 mL) was added NaH (0.38 g of 60% dispersion in oil). The resulting mixture was stirred for 15 minutes at 60°C, then **28**(2.68 g, 19.23 mmol) was added, and the mixture was heated at 70°C for another 45∼60 minutes (tracking with TLC). The reaction mixture was diluted with EA, and washed with H20 and saturated NaCl. The organic phase was dried over Na₂SO_{4;} filtered, and concentrated in vacuo to give crude product, which was purified via Prep-HPLC to give product **227**(1.45g, yield: 46.26).

### Procedure of preparation of Compound 96:

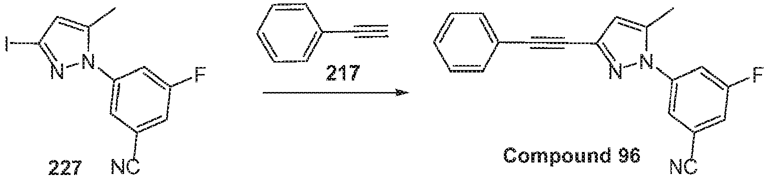

To a solution of **227**(1 00mg, 0.306mmol) and **217**(0.067ml, 0.611mmol) in 20mL of Et₃N was added Pd(PPh₃)₂Cl₂ (10.73mg, 0.015mmol) and CuI (5.82mg, 0.031mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 96**(29mg, yield: 31.5%).
**LCMS:** *m*/*z* 302 (M+H)⁺;
**¹H NMR** (400 MHz, CDCl₃): δ 7.67 (d, *J* = 0.8 Hz, 1H), 7.58-7.56 (m, 3H), 7.38-7.35 (m, 4H), 6.45 (d, *J*= 0.8 Hz, 1H), 2.46 (d, *J*= 0.8 Hz, 3H).

### Example Compound 97

### Preparation of 3-fluoro-5-(5-methyl-3-(pyridin-2-ylethynyl)-1H-pyrazol-1yl) benzonitrile:

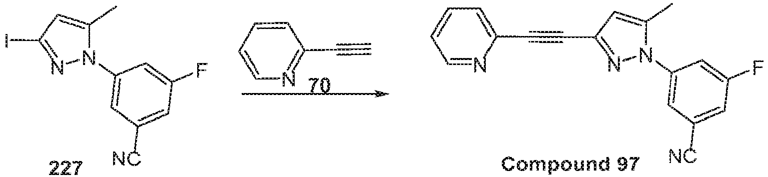

To a solution of **227**(100mg, 0.306mmol) and **70**(0.062ml, 0.611mmol) in 20mL of Et₃N was added Pd(PPh₃)₂Cl₂ (10.73mg, 0.015mmol) and CuI (5.82mg, 0.031mmol). The mixture was protected with N₂ atmosphere, then was heated at 70 °C for 24 hours. TLC analysis showed complete conversion of starting material to a major product. The reaction mixture was then concentrated in vacuo. The crude product was purified by Prep-HPLC to give the target product **Compound 97**(21mg, yield: 22.72%).
**LCMS**: *m*/*z* 303 (M+H)⁺;
**¹H NMR** (400 MHz, CDCl₃): δ 8.65-8.63 (m, 1H), 7.71-7.67 (m, 2H), 7.59-7.57 (m, 2H), 7.40 (d, *J*= 7.6 Hz, 1H), 7.28 (d, *J*= 7.6 Hz, 1H), 6.52 (d, *J*= 0.8 Hz, 1H), 2.46 (d, *J*= 0.8 Hz, 3H).

### Example Compound 98

### Preparation of 3-fluoro-5-(5-methyl-3-(pyridin-3-ylethynyl)-1H-pyrazol-1-yl) benzonitrile:

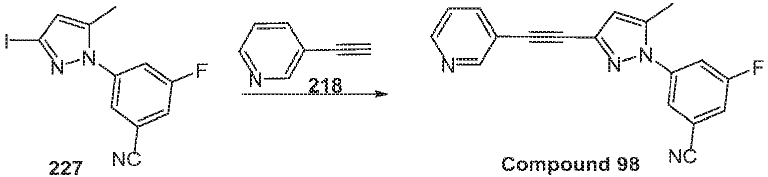

To a solution of **227**(100mg, 0.306mmol) and **218**(63.1mg, 0.611 mmol) in 20mL of Et₃N was added Pd(PPh₃)₂Cl₂ (10.73mg, 0.015mmol) and CuI (5.82mg, 0.031mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 98**(21mg, yield: 22.72%).
**LCMS:** *m*/*z* 303 (M+H)⁺;
**¹H NMR** (400 MHz, CDCl₃); δ 8.60-8.85 (m, 2H), 7.86 (d, 1H), 7.67 (s, 1H),7.58 (m, 1H), 7.40 (m, 1H), 7.35 (br, 1H), 6.48 (s, 1H), 2.46 (s, 3H).

### Example Compound 99

### Preparation of 3-(3-((3-chlorophenyl)ethynyl)-5-methyl-1H-pyrazol-1-yl)-5-fluorobenzonitrile:

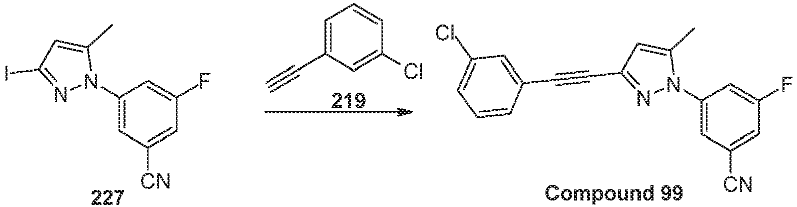

To a solution of **227**(1 00mg, 0.306mmol) and **2219**(0.075ml, 0.611mmol) in 20mL of Et₃N was added Pd(PPh₃)₂Cl₂ (10.73mg, 0.015mmol) and CuI (5.82mg, 0.031mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 99**(20mg, yield: 19.48%).
**LCMS:** *m*/*z* 336 (M+H)⁺;
**¹H NMR** (400 MHz, CDCl₃): δ 7.67 (s, 1H), 7.59-7.55 (m, 2H), 7.4-7.29 (m, 4H), 6.45 (s, 1H), 2.46 (s, 3H).

### Example Compound 100

### Preparation of 3-(3-((2-chloropyridin-4-yl)ethynyl)-5-methyl-1H-pyrazol-1-yl)-5-fluorobetizonitrile:

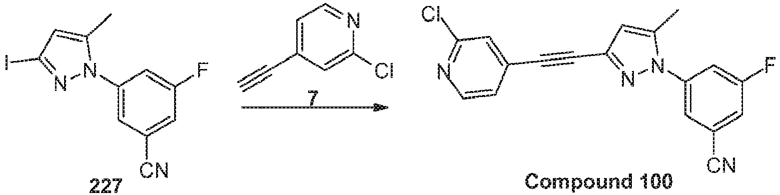

To a solution of **227**(100mg, 0.306mmol) and **7**(63.7mg, 0.611mmol) in 20mL of Et₃N was added Pd(PPh₃)₂Cl₂ (10.73 mg, 0.015mmol) and CuI (5.82mg, 0.031mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 100**(19mg, yield: 18.46%).
**LCMS:** *m*/*z* 337 (M+H)⁺;
**¹H NMR** (400 MHz, CDCl₃): δ 8.40-8.39 (m, 1H), 7.66 (s, 1H), 7.59-7.56 (m, 1H), 7.47 (s, 1H), 7.44-7.41 (m, 1H). 7.34-7.33 (m, 1H), 6.50 (s, 1H), 2.47 (s, 3H).

### Example Compound 101

### Preparation of 3-fluoro-5-(5-methyl-3-(pyrimidin-2-ylethynyl)-1H-pyrazol-1-yl) Benzonitrile:

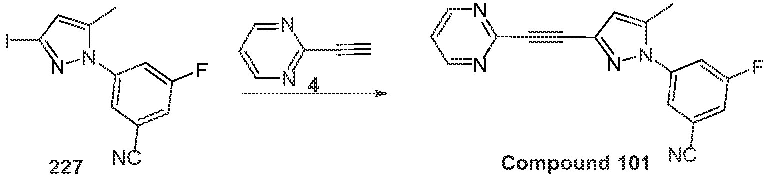

To a solution of **227**(100mg, 0.306mmol) and **4**(63.7mg, 0.611mmol) in 20mL of Et₃N was added Pd(PPh₃)₂Cl₂ (10.73mg, 0.015mmol) and CuI (5.82mg, 0.031mmol). The mixture was protected with N₂ atmosphere, then was heated at 100°C for 24 hours. TLC analysis showed complete conversion of starting material to a major product. The reaction mixture was then concentrated in vacuo. The crude product was purified by Prep-HPLC to give the target product **Compound 101**(15mg, yield: 16.2%).
**LCMS:** *m*/*z* 304 (M+H)⁺;
**¹H NMR** (400 MHz, CDCl₃); δ 8.77 (d, *J =* 4.9 Hz, 2H), 7.66 (s, 1H), 7.59 (dt, *J*= 9.2, 2.2 Hz, 1H), 7.45+7.34 (m, 1H), 7.30-7.26 (m, 1H), 6.55 (s, 1H), 2.46 (s, 4H).

### Example Compound 102

### Preparation of 3-fluoro-5-(5-methyl-3-(pyridin-4-ylethynyl)-1H-pyrazol-1-yl) benzonitrile:

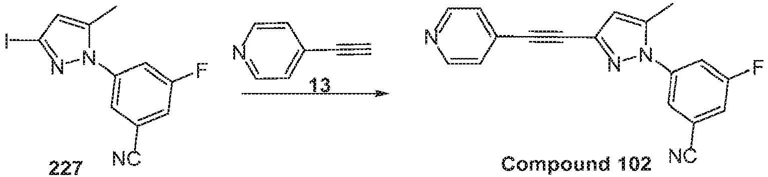

To a solution of **227**(100mg, 0.306mmol) and **13**(63.1mg, 0.61 1mmol) in 20mL of Et₃N was added Pd(PPh₃)₂Cl₂ (10.73mg, 0.015mmol) and CuI (5.82mg, 0.031mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 102**(16mg, yield: 17.31%).
**LCMS:** *m*/*z* 303 (M+H)⁺;
**¹H NMR** (400 MHz, CDCl₃): δ 8.64 (br, 2H), 7.67-7.66 (m, 1H), 7.60-7.56 (m, 1H), 7.44-7.40 (m, 3H), 6.49 (d, *J*= 1.2 Hz, 1H), 2.47 (d, *J*= 0.8 Hz, 3H).

### Example Compound 103

### Preparation of 3-fluoro-5-(5-methyl-3-(pyrazin-2-ylethynyl)-1H-pyrazol-1-yl) Benzonitrile:

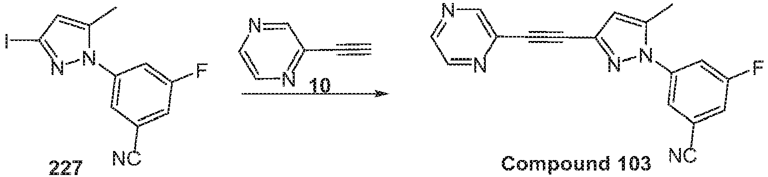

To a solution of **227**(100mg, 0.306mmol) and **10**(63.7mg, 0.61 1mmol) in 20mL of Et₃N was added Pd(PPh₃)₂Cl₂ (10.73mg, 0.015mmol) and CuI (5.82mg, 0.031mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product Compound **103**(18mg, yield: 19.41%).
**LCMS:** *m*/*z* 304 (M+H)⁺;
**¹H NMR** (400 MHz, CDCl₃); δ 8.66-8.65 (m, 3H), 7.67 (s, 1H), 7.60-7.57 (m, 1H), 7.43-7.40 (m, 1H). 6.55 (s, 1H), 2.47 (s, 3H).

### Example Compound 104

### Preparation of 3-fluoro-5-(3-(phenylethynyl)-1H-pyrazol-1-yl)benzonitrile:

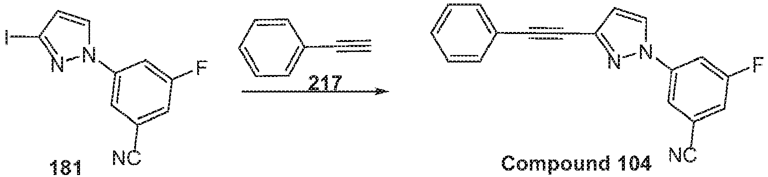

To a solution of **181**(100mg, 0.319mmol) and **217**(0.070ml, 0.639mmol) in 20mL of Et₃N was added Pd(PPh₃)₂Cl₂ (11.21mg, 0.01 mmol) and CuI (6.08mg, 0.032nimol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 104**(28 mg, yield: 30.5%).
**LCMS**: *m*/*z* 288 (M+H)⁺;
**¹H NMR** (400 MHz, CDCl₃): δ 7.94 (d, *J* = 2.8 Hz, 1H), 7.85 (d, *J* = 0.8 Hz, 1H), 7.79 (d, *J* = 9.2 Hz, 1H), 7.61-7.58 (m, 2H), 7.39-7.36 (m, 3H), 7.30-7.28 (m, 1H), 6.73 (d, *J* = 1.4 Hz, 1H).

### Example Compound 105

### Preparation of 3-fluoro-5-(3-(pyridin-2-ylethynyl)-1H-pyrazol-1-yl)benzonitrile:

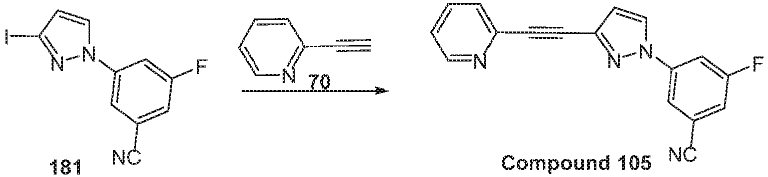

To a solution of **181**(100mg, 0.319mmol) and **70**(65.9mg, 0.639mmol) in 20mL of Et₃N was added Pd(PPh₃)₂Cl₂ (11.21mg, 0.016mmol) and CuI (6.08mg, 0.032mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 105**(18mg, yield: 19.55%).
**LCMS:** *m*/*z* 289 (M+H)⁺;
**¹H NMR** (400 MHz, CDCl₃): δ 8.66 (br, 1H), 7.95 (d, *J* = 2.4 Hz, 1H), 7.85 (s, 1H), 7.80-7.73 (m, 2H), 7.62 (d, *J* = 8.4 Hz, 1H), 7.33-7.29 (m, 2H), 6.81 (d, *J* = 2.4 Hz, 1H).

### Example Compound 106

### Preparation of 3-fluoro-5-(3-(pyridin-3-ylethynyl)-1H-pyrazol-1-yl)benzonitrile:

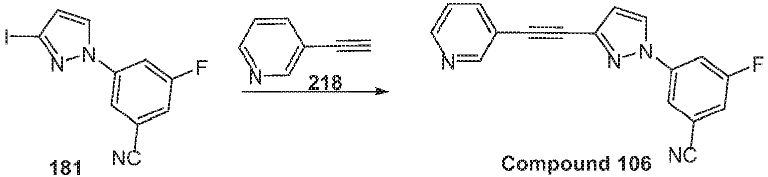

To a solution of **181**(100mg, 0.319mmol) and **218**(65.9mg, 0.639mmol) in 20mL of Et₃N was added Pd(PPh₃)₂Cl₂ (11.21mg, 0.016mmol) and CuI (6.08mg, 0.032mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 106**(15mg, yield: 16.29%).
**LCMS:** *m*/*z* 289 (M+H)⁺;
**¹H NMR** (400 MHz, CDCl₃): δ 8.83 (s, 1H), 8.60 (d, *J =* 3.6 Hz, 1H), 7.96 (d, *J* = 2.8 Hz, 1H), 7.89 (d, *J=* 8.0 Hz, 1H), 7.86-7.85 (m, 1H), 7.81-7.78 (m, 1H), 7.36-7.30 (m, 2H), 6.76 (d, *J=* 2.8 Hz, 1 H).

### Example Compound 107

### Preparation of 3-(3-((3-chlorophenyl)ethynyl)-1H-pyrazol-1-yl)-5-fluorobenzonitrile:

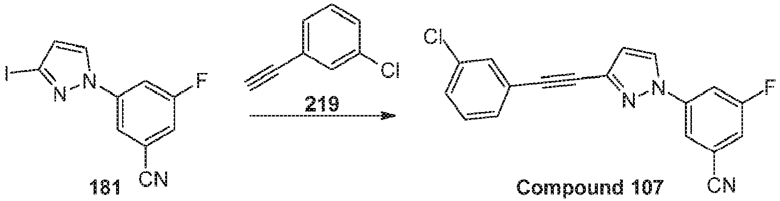

To a solution of **181**(100mg, 0.319mmol) and **219**(0.079ml, 0.639mmol) in 20mL of Et₃N was added Pd(PPh₃)₂Cl₂ (11.21mg, 0.016mmol) and CuI (6.08mg, 0.032mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 107**(25mg, yield: 24.33%).
**LCMS:** *m*/*z* 322(M+H)⁺;
**¹H NMR** (400 MHz, CDCl₃): δ 7.95 (d, *J* = 2.4 Hz, 1H), 7.84 (s, 1H), 7.80-7.77 (m, 1H), 7.58-7.57 (m, 1H), 7.48-7.46 (m, 1H), 7.36-7.29 (m, 3H), 6.73 (d, *J* = 2.8 Hz, 1H).

### Example Compound 108

### Preparation of 3-(3-((2-chloropyridin-4-yl)ethynyl)-1H-pyrazol-l-yl)- 5-fluorobenzonitrile:

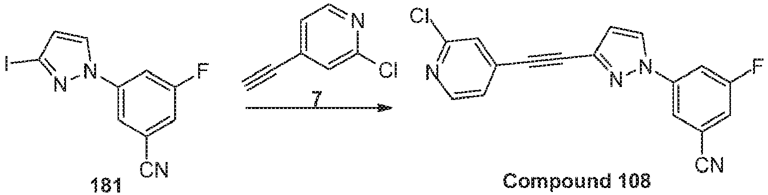

To a solution of **181**(1 00mg, 0.319mmol) and 7(88mg, 0.639mmol) in 20mL of Et₃N was added Pd(PPh₃)₂Cl₂ (11.21mg, 0.016mmol)and CuI (6.08mg, 0.032mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 108**(15mg, yield: 14.55%).
**LCMS:** *m*/*z* 323(M+H)⁺;
**¹H NMR** (400 MHz, CDCl₃), δ 8.43-8.41 (m, 1H), 7.98 (d, *J* = 2.8 Hz, 1H), 7.85 (d, *J=* 0.8 Hz, 1H), 7.80-7.77 (m, 1H), 7.50 (d, *J* = 0.8 Hz, 1H), 7.37-7.32 (m, 2H), 6.78 (d, *J* = 2.8 Hz, 1H).

### Example Compound 109

### Preparation of 3-fluoro-5-(3-(pyrimidin-2-ylethynyl)-1H-pyrazol-1-yl) benzonitrile:

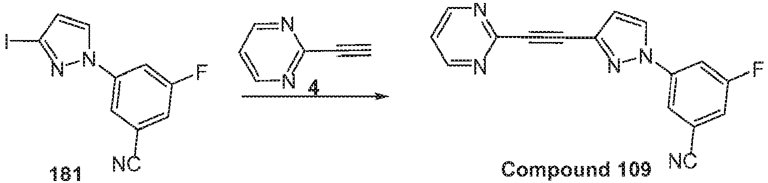

To a solution of **181**(100mg, 0.319mmol) and **4**(66.5mg, 0.639mmol) in 20mL of Et₃N was added Pd(PPh₃)₂Cl₂ (11.21mg, 0.016mmol)and CuI (6.08mg, 0.032mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 109**(20mg, yield: 21.7%).
**LCMS:** *m*/*z* 290 (M+H)⁺;
**¹H NMR** (400 MHz, CDCl₃): δ 8.79 (d, *J* = 5.2 Hz, 2H), 7.96 (d, *J* = 2.4 Hz, 1H), 7.84 (s, 1H), 7.80 (d, *J*= 9.2 Hz, 1H), 7.31-7.29 (m, 2H), 6.84 (d, *J* = *2.4* Hz, 1H).

### Example Compound 110

### Preparation of 3-fluoro-5-(3-(pyridin-4-ylethynyl)-1H-pyrazol-1-yl)benzonitrile:

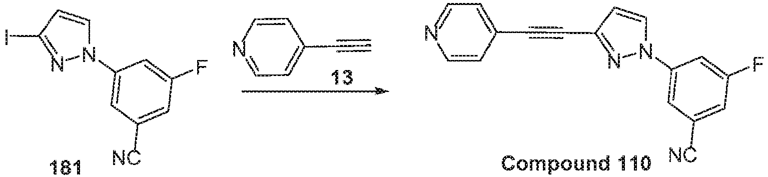

To a solution of **181**(100mg, 0.319mmol) and 13(65.9mg, 0.639mmol) in 20mL of Et₃N was added Pd(PPh₃)₂Cl₂ (11.21mg, 0.016mmol) and CuI (5.38mg, 0.028mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 110**(30mg, yield: 32.6%).
**LCMS:** *m*/*z* 289 (M+H)⁺_{;}
**¹H NMR** (400 MHz, CDCl₃) δ 8.65 (d, *J* = 4.8 Hz, 2H), 7.97 (d, *J* = 2.4 Hz, 1H), 7.86-7.85 (m, 1H), 7.80-7.78 (m, 1H), 7.47-7.45 (m, 2H), 7.33-7.31 (m, 1H), 6.78 (d, *J* = 2.4 Hz, 1H).

### Example Compound 111

### Preparation of 3-fluoro-5-(-3-(pyrazin-2-ylethynyl)-1H-pyrazol-1-yl)benzonitrile:

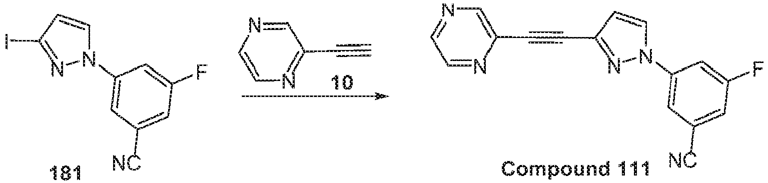

To a solution of **181**(1 00mg, 0.319mmol) and 10(66.5mg, 0.639mmol) in 20mL of Et₃N was added Pd(PPh₃)₂Cl₂ (11.21mg, 0.016mmol) and CuI (6.08mg, 0.032mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 111(18mg,** yield: 19.48%).
**LCMS:** *m*/*z* 290 (M+H)⁺;
**¹H NMR** (400 MHz, CDCl₃): δ 8.84 (d, *J =* 1.6 Hz, 1H), 8.63-8.62 (m, 1H), 8.55 (d, *J* = 2.4 Hz, 1H), 7.97 (d, *J* = 2.8 Hz, 1H), 7.85 (t, 1H), 7.79 (d, *J* = 9.2 Hz, 1H), 7.34-7.32 (m, 1H), 6.83 (d, *J=* 2.4 Hz, 1H).

### Example Compound 112

### Preparation of 2-((1-(4-fluorophenyl)-1H-pyrazol-3-yl)ethynyl)pyridine:

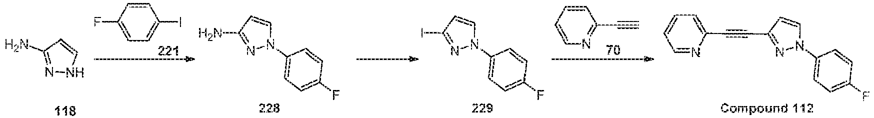

### Experimental section:

### Procedure for preparation 228:

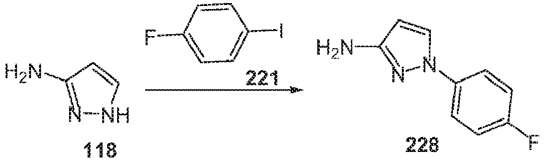

To a solution of **118**(2g, 24.07mmol), CuI (0.238g, 2.407mmol) and Cs₂CO₃ (9.41g, 28.9mmol) in 5mL of DMF was added **221**(5.34g, 24.07mmol), and the resulting mixture was heated at 140 °C via MW irradiation for 30 minutes. The mixture was cooled to room temperature, and diluted with EA, washed with H₂O (20mLx3). The combined organic layer was washed with saturated NaCl and dried over Na₂SO₄, filtered, and concentrated in vacuo. The residue was purified by column chromatography on silica gel to afford **228**(1g, yield: 23.45%).
**¹H NMR(400** MHz, DMSO-*d*₆): δ 8.09 (d, *J* = 2.5 Hz, 1H), 7.75-7.61 (m, 2H), 7.28- 7.16 (m, 2H), 5.73 (d, *J=* 2.5 Hz, 1H), 5.07 (s, 2H).

### Procedure for preparation 229:

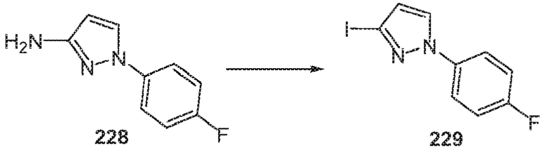

To a solution of **228**(0.5g, 2.82mmol) in concentrated HCI solution (18mL) was added a solution of NaNO₂ (0.195g, 2.82mmol) in water (2mL) over 3 minutes at 0°C. A solution of KI (0.468g, 2.82mmol) in water (3mL) was added to the reaction mixture over 5 minutes, resulting in nitrogen evolution. The reaction mixture was stirred for 5 minutes. Water was added, the aqueous mixture was extracted with EA, washed with NaS₂O₃two times, dried over Na₂SO₄, and concentrated in vacuo. The crude residue was purified by flash chromatography to obtain the target product **229**(0.1g, yield: 12.30%).
**¹H NMR** (400 MHz, CDCl₃): δ 7.67 (d, *J* = 2.5 Hz, 1H), 7.61-7.09 (m, 2H), 7.17-7.09 (m, 2H), 6.62 (d, *J* = 2.4 Hz, 1H).

### Procedure for preparation Compound 112:

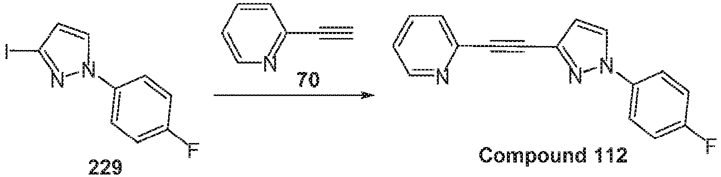

To a solution of **229**(100mg, 0.347mmol) and 70(0.070ml, 0.694mmol) in 20mL of Et₃N was added Pd(PPh₃)₂Cl₂ (12.18mg, 0.017mmol) and CuI (6.61mg, 0.035mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 112**(25mg, yield: 27.4 %).
**LCMS:** *m*/*z* 264 (M+H)⁺;
**¹HNMR** (400 MHz, CDCl₃): δ 8.64 (d, *J* = 4.8 Hz, 1H), 7.86 (d, *J* = 2.4 Hz, 1H), 7.72-7.67 (m, 3H), 7.61-7.59 (m, 1H), 7.30-7.27 (m, 1H), 7.19-7.14 (m, 2H), 6.75 (d, *J=* 2.4 Hz, 1H).

### Example Compound 113

### Preparation of 1-(4-fluorophenyl)-3-(phenylethynyl)-1H-pyrazole:

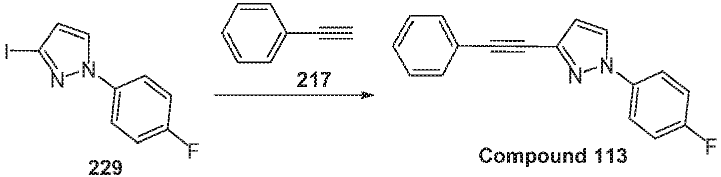

To a solution of **229**(75mg, 0.260mmol) and **217**(0.042mL, 0.382mmol) in 20mL of Et₃N was added Pd(PPh₃)₂Cl₂ (9.14mg, 0.013mmol) and CuI (4.96mg, 0.026mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 113**(20mg, yield: 29.3%).
**LCMS:** *m*/*z* 263 (M+H)⁺;
**¹H NMR** (400 MHz, CDCl₃): δ 7.85 (d, *J* = 2.5 Hz, 1H), 7.72-7.66 (m, 2H), 7.61-7.56 (m, 2H), 7.39-7.34 (m, 3H), 7.20-7.12 (m, 2H), 6.67 (d, *J* = 2.5 Hz, 1H).

### Example Compound 114

### Preparation of 3-((1-(4-fluorophenyl)-1H-pyrazol-3-yl)ethynyl)pyridine:

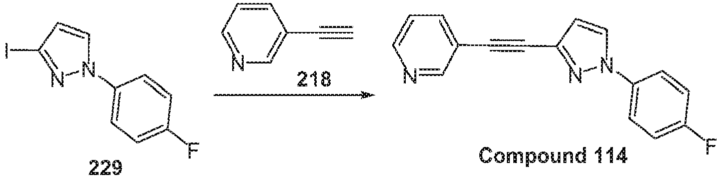

To a solution of **229**(100mg, 0.347mmol) and **218**(71.6mg, 0.694mmol) in 20mL of Et₃N was added Pd(PPh₃)₂Cl₂ (12.18mg, 0.017mmol) and CuI (6.61mg, 0.035mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 114**(19mg, yield: 20.79%).
**LCMS:** *m*/*z* 264 (M+H)⁺;
**¹H NMR** (400 MHz, DMSO-*d*₆): δ 8.83-8.61 (m, 3H), 8.03 (d, *J* = 7.6 Hz, 1H), 7.95-7.90 (m, 2H), 7.51-7.49 (m, 1H), 7.42-7.37 (m, 2H), 6.89 (d, *J* = 2.8 Hz, 1H).

### Example Compound 115

### Preparation of 3-((3-chlorophenyl)ethynyl)-1-(4-fluorophenyl)-1H-pyrazole:

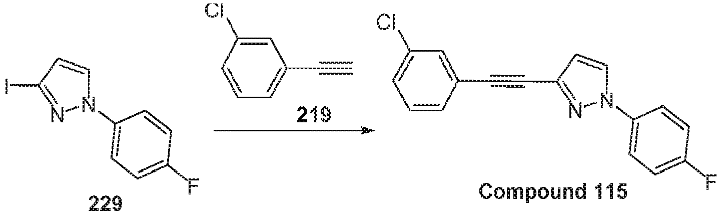

To a solution of **229**(100mg, 0.347mmol) and **219**(0.085ml, 0.694mmol) in 20mL of Et₃N was added Pd(PPh₃)₂Cl₂ (12.18mg, 0.017mmol) and CuI (6.61mg, 0.035mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 115**(21mg, yield: 20.39%).
**LCMS:** m/z 297 (M+H)⁺;
**¹HNMR** (400 MHz, CDCl₃): δ 7.86 (d, *J* = 2.4 Hz, 1H), 7.71-7.67 (m, 2H), 7.57-7.56 (m, 1H), 7.46 (d, *J* = 7.6 Hz, 1H), 7.33-7.29 (m, 2H), 7.19-7.14 (m, 2H), 6.67 (d, *J* = 2.4 Hz, 1H).

### Example Compound 116

### Preparation of 2-chloro-4-((1-(4₋fluorophenyl)-1H-pyrazol-3-yl)ethynyl)pyridine:

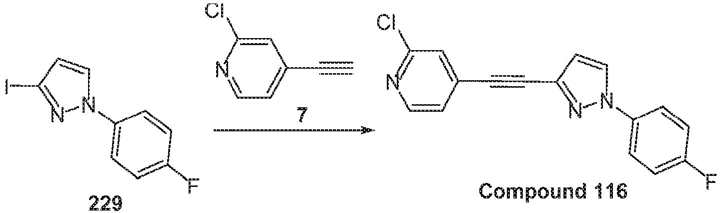

To a solution of **229**(100mg, 0.347mmol) and 7(96mg, 0.694mmol) in 20mL of Et₃N was added Pd(PPh₃)₂Cl₂ (12.18mg, 0.017mmol) and CuI (6.61mg, 0.035mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 116**(20mg, yield: 19.35%).
**LCMS:** *m*/*z* 298 (M+H)⁺;
**¹H NMR** (400 MHz, CDCl₃): δ 8.40-8.38 (m, 1H), 7.88 (d, *J =* 2.8 Hz, 1H), 7.70-7.67 (m, 2H), 7.48 (d, *J* = 0.8 Hz, 1H), 7.36-7.34 (m, 1H), 7.20-7.16 (m, 2H), 6.72 (d, *J* = 2.4 Hz, 1H).

### Example Compound 117

### Preparation of 2-((1-(4-fluorophenyl)-1H-pyrazol-3-yl)ethynyl)pyrimidine:

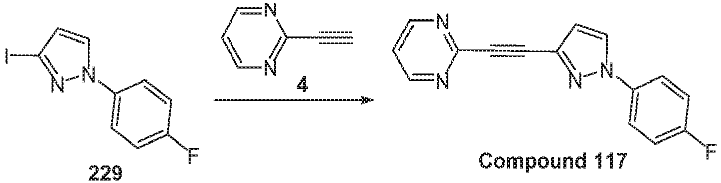

To a solution of **229**(100mg, 0.347mmol) and **4**(72.3mg, 0.694mmol) in 20mL of Et₃N was added Pd(PPh₃)₂Cl₂ (12.18mg, 0.017mmol) and CuI (6.61mg, 0.035mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 117**(18mg, yield: 19.62%).
**LCMS:** *m*/*z* 265 (M+H)⁺;
**¹H NMR** (400 MHz, CDCl₃): δ 8.77 (d, *J=* 4.8 Hz, 2H), 7.87 (d, *J* = 2.4 Hz, 1H), 7.71-7.68 (m, 2H), 7.28-7.26 (m, 1H), 7.19-7.15 (m, 2H), 6.79 (d, *J* = 2.8 Hz, 1H).

### Example Compound 118

### Preparation of-(4-fluorophenyl)-1H-pyrazol-3-yl)ethynyl)pyridine:

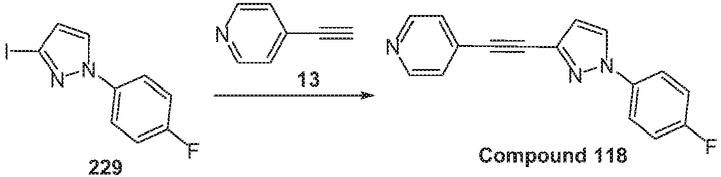

To a solution of **229**(100mg, 0.347mmol) and **13**(71.6mg, 0.694mmol) in 20mL of Et₃N was added Pd(PPh₃)₂Cl₂ (12.18mg, 0.017mmol) and CuI (6.61mg, 0.035mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 118**(20mg, yield: 21.88%).
**LCMS:** *m*/*z* 264 (M+H)⁺;
**¹H NMR** (400 MHz, CDCl₃); δ 8.63 (br, 2H), 7.88 (d, *J* = 2.4 Hz, 1H), 7.71-7.68 (m, 2H), 7.47 (d, *J* = 5.2 Hz, 2H), 7.20-7.16 (m, 2H), 6.72 (d, *J* = 2.4 Hz, 1H).

### Example Compound 119

### Preparation of 2-((1-(4-fluorophenyl)-1H-pyrazol-3-yl)ethynyl)pyrazine:

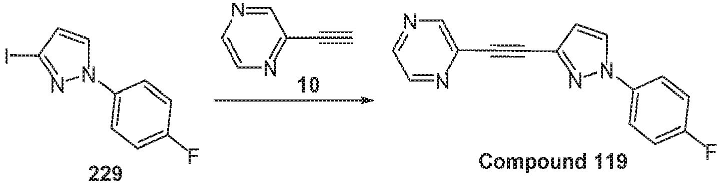

To a solution of **229**(100mg, 0.347mmol) and **10**(72.3mg, 0.694mmol) in 20mL of Et₃N was added Pd(PPh₃)₂Cl₂ (12.18mg, 0.017mmol) and CuI (6.61mg, 0.035mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 119**(23mg, yield: 25.07%).
**LCMS:** *m*/*z* 265 (M+H)⁺;
**¹H NMR** (400 MHz, CDCl₃): δ 8.82 (d, *J=* 1.2 Hz, 1H), 8.61-8.60 (m, 1H), 8.52 (d, *J* = 2.4 Hz, 1H), 7.88 (d, *J* = 2.4 Hz, 1H), 7.71-7.68 (m, 2H), 7.20-7.15 (m, 2H), 6.77 (d, *J*= 2.4 Hz, 1H).

### Example Compound 120

### Preparation of 3-(phenylethynyl)-1-(4-(trifluoromethoxy)phenyl)-1H-pyrazole:

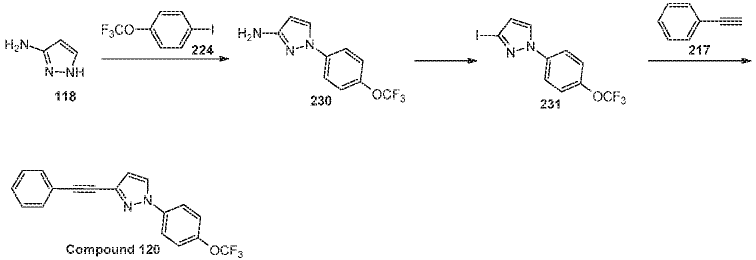

### Experimental section:

### Procedure for preparation of 230:

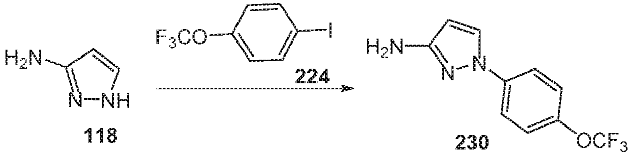

To a solution of **118**(2g, 24.07mmol), CuCl (0.238g, 2.407mmol) and Cs₂CO₃ (8.63g, 26.5mmol) in 5mL of DMF was added **224**(6.93g, 24.07mmol) and the resulting mixture was heated at 130 °C via MW irradiation for 30 minutes. The mixture was cooled to room temperature, and diluted with EA, washed with H₂O (20 mLx3). The combined organic layer was washed with saturated NaCl and dried over Na₂SO₄, filtered, and concentrated in vacuo. The residue was purified by column chromatography on silica gel to afford **230**(1g, yield: 17.08%).

### Procedure for preparation of 231:

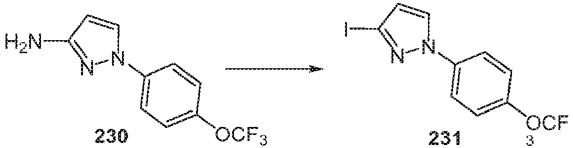

To a solution of **230**(0.5g, 2.056mmol) in concentrated HCl solution (10mL) was added a solution of NaNO₂ (0.170g, 2.467mmol) in water (2mL) over 3 minutes at 0°C. A solution of KI (0.444g, 2.67mmol) in water (3mL) was added to the reaction mixture over 5 minutes, resulting in nitrogen evolution. The reaction mixture was stirred for 5 minutes. Water was added, the aqueous mixture was extracted with EA, washed with NaS₂O₃ two times, dried over Na₂SO₄, and concentrated in vacuo. The crude residue was purified by flash chromatography to give the product **231**(0.2g, yield: 27.5%).
**LCMS:** *m*/*z* 355 (M+H)⁺.

### Preparation of Compound 120:

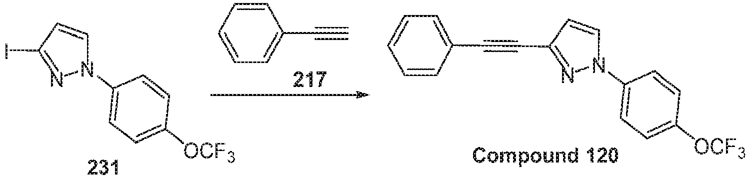

To a solution of 231(100mg, 0.282mmol) and 217(0.062ml, 0.565mmol) in 20mL of Et₃N was added Pd(PPh₃)₂Cl₂ (9.91mg, 0.014mmol) and CuI (5.38mg, 0.028mmol). The mixture was protected with N₂ atmosphere, then was heated at 70°C for 24 hours. TLC analysis showed complete conversion of starting material to a major product. The reaction mixture was then concentrated in vacuo. The crude product was purified by Prep-HPLC to give the target product **Compound 120**(23mg, yield: 24.81%).
**LCMS:** *m*/*z* 329 (M+H)⁺.
**¹H NMR** (400 MHz, CDCl₃): δ 7.90 (d, *J* = 2.4 Hz, 1H), 7.78-7.75 (m, 2H), 7.60-7.58 (m, 2H), 7.38-7.31 (m, 5H), 6.68 (d, *J* = 2.4 Hz, 1H).

### Example Compound 121

### Preparation of 3-((1-(4-(trifluoromethoxy)phenyl)-1H-pyrazol-3-yl) ethynyl) pyridine:

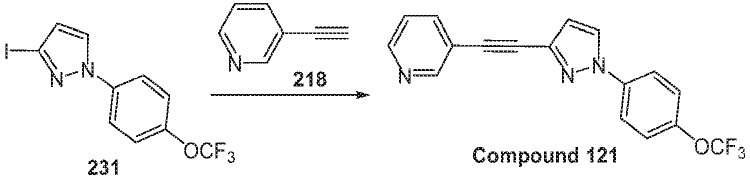

To a solution of **231**(100mg, 0.282mmol) and **218**(58.2mg, 0.565mmol) in 20mL of Et₃N was added Pd(PPh₃)₂Cl₂ (9.91mg, 0.014mmol) and CuI (5.38mg, 0.028mmol). The mixture was protected with N₂ atmosphere, then was heated at 70°C for 4 hours. TLC analysis showed complete conversion of starting material to a major product. The reaction mixture was then concentrated in vacuo. The crude product was purified by silica gel column chromatography to give the target product **Compound 121**(18mg, yield: 19.36%).
**LCMS:** *m*/*z* 330 (M+H)⁺;
*¹HNMR* (400 MHz, CDCl₃): δ 8.82 (s, 1H), 8.58 (s, 1H), 7.92 (d, *J* = 2.8 Hz, 1H), 7.88-7.85 (m, 1H), 7.78-7.74 (m, 2H), 7.34-7.29 (m, 3H), 6.71 (d, *J* = 2.4 Hz, 1H).

### Example Compound 122

### Preparation of 3-((3-chlorophenyl) ethynyl)-1-(4-(trifluoromethoxy)phenyl)-1H-pyrazole:

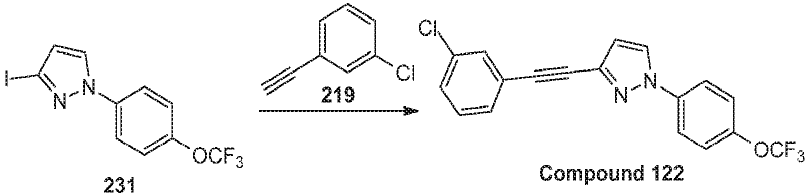

To a solution of **231**(100mg, 0.282mmol) and **219**(77mg, 0.565mmol) in 20mL of Et₃N was added Pd(PPh₃)₂Cl₂ (9.91mg, 0.014mmol) and CuI (5.38mg, 0.028mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 122**(22mg, yield: 21.47%).
**LCMS:** *m*/*z* 363 (M+H)⁺;
**¹H NMR** (400 MHz, CDCl₃): δ 7.91 (d, *J* = 2.4 Hz, 1H), 7.77-7.75 (m, 2H), 7.57 (d, *J=* 1.2 Hz, 1H), 7.46 (d, *J* = 7.2 Hz, 1H), 7.34-7.29 (m, 4H), 6.69 (d, *J* = 2.4 Hz, 1H).

### Example Compound 123

### Preparation of 2-((1-( 4-(trifluoromethoxy)phenyl)-1H-1H-pyrazol-3-yl)ethynyl) pyrimidine:

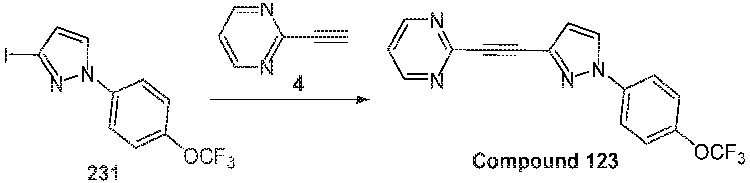

To a solution of **231**(100mg, 0.282mmol) and **4**(58.8mg, 0.565mmol) in 20mL of Et₃N was added Pd(PPh₃)₂Cl₂ (9.91mg, 0,0]4mmol) and CuI (5.38mg, 0.028mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 123**(22mg, yield: 23.59%).
**LCMS:** *m*/*z* 331 (M+H)⁺;
**¹H NMR** (400 MHz, CDCl₃): δ 8.77 (d, 4.8 Hz, 2H), 7.92 (d, *J* = 2.4 Hz, 1H), 7.78-7.76 (m, 2H), 7.34-7.32 (m, 2H), 7.29-7.26 (m, 1H), 6.80 (d, *J* = 2.8 Hz, 1H).

### Example Compound 124

### Preparation of 4-((1-(4-(trifluoromethoxy)phenyl)-1H-pyrazol-3-yl)ethynyl) pyridine:

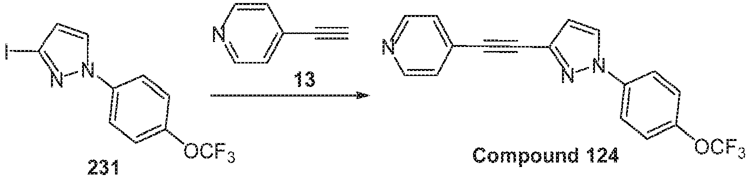

To a solution of **231**(100mg, 0.282mmol) and **13**(58.2mg, 0.565mmol) in 20mL of Et₃N was added Pd(PPh₃)₂Cl₂ (9.91mg, 0.014mmol) and CuI (5.38mg, 0.028mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 124**(19mg, yield: 20.43%).
**LCMS:** *m*/*z 330* (M+H)⁺*;*
**¹H NMR** (400 MHz, CDCl₃): δ 8.64 (s, 2H), 7.93 (d, *J* = 2.4 Hz, 1H), 7.78-7.74 (m, 2H), 7.45 (d, J = 5.2 Hz, 2H), 7.34 (d, *J* = 8.4 Hz, 2H), 6.73 (d, *J =* 2.4 Hz, 1H).

### Example Compound 125

### Preparation of2-((1-(4-(trifluoromethoxy)phenyl)-1H-pyrazol-3- yl)ethynyl) pyrazine:

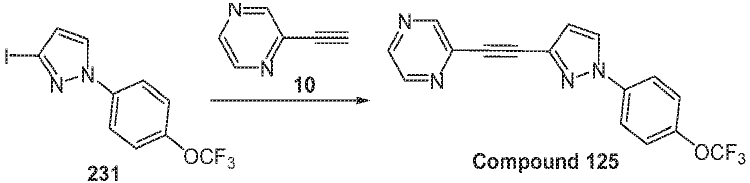

To a solution of **231**(100mg, 0.282mmol) and **10**(58.8mg, 0.565mmol) in 20mL of Et3N was added Pd(PPh₃)₂Cl₂ (9.91mg, 0.014mmol) and CuI (5.38mg, 0.028mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 125**(23mg, yield: 24.66%).
**LCMS**: *m*/*z* 331 (M+H)⁺;
¹**H NMR** (400 MHz, CDCl₃): δ 8.83 (d, *J* = 6.4 Hz, 1H), 8.62 (s, 1H), 8.54 (s, 1H), 7.93 (d, *J* = 2.4 Hz, 1H), 7.79-7.75 (m, 2H), 7.35-7.33 (m, 2H), 6.79 (d, *J* = 2.4 Hz, 1H).

### Example Compound 126

### Preparation of 2-((1-(4-fluorophenyl)-5-methyl-1H-pyrazol-3-yl)ethynyl)pyridine:

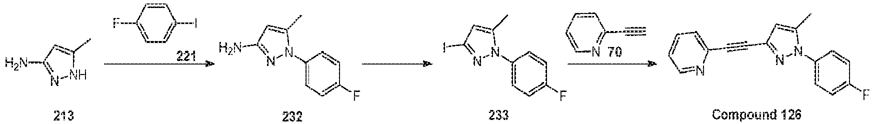

### Experimental section:

### Procedure for preparation of 232:

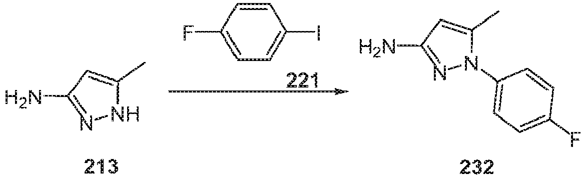

To a solution of **213**(1g, 10.30mmol), CuCl (0.102g, 1.030mmol), Cs₂CO₃ (4.03g, 12.36mmol) and quinolin-8-ol(0.149g, 1.030mmol) in 10mL of t-BuOH was added **221**(2.286g, 10.30mmol), and the resulting mixture was heated at 130°C via MW irradiation for 30 minutes. The mixture was cooled to room temperature, and diluted with EA, washed with H₂O (20 mLx3). The combined organic layer was washed with saturated NaCl and dried over Na₂SO₄, filtered, and concentrated in vacuo. The residue was purified by column chromatography on silica gel to afford 220(0.5g, yield: 25.4%).

### Procedure for preparation of 233:

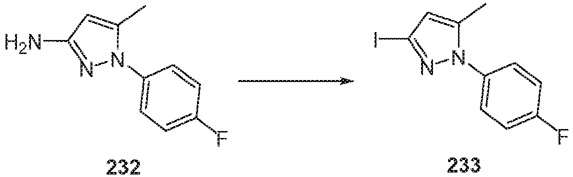

To a solution of **232**(1.2g, 6.28mmol) in concentrated HCl solution (18mL) was added a solution of NaNO₂ (0.650g, 9.41mmol) in water (2mL) over 3 minutes at 0°C. A solution of KI (1.302g, 7.84mmol) in water (3mL) was added to the reaction mixture over 5 minutes, resulting in nitrogen evolution. The reaction mixture was stirred for 15 minutes. Water was added, the aqueous mixture was extracted with EA, washed with NaS₂O₃ for two times, dried over Na₂SO₄, and concentrated in vacuo. The crude residue was purified by flash chromatography to give product **233**(0.5g, yield: 26.4%).
**LCMS**: *m*/*z* 303 (M+H)⁺.

### Preparation of Compound 126:

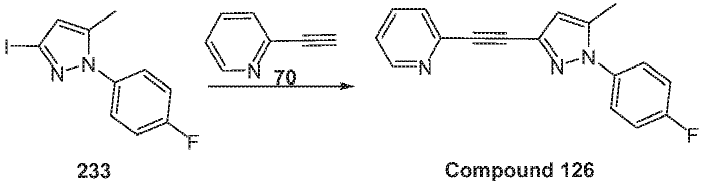

To a solution of **233**(100mg, 0.331mmol) and **70**(68.3mg, 0.662mmol) in 20mL of Et₃N was added Pd(PPh₃)₂Cl₂ (11.62mg, 0.017mmol) and CuI (6.30mg, 0.033mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 126**(25mg, yield: 27.2%).
**LCMS**: *m*/*z* 278 (M+H)⁺;
**¹H NMR** (400 MHz, CDCl₃): δ 8.61 (d, *J* = 4.0, 1H), 7.68-7.66 (m, 1H), 7.56-7.54 (m, 1H), 7.46-7.43 (m, 2H), 7.25 (d, *J* = 5.2 Hz, 1H), 7.19-7.15 (m, 2H), 6.47 (d, *J* = 0.8 Hz, 1H), 2.32 (d, *J* = 0.8 Hz, 3H).

### Example Compound 127

### Preparation of 3-((1-(4-fluorophenyl)-5-methyl-1H-pyrazol-3-yl)ethynyl)pyridine:

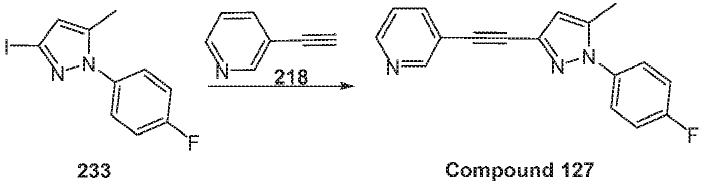

To a solution of **233**(100mg, 0.331mmol) and **218**(68.3mg, 0.662mmol) in 20mL of Et₃N was added Pd(PPh₃)₂Cl₂ (11.62mg, 0.017mmol) and CuI (6.30mg, 0.033mmol). The mixture was protected with N₂ atmosphere, then was heated at 70°C for 4 hours. TLC analysis showed complete conversion of starting material to major product. The reaction mixture was then concentrated in vacuo. The crude product was purified by Prep-HPLC to give the target product **Compound 127**(23mg, yield: 25.06%).
**LCMS:** *m*/*z* 278 (M+H)⁺;
¹**H NMR** (400 MHz, CDCl₃): δ 8.80 (br, 1H), 8.56 (br, 1H), 7.84 (d, *J* = 7.6 Hz, 1H), 7.47-7.43 (m, 2H), 7.31-7.28 (m, 1H), 7.20-7.16 (m, 2H), 6.43 (s, 1H), 2.33 (s, 3H).

### Example Compound 128

### Preparation of 2-((1-(4-fluorophenyl)-5-methyl-1H-pyrazol-3-yl)ethynyl) pyrimidine:

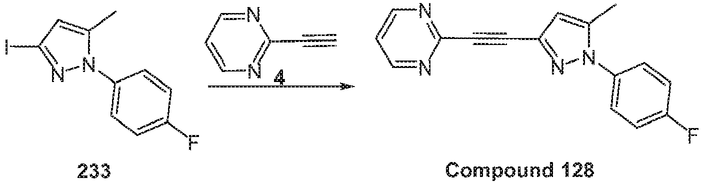

To a solution of **233**(100mg, 0.331mmol) and **4**(68.9mg, 0.662mmol) in 20mL of Et₃N was added Pd(PPh₃)₂Cl₂ (11.62mg, 0.017mmol) and CuI (6.30mg, 0.033mmol). The mixture was protected with N₂ atmosphere, then was heated at 70°C for 4 hours. TLC analysis showed complete conversion of starting material to major product. The reaction mixture was then concentrated in vacuo. The crude product was purified by Prep-HPLC to give the target product **Compound 128**(17mg, yield: 18.45%).
**LCMS**: *m*/*z* 279 (M+H)⁺;
¹**H NMR** (400MHz, CDCl₃): δ 8.75 (d, J = 5.2 Hz, 2H), 7.47-7.43 (m, 2H), 7.25 (d, J = 4.8 Hz, 1H), 7.17 (t, J = 8.6 Hz, 2H), 6.51 (s, 1H), 2.33 (s, 3H).

### Example Compound 129

### Preparation of 2-((1-(4-fluorophenyl)-5-methyl-1H-pyrazol-3-yl)ethynyl)pyrazine:

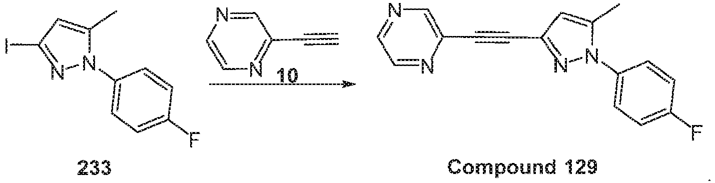

To a solution of **233**(100mg, 0.331mmol) and **10**(68.9mg, 0.662mmol) in 20mL of Et₃N was added Pd(PPh₃)₂Cl₂ (11.62mg, 0.017mmol) and CuI (6.30mg, 0.033mmol). The mixture was protected with N₂ atmosphere, then was heated at 70°C for 4 hours. TLC analysis showed complete conversion of starting material to major product. The reaction mixture was then concentrated in vacuo. The crude product was purified by Prep-HPLC to give the target product **Compound 129**(25mg, yield: 27.1%).
**LCMS:** *m*/*z* 279 (M+H)⁺;
**¹H NMR** (400 MHz, CDCl₃): δ 8.79 (br, 1H), 8.59 (br, 1H), 8.51 (br, 1H), 7.47-7.43 (m, 2H), 7.20-7.16 (m, 2H), 6.50 (s, 1H), 2.34 (s, 3H).

### Example Compound 130

### Preparation of 4-((1-(4-fluorophenyl)-5-methyl-1H-pyrazol-3-yl)ethynyl)pyridine:

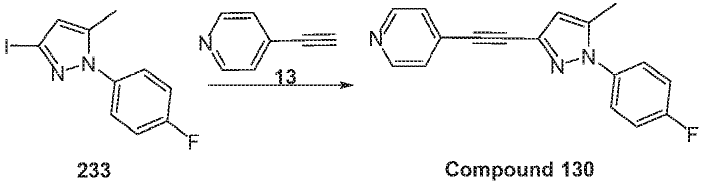

To a solution of **233**(100mg, 0.331mmol) and **13**(68.3mg, 0.662mmol) in 20mL of Et₃N was added Pd(PPh₃)₂Cl₂ (11.62mg, 0.017mmol) and CuI (6.30mg, 0.033mmol). The mixture was protected with N₂ atmosphere, then was heated at 70°C for 4 hours. TLC analysis showed complete conversion of starting material to major product. The reaction mixture was then concentrated in vacuo. The crude product was purified by Prep-HPLC to give the target product **Compound 130**(25mg, yield: 27.2%).
**LCMS:** *m*/*z* 278 (M+H)⁺;
**¹H NMR** (400 MHz, CDCl₃): δ 9.5-8.0 (br, 2H), 7.45-7.42 (m, 4H), 7.19-7.15 (t, J = 8.4 Hz, 2H), 6.43 (s, 1H), 2.32 (s, 3H).

### Example Compound 131

### Preparation of 2-chloro-4-((1-(4-fluorophenyl)-5-methyl-1H-pyrazol-3-yl)ethynyl) pyridine:

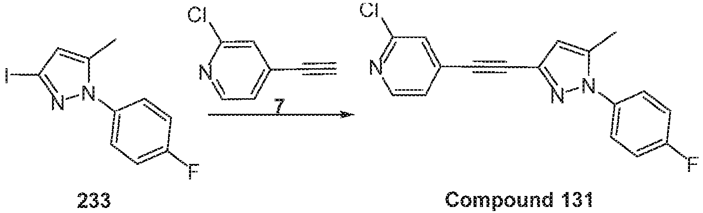

To a solution of **233**(100mg, 0.33 1mmol) and 7(91mg, 0.662mmol) in 20mL of Et₃N was added Pd(PPh₃)₂Cl₂ (11.62mg, 0.017mmol) and CuI (6.30mg, 0.033mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 131**(21mg, yield: 20.4%).
**LCMS:** *m*/*z* 312 (M+H)⁺;
¹**H NMR** (400 MHz, CDCl₃): δ 8.37-8.36 (m, 1H), 7.46-7.42 (m, 3H), 7.32-7.30 (m, 1H), 7.18 (t, *J* = 8.6 Hz, 2H), 6.44 (d, *J* = 0.8 Hz, 1H), 2.33 (d, *J* = 0.4 Hz, 3H).

### Example Compound 132

### Preparation of 3-((5-methyl-1-(4-(trifluoromethoxy)phenyl)-1H-pyrazol-3-yl) ethynyl)pyridine:

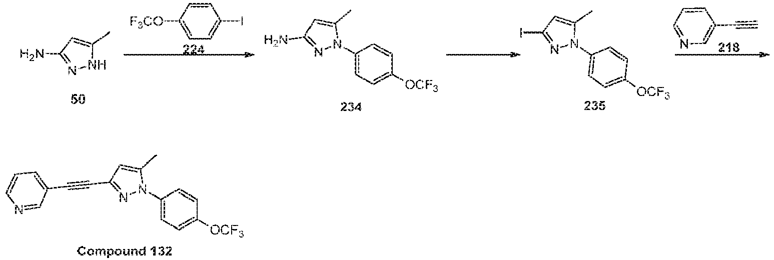

### Experimental section:

### Procedure for preparation of 234:

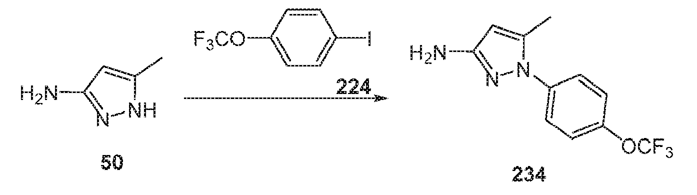

To a solution of **50**(1g, 10.30mmol), CuI (0.102g, 1.030mmol), Cs₂CO₃ (4.03g, 12.36mmol) and quinolin-8-ol (0.299g, 2.059mmol) in 5mL of t-BuOH was added **224**(2.67g, 9.27mmol), and the resulting mixture was heated at 130°C via MW irradiation for 30 minutes. The mixture was cooled to room temperature, and diluted with EA, washed with H₂O (20 mLx3). The combined the organic layer was washed with saturated NaCl and dried over Na₂SO₄, filtered, and concentrated in vacuo. The residue was purified by column chromatography on silica gel to afford **234**(0.4g, yield: 15.10%).

### Procedure for preparation of 235:

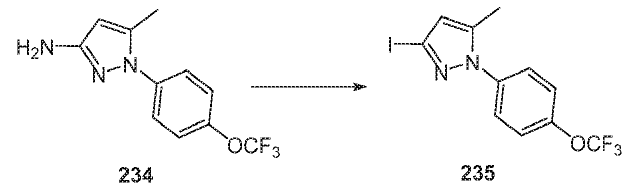

To a solution of **234**(1.3g, 5.05mmol) in concentrated HCl solution (15mL) was added a solution of NaNO₂ (0.384g, 5.56mmol) in water (2mL) over 3 minutes at 0°C. A solution of KI (1.007g, 6.07mmol) in water (3mL) was added to the reaction mixture over 5 minutes, resulting in nitrogen evolution. The reaction mixture was stirred for 15 minutes. Water was added, the aqueous mixture was extracted with EA, washed with NaS₂O₃ two times, dried over Na₂SO₄, and concentrated in vacuo. The crude residue was purified by flash chromatography to give product **235**(0.6g, yield: 32.3%).
**LCMS**: *m*/*z* 369(M+H)⁺.

### Preparation of Compound 132:

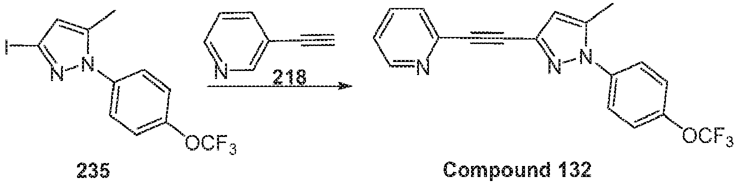

To a solution of **235**(100mg, 0.272mmol) and **218**(56.0mg, 0.543mmol) in 20mL of Et₃N was added Pd(PPh₃)₂Cl₂ (9.53mg, 0.014mmol) and CuI (5.17mg, 0.027mmol). The mixture was protected with N₂ atmosphere, then was heated at 70°C for 4 hours. TLC analysis showed complete conversion of starting material to major product. The reaction mixture was then concentrated in vacuo. The crude product was purified by Prep-HPLC to give the target product **Compound 132**(22mg, yield: 23.59%).
**LCMS**: *m*/*z* 344 (M+H)⁺;
**¹H NMR** (400 MHz, DMSO-*d*₆): δ 8.80 (s, 1H), 8.64 (s, 1H), 8.00 (d, *J* = 7.9 Hz, 1H), 7.78-7.70 (m, 2H), 7.56 (d, 2H), 7.53-7.43 (m, 1H), 6.64 (s, 1H), 2.37 (s, 3H).

### Example Compound 133

### Preparation of 2-((5-methyl-1-(4-(trifluoromethoxy)phenyl)-1H-pyrazol-3-yl) ethynyl)pyrimidine:

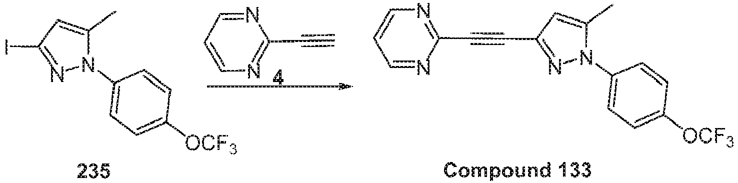

To a solution of **235**(100mg, 0.272mmol) and **4**(56.6mg, 0.543mmol) in 20mL of Et₃N was added Pd(PPh₃)₂Cl₂ (9.53mg, 0.014mmol) and CuI (5.17mg, 0.027mmol). The mixture was protected with N₂ atmosphere, then was heated at 70°C for 4 hours. TLC analysis showed complete conversion of starting material to major product. The reaction mixture was then concentrated in vacuo. The crude product was purified by Prep-HPLC to give the target product **Compound 133**(24mg, yield: 25.7%).
**LCMS:** *m*/*z* 345 (M+H)⁺;
**¹H NMR** (400 MHz, CDCl₃): δ 8.76 (d, *J* = 4.0 Hz, 2H), 7.54 - 7.52 (m, 2H), 7.35 - 7.32 (m, 2H), 7.25 (t, *J* = 4.6 Hz, 1H), 6.52 (d, *J* = 0.4 Hz, 1H), 2.37 (d, *J* = 0.4 Hz, 3H).

### Example Compound 134

### Preparation of 2-((5-methyl-1-(4-(trifluoromethoxy)phenyl)-1H-pyrazol-3-yl) ethynyl)pyrazine:

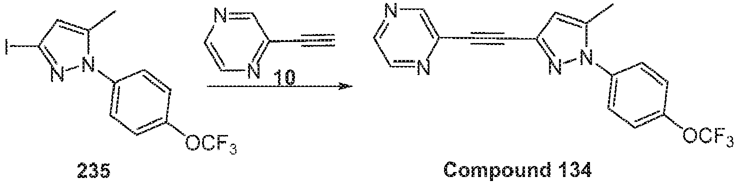

To a solution of **235**(100mg, 0.272mmol) and **10**(56.6mg, 0.543mmol) in 20mL of Et₃N was added Pd(PPh₃)₂Cl₂ (9.53mg, 0.014mmol) and CuI (5.17mg, 0.027mmol). The mixture was protected with N₂ atmosphere, then was heated at 70°C for 4 hours. TLC analysis showed complete conversion of starting material to major product. The reaction mixture was then concentrated in vacuo. The crude product was purified by Prep-HPLC to give the target product **Compound 134**(23mg, yield: 24.59%).
**LCMS**: *m*/*z* 345 (M+H)⁺;
¹**H NMR** (400 MHz, CDCl₃): δ 8.78 (s, 1H), 8.58 (s, 1H), 8.50 (s, 1H), 7.53 (d, *J* = 8.8 Hz, 2H), 7.35 (d,*J* = 8.0 Hz, 2H), 6.51 (s, 1H), 2.38 (s, 3H).

### Example Compound 135

### Preparation of 2-chloro-4-((5-methyl-1-(4-(trifluoromethoxy)phenyl)-1H-pyrazol-3-yl)ethynyl)pyridine:

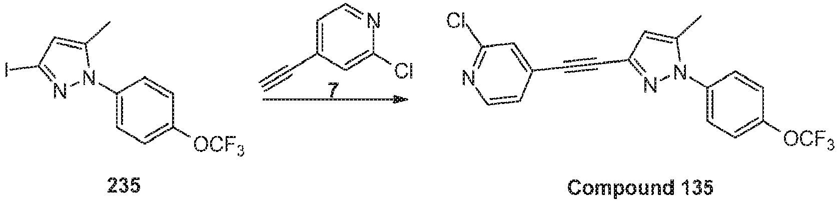

To a solution of **235**(100mg, 0.272mmol) and **7**(74.7mg, 0.543mmol) in 20mL of Et₃N was added Pd(PPh₃)₂Cl₂ (9.53mg, 0.014mmol) and CuI (5.17mg, 0.027mmol). The mixture was protected with N₂ atmosphere, then was heated at 70°C for 4 hours. TLC analysis showed complete conversion of starting material to major product. The reaction mixture was then concentrated in vacuo. The crude product was purified by Prep-HPLC to give the target product **Compound 135**(22mg, 21.44 % yield).
**LCMS**: *m*/*z* 378 (M+H)⁺;
**¹H NMR** (400 MHz, CDCl₃): δ 8.35 (d, *J* = 5.2 Hz, 1H), 7.52-7.50 (m, 2H), 7.43 (t, *J* = 0.8 Hz, 1H), 7.35 (s, 1H), 7.32-7.30 (m, 2H), 6.44 (s, 1H), 2.36 (s, 3H).

### Example Compound 136

### Preparation of 2-((5-methyl-1-(4-(trifluoromethoxy)phenyl)-1H-pyrazol-3-yl)ethynyl)pyridine:

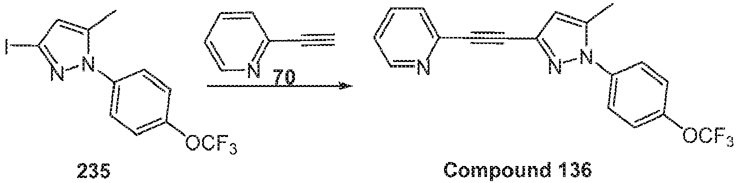

To a solution of **235**(100mg, 0.272mmol) and **70**(56.0mg, 0.543mmol) in 20mL of Et₃N was added Pd(PPh₃)₂Cl₂ (9.53mg, 0.014mmol) and CuI (5.17mg, 0.027mmol). The mixture was protected with N₂ atmosphere, then was heated at 70°C for 4 hours. TLC analysis showed complete conversion of starting material to a major product. The reaction mixture was then concentrated in vacuo. The crude product was purified by Prep-HPLC to give the target product **Compound 136**(23mg, yield: 24.66%).
**LCMS**: *m*/*z* 344 (M+H)⁺;
**¹H NMR** (400 MHz, CDCb): δ 8.60 (d, *J* = 4.4 Hz, 1H), 7.67-7.65 (m, 1H), 7.54-7.50 (m, 3H), 7.32 (d, *J* = 8.0 Hz, 2H), 7.26-7.21 (m, 1H), 6.46 (s, 1H), 2.35 (s, 3H).

### Example 8

### Functional Calcium Clux Assay Methodology

For functional assays, HEK293 cells stably expressing recombinant rat mGluR5 were seeded in 384-well plates and dye loaded using Fluo-8. Cells were then washed to remove the un-incorporated dye. Antagonist evaluation was performed following a 15 min incubation of the test compound followed by the addition of submaximal concentration of glutamate. Intacellular calcium ([Ca²⁺]ᵢ) measurements were performed using a fluorometric imaging plate reader (FLIPR, Molecular Devices). The glutamate-evoked increase in [Ca²⁺]_{¡} in the presence of the test compounds was compared to the response to glutamate alone (the positive control). Antagonist inhibition curves were fitted with a 4-parameter logistic equation giving IC₅₀ values, and Hill coefficients using an iterative nonlinear curve fitting algorithm.

The tables below provide IC50 data in this assay. In the activity column, A = IC₅₀ > 1,000 and ≤ 5,000 nM; B = IC₅₀ > 500 and ≤ 1,000 nM and C = IC₅₀ ≤ 500 nM.

**Table 1**

| # | **Example Compound** | **Structure** | **IC50 value (FLIPR assay)** |
|---|---|---|---|
| 1 | **18** | 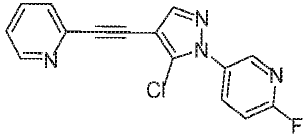 | **C** |
| 2 | **20** | 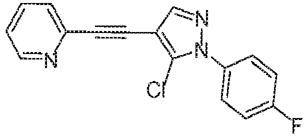 | **C** |
| 3 | **17** | 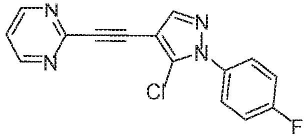 | **C** |
| 4 | **1** | 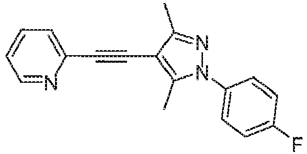 | **C** |
| 5 | **47** | 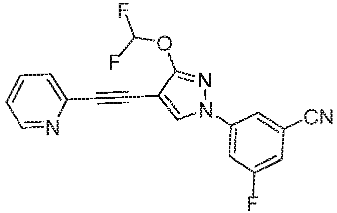 | **C** |
| 6 | **51** | 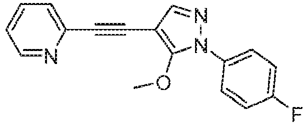 | **C** |
| 7 | **42** | 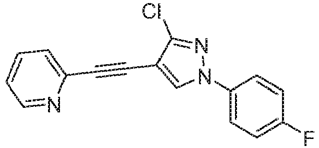 | **C** |
| 8 | **36** | 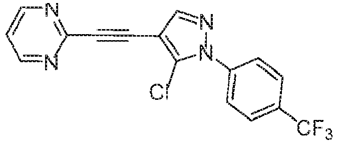 | **C** |
| 9 | **32** | 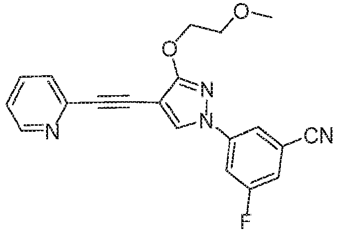 | **C** |
| 10 | **19** | 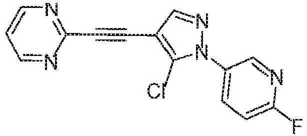 | **C** |
| 11 | **62** | 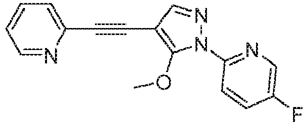 | **C** |
| 12 | **35** | 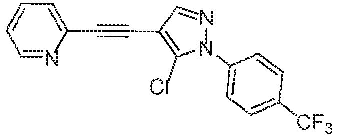 | **C** |
| 13 | **22** | 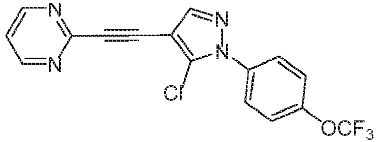 | **C** |
| 14 | **50** | 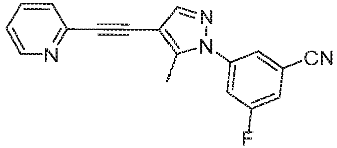 | **C** |
| 15 | **21** | 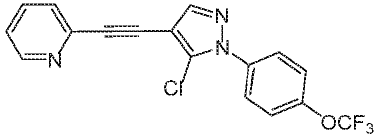 | **C** |
| 16 | **48** | 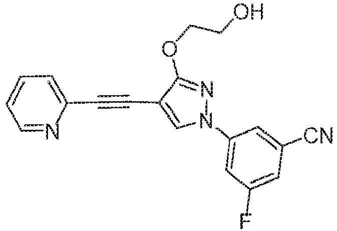 | **C** |
| 17 | **23** | 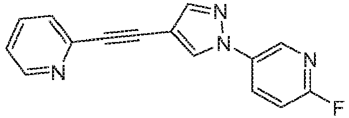 | **C** |
| 18 | **8** | 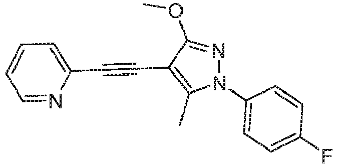 | **C** |
| 19 | **33** | 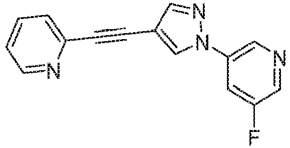 | **C** |
| 20 | **24** | 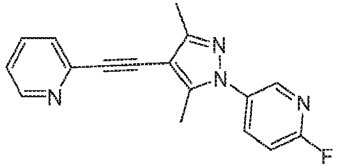 | **C** |
| 21 | **27** | 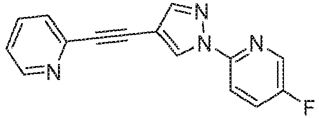 | **C** |
| 22 | **6** | 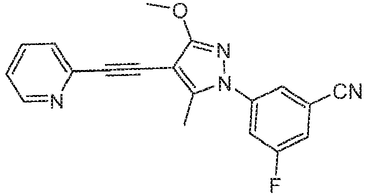 | **C** |
| 23 | **66** | 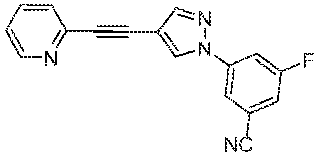 | **C** |
| 24 | **52** | 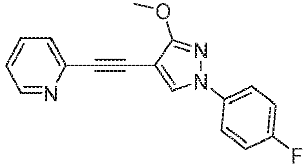 | **C** |
| 25 | **44** | 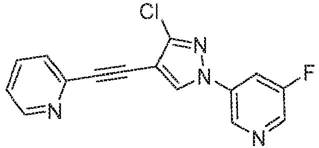 | **C** |
| 26 | **3** | 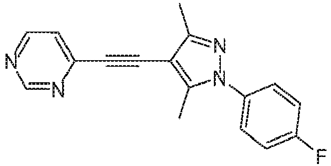 | **C** |
| 27 | **38** | 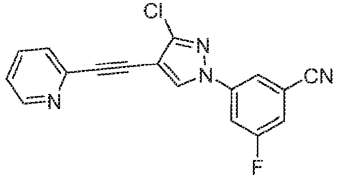 | **C** |
| 28 | **63** | 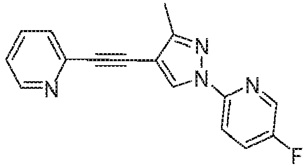 | **C** |
| 29 | **43** | 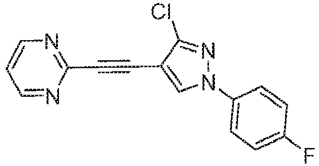 | **C** |
| 30 | **5** | 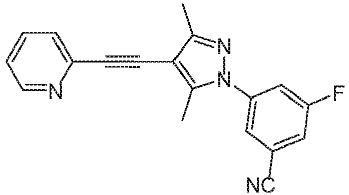 | **C** |
| 31 | **28** | 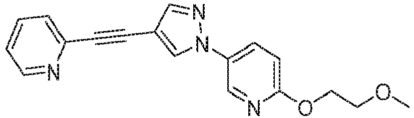 | **C** |
| 32 | **2** | 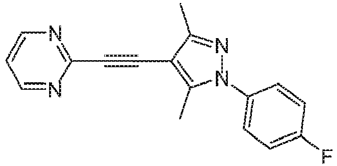 | **C** |
| 33 | **25** | 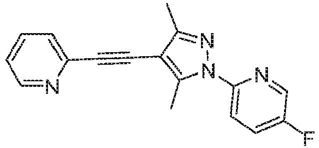 | **C** |
| 34 | **37** | 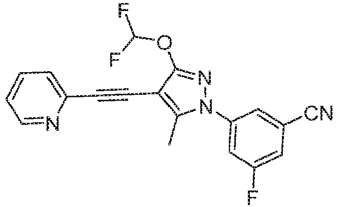 | **C** |
| 3 5 | **68** | 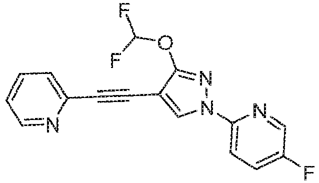 | **C** |
| 36 | **54** | 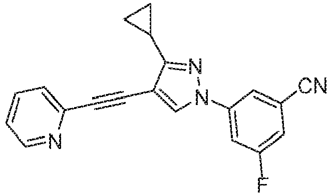 | **C** |
| 37 | **56** | 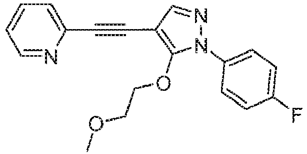 | **C** |
| 38 | **49** | 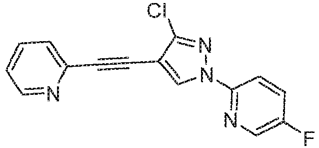 | **C** |
| 39 | **39** | 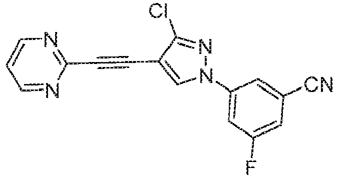 | **C** |
| 40 | **9** | 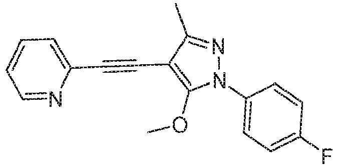 | **C** |
| 41 | **55** | 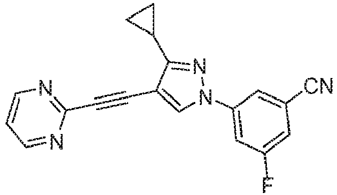 | **C** |
| 42 | **4** | 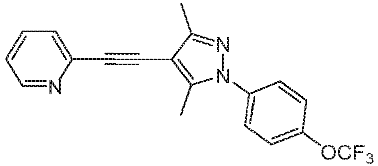 | **C** |
| 43 | **26** | 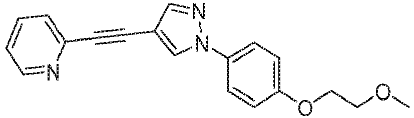 | **C** |
| 44 | **14** | 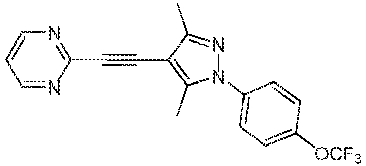 | **C** |
| 45 | **40** | 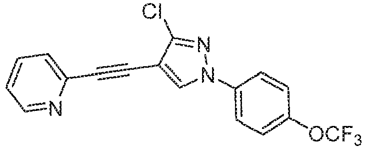 | **C** |
| 46 | **29** | 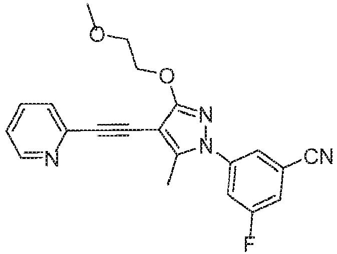 | **C** |
| 47 | **7** | 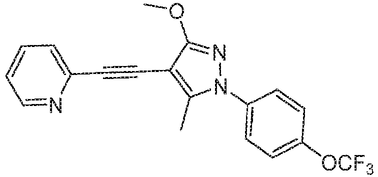 | **C** |
| 48 | **12** | 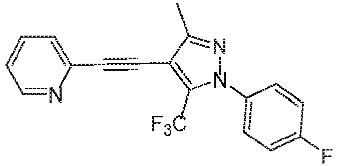 | **C** |
| 49 | **34** | 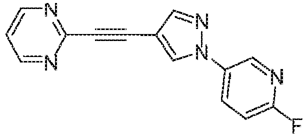 | **C** |
| 50 | **64** | 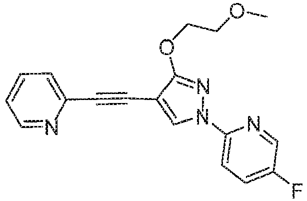 | **B** |
| 51 | **46** | 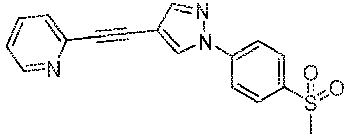 | **B** |
| 52 | **11** | 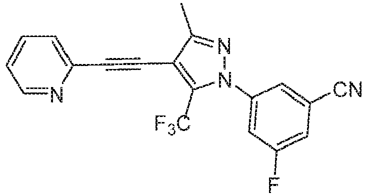 | **A** |
| 53 | **15** | 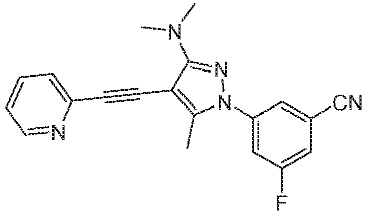 | **A** |
| 54 | **53** | 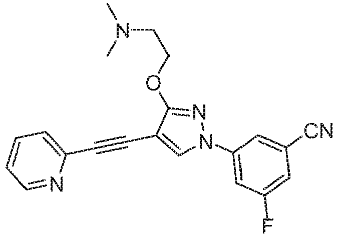 | **A** |
| 55 | **10** | 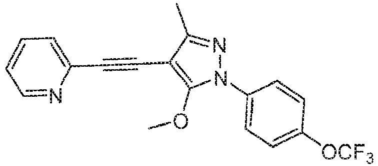 | **A** |
| 56 | **13** | 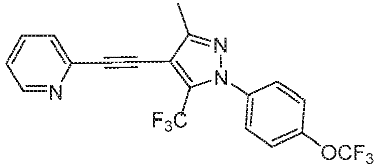 | **A** |
| 57 | **16** | 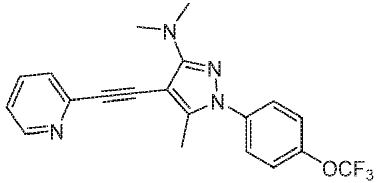 | **A** |
| 58 | **31** | 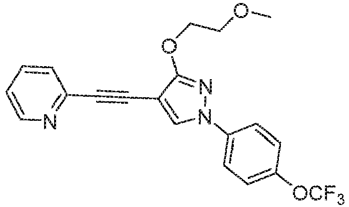 | **A** |
| 59 | **58** | 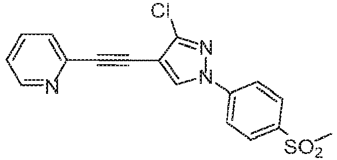 | **A** |
| 60 | **75** | 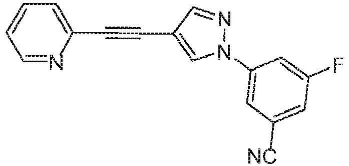 | **C** |
| 61 | **82** | 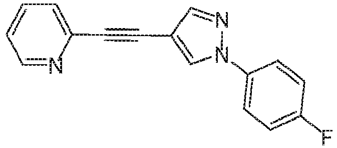 | **C** |
| 62 | **90** | 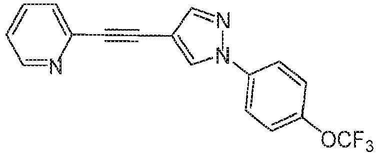 | **C** |
| 63 | **30** | 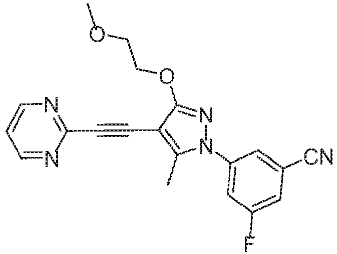 | **A** |
| 64 | **57** | 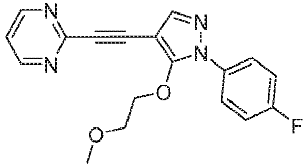 | **A** |
| 65 | **41** | 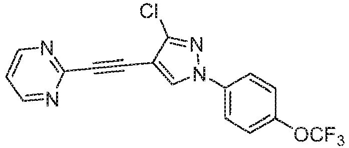 | **A** |
| 66 | **45** | 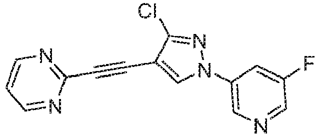 | **A** |
| 67 | **78** | 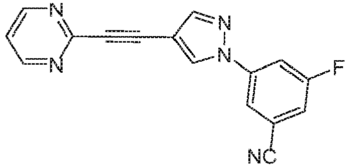 | **C** |
| 68 | **86** | 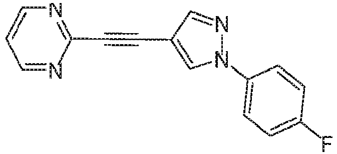 | **C** |
| 69 | **93** | 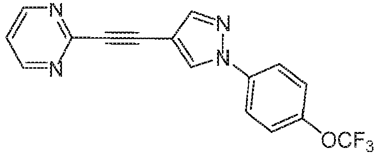 | **C** |
| 70 | **65** | 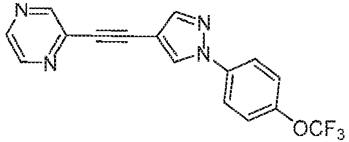 | **A** |
| 71 | **80** | 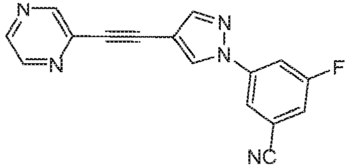 | **A** |
| 72 | **88** | 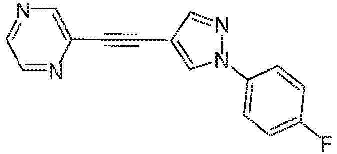 | **C** |
| 73 | **95** | 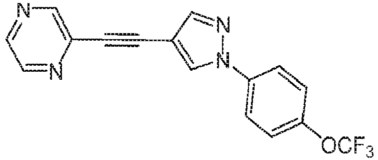 | **A** |
| 74 | **74** | 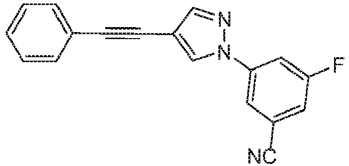 | **A** |
| 75 | **81** | 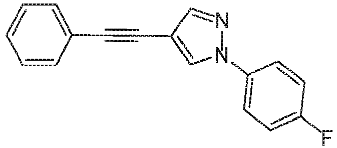 | **C** |
| 76 | **89** | 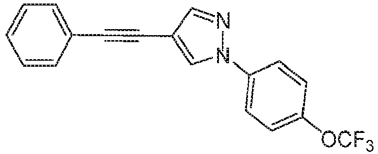 | **A** |
| 77 | **76** | 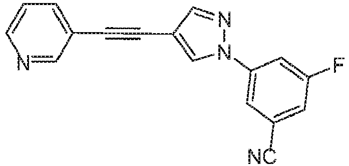 | **A** |
| 78 | **83** | 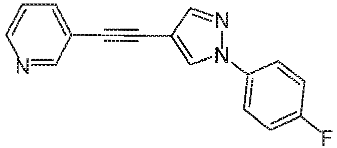 | **C** |
| 79 | **91** | 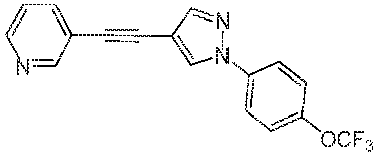 | **A** |
| 80 | **77** | 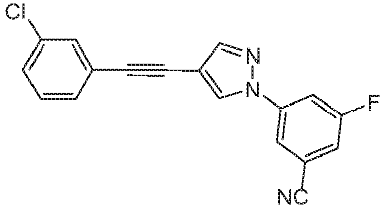 | **B** |
| 81 | **84** | 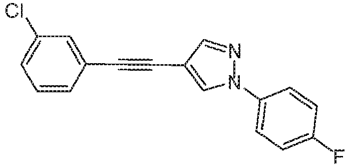 | **C** |
| 82 | **92** | 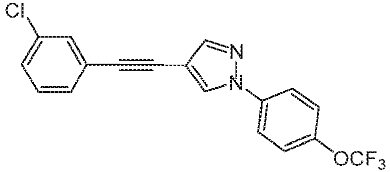 | **C** |
| 83 | **79** | 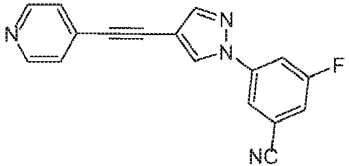 | **C** |
| 84 | **87** | 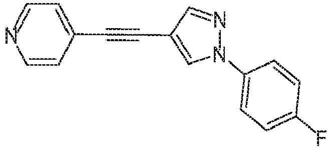 | **C** |
| 85 | **94** | 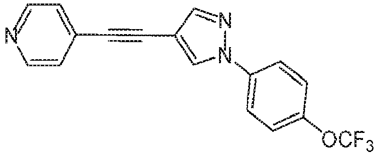 | **B** |
| 86 | **85** | 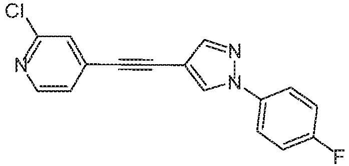 | **C** |

**Table 2**

| | | | |
|---|---|---|---|
| 1 | **61** | 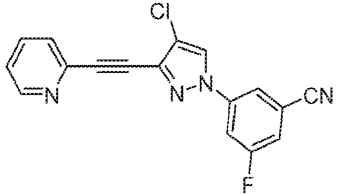 | **C** |
| 2 | **59** | 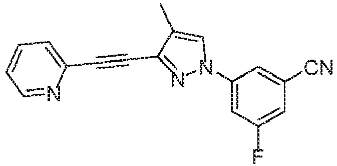 | **C** |
| 3 | **60** | 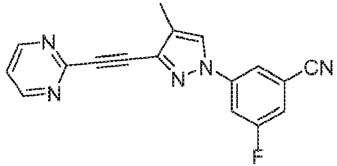 | **C** |
| 4 | **70** | 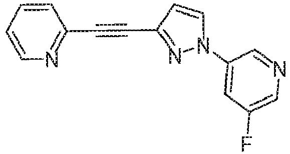 | **C** |
| 5 | **73** | 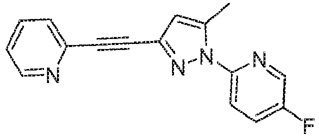 | **C** |
| 6 | **67** | 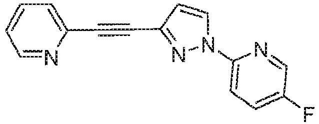 | **C** |
| 7 | **71** | 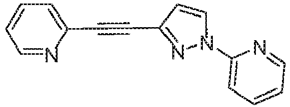 | **C** |
| 8 | **69** | 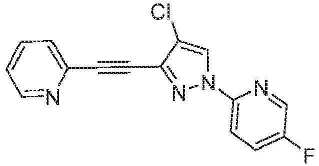 | **B** |
| 9 | **72** | 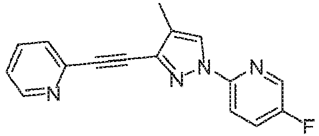 | **B** |
| 10 | **97** | 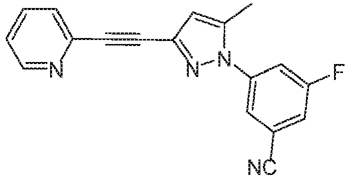 | **C** |
| 11 | **105** | 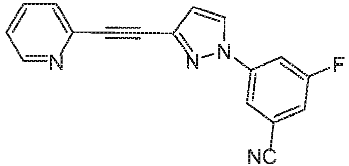 | **C** |
| 12 | **112** | 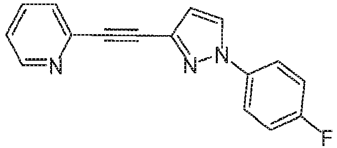 | **C** |
| 13 | **126** | 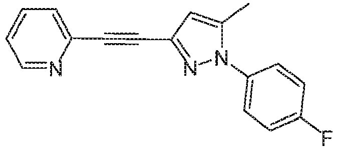 | **C** |
| 14 | **136** | 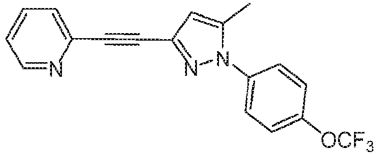 | **C** |
| 15 | **98** | 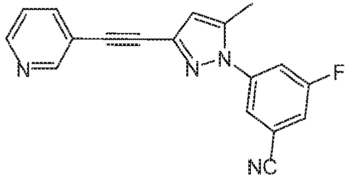 | **C** |
| 16 | **106** | 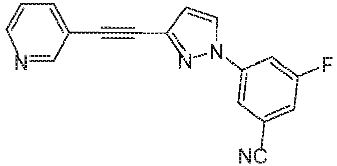 | **A** |
| 17 | **114** | 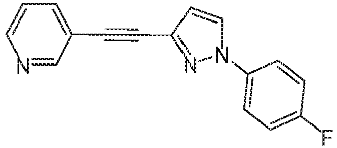 | **A** |
| 18 | **121** | 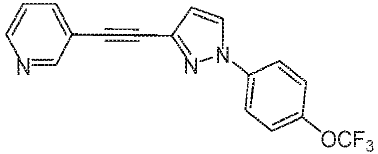 | **A** |
| 19 | **127** | 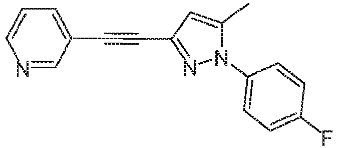 | **A** |
| 20 | **132** | 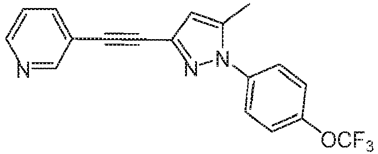 | **A** |
| 21 | **102** | 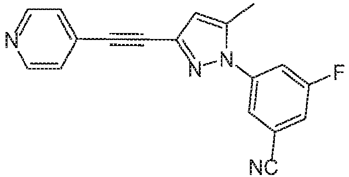 | **C** |
| 22 | **110** | 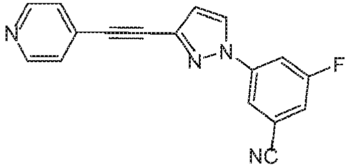 | C |
| **23** | **118** | 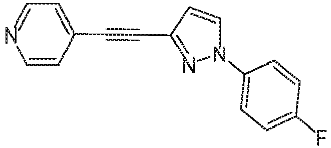 | **C** |
| 24 | **124** | 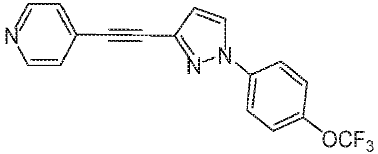 | **A** |
| 25 | **130** | 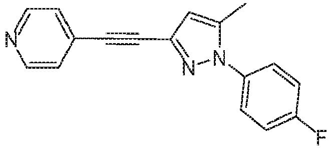 | **C** |
| 26 | **100** | 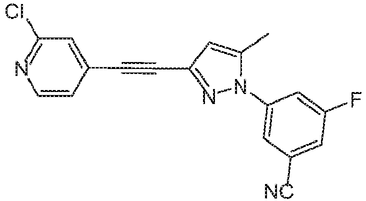 | **C** |
| 27 | **108** | 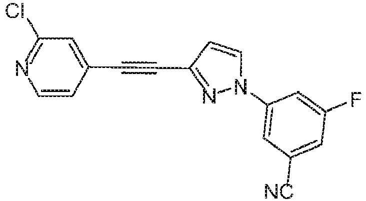 | **C** |
| 28 | **116** | 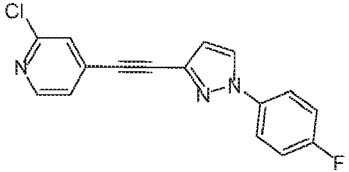 | **C** |
| 29 | **131** | 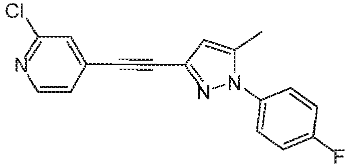 | **C** |
| 30 | **135** | 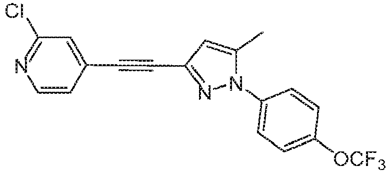 | **A** |
| 31 | **101** | 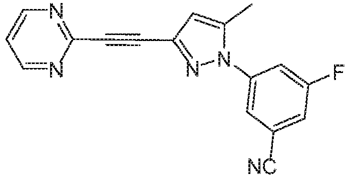 | **C** |
| 32 | **109** | 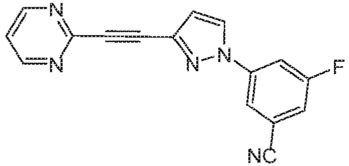 | **C** |
| 33 | **117** | 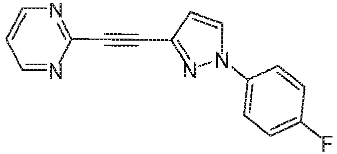 | **B** |
| 34 | **123** | 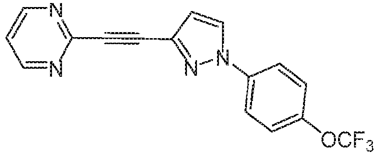 | **A** |
| 35 | **128** | 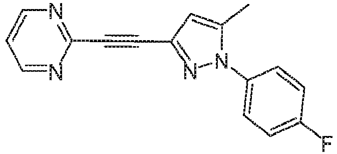 | **A** |
| 36 | **133** | 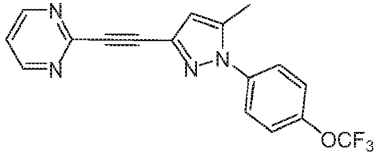 | **A** |
| 37 | **103** | 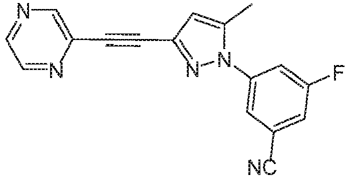 | **C** |
| 38 | **111** | 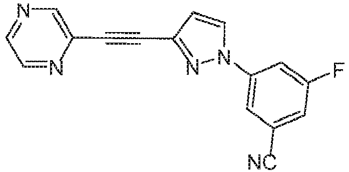 | **C** |
| 39 | **119** | 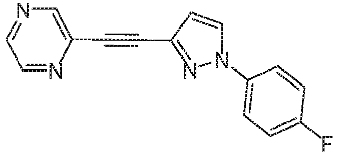 | **A** |
| 40 | **125** | 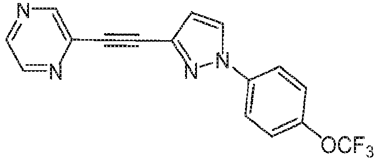 | **A** |
| 41 | **129** | 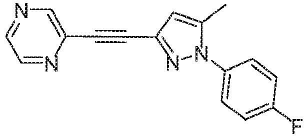 | **A** |
| 42 | **134** | 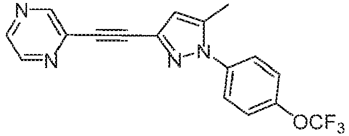 | **A** |
| 43 | **96** | 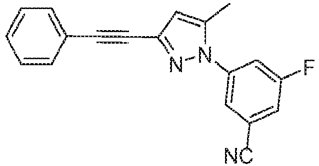 | **C** |
| 44 | **104** | 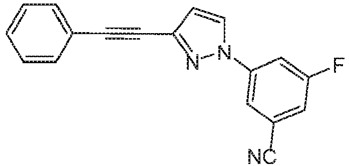 | **A** |
| 45 | **113** | 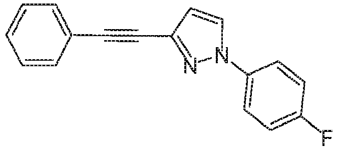 | **A** |
| 46 | **120** | 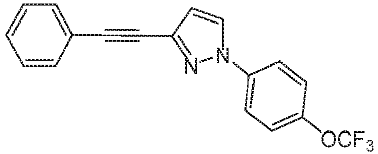 | **A** |
| 47 | **99** | 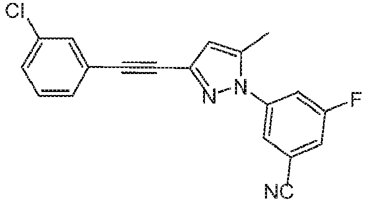 | **C** |
| 48 | **107** | 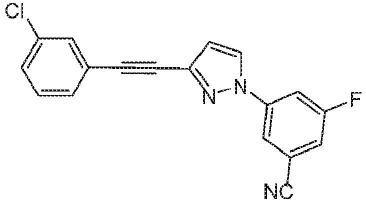 | **C** |
| 49 | **115** | 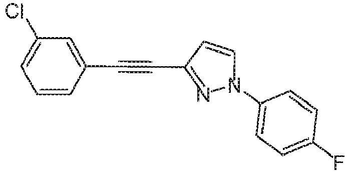 | **B** |
| 50 | **122** | 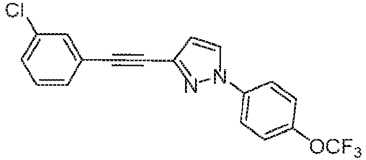 | **A** |

### Example 9

### Radioligand Binding Assay Using Membrane Preparations Expressing Rat mGluR5

The radiolabeled allosteric antagonist [³H]-2-Methyl-6-(phenylethynyl)pyridine (MPEP, American Radiolabeled Chemical) was used to evaluate the ability of test compounds to interact with the MPEP site on mGluR5 as described in Rodriguez et al. [Mol Pharmacol 78:1105-1123, 2010]. Membranes were prepared from HEK293 cells expressing rat mGluR5. Radioligand binding assays were performed in 96-well plates (Corning) containing binding buffer (15mM Tris pH 7.4, 120mM NaCl, 100mM KCl, 25mM MgCl₂, 25 mM CaCl₂) with a final assay volume of 250 µL and 40µg membranes/well.

Saturation isotherms were determined by incubation in presence of 12 increasing concentrations of [³H]-MPEP (0.1-100 nM), while competition experiments were performed with a fixed concentration (4nM) of [³H]-MPEP in presence of 12 increasing concentrations of test compound (1-30,000 nM). Incubations were performed at 4°C for 1h. Nonspecific binding was estimated using 100 µM MTEP. At the end of incubation, membranes were filtered over GF/C filter plates (Perkin Elmer) presoaked in 0.1% BSA for 2h at room temperature. Filter plates were then washed 5 times with ice cold buffer (15mM Tris, pH 7.4 plus 0.1% BSA) using the Packard Filtermate Harvester and dried overnight in a 37°C oven. Fifty µL microscint 20 (PerkinElmer) were added to each well and the plates were incubated on an orbital shaker for 15 min before counting on a Microbeta Trilux for 2 min/well.

## Claims

1. A compound of formula la: 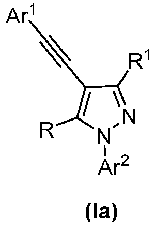 or a pharmaceutically acceptable salt thereof,
wherein:
R is - H, -halogen, -alkyl, -cycloalkyl, -haloalkyl, -OR², or -N(CH₃)₂, wherein R² is a branched or straight-chain alkyl radical of one to nine carbon atoms which may be optionally substituted with 1-3 substituents selected from the group consisting of -halogen, -OH, -alkoxy, and -N(CH₃)₂;
R¹ is -H, -halogen, -alkyl, -cycloalkyl, -haloalkyl, -OR^{2'}, or -N(CH₃)₂, wherein R^{2'} is a branched or straight-chain alkyl radical of one to nine carbon atomswhich may be optionally substituted with 1-3 substituents selected from the group consisting of -halogen, -OH, -alkoxy, and -N(CH₃)₂;
Ar¹ is a 5- to 10-membered mono- or bicyclic heteroaryl ring that contains 1-3 N heteroatoms, wherein the 5- to 10-membered mono- or bicyclic heteroaryl ring is optionally substituted with 1-3 substituents independently selected from the group consisting of -alkyl, -halogen, -OH, -CN, nitro, -CF₃, -O-CF₃, -O-alkyl, -O-aryl, -S-alkyl, -S-aryl, -S(O)-alkyl, -S(O)-aryl, -S(O₂)-alkyl, -S(O₂)-aryl, -CH2-aryl, aryl, heteroaryl, -O-CH₂-aryl, -N(CH₃)₂, cycloalkyl, heterocycloalkyl, -C(O)-alkyl, -C(O)-cycloalkyl, -C(O)-heterocycloalkyl, -C(O)-aryl, -C(O)-heteroaryl, -C(O)O-alkyl, -C(O)O-cycloalkyl, -C(O)O-heterocycloalkyl, -C(O)O-aryl, -C(O)O-heteroaryl, -C(O)NH₂, -C(O)NH-alkyl, -C(O)N(alkyl)₂, -C(O)NH-cycloalkyl, -C(O)N(cycloalkyl)₂, -C(O)NH-heterocycloalkyl, -C(O)N(heterocycloalkyl)₂, -C(O)NH-aryl, -C(O)N(aryl)₂, -C(O)NH-heteroaryl, -C(O)N(heteroaryl)₂, and substituted lower alkyl, wherein the substituents may combine to form an optionally substituted 5-7 membered fused carbacyclic or heterocyclic ring, or
a 5- to 10-membered mono- or bicyclic aryl ring, wherein the 5- to 10-membered mono- or bicyclic aryl ring is optionally substituted with 1-3 substituents independently selected from the group consisting of alkyl, -halogen, -OH, -CN, nitro, -CF₃, -O-CF₃, -O-alkyl, -O-aryl, -S-alkyl, -S-aryl, -S(O)-alkyl, -S(O)-aryl, -S(O₂)-alkyl, -S(O₂)-aryl, -CH2-aryl, aryl, heteroaryl, -O-CH₂-aryl, -N(CH₃)₂, cycloalkyl, heterocycloalkyl, -C(O)-alkyl, -C(O)-cycloalkyl, -C(O)-heterocycloalkyl, -C(O)-aryl, -C(O)-heteroaryl, -C(O)O-alkyl, -C(O)O-cycloalkyl, -C(O)O-heterocycloalkyl, -C(O)O-aryl, -C(O)O-heteroaryl, -C(O)NH₂, -C(O)NH-alkyl, -C(O)N(alkyl)₂, -C(O)NH-cycloalkyl, -C(O)N(cycloalkyl)₂, -C(O)NH-heterocycloalkyl, -C(O)N(heterocycloalkyl)₂, -C(O)NH-aryl, -C(O)N(aryl)₂, -C(O)NH-heteroaryl, -C(O)N(heteroaryl)₂, and substituted lower alkyl, wherein the substituents may combine to form a 5-7 membered fused and optionally substituted carbacyclic or heterocyclic ring;
Ar² is a 5- to 10-membered mono- or bicyclic heteroaryl ring that contains 1-3 N heteroatoms, wherein the 5- to 10-membered mono- or bicyclic heteroaryl ring is optionally substituted with 1-3 substituents independently selected from the group consisting of -alkyl, -halogen, -OH, -CN, nitro, -CF₃, -O-CF₃, -O-alkyl, -O-aryl, -S-alkyl, -S-aryl, -S(O)-alkyl, -S(O)-aryl, -S(O₂)-alkyl, -S(O₂)-aryl, -O-alkyl-OH, -O-alkyl-O-alkyl, -CH₂-aryl, aryl, heteroaryl, -O-CH₂-aryl, -N(CH₃)₂, cycloalkyl, heterocycloalkyl, -C(O)-alkyl, -C(O)-cycloalkyl, -C(O)-heterocycloalkyl, -C(O)-aryl, -C(O)-heteroaryl, -C(O)O-alkyl, -C(O)O-cycloalkyl, -C(O)O-heterocycloalkyl, -C(O)O-aryl, -C(O)O-heteroaryl, -C(O)NH₂, -C(O)NH-alkyl, -C(O)N(alkyl)₂, -C(O)NH-cycloalkyl, -C(O)N(cycloalkyl)₂, -C(O)NH-heterocycloalkyl, -C(O)N(heterocycloalkyl)₂, -C(O)NH-aryl, -C(O)N(aryl)₂, -C(O)NH-heteroaryl, -C(O)N(heteroaryl)₂, and substituted lower alkyl wherein the substituents may combine to form an optionally substituted 5-7 membered fused carbacyclic or heterocyclic ring, or
a 5- to 10-membered mono- or bicyclic aryl ring, wherein the 5- to 10-membered mono- or bicyclic aryl ring is optionally substituted with 1-3 substituents independently selected from the group consisting of -alkyl, -halogen, -OH, -CN, nitro, -CF₃, -O-CF₃, -O-alkyl, -O-aryl, -S-alkyl, -S-aryl, -S(O)-alkyl, -S(O)-aryl, -S(O₂)-alkyl, -S(O₂)-aryl, -O-alkyl-OH, -O-alkyl-O-alkyl, -CH₂-aryl, aryl, heteroaryl, -O-CH₂-aryl, -N(CH₃)₂, cycloalkyl, heterocycloalkyl, -C(O)-alkyl, -C(O)-cycloalkyl, -C(O)-heterocycloalkyl, -C(O)-aryl, -C(O)-heteroaryl, -C(O)O-alkyl, -C(O)O-cycloalkyl, -C(O)O-heterocycloalkyl, -C(O)O-aryl, -C(O)O-heteroaryl, -C(O)NH₂, -C(O)NH-alkyl, -C(O)N(alkyl)₂, -C(O)NH-cycloalkyl, -C(O)N(cycloalkyl)₂, -C(O)NH-heterocycloalkyl, -C(O)N(heterocycloalkyl)₂, -C(O)NH-aryl, -C(O)N(aryl)₂, -C(O)NH-heteroaryl, -C(O)N(heteroaryl)₂, and substituted lower alkyl wherein the substituents may combine to form an optionally substituted 5-7 membered fused carbacyclic or heterocyclic ring,
wherein in the above definitions, "lower alkyl" is a branched or straight chain alkyl of one to nine carbon atoms.

2. The compound according to claim 1 or a pharmaceutically acceptable salt thereof, wherein:
R is -H, -halogen, -alkyl, -cycloalkyl, -haloalkyl, -OR², or -N(CH₃)₂, wherein R² is a branched or straight-chain alkyl radical of one to nine carbon atoms which may be optionally substituted with 1-2 substituents selected from the group consisting of -OH and -alkoxy;
R¹ is -H, -halogen, -alkyl, -cycloalkyl, -haloalkyl, -OR^{2'}, or -N(CH₃)₂, wherein R^{2'} is a branched or straight-chain alkyl radical of one to nine carbon atoms which may be optionally substituted with 1-3 substituents selected from the group consisting of -halogen, -OH, -alkoxy, and -N(CH₃)₂;
Ar¹ is a 5- or 6-membered mono-heteroaryl ring that contains 1-3 N heteroatoms, wherein the 5- or 6-membered mono-heteroaryl ring is optionally substituted with 1-3 substituents independently selected from the group consisting of -alkyl, -halogen, -OH, -CN, nitro, -CF₃, -O-CF₃, and -O-alkyl, or
a 6-membered aryl ring, wherein the 6-membered aryl is optionally substituted with 1-3 substituents independently selected from the group consisting of -alkyl, -halogen, -OH, -CN, nitro, -CF₃, -O-CF₃, and -O-alkyl;
Ar² is a 5- or 6-membered mono-heteroaryl ring that contains 1-3 N heteroatoms, wherein the 5- or 6-membered mono-heteroaryl ring is optionally substituted with 1-3 substituents independently selected from the group consisting of -alkyl, -halogen, -OH, -CN, nitro, -CF₃, -O-CF₃, -O-alkyl, -S-alkyl, -S(O)-alkyl, -S(O₂)-alkyl, -O-alkyl-O-alkyl, aryl, -C(O)O-alkyl, -C(O)NH₂, -C(O)NH-alkyl, and -C(O)N(alkyl)₂, or.
a 6-membered aryl ring, wherein the 6-membered aryl is optionally substituted with 1-3 substituents independently selected from the group consisting of -alkyl, -halogen, -OH, -CN, nitro, -CF₃, -O-CF₃, -O-alkyl, -S-alkyl, -S(O)-alkyl, -S(O₂)-alkyl, -O-alkyl-O-alkyl, aryl, -C(O)O-alkyl, -C(O)NH₂, -C(O)NH-alkyl, and -C(O)N(alkyl)₂.

3. The compound according to claim 2 or a pharmaceutically acceptable salt thereof, wherein:
R is -H, -halogen, -alkyl, -cycloalkyl, -haloalkyl, -OR², or -N(lower alkyl)₂, wherein R² is a branched or straight-chain alkyl radical of one to nine carbon atomswhich may be optionally substituted with 1-2 substituents selected from the group consisting of -OH and -alkoxy;
R¹ is -H, -halogen, -alkyl, -cycloalkyl, -haloalkyl, -OR^{2'}, or -N(lower alkyl)2, wherein R^{2'} is a branched or straight-chain alkyl radical of one to nine carbon atomswhich may be optionally substituted with 1-3 substituents selected from the group consisting of -halogen, -OH, -alkoxy, and -N(CH₃)₂;
Ar¹ is a 6-membered aryl ring or a 5- or 6-membered mono-heteroaryl ring that contains 1, 2, or 3 N heteroatoms, wherein the 6-membered aryl or the 5- or 6-membered mono-heteroaryl ring is optionally substituted with 1, 2 or 3 substituents independently selected from the group consisting of branched or straight chain alkyl of one to nine carbon atoms, and halogen;
Ar² is a 6-membered aryl ring or a 5- or 6-membered mono-heteroaryl ring that contains 1, 2, or 3 N heteroatoms, wherein the 6-membered aryl ring or the 5- or 6-membered mono-heteroaryl ring is optionally substituted with 1, 2 or 3 substituents independently selected from the group consisting of -C₁-C₄alkyl, -F, -Cl, -Br, -OH, -CN, nitro, -CF₃, -OCF₃, -O-C₁-C₄alkyl, -SCH₃, -S(O)-CH₃, -S(O₂)-CH₃, -O-C₁-C₄alkyl-O-C₁-C₄alkyl, -C(O)OCH₃, -C(O)NH₂, -C(O)NH(CH₃), -C(O)N(CH₃)₂, phenyl.

4. The compound according to claim 3 or a pharmaceutically acceptable salt thereof, wherein:
R is -H, -halogen, -C₁-C₄alkyl, -CF₃, -O-C₁-C₄alkyl, or
-O-C₁-C₄alkyl-O-C₁-C₄alkyl;
R¹ is -H, -halogen, -C₁-C₄alkyl, -C₃-C₆cycloalkyl, -halo-C₁-C₄alkyl, -OR^{2'}, or -N(C₁-C₄alkyl)₂, wherein R^{2'} is C₁-C₄alkyl which may be optionally substituted with 1-2 substituents selected from the group consisting of -halogen, -OH, -C₁-C₄alkoxy, and -N(CH₃)₂;
Ar¹ is optionally substituted pyridinyl, pyrimidinyl, pyrazinyl, or phenyl, wherein the pyridinyl, pyrimidinyl, pyrazinyl, or phenyl is optionally substituted with a substituent selected from the group consisting of straight or branched chain alkyl of one to nine carbon atoms, and halogen;
Ar² is optionally substituted pyridinyl, or phenyl, wherein the pyridinyl, or phenyl is optionally substituted with 1-2 substituents independently selected from the group consisting of -C₁-C₄alkyl, -halogen, -CN, -CF₃, -O-CF₃, -S(O₂)-C₁-C₄alkyl, and -O-C₁-C₄alkyl-O-C₁-C₄alkyl.

5. The compound according to claim 1 or a pharmaceutically acceptable salt thereof, wherein said compound is:
2-((1-(4-fluorophenyl)-3,5-dimethyl-1H-pyrazol-4-yl)ethynyl)pyridine,
2-((3,5-dimethyl-1-(4-(trifluoromethoxy)phenyl)-1H-pyrazol-4-yl)ethynyl)pyridine,
3-(3,5-dimethyl-4-(2-(pyridin-2-yl)ethynyl)-1H-pyrazol-1-yl)-5-fluorobenzonitrile,
3-fluoro-5-(3-methoxy-5-methyl-4-(pyridin-2-ylethynyl)-1H-pyrazol-1-yl) benzonitrile,
2-((3-methoxy-5-methyl-1-(4-(trifluoromethoxy)phenyl)-1H-pyrazol-4-yl)ethynyl) pridine,
2-((1-(4-fluorophenyl)-3-methoxy-5-methyl-1H-pyrazol-4-yl)ethynyl)pyridine,
2-((1-(4-fluorophenyl)-5-methoxy-3-methyl-1H-pyrazol-4-yl)ethynyl)pyridine,
2-((3-methyl-5-methoxy-1-(4-(trifluoromethoxy)phenyl)-1H-pyrazol-4-yl)ethynyl) pyridine,
3-fluoro-5-(3-methyl-4-(pyridin-2-ylethynyl)-5-(trifluoromethyl)-1H-pyrazol-1-yl) benzonitrile,
2-((1-(4-fluorophenyl)-3-methyl-5-(trifluoromethyl)-1H-pyrazol-4-yl)ethynyl) pyridine,
2-((3-methyl-1-(4-(trifluoromethoxy)phenyl)-5-(trifluoromethyl)-1H-pyrazol-4-yl) ethynyl)pyridine,
3-(3-(dimethylamino)-5-methyl-4-(pyridin-2-ylethynyl)-1H-pyrazol-1-yl)-5-fluorobenzonitrile,
2-(3-(dimethylamino)-5-methyl-1-(4-(trifluoromethoxy)phenyl)-1H-pyrazol-4-yl) ethynyl)pyridine,
5-((5-chloro-4-(pyridin-2-ylethynyl)-1H-pyrazol-1-yl)-2-fluoropyridine,
2-((5-chloro-1-(4-fluorophenyl)-1H-pyrazol-4-yl)ethynyl)pyridine,
2-((5-chloro-1-(4-(trifluoromethoxy)phenyl)-1H-pyrazol-4-yl)ehynyl)pyridine,
2-fluoro-5-(4-(pyridin-2-yl)-1H-pyrazol-i-yl)pyridine,
5-(3,5-dimethyl-4-(pyridin-2-ylethynyl)-1H-pyrazol-1-yl)-2-fluoropyridine,
2-(3,5-dimethyl-4-(pyridin-2-ylethynyl)-1H-pyrazol-1-yl)-5-fluoropyridine,
2-((1-(4-(2-methoxyethoxy)phenyl)-1H-pyrazol-4-yl)ethynyl)pyridine,
5-fluoro-2-(4-(pyridin-2-ylethynyl)-1H-pyrazol-1-yl)pyridine,
2-(2-methoxyethoxy)-5-(4-(pyridin-2-ylethynyl)-1H-pyrazol-i-yl)pyridine,
3-fluoro-5-(3-(2-methoxyethoxy)-5-methyl-4-(pyridin-2-ylethynyl)-1H-pyrazol-1-yl) benzonitrile,
2-((3-(2-methoxyethoxy)-1-(4-(trifluoromethoxy)phenyl)-1H-pyrazol-4-yl) ethynyl)pyridine,
3-fluoro-5-(3-(2-methoxyethoxy)-4-(pyridin-2-ylethynyl)-1H-pyrazol-1-yl) benzonitrile,
3-fluoro-5-(4-(pyridin-2-ylethynyl)-1H-pyrazol-i-yl)pyridine,
2-((5-chloro-1-(4-(trifluoromethyl)phenyl)-1H-pyrazol-4-yl)ethynyl)pyridine,
3-(3-(difluoromethoxy)-5-methyl-4-(pyridin-2-ylethynyl)-1H-pyrazol-1-yl)-5-fluoro benzonitrile,
3-(3-chloro-4-(pyridin-2-ylethynyl)-1H-pyrazol-1-yl)-5-fluorobenzonitrile,
2-((3-chloro-1-(4-(trifluoromethoxy)phenyl)-1H-pyrazol-4-yl)ethynyl)pyridine,
2-((3-chloro-1-(4-fluorophenyl)-1H-pyrazol-4-yl)ethynyl)pyridine,
3-(3-chloro-4-(pyridin-2-ylethynyl)-1H-pyrazol-1-yl)-5-fluoropyridine,
2-((1-(4-(methylsulfonyl)phenyl)-1H-pyrazol-4-yl)ethynyl)pyridine,
3-(3-(difluoromethoxy)-4-(pyridin-2-ylethynyl)-1H-pyrazol-1-yl)-5-fluorobenzonitrile,
3-fluoro-5-(3-(2-hydroxyethoxy)-4-(pyridin-2-ylethynyl)-1H-pyrazol-1-yl) benzonitrile,
2-(3-chloro-4-(pyridin-2-ylethynyl)-1H-pyrazol-1-yl)-5-fluoropyridine,
3-fluoro-5-(5-methyl-4-(pyridin-2-ylethynyl)-1H-pyrazol-1-yl)benzonitrile,
2-((1-(4-fluorophenyl)-5-methoxy-1H-pyrazol-4-yl)ethynyl)pyridine,
2-((1-(4-fluorophenyl)-3-methoxy-1H-pyrazol-4-yl)ethynyl)pyridine,
3-(3-(2-(dimethylamino)ethoxy)-4-(pyridin-2-ylethynyl)-1H-pyrazol-1-yl)-5-fluoro benzonitrile,
3-(3-cyclopropyl-4-(pyridin-2-ylethynyl)-1H-pyrazol-1-yl)-5-fluorobenzonitrile,
2-((1-(4-fluorophenyl)-5-(2-methoxyethoxy)-1H-pyrazol-4-yl)ethynyl)pyridine,
2-((3-chloro-1-(4-(methylsulfonyl)phenyl)-1H-pyrazol-4-yl)ethynyl)pyridine,
5-fluoro-2-(5-methoxy-4-(pyridin-2-ylethynyl)-1H-pyrazol-1-yl)pyridine,
5-fluoro-2-(3-methyl-4-(pyridin-2-ylethynyl)-1H-pyrazol-1-yl)pyridine,
5-fluoro-2-(3-(2-methoxyethoxy)-4-(pyridin-2-ylethynyl)-1H-pyrazol-1-yl) pyridine,
3-fluoro-5-(4-(pyridin-2-ylethynyl)-1H-pyrazol-i-yl)benzonitrile,
2-(3-(difluoromethoxy)-4-(pyridin-2-ylethynyl)-1H-pyrazol-1-yl)-5-fluoropyridine,
3-fluoro-5-(4-(pyridin-2-ylethynyl)-1H-pyrazol-1-yl)benzonitrile,
3-fluoro-5-(4-(pyridin-3-ylethynyl)-1H-pyrazol-1-yl)benzonitrile,
3-fluoro-5-(4-(pyridin-4-ylethynyl)-1H-pyrazol-1-yl)benzonitrile,
2-((1-(4-fluorophenyl)-1H-pyrazol-4-yl)ethynyl)pyridine,
3-((1-(4-fluorophenyl)-1H-pyrazol-4-yl)ethynyl)pyridine,
2-chloro-4-((1-(4-fluorophenyl)-1H-pyrazol-4-yl)ethynyl)pyridine,
4-((1-(4-fluorophenyl)-1H-pyrazol-4-yl)ethynyl)pyridine,
2-((1-(4-(trifluoromethoxy)phenyl)-1H-pyrazol-4-yl)ethynyl)pyridine,
3-((1-(4-(trifluoromethoxy)phenyl)-1H-pyrazol-4-yl)ethynyl)pyridine,
4-((1-(4-(trifluoromethoxy)phenyl)-1H-pyrazol-4-yl)ethynyl)pyridine.
2-((1-(4-fluorophenyl)-3,5-dimethyl-1H-pyrazol-4-yl)ethynyl)pyrimidine,
4-((1-(4-fluorophenyl)-3,5-dimethyl-1H-pyrazol-4-yl)ethynyl)pyrimidine,
2-((3,5-dimethyl-1-(4-(trifluoromethoxy)phenyl)-1H-pyrazol-4-yl)ethynyl)pyrimidine,
2-((5-chloro-1-(4-fluorophenyl)-1H-pyrazol-4-yl)ethynyl)pyrimidine,
2-((5-chloro-1-(6-fluoropyridin-3-yl)-1H-pyrazol-4-yl)ethynyl)pyrimidine,
2-((5-chloro-1-(4-trifluoromethoxy)phenyl)-1H-pyrazol-4-yl)ethynyl)pyrimidine,
3-fluoro-5-(3-(2-methoxyethoxy)-5-methyl-4-(pyrimidin-2-ylethynyl)-1H-pyrazol-1-yl)benzonitrile,
2-((1-(6-fluoropyridin-3-yl)-1H-pyrazol-4-yl)ethynyl)pyrimidine,
2-((5-chloro-1-(4-(trifluoromethyl)phenyl)-1H-pyrazol-4-yl)ethynyl)pyrimidine, 3-(3-chloro-4-(pyrimidin-2-ylethynyl)-1H-pyrazol-1-yl)-5-fluorobenzonitrile,
2-((3-chloro-1-(4-(trifluoromethoxy)phenyl)-1H-pyrazol-4-yl)ethynyl) pyrimidine,
2-((3-chloro-1-(4-fluorophenyl)-1H-pyrazol-4-yl)ethynyl)pyrimidine,
2-((3-chloro-1-(5-fluoropyridin-3-yl)-1H-pyrazol-4-yl)ethynyl)pyrimidine,
3-(3-cyclopropyl-4-(pyrimidin-2-ylethynyl)-1H-pyrazol-1-yl)-5-fluorobenzonitrile
2-((1-(4-fluorophenyl)-5-(2-methoxyethoxy)-1H-pyrazol-4-yl)ethynyl)pyrimidine,
3-fluoro-5-(4-(pyrimidin-2-ylethynyl)-1H-pyrazol-1-yl)benzonitrile,
2-((1-(4-fluorophenyl)-1H-pyrazol-4-yl)ethynyl)pyrimidine,
2-((1-(4-(trifluorophenyl)-1H-pyrazol-4-yl)ethynyl)pyrimidine,
2-((1-(4-(trifluoromethoxy)phenyl)-1H-pyrazol-4-yl)ethynyl)pyrazine
3-fluoro-5-(4-(pyrazin-2-ylethynyl)-1H-pyrazol-1-yl)benzonitrile,
2-((1-(4-fluorophenyl)-1H-pyrazol-4-yl)ethynyl)pyrazine,
2-((1-(4-(trifluoromethoxy)phenyl)-1H-pyrazol-4-yl)ethynyl)pyrazine,
3-fluoro-5-(4-(phenylethynyl)-1H-pyrazol-1-yl)benzonitrile,
3-fluoro-5-(4-(3-chloro-phenylethynyl)-1H-pyrazol-1-yl)benzonitrile,
1-(4-fluorophenyl)-4-(phenylethynyl)-1H-pyrazole,
4-((3-chlorophenyl)ethynyl)-1-(4-fluorophenyl)-1H-pyrazole,
4-(phenylethynyl)-1-(4-(trifluoromethoxy)phenyl)-1H-pyrazole,
4-((3-chlorophenyl)ethynyl)-1-(4-(trifluoromethoxy)phenyl)-1H-pyrazole.

6. The compound according to claim 1 or a pharmaceutically acceptable salt thereof, wherein said compound is:
3-fluoro-5-(4-methyl-3-(pyridin-2-ylethynyl)-1H-pyrazol-1-yl)benzonitrile,
3-(4-chloro-3-(pyridin-2-ylethynyl)-1H-pyrazol-1-yl)-5-fluorobenzonitrile,
5-fluoro-2-(3-(pyridin-2-ylethynyl)-1H-pyrazol-1-yl)pyridine,
2-(4-chloro-3-(pyridin-2-ylethynyl)-1H-pyrazol-1-yl)-5-fluoropyridine,
3-fluoro-5-(3-(pyridin-2-ylethynyl)-1H-pyrazol-1-yl)pyridine,
2-((1-(pyridin-2-yl)-1H-pyrazol-3-yl)ethynyl)pyridine,
5-fluoro-2-(4-methyl-3-(pyridin-2-ylethynyl)-1H-pyrazol-1-yl)pyridine,
5-fluoro-2-(5-methyl-3-(pyridin-2-ylethynyl)-1H-pyrazol-1-yl)pyridine,
3-fluoro-5-(5-methyl-3-(pyridin-2-ylethynyl)-1H-pyrazol-1-yl)benzonitrile,
3-fluoro-5-(5-methyl-3-(pyridin-3-ylethynyl)-1H-pyrazol-1-yl)benzonitrile,
3-(3-((2-chloropyridin-4-yl)ethynyl)-5-methyl-1H-pyrazol-1-yl)-5-fluorobenzonitrile,
3-fluoro-5-(5-methyl-3-(pyridin-4-ylethynyl)-1H-pyrazol-1-yl)benzonitrile,
3-fluoro-5-(3-(pyridin-2-ylethynyl)-1H-pyrazol-1-yl)benzonitrile,
3-fluoro-5-(3-(pyridin-3-ylethynyl)-1H-pyrazol-1-yl)benzonitrile,
3-(3-((2-chloropyridin-4-yl)ethynyl)-1H-pyrazol-1-yl)-5-fluorobenzonitrile,
3-fluoro-5-(3-(pyridin-4-ylethynyl)-1H-pyrazol-i-yl)benzonitrile,
2-((1-(4-fluorophenyl)-1H-pyrazol-3-yl)ethynyl)pyridine,
3-((1-(4-fluorophenyl)-1H-pyrazol-3-yl)ethynyl)pyridine,
2-chloro-4-((1-(4-fluorophenyl)-1H-pyrazol-3-yl)ethynyl)pyridine,
4-((1-(4-fluorophenyl)-1H-pyrazol-3-yl)ethynyl)pyridine,
3-((1-(4-(trifluoromethoxy)phenyl)-1H-pyrazol-3-yl)ethynyl)pyridine,
4-((1-(4-(trifluoromethoxy)phenyl)-1H-pyrazol-3-yl)ethynyl)pyridine,
2-((1-(4-fluorophenyl)-5-methyl-1H-pyrazol-3-yl)ethynyl)pyridine,
3-((1-(4-fluorophenyl)-5-methyl-1H-pyrazol-3-yl)ethynyl)pyridine,
4-((1-(4-fluorophenyl)-5-methyl-1H-pyrazol-3-yl)ethynyl)pyridine,
2-chloro-4-((1-(4-fluorophenyl)-5-methyl-1H-pyrazol-3-yl)ethynyl)pyridine,
3-((5-methyl-1-(4-(trifluoromethoxy)phenyl)-1H-pyrazol-3-yl)ethynyl)pyridine,
2-chloro-4-((5-methyl-1-(4-(trifluoromethoxy)phenyl)-1H-pyrazol-3-yl)ethynyl) pyridine,
2-((5-methyl-1-(4-(trifluoromethoxy)phenyl)-1H-pyrazol-3-yl)ethynyl)pyridine.
3-fluoro-5-(4-methyl-3-(pyrimidin-2-ylethynyl)-1H-pyrazol-1-yl)benzonitrile,
3-fluoro-5-(5-methyl-3-(pyrimidin-2-ylethynyl)-1H-pyrazol-1-yl)benzonitrile,
3-fluoro-5-(3-(pyrimidin-2-ylethynyl)-1H-pyrazol-1-yl)benzonitrile,
2-((1-(4-fluorophenyl)-1H-pyrazol-3-yl)ethynyl)pyrimidine,
2-((1-(4-(trifluoromethoxy)phenyl)-1H-pyrazol-3-yl)ethynyl)pyrimidine,
2-((1-(4-fluorophenyl)-5-methyl-1H-pyrazol-3-yl)ethynyl)pyrimidine,
2-((5-methyl-1-(4-(trifluoromethoxy)phenyl)-1H-pyrazol-3-yl)ethynyl)pyrimidine,
3-fluoro-5-(5-methyl-3-(pyrazin-2-ylethynyl)-1H-pyrazol-1-yl)benzonitrile,
3-fluoro-5-(3-(pyrazin-2-ylethynyl)-1H-pyrazol-1-yl)benzonitrile,
2-((1-(4-fluorophenyl)-1H-pyrazol-3-yl)ethynyl)pyrazine,
2-((1-(4-(trifluoromethoxy)phenyl)-1H-pyrazol-3-yl)ethynyl)pyrazine,
2-((1-(4-fluorophenyl)-5-methyl-1H-pyrazol-3-yl)ethynyl)pyrazine,
2-((5-methyl-1-(4-(trifluoromethoxy)phenyl)-1H-pyrazol-3-yl)ethynyl)pyrazine,
3-fluoro-5-(5-methyl-3-(phenylethynyl)-1H-pyrazol-1-yl)benzonitrile,
3-(3-((3-chlorophenyl)ethynyl)-5-methyl-1H-pyrazol-1-yl)-5-fluorobenzonitrile,
3-fluoro-5-(3-(phenylethynyl)-1H-pyrazol-1-yl)benzonitrile,
3-(3-((3-chlorophenyl)ethynyl)-1H-pyrazol-1-yl)-5-fluorobenzonitrile,
1-(4-fluorophenyl)-3-(phenylethynyl)-1H-pyrazole,
3-((3-chlorophenyl)ethynyl)-1-(4-fluorophenyl)-1H-pyrazole,
3-(phenylethynyl)-1-(4-(trifluoromethoxy)phenyl)-1H-pyrazole,
3-((3-chlorophenyl)ethynyl)-1-(4-(trifluoromethoxy)phenyl)-1H-pyrazole.

7. A pharmaceutical composition, comprising a compound according to any one of claims 1-6 in free base or pharmaceutically acceptable salt form, in association with a pharmaceutical carrier or diluent.

8. A compound according to any one of claims 1-6 in free base or pharmaceutically acceptable salt form or a pharmaceutical composition of claim 7, for use in the prevention, treatment or delay of progression of disorders associated with irregularities of glutamatergic signal transmission in the digestive tract, urinary tract or central nervous system disorders mediated full or in part by mGlu5 receptors.

9. A compound according to any one of claims 1-6 in free base or pharmaceutically acceptable salt form, or a pharmaceutical composition according to claim 7, for use in the treatment of Alzheimer's disease, Parkinson's disease, levodopa-induced dyskinesia in Parkinson's disease (PD-LID), Huntington's chorea, psychiatric disorders, schizophrenia, mood disorders, emotion disorders, attention deficit disorders, Fragile X syndrome, autism spectrum disorders (ASDs), gastroesophageal reflux disease (GERD), drug addiction, depression disorders.

## Patentansprüche

1. Verbindung von Formel Ia: 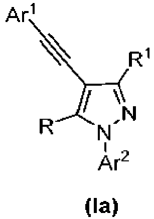 oder pharmazeutisch annehmbares Salz davon, wobei:
R -H, -Halogen, -Alkyl, -Cycloalkyl, -Halogenalkyl, -OR² oder -N(CH₃)₂ ist, wobei R² ein verzweigter oder geradkettiger Alkyl-Radikal mit einem bis neun Kohlenstoffatomen ist, der optional mit 1-3 Substituenten substituiert sein kann, ausgewählt aus der Gruppe, bestehend aus -Halogen, -OH, -Alkoxy und -N(CH₃)₂;
R¹ -H, -Halogen, -Alkyl, -Cycloalkyl, -Halogenalkyl, -OR² oder -N(CH₃)₂ ist, wobei R^{2'} ein verzweigter oder geradkettiger Alkyl-Radikal mit einem bis neun Kohlenstoffatomen ist, der optional mit 1-3 Substituenten substituiert sein kann, ausgewählt aus der Gruppe, bestehend aus -Halogen, -OH, -Alkoxy und -N(CH₃)₂;
Ar¹ ein 5- bis 10-gliedriger mono- oder bicyclischer Heteroaryl-Ring ist, der 1-3 N-Heteroatome enthält, wobei der 5- bis 10-gliedrige mono- oder bicyclische Heteroaryl-Ring optional mit 1-3 Substituenten substituiert ist, die unabhängig ausgewählt sind aus der Gruppe bestehend aus -Alkyl, -Halogen, -OH, -CN, Nitro, -CF₃, -O-CF₃, -O-Alkyl, -O-Aryl, -S-Alkyl, -S-Aryl, -S(O)-Alkyl, -S(O)-Aryl, -S(O₂)-Alkyl, -S(O₂)-Aryl, -CH₂-Aryl, Aryl, Heteroaryl, -O-CH₂-Aryl, -N(CH₃)₂, Cycloalkyl, Heterocycloalkyl, -C(O)-Alkyl, -C(O)-Cycloalkyl, -C(O)-Heterocycloalkyl, -C(O)-Aryl, -C(O)-Heteroaryl, -C(O)O-Alkyl, -C(O)O-Cycloalkyl, -C(O)O-Heterocycloalkyl, -C(O)O-Aryl, -C(O)O-Heteroaryl, -C(O)NH₂, -C(O)NH-Alkyl, -C(O)N(Alkyl)₂, -C(O)NH-Cycloalkyl, -C(O)N(Cycloalkyl)₂, -C(O)NH-Heterocycloalkyl, -C(O)N(Heterocycloalkyl)₂, -C(O)NH-Aryl, -C(O)N(Aryl)_{2,} -C(O)NH-Heteroaryl, -C(O)N(Heteroaryl)₂ und substituiertem Niederalkyl, wobei die Substituenten zusammen einen optional substituierten 5-7-gliedrigen kondensierten carbacyclischen oder heterocyclischen Ring bilden können, oder
ein 5- bis 10-gliedriger mono- oder bicyclischer Aryl-Ring, wobei der 5- bis 10-gliedrige mono- oder bicyclische Aryl-Ring optional mit 1-3 Substituenten substituiert ist, unabhängig ausgewählt aus der Gruppe bestehend aus Alkyl, -Halogen, -OH, -CN, Nitro, -CF₃, -O-CF₃, -O-Alkyl, -O-Aryl, -S-Alkyl, -S-Aryl, -S(O)-Alkyl, -S(O)-Aryl, -S(O₂)-Alkyl, -S(O₂)-Aryl, -CH₂-Aryl, Aryl, Heteroaryl, -O-CH₂-Aryl, -N(CH₃)₂, Cycloalkyl, Heterocycloalkyl, -C(O)-alkyl, -C(O)-Cycloalkyl, -C(O)-Heterocycloalkyl, -C(O)-aryl, -C(O)-Heteroaryl, -C(O)O-Alkyl, -C(O)O-Cycloalkyl, -C(O)O-Heterocycloalkyl, -C(O)O-Aryl, -C(O)O-Heteroaryl, -C(O)NH₂, -C(O)NH-Alkyl, -C(O)N(Alkyl)₂, -C(O)NH-Cycloalkyl, -C(O)N(Cycloalkyl)₂, -C(O)NH-Heterocycloalkyl, -C(O)N(Heterocycloalkyl)₂, -C(O)NH-Aryl, -C(O)N(Aryl)_{2,} -C(O)NH-Heteroaryl, -C(O)N(Heteroaryl)₂ und substituiertem Niederalkyl, wobei die Substituenten zusammen einen 5-7-gliedrigen kondensierten und optional substituierten carbacyclischen oder heterocyclischen Ring bilden können;
Ar² ein 5- bis 10-gliedriger mono- oder bicyclischer Heteroaryl-Ring ist, der 1-3 N-Heteroatome enthält, wobei der 5- bis 10-gliedrige mono- oder bicyclische Heteroaryl-Ring optional mit 1-3 Substituenten substituiert ist, unabhängig ausgewählt sind aus der Gruppe bestehend aus -Alkyl, -Halogen, -OH, -CN, Nitro, -CF₃, -O-CF₃, -O-Alkyl, -O-Aryl, -S-Alkyl, -S-Aryl, -S(O)-Alkyl, -S(O)-Aryl, -S(O₂)-Alkyl, -S(O₂)-Aryl, -O-Alkyl-OH, -O-Alkyl-O-Alkyl, -CH₂-Aryl, Aryl, Heteroaryl, -O-CH₂-Aryl, -N(CH₃)₂, Cycloalkyl, Heterocycloalkyl, -C(O)-Alkyl, -C(O)-Cycloalkyl, -C(O)-Heterocycloalkyl, -C(O)-Aryl, -C(O)-Heteroaryl, -C(O)O-Alkyl, -C(O)O-Cycloalkyl, -C(O)O-Heterocycloalkyl, -C(O)O-Aryl, -C(O)O-Heteroaryl, -C(O)NH₂, -C(O)NH-Alkyl, -C(O)N(Alkyl)₂, -C(O)NH-Cycloalkyl, -C(O)N(Cycloalkyl)₂, -C(O)NH-Heterocycloalkyl, -C(O)N(Heterocycloalkyl)₂, -C(O)NH-Aryl, -C(O)N(Aryl)_{2,} -C(O)NH-Heteroaryl, -C(O)N(Heteroaryl)₂ und substituiertem Niederalkyl, wobei die Substituenten zusammen einen optional substituierten 5-7-gliedrigen kondensierten carbacyclischen oder heterocyclischen Ring bilden können, oder
ein 5- bis 10-gliedriger mono- oder bicyclischer Aryl-Ring, wobei der 5- bis 10-gliedrige mono- oder bicyclische Aryl-Ring optional mit 1-3 Substituenten substituiert ist, unabhängig ausgewählt aus der Gruppe, bestehend aus -Alkyl, -Halogen, -OH, -CN, Nitro, -CF₃, -O-CF₃, -O-Alkyl, -O-Aryl, -S-Alkyl, -S-Aryl, -S(O)-Alkyl, -S(O)-Aryl, -S(O₂)-Alkyl, -S(O₂)-Aryl, -O-Alkyl-OH, -O-Alkyl-O-Alkyl, -CH₂-Aryl, Aryl, Heteroaryl, -O-CH₂-Aryl, -N(CH₃)₂, Cycloalkyl, Heterocycloalkyl, -C(O)-Alkyl, -C(O)-Cycloalkyl, -C(O)-Heterocycloalkyl, -C(O)-Aryl, -C(O)-Heteroaryl, -C(O)O-Alkyl, -C(O)O-Cycloalkyl, -C(O)O-Heterocycloalkyl, -C(O)O-Aryl, -C(O)O-Heteroaryl, -C(O)NH₂, -C(O)NH-Alkyl, -C(O)N(Alkyl)₂, -C(O)NH-Cycloalkyl, -C(O)N(Cycloalkyl)₂, -C(O)NH-Heterocycloalkyl, -C(O)N(Heterocycloalkyl)₂, -C(O)NH-Aryl, -C(O)N(Aryl)_{2,} -C(O)NH-Heteroaryl, -C(O)N(Heteroaryl)₂ und substituiertem Niederalkyl, wobei die Substituenten zusammen einen optional substituierten 5-7-gliedrigen kondensierten carbacyclischen oder heterocyclischen Ring bilden können,
wobei in den obigen Definitionen "Niederalkyl" ein verzweigtes oder geradkettiges Alkyl mit einem bis neun Kohlenstoffatomen ist.

2. Verbindung nach Anspruch 1 oder pharmazeutisch annehmbares Salz davon, wobei:
R -H, -Halogen, -Alkyl, -Cycloalkyl, -Halogenalkyl, -OR² oder -N(CH₃)₂ ist, wobei R² ein verzweigter oder geradkettiger Alkyl-Radikal mit einem bis neun Kohlenstoffatomen ist, der optional mit 1-2 Substituenten, ausgewählt aus der Gruppe bestehend aus -OH und -Alkoxy, substituiert sein kann;
R¹ -H, -Halogen, -Alkyl, -Cycloalkyl, -Halogenalkyl, -OR^{2'} oder -N(CH₃)₂ ist, wobei R^{2'} ein verzweigter oder geradkettiger Alkyl-Radikal mit einem bis neun Kohlenstoffatomen ist, der optional mit 1-3 Substituenten substituiert sein kann, ausgewählt aus der Gruppe, bestehend aus -Halogen, -OH, -Alkoxy und -N(CH₃)₂;
Ar¹ ein 5- oder 6-gliedriger Monoheteroaryl-Ring ist, der 1-3 N-Heteroatome enthält, wobei der 5- oder 6-gliedrige Monoheteroaryl-Ring optional mit 1-3 Substituenten substituiert ist, die unabhängig ausgewählt sind aus der Gruppe, bestehend aus -Alkyl, -Halogen, -OH, -CN, Nitro, -CF₃, -O-CF₃ und -O-Alkyl, oder
einen 6-gliedrigen Aryl-Ring, wobei das 6-gliedrige Aryl optional mit 1-3 Substituenten substituiert ist, unabhängig ausgewählt aus der Gruppe, bestehend aus -Alkyl, -Halogen, -OH, -CN, Nitro, -CF₃, -O-CF₃ und -O-Alkyl;
Ar² ein 5- oder 6-gliedriger Monoheteroaryl-Ring ist, der 1-3 N-Heteroatome enthält, wobei der 5- oder 6-gliedrige Monoheteroaryl-Ring optional mit 1-3 Substituenten substituiert ist, unabhängig ausgewählt aus der Gruppe, bestehend aus -Alkyl, -Halogen, -OH, -CN, Nitro, -CF₃, -O-CF₃, -O-Alkyl, -S-Alkyl, -S(O)-Alkyl, -S(O₂)-Alkyl, -O-Alkyl-O-Alkyl, Aryl, -C(O)O-Alkyl, -C(O)NH₂, -C(O)NH-Alkyl und -C(O)N(Alkyl)₂, oder
ein 6-gliedriger Aryl-Ring, wobei das 6-gliedrige Aryl optional mit 1-3 Substituenten substituiert ist, unabhängig ausgewählt aus der Gruppe, bestehend aus -Alkyl, -Halogen, -OH, -CN, Nitro, -CF₃, -O-CF₃, -O-Alkyl, -S-Alkyl, -S(O)-Alkyl, -S(O₂)-Alkyl, -O-Alkyl-O-Alkyl, Aryl, -C(O)O-Alkyl, -C(O)NH₂, -C(O)NH-Alkyl und -C(O)N(Alkyl)₂.

3. Verbindung nach Anspruch 2 oder pharmazeutisch annehmbares Salz davon, wobei:
R -H, -Halogen, -Alkyl, -Cycloalkyl, -Halogenalkyl, -OR² oder -N(Niederalkyl)₂ ist, wobei R² ein verzweigter oder geradkettiger Alkyl-Radikal mit einem bis neun Kohlenstoffatomen ist, der optional mit 1-2 Substituenten, ausgewählt aus der Gruppe bestehend aus -OH und -Alkoxy, substituiert sein kann;
R¹ -H, -Halogen, -Alkyl, -Cycloalkyl, -Halogenalkyl, -OR^{2'} oder -N(Niederalkyl)₂ ist, wobei R^{2'} ein verzweigter oder geradkettiger Alkyl-Radikal mit einem bis neun Kohlenstoffatomen ist, der optional mit 1-3 Substituenten substituiert sein kann, ausgewählt aus der Gruppe, bestehend aus -Halogen, -OH, -Alkoxy und -N(CH₃)₂;
Ar¹ ein 6-gliedriger Aryl-Ring oder ein 5- oder 6-gliedriger Monoheteroaryl-Ring ist, der 1, 2 oder 3 N-Heteroatome enthält, wobei der 6-gliedrige Aryl- oder der 5- oder 6-gliedrige Monoheteroaryl-Ring optional mit 1, 2 oder 3 Substituenten substituiert ist, unabhängig ausgewählt aus der Gruppe bestehend aus verzweigtem oder geradkettigem Alkyl mit eins bis neun Kohlenstoffatomen und Halogen:
Ar² ein 6-gliedriger Aryl-Ring oder ein 5- oder 6-gliedriger Monoheteroaryl-Ring ist, der 1, 2 oder 3 N-Heteroatome enthält, wobei der 6-gliedrige Aryl-Ring oder der 5- oder 6-gliedrige Monoheteroaryl-Ring optional mit 1, 2 oder 3 Substituenten substituiert ist, unabhängig ausgewählt aus der Gruppe bestehend aus -C₁-C₄-Alkyl, -F, -Cl, -Br, -OH, -CN, Nitro, -CF₃, -OCF₃, -O-C₁-C₄-Alkyl, -SCH₃, -S(O)-CH₃, -S(O₂)-CH₃, -O-C₁-C₄-Alkyl-O-C₁-C₄-Alkyl, -C(O)OCH₃, -C(O)NH₂, -C(O)NH(CH₃), -C(O)N(CH₃)₂, Phenyl.

4. Verbindung nach Anspruch 3 oder ein pharmazeutisch annehmbares Salz davon, wobei:
R -H, -Halogen, -C₁-C₄-Alkyl, -CF₃, -O-C₁-C₄-Alkyl oder -O-C₁-C₄-Alkyl-O-C₁-C₄-Alkyl;
R¹ -H, -Halogen, -C₁-C₄-Alkyl, -C₃-C₆-Cycloalkyl, -Halo-C₁-C₄-Alkyl, -OR² oder -N(C₁-C₄-Alkyl)₂ ist, wobei R^{2'} C₁-C₄-Alkyl ist, das optional mit 1-2 Substituenten substituiert sein kann, ausgewählt aus der Gruppe bestehend aus -Halogen, -OH, -C₁-C₄-Alkoxy und -N(CH₃)₂;
Ar¹ optional substituiertes Pyridinyl, Pyrimidinyl, Pyrazinyl oder Phenyl ist, wobei das Pyridinyl, Pyrimidinyl, Pyrazinyl oder Phenyl optional mit einem Substituenten substituiert ist, ausgewählt aus der Gruppe bestehend aus gerad- oder verzweigtkettigem Alkyl aus einem bis neun Kohlenstoffatomen und Halogen;
Ar² optional substituiertes Pyridinyl oder Phenyl ist, wobei das Pyridinyl oder Phenyl optional mit 1-2 Substituenten substituiert ist, unabhängig ausgewählt aus der Gruppe bestehend aus -C₁-C₄-Alkyl, -Halogen, -CN, -CF₃, -O-CF₃, -S(O₂)-C₁-C₄-Alkyl und -O-C₁-C₄-Alkyl-O-C₁-C₄-Alkyl.

5. Verbindung nach Anspruch 1 oder pharmazeutisch annehmbares Salz davon, wobei die Verbindung wie folgt ist:
2-((1-(4-Fluorphenyl)-3,5-dimethyl-1H-pyrazol-4-yl)ethinyl)pyridin,
2-((3,5-Dimethyl-1-(4-(trifluormethoxy)phenyl)-1H-pyrazol-4-yl)ethinyl)pyridin,
3-(3,5-Dimethyl-4-(2-(pyridin-2-yl)ethinyl)-1H-pyrazol-1-yl)-5-fluorbenzonitril,
3-Fluor-5-(3-methoxy-5-methyl-4-(pyridin-2-ylethinyl)-1H-pyrazol-1-yl)benzonitril,
2-((3-Methoxy-5-methyl-1-(4-(trifluormethoxy)phenyl)-1H-pyrazol-4-yl)ethinyl)pridin,
2-((1-(4-Fluorphenyl)-3-methoxy-5-methyl-1H-pyrazol-4-yl)ethinyl)pyridin,
2-((1-(4-Fluorphenyl)-5-methoxy-3-methyl-1H-pyrazol-4-yl)ethinyl)pyridin,
2-((3-Methyl-5-methoxy-1-(4-(trifluormethoxy)phenyl)-1H-pyrazol-4-yl)ethinyl)pyridin,
3-Fluor-5-(3-methyl-4-(pyridin-2-ylethinyl)-5-(trifluormethyl)-1H-pyrazol-1-yl)benzonitr il,
2-((1-(4-Fluorphenyl)-3-methyl-5-(trifluormethyl)-1H-pyrazol-4-yl)ethinyl)pyridin,
2-((3-Methyl-1-(4-(trifluormethoxy)phenyl)-5-(trifluormethyl)-1H-pyrazol-4-yl)ethinyl)p yridin,
3-(3-(Dimethylamino)-5-methyl-4-(pyridin-2-ylethinyl)-1H-pyrazol-1-yl)-5-fluorbenzoni tril,
2-(3-(Dimethylamino)-5-methyl-1-(4-(trifluoromethoxy)phenyl)-1H-pyrazol-4-yl)ethinyl )pyridin,
5 -((5-Chlor-4-(pyridin-2-ylethinyl)-1H-pyrazol-1-yl)-2-fluorpyridin,
2-((5-Chlor-1-(4-fluorphenyl)-1H-pyrazol-4-yl)ethinyl)pyridin,
2-((5-Chlor-1-(4-(trifluormethoxy)phenyl)-1H-pyrazol-4-yl)ehynyl)pyridin,
2-Fluor-5-(4-(pyridin-2-yl)-1H-pyrazol-1-yl)pyridin,
5-(3,5-Dimethyl-4-(pyridin-2-ylethinyl)-1H-pyrazol-1-yl)-2-fluorpyridin,
2-(3,5-Dimethyl-4-(pyridin-2-ylethinyl)-1H-pyrazol-1-yl)-5-fluorpyridin,
2-((1-(4-(2-Methoxyethoxy)phenyl)-1H-pyrazol-4-yl)ethinyl)pyridin,
5-Fluor-2-(4-(pyridin-2-ylethinyl)-1H-pyrazol-1-yl)pyridin,
2-(2-Methoxyethoxy)-5-(4-(Pyridin-2-ylethinyl)-1H-pyrazol-1-yl)pyridin,
3-Fluor-5-(3-(2-methoxyethoxy)-5-methyl-4-(pyridin-2-ylethinyl)-1H-pyrazol-1-yl)benzo nitril,
2-((3-(2-Methoxyethoxy)-1-(4-(trifluormethoxy)phenyl)-1H-pyrazol-4-yl)ethinyl)pyridin,
3-Fluor-5-(3-(2-methoxyethoxy)-4-(pyridin-2-ylethinyl)-1H-pyrazol-1-yl)benzonitril,
3 -Fluor-5 -(4-(pyridin-2-ylethinyl)-1H-pyrazol-1-yl)pyridin,
2-((5-Chlor-1-(4-(trifluormethyl)phenyl)-1H-pyrazol-4-yl)ethinyl)pyridin,
3-(3-(Difluormethoxy)-5-methyl-4-(pyridin-2-ylethinyl)-1H-pyrazol-1-yl)-5-fluorbenzoni tril,
3 -(3 -Chlor-4-(pyridin-2-ylethinyl)-1H-pyrazol-1-yl)-5-fluorbenzonitril,
2-((3-Chlor-1-(4-(trifluormethoxy)phenyl)-1H-pyrazol-4-yl)ethinyl)pyridin,
2-((3-Chlor-1-(4-fluorphenyl)-1H-pyrazol-4-yl)ethinyl)pyridin,
3-(3-Chlor-4-(pyridin-2-ylethinyl)-1H-pyrazol-1-yl)-5-fluoropyridin,
2-((1-(4-(Methylsulfonyl)phenyl)-lH-pyrazol-4-yl)ethinyl)pyridin,
3 -(3 -(Difluormethoxy)-4-(pyridin-2-ylethinyl)-1H-pyrazol-1-yl)-5-fluorbenzonitril,
3-Fluor-5-(3-(2-hydroxyethoxy)-4-(pyridin-2-ylethinyl)-1H-pyrazol-1-yl)benzonitril,
2-(3-Chlor-4-(pyridin-2-ylethinyl)-1H-pyrazol-1-yl)-5-fluorpyridin,
3-Fluor-5-(5-methyl-4-(pyridin-2-ylethinyl)-1H-pyrazol-1-yl)benzonitril,
2-((1-(4-Fluorphenyl)-5-methoxy-lH-pyrazol-4-yl)ethinyl)pyridin,
2-((1-(4-Fluorphenyl)-3-methoxy-1H-pyrazol-4-yl)ethinyl)pyridin,
3-(3-(2-(Dimethylamino)ethoxy)-4-(pyridin-2-ylethinyl)-1H-pyrazol-1-yl)-5-fluor-benzo nitril,
3-(3-Cyclopropyl-4-(pyridin-2-ylethinyl)-1H-pyrazol-1-yl)-5-fluorbenzonitril,
2-((1-(4-Fluorphenyl)-5-(2-methoxyethoxy)-1H-pyrazol-4-yl)ethinyl)pyridin,
2-((3-Chlor-1-(4-(methylsulfonyl)phenyl)-1H-pyrazol-4-yl)ethinyl)pyridin,
5-Fluor-2-(5-methoxy-4-(pyridin-2-ylethinyl)-1H-pyrazol-1-yl)pyridin,
5 -Fluor-2-(3 -methyl-4-(pyridin-2-ylethinyl)-1H-pyrazol-1-yl)pyridin,
5 -Fluor-2-(3-(2-methoxyethoxy)-4-(pyridin-2-ylethinyl)-1H-pyrazol-1-yl)pyridin,
3 -Fluor-5-(4-(Pyridin-2-ylethinyl)-1H-pyrazol-1-yl)benzonitril,
2-(3-(Difluormethoxy)-4-(pyridin-2-ylethinyl)-1H-pyrazol-1-yl)-5-fluoropyridin,
3 -Fluor-5 -(4-(Pyridin-2-ylethinyl)- 1H-pyrazol-1-yl)benzonitril,
3 -Fluor-5 -(4-(Pyridin-3 -ylethinyl)-1H-pyrazol-1-yl)benzonitril,
3 -Fluor-5 -(4-(Pyridin-4-ylethinyl)-1H-pyrazol-1-yl)benzonitril,
2-((1-(4-Fluorphenyl)-1H-pyrazol-4-yl)ethinyl)pyridin,
3-(1-(4-Fluorphenyl)-1H-pyrazol-4-yl)ethinyl)pyridin,
2-Chlor-4-((1-(4-fluorphenyl)-1H-pyrazol-4-yl)ethinyl)pyridin,
4-((1-(4-Fluorphenyl)-1H-pyrazol-4-yl)ethinyl)pyridin,
2-((1-(4-(Trifluormethoxy)phenyl)-1H-pyrazol-4-yl)ethinyl)pyridin,
3-((1-(4-(Trifluormethoxy)phenyl)-1H-pyrazol-4-yl)ethinyl)pyridin,
4-((1-(4-(Trifluormethoxy)phenyl)-1H-pyrazol-4-yl)ethinyl)pyridin,
2-((1-(4-Fluorphenyl)-3,5-dimethyl-1H-pyrazol-4-yl)ethinyl)pyrimidin,
4-((1-(4-Fluorphenyl)-3,5-dimethyl-1H-pyrazol-4-yl)ethinyl)pyrimidin,
2-((3,5-Dimethyl-1-(4-(trifluormethoxy)phenyl)-1H-pyrazol-4-yl)ethinyl)pyrimidin,
2-((5-Chlor-1-(4-fluorphenyl)-1H-pyrazol-4-yl)ethinyl)pyrimidin,
2-((5-Chlor-1-(6-fluorpyridin-3-yl)-1H-pyrazol-4-yl)ethinyl)pyrimidin,
2-((5-Chlor-1-(4-trifluormethoxy)phenyl)-1H-pyrazol-4-yl)ethinyl)pyrimidin,
3 -Fluor-5 -(3 -(2-methoxyethoxy)-5-methyl-4-(pyrimidin-2-ylethinyl)-1H-pyrazol-1-yl)be nzonitril,
2-((1-(6-Fluorpyridin-3-yl)-1H-pyrazol-4-yl)ethinyl)pyrimidin,
2-((5-Chlor-1-(4-(trifluormethyl)phenyl)-1H-pyrazol-4-yl)ethinyl)pyrimidin,
3 -(3 -Chlor-4-(pyrimidin-2-ylethinyl)-1H-pyrazol-1-yl)-5-fluorbenzonitril,
2-((3-Chlor-1-(4-(trifluormethoxy)phenyl)-1H-pyrazol-4-yl)ethinyl)pyrimidin,
2-((3-Chlor-1-(4-fluorphenyl)-1H-pyrazol-4-yl)ethinyl)pyrimidin,
2-((3-Chlor-1-(5-fluorpyridin-3-yl)-1H-pyrazol-4-yl)ethinyl)pyrimidin,
3-(3-Cyclopropyl-4-(pyrimidin-2-ylethinyl)-1H-pyrazol-1-yl)-5-fluorbenzonitril
2-((1-(4-Fluorphenyl)-5-(2-methoxyethoxy)-1H-pyrazol-4-yl)ethinyl)pyrimidin,
3-Fluor-5-(4-(Pyrimidin-2-ylethinyl)-1H-pyrazol-1-yl)benzonitril,
2-((1-(4-Fluorphenyl)-1H-pyrazol-4-yl)ethinyl)pyrimidin,
2-((1-(4-(Trifluorphenyl)-1H-pyrazol-4-yl)ethinyl)pyrimidin,
2-((1-(4-(Trifluormethoxy)phenyl)-1H-pyrazol-4-yl)ethinyl)pyrazin
3 -Fluor-5-(4-(pyrazin-2-ylethinyl)-1H-pyrazol-1-yl)benzonitril,
2-((1-(4-Fluorphenyl)-1H-pyrazol-4-yl)ethinyl)pyrazin,
2-((1-(4-(Trifluormethoxy)phenyl)-1H-pyrazol-4-yl)ethinyl)pyrazin,
3-Fluor-5-(4-(phenylethinyl)-1H-pyrazol-1-yl)benzonitril,
3-Fluor-5-(4-(3-chlor-phenylethinyl)-1H-pyrazol-1-yl)benzonitril,
1-(4-Fluorphenyl)-4-(phenylethinyl)-1H-pyrazol,
4-((3-Chlorphenyl)ethinyl)-1-(4-fluorphenyl)-1H-pyrazol,
4-(Phenylethinyl)-1-(4-(Trifluormethoxy)phenyl)- 1H-pyrazol,
4-((3-Chlorphenyl)ethinyl)-1-(4-(Trifluormethoxy)phenyl)-1H-pyrazol.

6. Verbindung nach Anspruch 1 oder pharmazeutisch annehmbares Salz davon, wobei die Verbindung wie folgt ist:
3-Fluor-5-(4-methyl-3-(pyridin-2-ylethinyl)-1H-pyrazol-1-yl)benzonitril,
3-(4-Chlor-3-(pyridin-2-ylethinyl)-1H-pyrazol-1-yl)-5-fluorbenzonitril,
5 -Fluor-2-(3-(pyridin-2-ylethinyl)-1H-pyrazol-1-yl)pyridin,
2-(4-Chlor-3-(pyridin-2-ylethinyl)-1H-pyrazol-1-yl)-5-fluoropyridin,
3-Fluor-5-(3-(Pyridin-2-ylethinyl)-1H-pyrazol-1-yl)pyridin,
2-((1-(Pyridin-2-yl)-1H-pyrazol-3-yl)ethinyl)pyridin,
5-Fluor-2-(4-methyl-3-(pyridin-2-ylethinyl)-1H-pyrazol-1-yl)pyridin,
5 -Fluor-2-(5 -methyl-3 -(pyridin-2-ylethinyl)-1H-pyrazol-1-yl)pyridin,
3-Fluor-5-(5-methy1-3-(pyridin-2-ylethinyl)-1H-pyrazol-1-yl)benzonitril,
3 -Fluor-5-(5-methyl-3-(pyridin-3-ylethinyl)-1H-pyrazol-1-yl)benzonitril,
3-(3-((2-Chlorpyridin-4-yl)ethinyl)-5-methyl-1H-pyrazol-1-yl)-5-fluorbenzonitril,
3-Fluor-5-(5-methyl-3-(pyridin-4-ylethinyl)-1H-pyrazol-1-yl)benzonitril,
3-Fluor-5-(3-(pyridin-2-ylethinyl)-1H-pyrazol-1-yl)benzonitril,
3-Fluor-5-(3-(pyridin-3-ylethinyl)-1H-pyrazol-1-yl)benzonitril,
3-(3-((2-Chlorpyridin-4-yl)ethinyl)-1H-pyrazol-1-yl)-5-fluorbenzonitril,
3-Fluor-5-(3-(Pyridin-4-ylethinyl)-1H-pyrazol-1-yl)benzonitril,
2-((1 -(4-Fluorphenyl)-1H-pyrazol-3-yl)ethinyl)pyridin,
3-(1-(4-Fluorphenyl)-1H-pyrazol-3-yl)ethinyl)pyridin,
2-Chlor-4-((1-(4-fluorphenyl)-1H-pyrazol-3-yl)ethinyl)pyridin,
4-((1-(4-Fluorphenyl)-1H-pyrazol-3-yl)ethinyl)pyridin,
3-((1-(rifluormethoxy)phenyl)-1H-pyrazol-3-yl)ethinyl)pyridin,
4-((1-(4-(Trifluormethoxy)phenyl)-1H-pyrazol-3-yl)ethinyl)pyridin,
2-((1-(4-Fluorphenyl)-5-methyl-1H-pyrazol-3-yl)ethinyl)pyridin,
3-(1-(4-Fluorphenyl)-5-methyl-1H-pyrazol-3-yl)ethinyl)pyridin,
4-((1-(4-Fluorphenyl)-5-methyl-1H-pyrazol-3-yl)ethinyl)pyridin,
2-Chlor-4-((1-(4-fluorphenyl)-5-methyl-1H-pyrazol-3-yl)ethinyl)pyridin,
3-((5-Methyl-1-(4-(trifluormethoxy)phenyl)-1H-pyrazol-3-yl)ethinyl)pyridin,
2-Chlor-4-((5-methyl-1-(4-(trifluormethoxy)phenyl)-1H-pyrazol-3-yl)ethinyl)pyridin,
2-((5-Methyl-1-(4-(trifluoromethoxy)phenyl)-1H-pyrazol-3-yl)ethinyl)pyridin,
3-Fluor-5-(4-methyl-3-(pyrimidin-2-ylethinyl)-1H-pyrazol-1-yl)benzonitril,
3-Fluor-5-(5-methyl-3-(pyrimidin-2-ylethinyl)-1H-pyrazol-1-yl)benzonitril,
3-Fluor-5-(3-(pyrimidin-2-ylethinyl)-1H-pyrazol-1-yl)benzonitril,
2-((1 -(4-Fluorphenyl)-1H-pyrazol-3 -yl)ethinyl)pyrimidin,
2-((1-(4-(Trifluormethoxy)phenyl)-1H-pyrazol-3-yl)ethinyl)pyrimidin,
2-((1 -(4-Fluorphenyl)-5 -methyl-1H-pyrazol-3 -yl)ethinyl)pyrimidin,
2-((5-Methyl-1-(4-(trifluormethoxy)phenyl)-1H-pyrazol-3-yl)ethinyl)pyrimidin,
3-Fluor-5-(5-methyl-3-(pyrazin-2-ylethinyl)-1H-pyrazol-1-yl)benzonitril,
3-Fluor-5-(3-(pyrazin-2-ylethinyl)-1H-pyrazol-1-yl)benzonitril,
2-((1 -(4-Fluorphenyl)-1H-pyrazol-3 -yl)ethinyl)pyrazin,
2-((1-(4-(Trifluormethoxy)phenyl)-1H-pyrazol-3-yl)ethinyl)pyrazin,
2-((1-(4-Fluorphenyl)-5-methyl-1H-pyrazol-3-yl)ethinyl)pyrazin,
2-((5-Methyl-1-(4-(trifluormethoxy)phenyl)-1H-pyrazol-3-yl)ethinyl)pyrazin,
3-Fluor-5-(5-methyl-3-(phenylethinyl)-1H-pyrazol-1-yl)benzonitril,
3-(3-((3-Chlorphenyl)ethinyl)-5-methyl-1H-pyrazol-1-yl)-5-fluorbenzonitril,
3-Fluor-5-(3-(phenylethinyl)-1H-pyrazol-1-yl)benzonitril,
3-(3-((3-Chlorphenyl)ethinyl)-1H-pyrazol-1-yl)-5-fluorbenzonitril,
1-(4-Fluorphenyl)-3-(phenylethinyl)-1H-pyrazol,
3-((3-Chlorphenyl)ethinyl)-1-(4-fluorphenyl)-1H-pyrazol,
3-(Phenylethinyl)-1-(4-(trifluormethoxy)phenyl)-1H-pyrazol,
3-((3-Chlorphenyl)ethinyl)-1-(4-(trifluormethoxy)phenyl)-1H-pyrazol.

7. Pharmazeutische Zusammensetzung, umfassend eine Verbindung nach einem der Ansprüche 1-6 in Form einer freien Base oder eines pharmazeutisch annehmbaren Salzes in Verbindung mit einem pharmazeutischen Träger oder Verdünnungsmittel.

8. Verbindung nach einem der Ansprüche 1-6 in Form einer freien Base oder eines pharmazeutisch annehmbaren Salzes oder eine pharmazeutische Zusammensetzung nach Anspruch 7 zur Verwendung bei der Vorbeugung, Behandlung oder Verzögerung eines Fortschreitens von Störungen, die mit Unregelmäßigkeiten der glutamatergen Signalübertragung im Verdauungstrakt, den Harnwegen oder Störungen des zentralen Nervensystems, die ganz oder teilweise durch mGlu5-Rezeptoren vermittelt werden, assoziiert sind.

9. Verbindung nach einem der Ansprüche 1 bis 6 in Form einer freien Base oder eines pharmazeutisch annehmbaren Salzes oder eine pharmazeutische Zusammensetzung nach Anspruch 7 zur Verwendung bei der Behandlung von Alzheimer-Krankheit, Parkinson-Krankheit, Levodopa-induzierter Dyskinesie bei Parkinson-Krankheit (PD-LID), Chorea Huntington, psychiatrischen Störungen, Schizophrenie, Stimmungsstörungen, Emotionsstörungen, Aufmerksamkeitsdefizitstörungen, fragilem X-Syndrom, Autismus-Spektrum-Störungen (ASD), gastroösophagealer Reflux-Krankheit (GERD), Drogenabhängigkeit, Depressionsstörungen.

## Revendications

1. Composé de formule la : 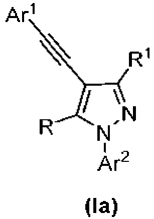 ou un sel pharmaceutiquement acceptable de celui-ci, dans lequel :
R est - H, -halogène, -alkyle, -cycloalkyle, -haloalkyle, -OR², ou -N(CH₃)₂, dans lequel R² est un radical alkyle à chaîne ramifiée ou linéaire de un à neuf atomes de carbone qui peut être facultativement substitué par 1 à 3 substituants choisis parmi le groupe constitué par -halogène, -OH, -alcoxy, et -N(CH₃)₂ ;
R¹ est -H, -halogène, -alkyle, -cycloalkyle, -haloalkyle, -OR², ou -N(CH₃)₂, dans lequel R^{2'} est un radical alkyle à chaîne ramifiée ou linéaire de un à neuf atomes de carbone qui peut être facultativement substitué par 1 à 3 substituants choisis parmi le groupe constitué par -halogène, -OH, -alcoxy, et -N(CH₃)₂ ;
Ar¹ est un noyau hétéroaryle monocyclique ou bicyclique de 5 à 10 éléments qui contient 1 à 3 hétéroatomes N, dans lequel le noyau hétéroaryle monocyclique ou bicyclique de 5 à 10 éléments est facultativement substitué par 1 à 3 substituants choisis indépendamment parmi le groupe constitué par -alkyle, -halogène, -OH, -CN, un nitro, -CF₃, -O-CF₃, -O-alkyle, -O-aryle, -S-alkyle, -S-aryle, -S(O)-alkyle, -S(O)-aryle, -S(O₂)-alkyle, -S(O₂)-aryle, -CH₂-aryle, un aryle, un hétéroaryle, -O-CH₂-aryle, -N(CH₃)₂, un cycloalkyle, un hétérocycloalkyle, -C(O)-alkyle, -C(O)-cycloalkyle, -C(O)-hétérocycloalkyle, -C(O)-aryle, -C(O)-hétéroaryle, -C(O)O-alkyle, -C(O)O-cycloalkyle, -C(O)O-hétérocycloalkyle, -C(O)O-aryle, -C(O)O-hétéroaryle, -C(O)NH₂, -C(O)NH-alkyle, -C(O)N(alkyl)₂, -C(O)NH-cycloalkyle, -C(O)N(cycloalkyle)₂, -C(O)NH-hétérocycloalkyle, -C(O)N(hétérocycloalkyle)₂, -C(O)NH-aryle, -C(O)N(aryle)₂, -C(O)NH-hétéroaryle, -C(O)N(hétéroaryle)₂, et un alkyle inférieur substitué, dans lequel les substituants peuvent se combiner pour former un noyau hétérocyclique ou carbacyclique condensé de 5 à 7 éléments facultativement substitué, ou
un noyau aryle monocyclique ou bicyclique de 5 à 10 éléments, dans lequel le noyau aryle monocyclique ou bicyclique de 5 à 10 éléments est facultativement substitué par 1 à 3 substituants choisis indépendamment parmi le groupe constitué par un alkyle, -halogène, -OH, -CN, un nitro, -CF₃, -O-CF₃, -O-alkyle, -O-aryle, -S-alkyle, -S-aryle, -S(O)-alkyle, -S(O)-aryle, -S(O₂)-alkyle, -S(O₂)-aryle, -CH₂-aryle, un aryle, un hétéroaryle, -O-CH₂-aryle, -N(CH₃)₂, un cycloalkyle, un hétérocycloalkyle, -C(O)-alkyle, -C(O)-cycloalkyle, -C(O)-hétérocycloalkyle, -C(O)-aryle, -C(O)-hétéroaryle, -C(O)O-alkyle, -C(O)O-cycloalkyle, -C(O)O-hétérocycloalkyle, -C(O)O-aryle, -C(O)O-hétéroaryle, -C(O)NH₂, -C(O)NH-alkyle, -C(O)N(alkyle)₂, -C(O)NH-cycloalkyle, -C(O)N(cycloalkyle)₂, -C(O)NH-hétérocycloalkyle, -C(O)N(hétérocycloalkyle)₂, -C(O)NH-aryle, -C(O)N(aryle)₂, -C(O)NH-hétéroaryle, -C(O)N(hétéroaryle)₂, et un alkyle inférieur substitué, dans lequel les substituants peuvent se combiner pour former un noyau hétérocyclique ou carbacyclique facultativement substitué et condensé de 5 à 7 éléments ;
Ar² est un noyau hétéroaryle monocyclique ou bicyclique de 5 à 10 éléments qui contient 1 à 3 hétéroatomes N, dans lequel le noyau hétéroaryle monocyclique ou bicyclique de 5 à 10 éléments est facultativement substitué par 1 à 3 substituants choisis indépendamment parmi le groupe constitué par -alkyle, -halogène, -OH, -CN, un nitro, -CF₃, -O-CF₃, -O-alkyle, -O-aryle, -S-alkyle, -S-aryle, -S(O)-alkyle, -S(O)-aryle, -S(O₂)-alkyle, -S(O₂)-aryle, -O-alkyl-OH, -O-alkyl-O-alkyle, -CH₂-aryle, un aryle, un hétéroaryle, -O-CH₂-aryle, -N(CH₃)₂, un cycloalkyle, un hétérocycloalkyle, -C(O)-alkyle, -C(O)-cycloalkyle, -C(O)-hétérocycloalkyle, -C(O)-aryle, -C(O)-hétéroaryle, -C(O)O-alkyle, -C(O)O-cycloalkyle, -C(O)O-hétérocycloalkyle, -C(O)O-aryle, -C(O)O-hétéroaryle, -C(O)NH₂, -C(O)NH-alkyle, -C(O)N(alkyle)₂, -C(O)NH-cycloalkyle, -C(O)N(cycloalkyle)₂, -C(O)NH-hétérocycloalkyle, -C(O)N(hétérocycloalkyle)₂, -C(O)NH-aryle, -C(O)N(aryle)₂, -C(O)NH-hétéroaryle, -C(O)N(hétéroaryle)₂, et un alkyle inférieur substitué dans lequel les substituants peuvent se combiner pour former un noyau hétérocyclique ou carbacyclique condensé de 5 à 7 éléments facultativement substitué, ou
un noyau aryle monocyclique ou bicyclique de 5 à 10 éléments, dans lequel le noyau aryle monocyclique ou bicyclique de 5 à 10 éléments est facultativement substitué par 1 à 3 substituants choisis indépendamment parmi le groupe constitué par -alkyle, -halogène, -OH, -CN, un nitro, -CF₃, -O-CF₃, -O-alkyle, -O-aryle, -S-alkyle, -S-aryle, -S(O)-alkyle, -S(O)-aryle, -S(O₂)-alkyle, -S(O₂)-aryle, -O-alkyl-OH, -O-alkyl-O-alkyle, -CH₂-aryle, un aryle, un hétéroaryle, -O-CH₂-aryle, -N(CH₃)₂, un cycloalkyle, un hétérocycloalkyle, -C(O)-alkyle, -C(O)-cycloalkyle, -C(O)-hétérocycloalkyle, -C(O)-aryle, -C(O)-hétéroaryle, -C(O)O-alkyle, -C(O)O-cycloalkyle, -C(O)O-hétérocycloalkyle, -C(O)O-aryle, -C(O)O-hétéroaryle, -C(O)NH₂, -C(O)NH-alkyle, -C(O)N(alkyle)₂, -C(O)NH-cycloalkyle, -C(O)N(cycloalkyle)₂, -C(O)NH-hétérocycloalkyle, -C(O)N(hétérocycloalkyle)₂, -C(O)NH-aryle, -C(O)N(aryle)₂, -C(O)NH-hétéroaryle, -C(O)N(hétéroaryle)₂, et un alkyle inférieur substitué dans lequel les substituants peuvent se combiner pour former un noyau hétérocyclique ou carbacyclique condensé de 5 à 7 éléments facultativement substitué,
dans lequel dans les définitions susmentionnées, un « alkyle inférieur » est un alkyle à chaîne ramifiée ou linéaire de un à neuf atomes de carbone.

2. Composé selon la revendication 1 ou sel pharmaceutiquement acceptable de celui-ci, dans lequel :
R est -H, -halogène, -alkyle, -cycloalkyle, -haloalkyle, -OR², ou -N(CH₃)₂, dans lequel R² est un radical alkyle à chaîne ramifiée ou linéaire de un à neuf atomes de carbone qui peut être facultativement substitué par 1 à 2 substituants choisis parmi le groupe constitué par -OH et -alcoxy ;
R¹ est -H, -halogène, -alkyle, -cycloalkyle, -haloalkyle, -OR^{2'}, ou -N(CH₃)₂, dans lequel R^{2'} est un radical alkyle à chaîne ramifiée ou linéaire de un à neuf atomes de carbone qui peut être facultativement substitué par 1 à 3 substituants choisis parmi le groupe constitué par -halogène, -OH, -alcoxy, et -N(CH₃)₂ ;
Ar¹ est un noyau mono-hétéroaryle de 5 ou 6 éléments qui contient 1 à 3 hétéroatomes N, dans lequel le noyau mono-hétéroaryle de 5 ou 6 éléments est facultativement substitué par 1 à 3 substituants choisis indépendamment parmi le groupe constitué par -alkyle, -halogène, -OH, -CN, un nitro, -CF₃, -O-CF₃, et -O-alkyle, ou un noyau aryle de 6 éléments, dans lequel l'aryle de 6 éléments est facultativement substitué par 1 à 3 substituants choisis indépendamment parmi le groupe constitué par -alkyle, -halogène, -OH, -CN, un nitro, -CF₃, -O-CF₃, et -O-alkyle;
Ar² est un noyau mono-hétéroaryle de 5 ou 6 éléments qui contient 1 à 3 hétéroatomes N, dans lequel le noyau mono-hétéroaryle de 5 ou 6 éléments est facultativement substitué par 1 à 3 substituants choisis indépendamment parmi le groupe constitué par -alkyle, -halogène, -OH, -CN, un nitro, -CF₃, -O-CF₃, -O-alkyle, -S-alkyle, -S(O)-alkyle, -S(O₂)-alkyle, -O-alkyl-O-alkyle, un aryle, -C(O)O-alkyle, -C(O)NH₂, -C(O)NH-alkyle, et -C(O)N(alkyle)₂, ou
un noyau aryle de 6 éléments, dans lequel l'aryle de 6 éléments est facultativement substitué par 1 à 3 substituants choisis indépendamment parmi le groupe constitué par -alkyle, -halogène, -OH, -CN, un nitro, -CF₃, -O-CF₃, -O-alkyle, -S-alkyle, -S(O)-alkyle, -S(O₂)-alkyle, -O-alkyl-O-alkyle, un aryle, -C(O)O-alkyle, -C(O)NH₂, -C(O)NH-alkyle, et -C(O)N(alkyle)₂.

3. Composé selon la revendication 2 ou sel pharmaceutiquement acceptable de celui-ci, dans lequel :
R est -H, -halogène, -alkyle, -cycloalkyle, -haloalkyle, -OR², ou -N(alkyle inférieur)₂, dans lequel R² est un radical alkyle à chaîne ramifiée ou linéaire de un à neuf atomes de carbone qui peut être facultativement substitué par 1 à 2 substituants choisis parmi le groupe constitué par -OH et -alcoxy ;
R¹ est -H, -halogène, -alkyle, -cycloalkyle, -haloalkyle, -OR^{2'}, ou -N(alkyle inférieur)₂, dans lequel R^{2'} est un radical alkyle à chaîne ramifiée ou linéaire de un à neuf atomes de carbone qui peut être facultativement substitué par 1 à 3 substituants choisis parmi le groupe constitué par -halogène, -OH, -alcoxy, et -N(CH₃)₂ ;
Ar¹ est un noyau aryle de 6 éléments ou un noyau mono-hétéroaryle de 5 ou 6 éléments qui contient 1, 2, ou 3 hétéroatomes N, dans lequel le noyau aryle de 6 éléments ou le noyau mono-hétéroaryle de 5 ou 6 éléments est facultativement substitué par 1, 2 ou 3 substituants choisis indépendamment parmi le groupe constitué par un alkyle à chaîne ramifiée ou linéaire de un à neuf atomes de carbone, et un halogène ;
Ar² est un noyau aryle de 6 éléments ou un noyau mono-hétéroaryle de 5 ou 6 éléments qui contient 1, 2, ou 3 hétéroatomes N, dans lequel le noyau aryle de 6 éléments ou le noyau mono-hétéroaryle de 5 ou 6 éléments est facultativement substitué par 1, 2 ou 3 substituants choisis indépendamment parmi le groupe constitué par -alkyle en C₁-C₄, -F, -Cl, -Br, -OH, -CN, un nitro, -CF₃, -OCF₃, -O-alkyle en C₁-C₄, -SCH₃, -S(O)-CH₃, -S(O₂)-CH₃, -O-alkyle en C₁-C₄-O-alkyle en C₁-C₄, -C(O)OCH₃, -C(O)NH₂, -C(O)NH(CH₃), -C(O)N(CH₃)₂, un phényle.

4. Composé selon la revendication 3 ou sel pharmaceutiquement acceptable de celui-ci, dans lequel :
R est -H, -halogène, -alkyle en C₁-C₄, -CF₃, -O-alkyle en C₁-C₄, ou -O-alkyle en C₁-C₄-O-alkyle en C₁-C₄ ;
R¹ est -H, -halogène, -alkyle en C₁-C₄, -cycloalkyle en C₃-C₆, -halo-alkyle en C₁-C₄, -OR^{2'}, ou -N(alkyle en C₁-C₄)₂, dans lequel R^{2'} est un alkyle en C₁-C₄ qui peut être facultativement substitué par 1 à 2 substituants choisis parmi le groupe constitué par -halogène, -OH, -alcoxy en C₁-C₄, et -N(CH₃)₂ ;
Ar¹ est un pyridinyle, un pyrimidinyle, un pyrazinyle, ou un phényle, facultativement substitué, dans lequel le pyridinyle, le pyrimidinyle, le pyrazinyle, ou le phényle est facultativement substitué par un substituant choisi parmi le groupe constitué par un alkyle à chaîne ramifiée ou linéaire de un à neuf atomes de carbone, et un halogène ;
Ar² est un pyridinyle, ou un phényle, facultativement substitué, dans lequel le pyridinyle, ou le phényle est facultativement substitué par 1 à 2 substituants choisis indépendamment parmi le groupe constitué par -alkyle en C₁-C₄, -halogène, -CN, -CF₃, -O-CF₃, -S(O₂)-alkyle en C₁-C₄, et -O-alkyle en C₁-C₄-O-alkyle en C₁-C₄.

5. Composé selon la revendication 1 ou sel pharmaceutiquement acceptable de celui-ci, dans lequel ledit composé est :
2-((1-(4-fluorophényl)-3,5-diméthyl-1H-pyrazol-4-yl)éthynyl)pyridine,
2-((3,5-diméthyl-l-(4-(trifluorométhoxy)phényl)-1H-pyrazol-4-yl)éthynyl)pyridine,
3-(3,5-diméthyl-4-(2-(pyridin-2-yl)éthynyl)-1H-pyrazol-1-yl)-5-fluorobenzonitrile,
3-fluoro-5-(3-méthoxy-5-méthyl-4-(pyridin-2-yléthynyl)-1H-pyrazol-1-yl)benzonitrile,
2-((3-méthoxy-5-méthyl-1-(4-(trifluorométhoxy)phényl)-1H-pyrazol-4-yl)éthynyl) pridine,
2-((1-(4-fluorophényl)-3-méthoxy-5-méthyl-1H-pyrazol-4-yl)éthynyl)pyridine,
2-((1-(4-fluorophényl)-5-méthoxy-3-méthyl-1H-pyrazol-4-yl)éthynyl)pyridine,
2-((3-méthyl-5-méthoxy-1-(4-(trifluorométhoxy)phényl)-1H-pyrazol-4-yl)éthynyl) pyridine,
3-fluoro-5-(3-méthyl-4-(pyridin-2-yléthynyl)-5-(trifluorométhyl)-1H-pyrazol-1-yl) benzonitrile,
2-((1-(4-fluorophényl)-3-méthyl-5-(trifluorométhyl)-1H-pyrazol-4-yl)éthynyl)pyridine,
2-((3-méthyl-1-(4-(trifluorométhoxy)phényl)-5-(trifluorométhyl)-1H-pyrazol-4-yl) éthynyl)pyridine,
3-(3-(diméthylamino)-5-méthyl-4-(pyridin-2-yléthynyl)-1H-pyrazol-1-yl)-5-fluorobenzonitrile,
2-(3-(diméthylamino)-5-méthyl-1-(4-(trifluorométhoxy)phényl)-1H-pyrazol-4-yl) éthynyl)pyridine,
5-((5-chloro-4-(pyridin-2-yléthynyl)-1H-pyrazol-1-yl)-2-fluoropyridine,
2-((5-chloro-1-(4-fluorophényl)-1H-pyrazol-4-yl)éthynyl)pyridine,
2-((5-chloro-1-(4-(trifluorométhoxy)phényl)-1H-pyrazol-4-yl)éhynyl)pyridine,
2-fluoro-5-(4-(pyridin-2-yl)-1H-pyrazol-1-yl)pyridine,
5-(3,5-diméthyl-4-(pyridin-2-yléthynyl)-1H-pyrazol-1-yl)-2-fluoropyridine,
2-(3,5-diméthyl-4-(pyridin-2-yléthynyl)-1H-pyrazol-1-yl)-5-fluoropyridine,
2-((1-(4-(2-méthoxyéthoxy)phényl)-1H-pyrazol-4-yl)éthynyl)pyridine,
5-fluoro-2-(4-(pyridin-2-yléthynyl)-1H-pyrazol-1-yl)pyridine,
2-(2-méthoxyéthoxy)-5-(4-(pyridin-2-yléthynyl)-1H-pyrazol-1-yl)pyridine,
3-fluoro-5-(3-(2-méthoxyéthoxy)-5-méthyl-4-(pyridin-2-yléthynyl)-1H-pyrazol-1-yl) benzonitrile,
2-((3-(2-méthoxyéthoxy)-1-(4-(trifluorométhoxy)phényl)-1H-pyrazol-4-yl) éthynyl)pyridine,
3-fluoro-5-(3-(2-méthoxyéthoxy)-4-(pyridin-2-yléthynyl)-1H-pyrazol-1-yl)benzonitrile,
3-fluoro-5-(4-(pyridin-2-yléthynyl)-1H-pyrazol-1-yl)pyridine,
2-((5-chloro-1-(4-(trifluorométhyl)phényl)-1H-pyrazol-4-yl)éthynyl)pyridine,
3-(3-(difluorométhoxy)-5-méthyl-4-(pyridin-2-yléthynyl)-1H-pyrazol-1-yl)-5-fluorobenzonitrile,
3-(3-chloro-4-(pyridin-2-yléthynyl)-1H-pyrazol-1-yl)-5-fluorobenzonitrile,
2-((3-chloro-1-(4-(trifluorométhoxy)phényl)-1H-pyrazol-4-yl)éthynyl)pyridine,
2-((3-chloro-1-(4-fluorophényl)-1H-pyrazol-4-yl)éthynyl)pyridine,
3-(3-chloro-4-(pyridin-2-yléthynyl)-1H-pyrazol-1-yl)-5-fluoropyridine,
2-((1-(4-(méthylsulfonyl)phényl)-1H-pyrazol-4-yl)éthynyl)pyridine,
3-(3-(difluorométhoxy)-4-(pyridin-2-yléthynyl)-1H-pyrazol-1-yl)-5-fluorobenzonitrile,
3-fluoro-5-(3-(2-hydroxyéthoxy)-4-(pyridin-2-yléthynyl)-1H-pyrazol-1-yl)benzonitrile,
2-(3-chloro-4-(pyridin-2-yléthynyl)-1H-pyrazol-1-yl)-5-fluoropyridine,
3-fluoro-5-(5-méthyl-4-(pyridin-2-yléthynyl)-1H-pyrazol-1-yl)benzonitrile,
2-((1-(4-fluorophényl)-5-méthoxy-1H-pyrazol-4-yl)éthynyl)pyridine,
2-((1-(4-fluorophényl)-3-méthoxy-1H-pyrazol-4-yl)éthynyl)pyridine,
3-(3-(2-(diméthylamino)éthoxy)-4-(pyridin-2-yléthynyl)-1H-pyrazol-1-yl)-5-fluorobenzonitrile,
3-(3-cyclopropyl-4-(pyridin-2-yléthynyl)-1H-pyrazol-1-yl)-5-fluorobenzonitrile,
2-((1-(4-fluorophényl)-5-(2-méthoxyéthoxy)-1H-pyrazol-4-yl)éthynyl)pyridine,
2-((3-chloro-1-(4-(méthylsulfonyl)phényl)-1H-pyrazol-4-yl)éthynyl)pyridine,
5-fluoro-2-(5-méthoxy-4-(pyridin-2-yléthynyl)-1H-pyrazol-1-yl)pyridine,
5-fluoro-2-(3-méthyl-4-(pyridin-2-yléthynyl)-1H-pyrazol-1-yl)pyridine,
5-fluoro-2-(3-(2-méthoxyéthoxy)-4-(pyridin-2-yléthynyl)-1H-pyrazol-1-yl)pyridine,
3-fluoro-5-(4-(pyridin-2-yléthynyl)-1H-pyrazol-1-yl)benzonitrile,
2-(3-(difluorométhoxy)-4-(pyridin-2-yléthynyl)-1H-pyrazol-1-yl)-5-fluoropyridine,
3-fluoro-5-(4-(pyridin-2-yléthynyl)-1H-pyrazol-1-yl)benzonitrile,
3-fluoro-5-(4-(pyridin-3-yléthynyl)-1H-pyrazol-1-yl)benzonitrile,
3-fluoro-5-(4-(pyridin-4-yléthynyl)-1H-pyrazol-1-yl)benzonitrile,
2-((1-(4-fluorophényl)-1H-pyrazol-4-yl)éthynyl)pyridine,
3-((1-(4-fluorophényl)-1H-pyrazol-4-yl)éthynyl)pyridine,
2-chloro-4-((1-(4-fluorophényl)-1H-pyrazol-4-yl)éthynyl)pyridine,
4-((1-(4-fluorophényl)-1H-pyrazol-4-yl)éthynyl)pyridine,
2-((1-(4-(trifluorométhoxy)phényl)-1H-pyrazol-4-yl)éthynyl)pyridine,
3-((1-(4-(trifluorométhoxy)phényl)-1H-pyrazol-4-yl)éthynyl)pyridine,
4-((1-(4-(trifluorométhoxy)phényl)-1H-pyrazol-4-yl)éthynyl)pyridine,
2-((1-(4-fluorophényl)-3,5-diméthyl-1H-pyrazol-4-yl)éthynyl)pyrimidine,
4-((1-(4-fluorophényl)-3,5-diméthyl-1H-pyrazol-4-yl)éthynyl)pyrimidine,
2-((3,5-diméthyl-1-(4-(trifluorométhoxy)phényl)-1H-pyrazol-4-yl)éthynyl)pyrimidine,
2-((5-chloro-1-(4-fluorophényl)-1H-pyrazol-4-yl)éthynyl)pyrimidine,
2-((5-chloro-1-(6-fluoropyridin-3-yl)-1H-pyrazol-4-yl)éthynyl)pyrimidine,
2-((5-chloro-1-(4-trifluorométhoxy)phényl)-1H-pyrazol-4-yl)éthynyl)pyrimidine,
3-fluoro-5-(3-(2-méthoxyéthoxy)-5-méthyl-4-(pyrimidin-2-yléthynyl)-1H-pyrazol-1-yl)benzonitrile,
2-((1-(6-fluoropyridin-3-yl)-1H-pyrazol-4-yl)éthynyl)pyrimidine,
2-((5-chloro-1-(4-(trifluorométhyl)phényl)-1H-pyrazol-4-yl)éthynyl)pyrimidine,
3-(3-chloro-4-(pyrimidin-2-yléthynyl)-1H-pyrazol-1-yl)-5-fluorobenzonitrile,
2-((3-chloro-1-(4-(trifluorométhoxy)phényl)-1H-pyrazol-4-yl)éthynyl)pyrimidine,
2-((3-chloro-1-(4-fluorophényl)-1H-pyrazol-4-yl)éthynyl)pyrimidine,
2-((3-chloro-1-(5-fluoropyridin-3-yl)-1H-pyrazol-4-yl)éthynyl)pyrimidine,
3-(3-cyclopropyl-4-(pyrimidin-2-yléthynyl)-1H-pyrazol-1-yl)-5-fluorobenzonitrile
2-((1-(4-fluorophényl)-5-(2-méthoxyéthoxy)-1H-pyrazol-4-yl)éthynyl)pyrimidine,
3-fluoro-5-(4-(pyrimidin-2-yléthynyl)-1H-pyrazol-1-yl)benzonitrile,
2-((1-(4-fluorophényl)-1H-pyrazol-4-yl)éthynyl)pyrimidine,
2-((1-(4-(trifluorophényl)-1H-pyrazol-4-yl)éthynyl)pyrimidine,
2-((1-(4-(trifluorométhoxy)phényl)-1H-pyrazol-4-yl)éthynyl)pyrazine
3-fluoro-5-(4-(pyrazin-2-yléthynyl)-1H-pyrazol-1-yl)benzonitrile,
2-((1-(4-fluorophény1)-1H-pyrazol-4-yl)éthynyl)pyrazine,
2-((1-(4-(trifluorométhoxy)phényl)-1H-pyrazol-4-yl)éthynyl)pyrazine,
3-fluoro-5-(4-(phényléthynyl)-1H-pyrazol-1-yl)benzonitrile,
3-fluoro-5-(4-(3-chloro-phényléthynyl)-1H-pyrazol-1-yl)benzonitrile,
1-(4-fluorophényl)-4-(phényléthynyl)-1H-pyrazole,
4-((3-chlorophényl)éthynyl)-1-(4-fluorophényl)-1H-pyrazole,
4-(phényléthynyl)-1-(4-(trifluorométhoxy)phényl)-1H-pyrazole,
4-((3-chlorophényl)éthynyl)-1-(4-(trifluorométhoxy)phényl)-1H-pyrazole.

6. Composé selon la revendication 1 ou sel pharmaceutiquement acceptable de celui-ci, dans lequel ledit composé est :
3-fluoro-5-(4-méthyl-3-(pyridin-2-yléthynyl)-1H-pyrazol-1-yl)benzonitrile,
3-(4-chloro-3-(pyridin-2-yléthynyl)-1H-pyrazol-1-yl)-5-fluorobenzonitrile,
5-fluoro-2-(3-(pyridin-2-yléthynyl)-1H-pyrazol-1-yl)pyridine,
2-(4-chloro-3-(pyridin-2-yléthynyl)-1H-pyrazol-1-yl)-5-fluoropyridine,
3-fluoro-5-(3-(pyridin-2-yléthynyl)-1H-pyrazol-1-yl)pyridine,
2-((1-(pyridin-2-yl)-1H-pyrazol-3-yl)éthynyl)pyridine,
5-fluoro-2-(4-méthyl-3-(pyridin-2-yléthynyl)-1H-pyrazol-1l-yl)pyridine,
5-fluoro-2-(5-méthyl-3-(pyridin-2-yléthynyl)-1H-pyrazol-1-yl)pyridine,
3-fluoro-5-(5-méthyl-3-(pyridin-2-yléthynyl)-1H-pyrazol-1-yl)benzonitrile,
3-fluoro-5-(5-méthy1-3-(pyridin-3-yléthyny1)-1H-pyrazol-1-yl)benzonitrile,
3-(3-((2-chloropyridin-4-yl)éthynyl)-5-méthyl-1H-pyrazol-1-yl)-5-fluorobenzonitrile,
3-fluoro-5-(5-méthyl-3-(pyridin-4-yléthynyl)-1H-pyrazol-1-yl)benzonitrile,
3-fluoro-5-(3-(pyridin-2-yléthynyl)-1H-pyrazol-1-yl)benzonitrile,
3-fluoro-5-(3-(pyridin-3-yléthynyl)-1H-pyrazol-1-yl)benzonitrile,
3-(3-((2-chloropyridin-4-yl)éthynyl)-1H-pyrazol-1-yl)-5-fluorobenzonitrile,
3-fluoro-5-(3-(pyridin-4-yléthynyl)-1H-pyrazol-1-yl)benzonitrile,
2-((1-(4-fluorophényl)-1H-pyrazol-3-yl)éthynyl)pyridine,
3-((1-(4-fluorophényl)-1H-pyrazol-3-yl)éthynyl)pyridine,
2-chloro-4-((1-(4-fluorophényl)-1H-pyrazol-3-yl)éthynyl)pyridine,
4-((1-(4-fluorophényl)-1H-pyrazol-3-yl)éthynyl)pyridine,
3-((1-(4-(trifluorométhoxy)phényl)-1H-pyrazol-3-yl)tthynyl)pyridine,
4-((1-(4-(trifluorométhoxy)phényl)-1H-pyrazol-3-yl)éthynyl)pyridine,
2-((1-(4-fluorophényl)-5-méthyl-1H-pyrazol-3-yl)éthynyl)pyridine,
3-((1-(4-fluorophényl)-5-méthyl-1H-pyrazol-3-yl)éthynyl)pyridine,
4-((1-(4-fluorophényl)-5-méthyl-1H-pyrazol-3-yl)éthynyl)pyridine,
2-chloro-4-((1-(4-fluorophényl)-5-méthyl-1H-pyrazol-3-yl)éthynyl)pyridine,
3-((5-méthyl-1-(4-(trifluorométhoxy)phényl)-1H-pyrazol-3-yl)éthynyl)pyridine,
2-chloro-4-((5-méthyl-1-(4-(trifluorométhoxy)phényl)-1H-pyrazol-3-yl)éthynyl) pyridine,
2-((5-méthyl-1-(4-(trifluorométhoxy)phényl)-1H-pyrazol-3-yl)éthynyl)pyridine,
3-fluoro-5-(4-méthyl-3-(pyrimidin-2-yléthynyl)-1H-pyrazol-1-yl)benzonitrile,
3-fluoro-5-(5-méthyl-3-(pyrimidin-2-yléthynyl)-1H-pyrazol-1-yl)benzonitrile,
3-fluoro-5-(3-(pyrimidin-2-yléthynyl)-1H-pyrazol-1-yl)benzonitrile,
2-((1-(4-fluorophényl)-1H-pyrazol-3-yl)éthynyl)pyrimidine,
2-((1-(4-(trifluorométhoxy)phényl)-1H-pyrazol-3-yl)éthynyl)pyrimidine,
2-((1-(4-fluorophényl)-5-méthyl-1H-pyrazol-3-yl)éthynyl)pyrimidine,
2-((5-méthyl-1-(4-(trifluorométhoxy)phényl)-1H-pyrazol-3-yl)éthynyl)pyrimidine,
3-fluoro-5-(5-méthyl-3-(pyrazin-2-yléthynyl)-1H-pyrazol-1-yl)benzonitrile,
3-fluoro-5-(3-(pyrazin-2-yléthynyl)-1H-pyrazol-1-yl)benzonitrile,
2-((1-(4-fluorophényl)-1H-pyrazol-3-yl)éthynyl)pyrazine,
2-((1-(4-(trifluorométhoxy)phényl)-1H-pyrazol-3-yl)éthynyl)pyrazine,
2-((1-(4-fluorophényl)-5-méthyl-1H-pyrazol-3-yl)éthynyl)pyrazine,
2-((5-méthyl-1-(4-(trifluorométhoxy)phényl)-1H-pyrazol-3-yl)éthynyl)pyrazine,
3-fluoro-5-(5-méthyl-3-(phényléthynyl)-1H-pyrazol-1-yl)benzonitrile,
3-(3-((3-chlorophényl)éthynyl)-5-méthyl-1H-pyrazol-1-yl)-5-fluorobenzonitrile,
3-fluoro-5-(3-(phényléthynyl)-1H-pyrazol-1-yl)benzonitrile,
3-(3-((3-chlorophényl)éthynyl)-1H-pyrazol-1-yl)-5-fluorobenzonitrile,
1-(4-fluorophényl)-3-(phényléthynyl)-1H-pyrazole,
3-((3-chlorophényl)éthynyl)-1-(4-fluorophényl)-1H-pyrazole,
3-(phényléthynyl)-1-(4-(trifluorométhoxy)phényl)-1H-pyrazole,
3-((3-chlorophényl)éthynyl)-1-(4-(trifluorométhoxy)phényl)-1H-pyrazole.

7. Composition pharmaceutique, comprenant un composé selon l'une quelconque des revendications 1 à 6 sous la forme d'une base libre ou d'un sel pharmaceutiquement acceptable, en association avec un support pharmaceutique ou diluant.

8. Composé selon l'une quelconque des revendications 1 à 6 sous la forme d'une base libre ou d'un sel pharmaceutiquement acceptable ou composition pharmaceutique selon la revendication 7, destiné à être utilisé dans la prévention, le traitement ou le retard de la progression de troubles associés à des irrégularités de la transmission des signaux glutamatergiques dans les troubles du tube digestif, de l'appareil urinaire ou du système nerveux central régulés entièrement ou en partie par les récepteurs mGlu5.

9. Composé selon l'une quelconque des revendications 1 à 6 sous la forme d'une base libre ou d'un sel pharmaceutiquement acceptable ou composition pharmaceutique selon la revendication 7, destiné à être utilisé dans le traitement de la maladie d'Alzheimer, de la maladie de Parkinson, de la dyskinésie induite par la lévodopa dans la maladie de Parkinson (PD-LID), de la chorée de Huntington, des troubles psychiatriques, de la schizophrénie, des troubles de l'humeur, des troubles émotionnels, des troubles de déficit de l'attention, du syndrome de l'X fragile, des troubles du spectre autistique (TSA), de la maladie du reflux gastro-œsophagien (MRGO), de la dépendance aux drogues, des troubles de dépression.
